# EUROPEAN PATENT APPLICATION

(11) **EP 3 708 665 A1**
(43) Date of publication of application: **16.09.2020**
(21) Application number: 20155737.8
(22) Date of filing: 08.12.2016
(51) Int. Cl.: C12N 15/11, C12N 15/113, C12N 15/10, C12N 9/22, C12N 15/81

(54) **METHODS AND COMPOSITIONS FOR T-RNA BASED GUIDE RNA EXPRESSION**

(30) Priority: 18.12.2015 US 201562269121 P
(62) Divisional of application: 16822827.8
(71) Applicant: Danisco US Inc., Palo Alto, CA 94304 (US)
(72) Inventor: BENDEZU, Felipe Oseas, Palo Alto, CA 94304 (US); FAN, Xiaochun, Palo Alto, CA 94304 (US); FRISCH, Ryan L., Palo Alto, CA 94304 (US); HONG, Seung-Pyo, Palo Alto, CA 94304 (US)
(74) Representative: Mewburn Ellis LLP

(57) **Abstract**

Compositions and methods are provided for editing nucleotides and/or altering target sites in the genome of a cell. The methods and compositions employ a recombinant DNA construct comprising a tRNA promoter operably linked to a polynuc-leotide encoding a single guide RNA is capable of forming a guide RNA/Cas endonuclease complex, wherein said complex can bind to and cleave a target site sequence in the genome of a cell such as a microbial cell. The present disclosure further describes methods and compositions employing a recombinant DNA construct compromising a tRNA promoter operably linked to a spacer sequence and a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast.

SEQUENCE LISTING

<110>  E. I. du Pont de Nemours and Company
       Bendezu, Felipe  Oseas
       Fan, Xiaochun
       Frisch, Ryan L
       HONG, SEUNG-PYO

<120>  METHODS AND COMPOSITIONS FOR T-RNA BASED GUIDE RNA EXPRESSION

<130>  007591654

<140> EP
<141> Not assigned yet

<150> EP 16822827.8
<151> 2015-12-08

<150>  US 62/269,121
<151>  2015-12-18

<160>  133

<170>  PatentIn version 3.5

<210>  1
<211>  1372
<212>  PRT
<213>  Streptococcus pyogenes

<400>  1

Met Asp Lys Lys Tyr Ser Ile Gly Leu Asp Ile Gly Thr Asn Ser Val
1               5                   10                  15

Gly Trp Ala Val Ile Thr Asp Glu Tyr Lys Val Pro Ser Lys Lys Phe
            20                  25                  30

Lys Val Leu Gly Asn Thr Asp Arg His Ser Ile Lys Lys Asn Leu Ile
        35                  40                  45

Gly Ala Leu Leu Phe Asp Ser Gly Glu Thr Ala Glu Ala Thr Arg Leu
    50                  55                  60

Lys Arg Thr Ala Arg Arg Arg Tyr Thr Arg Arg Lys Asn Arg Ile Cys
65                  70                  75                  80

Tyr Leu Gln Glu Ile Phe Ser Asn Glu Met Ala Lys Val Asp Asp Ser
                85                  90                  95

Phe Phe His Arg Leu Glu Glu Ser Phe Leu Val Glu Glu Asp Lys Lys
            100                 105                 110

His Glu Arg His Pro Ile Phe Gly Asn Ile Val Asp Glu Val Ala Tyr
        115                 120                 125

His Glu Lys Tyr Pro Thr Ile Tyr His Leu Arg Lys Lys Leu Val Asp
    130                 135                 140

Ser Thr Asp Lys Ala Asp Leu Arg Leu Ile Tyr Leu Ala Leu Ala His
145                 150                 155                 160

Met Ile Lys Phe Arg Gly His Phe Leu Ile Glu Gly Asp Leu Asn Pro
                165                 170                 175

Asp Asn Ser Asp Val Asp Lys Leu Phe Ile Gln Leu Val Gln Thr Tyr
            180                 185                 190

Asn Gln Leu Phe Glu Glu Asn Pro Ile Asn Ala Ser Gly Val Asp Ala
        195                 200                 205

Lys Ala Ile Leu Ser Ala Arg Leu Ser Lys Ser Arg Arg Leu Glu Asn
    210                 215                 220

Leu Ile Ala Gln Leu Pro Gly Glu Lys Lys Asn Gly Leu Phe Gly Asn
225                 230                 235                 240

Leu Ile Ala Leu Ser Leu Gly Leu Thr Pro Asn Phe Lys Ser Asn Phe
                245                 250                 255

Asp Leu Ala Glu Asp Ala Lys Leu Gln Leu Ser Lys Asp Thr Tyr Asp
            260                 265                 270

Asp Asp Leu Asp Asn Leu Leu Ala Gln Ile Gly Asp Gln Tyr Ala Asp
        275                 280                 285

Leu Phe Leu Ala Ala Lys Asn Leu Ser Asp Ala Ile Leu Leu Ser Asp
    290                 295                 300

Ile Leu Arg Val Asn Thr Glu Ile Thr Lys Ala Pro Leu Ser Ala Ser
305                 310                 315                 320

Met Ile Lys Arg Tyr Asp Glu His His Gln Asp Leu Thr Leu Leu Lys
                325                 330                 335

Ala Leu Val Arg Gln Gln Leu Pro Glu Lys Tyr Lys Glu Ile Phe Phe
            340                 345                 350

Asp Gln Ser Lys Asn Gly Tyr Ala Gly Tyr Ile Asp Gly Gly Ala Ser
        355                 360                 365

Gln Glu Glu Phe Tyr Lys Phe Ile Lys Pro Ile Leu Glu Lys Met Asp
    370                 375                 380

Gly Thr Glu Glu Leu Leu Val Lys Leu Asn Arg Glu Asp Leu Leu Arg
385                 390                 395                 400

Lys Gln Arg Thr Phe Asp Asn Gly Ser Ile Pro His Gln Ile His Leu
                405                 410                 415

Gly Glu Leu His Ala Ile Leu Arg Arg Gln Glu Asp Phe Tyr Pro Phe
            420                 425                 430

Leu Lys Asp Asn Arg Glu Lys Ile Glu Lys Ile Leu Thr Phe Arg Ile
        435                 440                 445

Pro Tyr Tyr Val Gly Pro Leu Ala Arg Gly Asn Ser Arg Phe Ala Trp
    450                 455                 460

Met Thr Arg Lys Ser Glu Glu Thr Ile Thr Pro Trp Asn Phe Glu Glu
465                 470                 475                 480

Val Val Asp Lys Gly Ala Ser Ala Gln Ser Phe Ile Glu Arg Met Thr
                485                 490                 495

Asn Phe Asp Lys Asn Leu Pro Asn Glu Lys Val Leu Pro Lys His Ser
            500                 505                 510

Leu Leu Tyr Glu Tyr Phe Thr Val Tyr Asn Glu Leu Thr Lys Val Lys
        515                 520                 525

Tyr Val Thr Glu Gly Met Arg Lys Pro Ala Phe Leu Ser Gly Glu Gln
    530                 535                 540

Lys Lys Ala Ile Val Asp Leu Leu Phe Lys Thr Asn Arg Lys Val Thr
545                 550                 555                 560

Val Lys Gln Leu Lys Glu Asp Tyr Phe Lys Lys Ile Glu Cys Phe Asp
                565                 570                 575

Ser Val Glu Ile Ser Gly Val Glu Asp Arg Phe Asn Ala Ser Leu Gly
            580                 585                 590

Thr Tyr His Asp Leu Leu Lys Ile Ile Lys Asp Lys Asp Phe Leu Asp
        595                 600                 605

Asn Glu Glu Asn Glu Asp Ile Leu Glu Asp Ile Val Leu Thr Leu Thr
    610                 615                 620

Leu Phe Glu Asp Arg Glu Met Ile Glu Glu Arg Leu Lys Thr Tyr Ala

625                 630                 635                 640

His Leu Phe Asp Asp Lys Val Met Lys Gln Leu Lys Arg Arg Arg Tyr
                645                 650                 655

Thr Gly Trp Gly Arg Leu Ser Arg Lys Leu Ile Asn Gly Ile Arg Asp
            660                 665                 670

Lys Gln Ser Gly Lys Thr Ile Leu Asp Phe Leu Lys Ser Asp Gly Phe
        675                 680                 685

Ala Asn Arg Asn Phe Met Gln Leu Ile His Asp Asp Ser Leu Thr Phe
    690                 695                 700

Lys Glu Asp Ile Gln Lys Ala Gln Val Ser Gly Gln Gly Asp Ser Leu
705                 710                 715                 720

His Glu His Ile Ala Asn Leu Ala Gly Ser Pro Ala Ile Lys Lys Gly
                725                 730                 735

Ile Leu Gln Thr Val Lys Val Val Asp Glu Leu Val Lys Val Met Gly
            740                 745                 750

Arg His Lys Pro Glu Asn Ile Val Ile Glu Met Ala Arg Glu Asn Gln
        755                 760                 765

Thr Thr Gln Lys Gly Gln Lys Asn Ser Arg Glu Arg Met Lys Arg Ile
    770                 775                 780

Glu Glu Gly Ile Lys Glu Leu Gly Ser Gln Ile Leu Lys Glu His Pro
785                 790                 795                 800

Val Glu Asn Thr Gln Leu Gln Asn Glu Lys Leu Tyr Leu Tyr Tyr Leu
                805                 810                 815

Gln Asn Gly Arg Asp Met Tyr Val Asp Gln Glu Leu Asp Ile Asn Arg
            820                 825                 830

Leu Ser Asp Tyr Asp Val Asp His Ile Val Pro Gln Ser Phe Leu Lys
        835                 840                 845

Asp Asp Ser Ile Asp Asn Lys Val Leu Thr Arg Ser Asp Lys Asn Arg
    850                 855                 860

Gly Lys Ser Asp Asn Val Pro Ser Glu Glu Val Val Lys Lys Met Lys
865                 870                 875                 880

Asn Tyr Trp Arg Gln Leu Leu Asn Ala Lys Leu Ile Thr Gln Arg Lys
                885                 890                 895

Phe Asp Asn Leu Thr Lys Ala Glu Arg Gly Gly Leu Ser Glu Leu Asp
            900                 905                 910

Lys Ala Gly Phe Ile Lys Arg Gln Leu Val Glu Thr Arg Gln Ile Thr
        915                 920                 925

Lys His Val Ala Gln Ile Leu Asp Ser Arg Met Asn Thr Lys Tyr Asp
    930                 935                 940

Glu Asn Asp Lys Leu Ile Arg Glu Val Lys Val Ile Thr Leu Lys Ser
945                 950                 955                 960

Lys Leu Val Ser Asp Phe Arg Lys Asp Phe Gln Phe Tyr Lys Val Arg
                965                 970                 975

Glu Ile Asn Asn Tyr His His Ala His Asp Ala Tyr Leu Asn Ala Val
            980                 985                 990

Val Gly Thr Ala Leu Ile Lys Lys Tyr Pro Lys Leu Glu Ser Glu Phe
        995                 1000                1005

Val Tyr Gly Asp Tyr Lys Val Tyr Asp Val Arg Lys Met Ile Ala
    1010                1015                1020

Lys Ser Glu Gln Glu Ile Gly Lys Ala Thr Ala Lys Tyr Phe Phe
    1025                1030                1035

Tyr Ser Asn Ile Met Asn Phe Phe Lys Thr Glu Ile Thr Leu Ala
    1040                1045                1050

Asn Gly Glu Ile Arg Lys Arg Pro Leu Ile Glu Thr Asn Gly Glu
    1055                1060                1065

Thr Gly Glu Ile Val Trp Asp Lys Gly Arg Asp Phe Ala Thr Val
    1070                1075                1080

Arg Lys Val Leu Ser Met Pro Gln Val Asn Ile Val Lys Lys Thr
    1085                1090                1095

Glu Val Gln Thr Gly Gly Phe Ser Lys Glu Ser Ile Leu Pro Lys
    1100                1105                1110

Arg Asn Ser Asp Lys Leu Ile Ala Arg Lys Lys Asp Trp Asp Pro
    1115                1120                1125

Lys Lys Tyr Gly Gly Phe Asp Ser Pro Thr Val Ala Tyr Ser Val
    1130                1135                1140

Leu Val Val Ala Lys Val Glu Lys Gly Lys Ser Lys Lys Leu Lys
    1145                1150                1155

Ser Val Lys Glu Leu Leu Gly Ile Thr Ile Met Glu Arg Ser Ser
    1160                1165                1170

Phe Glu Lys Asn Pro Ile Asp Phe Leu Glu Ala Lys Gly Tyr Lys
    1175                1180                1185

Glu Val Lys Lys Asp Leu Ile Ile Lys Leu Pro Lys Tyr Ser Leu
    1190                1195                1200

Phe Glu Leu Glu Asn Gly Arg Lys Arg Met Leu Ala Ser Ala Gly
    1205                1210                1215

Glu Leu Gln Lys Gly Asn Glu Leu Ala Leu Pro Ser Lys Tyr Val
    1220                1225                1230

Asn Phe Leu Tyr Leu Ala Ser His Tyr Glu Lys Leu Lys Gly Ser
    1235                1240                1245

Pro Glu Asp Asn Glu Gln Lys Gln Leu Phe Val Glu Gln His Lys
    1250                1255                1260

His Tyr Leu Asp Glu Ile Ile Glu Gln Ile Ser Glu Phe Ser Lys
    1265                1270                1275

Arg Val Ile Leu Ala Asp Ala Asn Leu Asp Lys Val Leu Ser Ala
    1280                1285                1290

Tyr Asn Lys His Arg Asp Lys Pro Ile Arg Glu Gln Ala Glu Asn
    1295                1300                1305

Ile Ile His Leu Phe Thr Leu Thr Asn Leu Gly Ala Pro Ala Ala
    1310                1315                1320

Phe Lys Tyr Phe Asp Thr Thr Ile Asp Arg Lys Arg Tyr Thr Ser
    1325                1330                1335

Thr Lys Glu Val Leu Asp Ala Thr Leu Ile His Gln Ser Ile Thr
    1340                1345                1350

Gly Leu Tyr Glu Thr Arg Ile Asp Leu Ser Gln Leu Gly Gly Asp
    1355                1360                1365

Ser Arg Ala Asp
    1370

<210> 2
<211> 4140
<212> DNA
<213> Artificial sequence

<220>
<223> Yarrowia codon optimized Cas9

<400> 2
atggacaaga aatactccat cggcctggac attggaacca actctgtcgg ctgggctgtc 60

atcaccgacg agtacaaggt gccctccaag aaattcaagg tcctcggaaa caccgatcga 120

cactccatca agaaaaacct cattggtgcc ctgttgttcg attctggcga gactgccgaa 180

gctaccagac tcaagcgaac tgctcggcga cgttacaccc gacggaagaa ccgaatctgc 240

tacctgcagg agatcttttc caacgagatg gccaaggtgg acgattcgtt ctttcatcga 300

ctggaggaat ccttcctcgt cgaggaagac aagaaacacg agcgtcatcc catctttggc 360

aacattgtgg acgaggttgc ttaccacgag aagtatccta ccatctacca tctccgaaag 420

aaactcgtcg attccaccga caaggcggat ctcagactta tctacctcgc tctggcacac 480

atgatcaagt ttcgaggtca tttcctcatc gagggcgatc tcaatcccga caacagcgat 540

gtggacaagc tgttcattca gctcgttcag acctacaacc agctgttcga ggaaaacccc 600

atcaatgcct ccggagtcga tgcaaaggcc atcttgtctg ctcgactctc gaagagcaga 660

cgactggaga acctcattgc ccaacttcct ggcgagaaaa agaacggact gtttggcaac 720

ctcattgccc tttctcttgg tctcacaccc aacttcaagt ccaacttcga tctggcggag 780

gacgccaagc tccagctgtc caaggacacc tacgacgatg acctcgacaa cctgcttgca 840

cagattggcg atcagtacgc cgacctgttt ctcgctgcca agaacctttc ggatgctatt 900

ctcttgtctg acattctgcg agtcaacacc gagatcacaa aggctcccct ttctgcctcc 960

atgatcaagc gatacgacga gcaccatcag gatctcacac tgctcaaggc tcttgtccga 1020

cagcaactgc ccgagaagta caaggagatc tttttcgatc agtcgaagaa cggctacgct 1080

ggatacatcg acggcggagc ctctcaggaa gagttctaca agttcatcaa gccaattctc 1140

gagaagatgg acggaaccga ggaactgctt gtcaagctca atcgagagga tctgcttcgg 1200

aagcaacgaa ccttcgacaa cggcagcatt cctcatcaga tccacctcgg tgagctgcac 1260

gccattcttc gacgtcagga agacttctac ccctttctca aggacaaccg agagaagatc 1320

gagaagattc ttacctttcg aatcccctac tatgttggtc ctcttgccag aggaaactct 1380

cgatttgctt ggatgactcg aaagtccgag gaaaccatca ctccctggaa cttcgaggaa 1440

gtcgtggaca agggtgcctc tgcacagtcc ttcatcgagc gaatgaccaa cttcgacaag 1500

aatctgccca acgagaaggt tcttcccaag cattcgctgc tctacgagta ctttacagtc 1560

tacaacgaac tcaccaaagt caagtacgtt accgagggaa tgcgaaagcc tgccttcttg 1620

tctggcgaac agaagaaagc cattgtcgat ctcctgttca agaccaaccg aaaggtcact 1680

gttaagcagc tcaaggagga ctacttcaag aaaatcgagt gtttcgacag cgtcgagatt 1740

tccggagttg aggaccgatt caacgcctct ttgggcacct atcacgatct gctcaagatt 1800

atcaaggaca aggattttct cgacaacgag gaaaacgagg acattctgga ggacatcgtg 1860

ctcactctta ccctgttcga agatcgggag atgatcgagg aacgactcaa gacatacgct 1920

cacctgttcg acgacaaggt catgaaacaa ctcaagcgac gtagatacac cggctgggga 1980

agactttcgc gaaagctcat caacggcatc agagacaagc agtccggaaa gaccattctg 2040

gactttctca agtccgatgg ctttgccaac cgaaacttca tgcagctcat tcacgacgat 2100

tctcttacct tcaaggagga catccagaag gcacaagtgt ccggtcaggg cgacagcttg 2160

cacgaacata ttgccaacct ggctggttcg ccagccatca agaaaggcat tctccagact 2220

gtcaaggttg tcgacgagct ggtgaaggtc atgggacgtc acaagcccga gaacattgtg 2280

atcgagatgg ccagagagaa ccagacaact caaaagggtc agaaaaactc gcgagagcgg 2340

atgaagcgaa tcgaggaagg catcaaggag ctgggatccc agattctcaa ggagcatccc 2400

gtcgagaaca ctcaactgca gaacgagaag ctgtatctct actatctgca gaatggtcga 2460

gacatgtacg tggatcagga actggacatc aatcgtctca gcgactacga tgtggaccac 2520

attgtccctc aatcctttct caaggacgat tctatcgaca acaaggtcct tacacgatcc 2580

gacaagaaca gaggcaagtc ggacaacgtt cccagcgaag aggtggtcaa aaagatgaag 2640

aactactggc gacagctgct caacgccaag ctcattaccc agcgaaagtt cgacaatctt 2700

accaaggccg agcgaggcgg tctgtccgag ctcgacaagg ctggcttcat caagcgtcaa 2760

ctcgtcgaga ccagacagat cacaaagcac gtcgcacaga ttctcgattc tcggatgaac 2820

accaagtacg acgagaacga caagctcatc cgagaggtca aggtgattac tctcaagtcc 2880

aaactggtct ccgatttccg aaaggacttt cagttctaca aggtgcgaga gatcaacaat 2940

taccaccatg cccacgatgc ttacctcaac gccgtcgttg gcactgcgct catcaagaaa 3000

taccccaagc tcgaaagcga gttcgtttac ggcgattaca aggtctacga cgttcgaaag 3060

atgattgcca agtccgaaca ggagattggc aaggctactg ccaagtactt cttttactcc 3120

aacatcatga actttttcaa gaccgagatc accttggcca acggagagat tcgaaagaga 3180

ccacttatcg agaccaacgg cgaaactgga gagatcgtgt gggacaaggg tcgagacttt 3240

gcaaccgtgc gaaaggttct gtcgatgcct caggtcaaca tcgtcaagaa aaccgaggtt 3300

cagactggcg gattctccaa ggagtcgatt ctgcccaagc gaaactccga caagctcatc 3360

gctcgaaaga aagactggga tcccaagaaa tacggtggct tcgattctcc taccgtcgcc 3420

tattccgtgc ttgtcgttgc gaaggtcgag aagggcaagt ccaaaaagct caagtccgtc 3480

aaggagctgc tcggaattac catcatggag cgatcgagct tcgagaagaa tcccatcgac 3540

ttcttggaag ccaagggtta caaggaggtc aagaaagacc tcattatcaa gctgcccaag 3600

tactctctgt tcgaactgga gaacggtcga aagcgtatgc tcgcctccgc tggcgagctg 3660

cagaagggaa acgagcttgc cttgccttcg aagtacgtca actttctcta tctggcttct 3720

cactacgaga agctcaaggg ttctcccgag gacaacgaac agaagcaact cttcgttgag 3780

cagcacaaac attacctcga cgagattatc gagcagattt ccgagttttc gaagcgagtc 3840

atcctggctg atgccaactt ggacaaggtg ctctctgcct acaacaagca tcgggacaaa 3900

cccattcgag aacaggcgga gaacatcatt cacctgttta ctcttaccaa cctgggtgct 3960

cctgcagctt tcaagtactt cgataccact atcgaccgaa agcggtacac atccaccaag 4020

gaggttctcg atgccaccct gattcaccag tccatcactg gcctgtacga gacccgaatc 4080

gacctgtctc agcttggtgg cgactccaga gccgatccca agaaaaagcg aaaggtctaa 4140

<210> 3
<211> 7
<212> PRT
<213> SV40

<400> 3

Pro Lys Lys Lys Arg Lys Val
1               5

<210> 4
<211> 543
<212> DNA
<213> YArrowia lipolytica

<400> 4
tcgacgttta aaccatcatc taagggcctc aaaactacct cggaactgct gcgctgatct 60

ggacaccaca gaggttccga gcactttagg ttgcaccaaa tgtcccacca ggtgcaggca 120

gaaaacgctg gaacagcgtg tacagtttgt cttaacaaaa agtgagggcg ctgaggtcga 180

gcagggtggt gtgacttgtt atagccttta gagctgcgaa agcgcgtatg gatttggctc 240

atcaggccag attgagggtc tgtggacaca tgtcatgtta gtgtacttca atcgccccct 300

ggatatagcc ccgacaatag gccgtggcct catttttttg ccttccgcac atttccattg 360

ctcggtaccc acaccttgct tctcctgcac ttgccaacct taatactggt ttacattgac 420

caacatctta caagcggggg gcttgtctag ggtatatata aacagtggct ctcccaatcg 480

gttgccagtc tcttttttcc tttctttccc cacagattcg aaatctaaac tacacatcac 540

acc                                                                543

<210>  5
<211>  4683
<212>  DNA
<213>  Artificial sequence

<220>
<223>  Yarrowia optimized expression cassette

<400>  5
tcgacgttta aaccatcatc taagggcctc aaaactacct cggaactgct gcgctgatct      60

ggacaccaca gaggttccga gcactttagg ttgcaccaaa tgtcccacca ggtgcaggca     120

gaaaacgctg gaacagcgtg tacagtttgt cttaacaaaa agtgagggcg ctgaggtcga     180

gcagggtggt gtgacttgtt atagccttta gagctgcgaa agcgcgtatg gatttggctc     240

atcaggccag attgagggtc tgtggacaca tgtcatgtta gtgtacttca atcgccccct     300

ggatatagcc ccgacaatag gccgtggcct catttttttg ccttccgcac atttccattg     360

ctcggtaccc acaccttgct tctcctgcac ttgccaacct taatactggt ttacattgac     420

caacatctta caagcggggg gcttgtctag ggtatatata aacagtggct ctcccaatcg     480

gttgccagtc tcttttttcc tttctttccc cacagattcg aaatctaaac tacacatcac     540

accatggaca agaaatactc catcggcctg gacattggaa ccaactctgt cggctgggct     600

gtcatcaccg acgagtacaa ggtgccctcc aagaaattca aggtcctcgg aaacaccgat     660

cgacactcca tcaagaaaaa cctcattggt gccctgttgt tcgattctgg cgagactgcc     720

gaagctacca gactcaagcg aactgctcgg cgacgttaca cccgacggaa gaaccgaatc     780

tgctacctgc aggagatctt ttccaacgag atggccaagg tggacgattc gttctttcat     840

cgactggagg aatccttcct cgtcgaggaa gacaagaaac acgagcgtca tcccatcttt     900

ggcaacattg tggacgaggt tgcttaccac gagaagtatc ctaccatcta ccacctgcga     960

aagaaactcg tcgattccac cgacaaggcg gatctcagac ttatctacct cgctctggca    1020

cacatgatca agtttcgagg tcatttcctc atcgagggcg atctcaatcc cgacaacagc    1080

gatgtggaca agctgttcat tcagctcgtt cagacctaca accagctgtt cgaggaaaac    1140

cccatcaatg cctccggagt cgatgcaaag gccatcttgt ctgctcgact ctcgaagagc    1200

agacgactgg agaacctcat tgcccaactt cctggcgaga aaaagaacgg actgtttggc    1260

aacctcattg ccctttctct tggtctcaca cccaacttca agtccaactt cgatctggcg    1320

gaggacgcca agctccagct gtccaaggac acctacgacg atgacctcga caacctgctt    1380

gcacagattg gcgatcagta cgccgacctg tttctcgctg ccaagaacct ttcggatgct    1440

attctcttgt ctgacattct gcgagtcaac accgagatca caaaggctcc cctttctgcc    1500

tccatgatca agcgatacga cgagcaccat caggatctca cactgctcaa ggctcttgtc    1560

cgacagcaac tgcccgagaa gtacaaggag atctttttcg atcagtcgaa gaacggctac 1620

gctggataca tcgacggcgg agcctctcag gaagagttct acaagttcat caagccaatt 1680

ctcgagaaga tggacggaac cgaggaactg cttgtcaagc tcaatcgaga ggatctgctt 1740

cggaagcaac gaaccttcga caacggcagc attcctcatc agatccacct cggtgagctg 1800

cacgccattc ttcgacgtca ggaagacttc tacccctttc tcaaggacaa ccgagagaag 1860

atcgagaaga ttcttacctt tcgaatcccc tactatgttg gtcctcttgc cagaggaaac 1920

tctcgatttg cttggatgac tcgaaagtcc gaggaaacca tcactccctg gaacttcgag 1980

gaagtcgtgg acaagggtgc ctctgcacag tccttcatcg agcgaatgac caacttcgac 2040

aagaatctgc ccaacgagaa ggttcttccc aagcattcgc tgctctacga gtactttaca 2100

gtctacaacg aactcaccaa agtcaagtac gttaccgagg gaatgcgaaa gcctgccttc 2160

ttgtctggcg aacagaagaa agccattgtc gatctcctgt tcaagaccaa ccgaaaggtc 2220

actgttaagc agctcaagga ggactacttc aagaaaatcg agtgtttcga cagcgtcgag 2280

atttccggag ttgaggaccg attcaacgcc tctttgggca cctatcacga tctgctcaag 2340

attatcaagg acaaggattt tctcgacaac gaggaaaacg aggacattct ggaggacatc 2400

gtgctcactc ttaccctgtt cgaagatcgg gagatgatcg aggaacgact caagacatac 2460

gctcacctgt tcgacgacaa ggtcatgaaa caactcaagc gacgtagata caccggctgg 2520

ggaagacttt cgcgaaagct catcaacggc atcagagaca agcagtccgg aaagaccatt 2580

ctggactttc tcaagtccga tggctttgcc aaccgaaact tcatgcagct cattcacgac 2640

gattctctta ccttcaagga ggacatccag aaggcacaag tgtccggtca gggcgacagc 2700

ttgcacgaac atattgccaa cctggctggt tcgccagcca tcaagaaagg cattctccag 2760

actgtcaagg ttgtcgacga gctggtgaag gtcatgggac gtcacaagcc cgagaacatt 2820

gtgatcgaga tggccagaga gaaccagaca actcaaaagg gtcagaaaaa ctcgcgagag 2880

cggatgaagc gaatcgagga aggcatcaag gagctgggat cccagattct caaggagcat 2940

cccgtcgaga acactcaact gcagaacgag aagctgtatc tctactatct gcagaatggt 3000

cgagacatgt acgtggatca ggaactggac atcaatcgtc tcagcgacta cgatgtggac 3060

cacattgtcc ctcaatcctt tctcaaggac gattctatcg acaacaaggt ccttacacga 3120

tccgacaaga acagaggcaa gtcggacaac gttcccagcg aagaggtggt caaaaagatg 3180

aagaactact ggcgacagct gctcaacgcc aagctcatta cccagcgaaa gttcgacaat 3240

cttaccaagg ccgagcgagg cggtctgtcc gagctcgaca aggctggctt catcaagcgt 3300

caactcgtcg agaccagaca gatcacaaag cacgtcgcac agattctcga ttctcggatg 3360

aacaccaagt acgacgagaa cgacaagctc atccgagagg tcaaggtgat tactctcaag 3420

tccaaactgg tctccgattt ccgaaaggac tttcagttct acaaggtgcg agagatcaac 3480

aattaccacc atgcccacga tgcttacctc aacgccgtcg ttggcactgc gctcatcaag 3540

aaatacccca agctcgaaag cgagttcgtt tacggcgatt acaaggtcta cgacgttcga 3600

aagatgattg ccaagtccga acaggagatt ggcaaggcta ctgccaagta cttcttttac 3660

tccaacatca tgaacttttt caagaccgag atcaccttgg ccaacggaga gattcgaaag 3720

agaccactta tcgagaccaa cggcgaaact ggagagatcg tgtgggacaa gggtcgagac 3780

tttgcaaccg tgcgaaaggt tctgtcgatg cctcaggtca acatcgtcaa gaaaaccgag 3840

gttcagactg gcggattctc caaggagtcg attctgccca agcgaaactc cgacaagctc 3900

atcgctcgaa agaaagactg ggatcccaag aaatacggtg gcttcgattc tcctaccgtc 3960

gcctattccg tgcttgtcgt tgcgaaggtc gagaagggca agtccaaaaa gctcaagtcc 4020

gtcaaggagc tgctcggaat taccatcatg gagcgatcga gcttcgagaa gaatcccatc 4080

gacttcttgg aagccaaggg ttacaaggag gtcaagaaag acctcattat caagctgccc 4140

aagtactctc tgttcgaact ggagaacggt cgaaagcgta tgctcgcctc cgctggcgag 4200

ctgcagaagg gaaacgagct tgccttgcct tcgaagtacg tcaactttct ctatctggct 4260

tctcactacg agaagctcaa gggttctccc gaggacaacg aacagaagca actcttcgtt 4320

gagcagcaca aacattacct cgacgagatt atcgagcaga tttccgagtt ttcgaagcga 4380

gtcatcctgg ctgatgccaa cttggacaag gtgctctctg cctacaacaa gcatcgggac 4440

aaacccattc gagaacaggc ggagaacatc attcacctgt ttactcttac caacctgggt 4500

gctcctgcag ctttcaagta cttcgatacc actatcgacc gaaagcggta cacatccacc 4560

aaggaggttc tcgatgccac cctgattcac cagtccatca ctggcctgta cgagacccga 4620

atcgacctgt ctcagcttgg tggcgactcc agagccgatc ccaagaaaaa gcgaaaggtc 4680

taa 4683

<210> 6
<211> 10706
<212> DNA
<213> Artificial sequence

<220>
<223> pZufCas9

<400> 6
catggacaag aaatactcca tcggcctgga cattggaacc aactctgtcg gctgggctgt 60

catcaccgac gagtacaagg tgccctccaa gaaattcaag gtcctcggaa acaccgatcg 120

acactccatc aagaaaaacc tcattggtgc cctgttgttc gattctggcg agactgccga 180

agctaccaga ctcaagcgaa ctgctcggcg acgttacacc cgacggaaga accgaatctg 240

ctacctgcag gagatctttt ccaacgagat ggccaaggtg gacgattcgt tctttcatcg 300

actggaggaa tccttcctcg tcgaggaaga caagaaacac gagcgtcatc ccatctttgg 360

caacattgtg gacgaggttg cttaccacga gaagtatcct accatctacc acctgcgaaa 420

gaaactcgtc gattccaccg acaaggcgga tctcagactt atctacctcg ctctggcaca 480

catgatcaag tttcgaggtc atttcctcat cgagggcgat ctcaatcccg acaacagcga 540

tgtggacaag ctgttcattc agctcgttca gacctacaac cagctgttcg aggaaaaccc 600

catcaatgcc tccggagtcg atgcaaaggc catcttgtct gctcgactct cgaagagcag 660

acgactggag aacctcattg cccaacttcc tggcgagaaa aagaacggac tgtttggcaa 720

cctcattgcc ctttctcttg gtctcacacc caacttcaag tccaacttcg atctggcgga 780

ggacgccaag ctccagctgt ccaaggacac ctacgacgat gacctcgaca acctgcttgc 840

acagattggc gatcagtacg ccgacctgtt tctcgctgcc aagaaccttt cggatgctat 900

tctcttgtct gacattctgc gagtcaacac cgagatcaca aaggctcccc tttctgcctc 960

catgatcaag cgatacgacg agcaccatca ggatctcaca ctgctcaagg ctcttgtccg 1020

acagcaactg cccgagaagt acaaggagat ctttttcgat cagtcgaaga acggctacgc 1080

tggatacatc gacggcggag cctctcagga agagttctac aagttcatca agccaattct 1140

cgagaagatg gacggaaccg aggaactgct tgtcaagctc aatcgagagg atctgcttcg 1200

gaagcaacga accttcgaca acggcagcat tcctcatcag atccacctcg gtgagctgca 1260

cgccattctt cgacgtcagg aagacttcta cccctttctc aaggacaacc gagagaagat 1320

cgagaagatt cttacctttc gaatccccta ctatgttggt cctcttgcca gaggaaactc 1380

tcgatttgct tggatgactc gaaagtccga ggaaaccatc actccctgga acttcgagga 1440

agtcgtggac aagggtgcct ctgcacagtc cttcatcgag cgaatgacca acttcgacaa 1500

gaatctgccc aacgagaagg ttcttcccaa gcattcgctg ctctacgagt actttacagt 1560

ctacaacgaa ctcaccaaag tcaagtacgt taccgaggga atgcgaaagc ctgccttctt 1620

gtctggcgaa cagaagaaag ccattgtcga tctcctgttc aagaccaacc gaaaggtcac 1680

tgttaagcag ctcaaggagg actacttcaa gaaaatcgag tgtttcgaca gcgtcgagat 1740

ttccggagtt gaggaccgat tcaacgcctc tttgggcacc tatcacgatc tgctcaagat 1800

tatcaaggac aaggattttc tcgacaacga ggaaaacgag gacattctgg aggacatcgt 1860

gctcactctt accctgttcg aagatcggga gatgatcgag gaacgactca agacatacgc 1920

tcacctgttc gacgacaagg tcatgaaaca actcaagcga cgtagataca ccggctgggg 1980

aagactttcg cgaaagctca tcaacggcat cagagacaag cagtccggaa agaccattct 2040

ggactttctc aagtccgatg gctttgccaa ccgaaacttc atgcagctca ttcacgacga 2100

ttctcttacc ttcaaggagg acatccagaa ggcacaagtg tccggtcagg gcgacagctt 2160

gcacgaacat attgccaacc tggctggttc gccagccatc aagaaaggca ttctccagac 2220

tgtcaaggtt gtcgacgagc tggtgaaggt catgggacgt cacaagcccg agaacattgt 2280

gatcgagatg gccagagaga accagacaac tcaaaagggt cagaaaaact cgcgagagcg 2340

gatgaagcga atcgaggaag gcatcaagga gctgggatcc cagattctca aggagcatcc 2400

cgtcgagaac actcaactgc agaacgagaa gctgtatctc tactatctgc agaatggtcg 2460

agacatgtac gtggatcagg aactggacat caatcgtctc agcgactacg atgtggacca 2520

cattgtccct caatcctttc tcaaggacga ttctatcgac aacaaggtcc ttacacgatc 2580

cgacaagaac agaggcaagt cggacaacgt tcccagcgaa gaggtggtca aaaagatgaa 2640

gaactactgg cgacagctgc tcaacgccaa gctcattacc cagcgaaagt tcgacaatct 2700

taccaaggcc gagcgaggcg gtctgtccga gctcgacaag gctggcttca tcaagcgtca 2760

actcgtcgag accagacaga tcacaaagca cgtcgcacag attctcgatt ctcggatgaa 2820

caccaagtac gacgagaacg acaagctcat ccgagaggtc aaggtgatta ctctcaagtc 2880

caaactggtc tccgatttcc gaaaggactt tcagttctac aaggtgcgag agatcaacaa 2940

ttaccaccat gcccacgatg cttacctcaa cgccgtcgtt ggcactgcgc tcatcaagaa 3000

ataccccaag ctcgaaagcg agttcgttta cggcgattac aaggtctacg acgttcgaaa 3060

gatgattgcc aagtccgaac aggagattgg caaggctact gccaagtact tcttttactc 3120

caacatcatg aactttttca agaccgagat caccttggcc aacggagaga ttcgaaagag 3180

accacttatc gagaccaacg gcgaaactgg agagatcgtg tgggacaagg gtcgagactt 3240

tgcaaccgtg cgaaaggttc tgtcgatgcc tcaggtcaac atcgtcaaga aaaccgaggt 3300

tcagactggc ggattctcca aggagtcgat tctgcccaag cgaaactccg acaagctcat 3360

cgctcgaaag aaagactggg atcccaagaa atacggtggc ttcgattctc ctaccgtcgc 3420

ctattccgtg cttgtcgttg cgaaggtcga gaagggcaag tccaaaaagc tcaagtccgt 3480

caaggagctg ctcggaatta ccatcatgga gcgatcgagc ttcgagaaga atcccatcga 3540

cttcttggaa gccaagggtt acaaggaggt caagaaagac ctcattatca agctgcccaa 3600

gtactctctg ttcgaactgg agaacggtcg aaagcgtatg ctcgcctccg ctggcgagct 3660

gcagaaggga aacgagcttg ccttgccttc gaagtacgtc aactttctct atctggcttc 3720

tcactacgag aagctcaagg gttctcccga ggacaacgaa cagaagcaac tcttcgttga 3780

gcagcacaaa cattacctcg acgagattat cgagcagatt tccgagtttt cgaagcgagt 3840

catcctggct gatgccaact tggacaaggt gctctctgcc tacaacaagc atcgggacaa 3900

acccattcga gaacaggcgg agaacatcat tcacctgttt actcttacca acctgggtgc 3960

tcctgcagct ttcaagtact tcgataccac tatcgaccga aagcggtaca catccaccaa 4020

ggaggttctc gatgccaccc tgattcacca gtccatcact ggcctgtacg agacccgaat 4080

cgacctgtct cagcttggtg gcgactccag agccgatccc aagaaaaagc gaaaggtcta 4140

agcggccgca agtgtggatg gggaagtgag tgcccggttc tgtgtgcaca attggcaatc 4200

caagatggat ggattcaaca cagggatata gcgagctacg tggtggtgcg aggatatagc 4260

aacggatatt tatgtttgac acttgagaat gtacgataca agcactgtcc aagtacaata 4320

ctaaacatac tgtacatact catactcgta cccgggcaac ggtttcactt gagtgcagtg 4380

gctagtgctc ttactcgtac agtgtgcaat actgcgtatc atagtctttg atgtatatcg 4440

tattcattca tgttagttgc gtacgagccg gaagcataaa gtgtaaagcc tggggtgcct 4500

aatgagtgag ctaactcaca ttaattgcgt tgcgctcact gcccgctttc cagtcgggaa 4560

acctgtcgtg ccagctgcat taatgaatcg gccaacgcgc ggggagaggc ggtttgcgta 4620

ttgggcgctc ttccgcttcc tcgctcactg actcgctgcg ctcggtcgtt cggctgcggc 4680

gagcggtatc agctcactca aaggcggtaa tacggttatc cacagaatca ggggataacg 4740

caggaaagaa catgtgagca aaaggccagc aaaaggccag gaaccgtaaa aaggccgcgt 4800

tgctggcgtt tttccatagg ctccgccccc ctgacgagca tcacaaaaat cgacgctcaa 4860

gtcagaggtg gcgaaacccg acaggactat aaagatacca ggcgtttccc cctggaagct 4920

ccctcgtgcg ctctcctgtt ccgaccctgc cgcttaccgg atacctgtcc gcctttctcc 4980

cttcgggaag cgtggcgctt tctcatagct cacgctgtag gtatctcagt tcggtgtagg 5040

tcgttcgctc caagctgggc tgtgtgcacg aaccccccgt tcagcccgac cgctgcgcct 5100

tatccggtaa ctatcgtctt gagtccaacc cggtaagaca cgacttatcg ccactggcag 5160

cagccactgg taacaggatt agcagagcga ggtatgtagg cggtgctaca gagttcttga 5220

agtggtggcc taactacggc tacactagaa ggacagtatt tggtatctgc gctctgctga 5280

agccagttac cttcggaaaa agagttggta gctcttgatc cggcaaacaa accaccgctg 5340

gtagcggtgg tttttttgtt tgcaagcagc agattacgcg cagaaaaaaa ggatctcaag 5400

aagatccttt gatcttttct acggggtctg acgctcagtg gaacgaaaac tcacgttaag 5460

ggattttggt catgagatta tcaaaaagga tcttcaccta gatcctttta aattaaaaat 5520

gaagttttaa atcaatctaa agtatatatg agtaaacttg gtctgacagt taccaatgct 5580

taatcagtga ggcacctatc tcagcgatct gtctatttcg ttcatccata gttgcctgac 5640

tccccgtcgt gtagataact acgatacggg agggcttacc atctggcccc agtgctgcaa 5700

tgataccgcg agacccacgc tcaccggctc cagatttatc agcaataaac cagccagccg 5760

gaagggccga gcgcagaagt ggtcctgcaa ctttatccgc ctccatccag tctattaatt 5820

gttgccggga agctagagta agtagttcgc cagttaatag tttgcgcaac gttgttgcca 5880

ttgctacagg catcgtggtg tcacgctcgt cgtttggtat ggcttcattc agctccggtt 5940

cccaacgatc aaggcgagtt acatgatccc ccatgttgtg caaaaaagcg gttagctcct 6000

tcggtcctcc gatcgttgtc agaagtaagt tggccgcagt gttatcactc atggttatgg 6060

cagcactgca taattctctt actgtcatgc catccgtaag atgcttttct gtgactggtg 6120

agtactcaac caagtcattc tgagaatagt gtatgcggcg accgagttgc tcttgcccgg 6180

cgtcaatacg ggataatacc gcgccacata gcagaacttt aaaagtgctc atcattggaa 6240

aacgttcttc ggggcgaaaa ctctcaagga tcttaccgct gttgagatcc agttcgatgt 6300

aacccactcg tgcacccaac tgatcttcag catcttttac tttcaccagc gtttctgggt 6360

gagcaaaaac aggaaggcaa aatgccgcaa aaaagggaat aagggcgaca cggaaatgtt 6420

gaatactcat actcttcctt tttcaatatt attgaagcat ttatcagggt tattgtctca 6480

tgagcggata catatttgaa tgtatttaga aaaataaaca aataggggtt ccgcgcacat 6540

ttccccgaaa agtgccacct gacgcgccct gtagcggcgc attaagcgcg gcgggtgtgg 6600

tggttacgcg cagcgtgacc gctacacttg ccagcgccct agcgcccgct cctttcgctt 6660

tcttcccttc ctttctcgcc acgttcgccg gctttccccg tcaagctcta aatcgggggc 6720

tccctttagg gttccgattt agtgctttac ggcacctcga ccccaaaaaa cttgattagg 6780

gtgatggttc acgtagtggg ccatcgccct gatagacggt ttttcgccct ttgacgttgg 6840

agtccacgtt ctttaatagt ggactcttgt tccaaactgg aacaacactc aaccctatct 6900

cggtctattc ttttgattta taagggattt tgccgatttc ggcctattgg ttaaaaaatg 6960

agctgattta acaaaaattt aacgcgaatt ttaacaaaat attaacgctt acaatttcca 7020

ttcgccattc aggctgcgca actgttggga agggcgatcg gtgcgggcct cttcgctatt 7080

acgccagctg gcgaaagggg gatgtgctgc aaggcgatta agttgggtaa cgccagggtt 7140

ttcccagtca cgacgttgta aaacgacggc cagtgaattg taatacgact cactataggg 7200

cgaattgggt accgggcccc ccctcgaggt cgatggtgtc gataagcttg atatcgaatt 7260

catgtcacac aaaccgatct tcgcctcaag gaaacctaat tctacatccg agagactgcc 7320

gagatccagt ctacactgat taattttcgg gccaataatt taaaaaaatc gtgttatata 7380

atattatatg tattatatat atacatcatg atgatactga cagtcatgtc ccattgctaa 7440

atagacagac tccatctgcc gcctccaact gatgttctca atatttaagg ggtcatctcg 7500

cattgtttaa taataaacag actccatcta ccgcctccaa atgatgttct caaaatatat 7560

tgtatgaact tatttttatt acttagtatt attagacaac ttacttgctt tatgaaaaac 7620

acttcctatt taggaaacaa tttataatgg cagttcgttc atttaacaat ttatgtagaa 7680

taaatgttat aaatgcgtat gggaaatctt aaatatggat agcataaatg atatctgcat 7740

tgcctaattc gaaatcaaca gcaacgaaaa aaatcccttg tacaacataa atagtcatcg 7800

agaaatatca actatcaaag aacagctatt cacacgttac tattgagatt attattggac 7860

gagaatcaca cactcaactg tctttctctc ttctagaaat acaggtacaa gtatgtacta 7920

ttctcattgt tcatacttct agtcatttca tcccacatat tccttggatt tctctccaat 7980

gaatgacatt ctatcttgca aattcaacaa ttataataag atataccaaa gtagcggtat 8040

agtggcaatc aaaaagcttc tctggtgtgc ttctcgtatt tatttttatt ctaatgatcc 8100

attaaaggta tatatttatt tcttgttata taatcctttt gtttattaca tgggctggat 8160

acataaaggt attttgattt aattttttgc ttaaattcaa tcccccctcg ttcagtgtca 8220

actgtaatgg taggaaatta ccatactttt gaagaagcaa aaaaaatgaa agaaaaaaaa 8280

aatcgtattt ccaggttaga cgttccgcag aatctagaat gcggtatgcg gtacattgtt 8340

cttcgaacgt aaaagttgcg ctccctgaga tattgtacat ttttgctttt acaagtacaa 8400

gtacatcgta caactatgta ctactgttga tgcatccaca acagtttgtt ttgttttttt 8460

ttgttttttt tttttctaat gattcattac cgctatgtat acctacttgt acttgtagta 8520

agccgggtta ttggcgttca attaatcata gacttatgaa tctgcacggt gtgcgctgcg 8580

agttactttt agcttatgca tgctacttgg gtgtaatatt gggatctgtt cggaaatcaa 8640

cggatgctca atcgatttcg acagtaatta attaagtcat acacaagtca gctttcttcg 8700

agcctcatat aagtataagt agttcaacgt attagcactg tacccagcat ctccgtatcg 8760

agaaacacaa caacatgccc cattggacag atcatgcgga tacacaggtt gtgcagtatc 8820

atacatactc gatcagacag gtcgtctgac catcatacaa gctgaacaag cgctccatac 8880

ttgcacgctc tctatataca cagttaaatt acatatccat agtctaacct ctaacagtta 8940

atcttctggt aagcctccca gccagccttc tggtatcgct tggcctcctc aataggatct 9000

cggttctggc cgtacagacc tcggccgaca attatgatat ccgttccggt agacatgaca 9060

tcctcaacag ttcggtactg ctgtccgaga gcgtctccct tgtcgtcaag acccaccccg 9120

ggggtcagaa taagccagtc ctcagagtcg cccttaggtc ggttctgggc aatgaagcca 9180

accacaaact cggggtcgga tcgggcaagc tcaatggtct gcttggagta ctcgccagtg 9240

gccagagagc ccttgcaaga cagctcggcc agcatgagca gacctctggc cagcttctcg 9300

ttgggagagg ggactaggaa ctccttgtac tgggagttct cgtagtcaga gacgtcctcc 9360

ttcttctgtt cagagacagt ttcctcggca ccagctcgca ggccagcaat gattccggtt 9420

ccgggtacac cgtgggcgtt ggtgatatcg gaccactcgg cgattcggtg acaccggtac 9480

tggtgcttga cagtgttgcc aatatctgcg aactttctgt cctcgaacag gaagaaaccg 9540

tgcttaagag caagttcctt gagggggagc acagtgccgg cgtaggtgaa gtcgtcaatg 9600

atgtcgatat gggttttgat catgcacaca taaggtccga ccttatcggc aagctcaatg 9660

agctccttgg tggtggtaac atccagagaa gcacacaggt tggttttctt ggctgccacg 9720

agcttgagca ctcgagcggc aaaggcggac ttgtggacgt tagctcgagc ttcgtaggag 9780

ggcattttgg tggtgaagag gagactgaaa taaatttagt ctgcagaact ttttatcgga 9840

accttatctg gggcagtgaa gtatatgtta tggtaatagt tacgagttag ttgaacttat 9900

agatagactg gactatacgg ctatcggtcc aaattagaaa gaacgtcaat ggctctctgg 9960

gcgtcgcctt tgccgacaaa aatgtgatca tgatgaaagc cagcaatgac gttgcagctg 10020

atattgttgt cggccaaccg cgccgaaaac gcagctgtca gacccacagc ctccaacgaa 10080

gaatgtatcg tcaaagtgat ccaagcacac tcatagttgg agtcgtactc caaaggcggc 10140

aatgacgagt cagacagata ctcgtcgacg tttaaaccat catctaaggg cctcaaaact 10200

acctcggaac tgctgcgctg atctggacac cacagaggtt ccgagcactt taggttgcac 10260

caaatgtccc accaggtgca ggcagaaaac gctggaacag cgtgtacagt ttgtcttaac 10320

aaaaagtgag ggcgctgagg tcgagcaggg tggtgtgact tgttatagcc tttagagctg 10380

cgaaagcgcg tatggatttg gctcatcagg ccagattgag ggtctgtgga cacatgtcat 10440

gttagtgtac ttcaatcgcc ccctggatat agccccgaca ataggccgtg gcctcatttt 10500

tttgccttcc gcacatttcc attgctcggt acccacacct tgcttctcct gcacttgcca 10560

accttaatac tggtttacat tgaccaacat cttacaagcg gggggcttgt ctagggtata 10620

tataaacagt ggctctccca atcggttgcc agtctctttt ttcctttctt tccccacaga 10680

ttcgaaatct aaactacaca tcacac 10706

<210> 7
<211> 47
<212> DNA
<213> Artificial sequence

<220>
<223> AarI-removal 1

<400> 7
agaagtatcc taccatctac catctccgaa agaaactcgt cgattcc 47

<210> 8
<211> 47
<212> DNA
<213> Artificial sequence

<220>
<223> AarI-removal 2

<400> 8
ggaatcgacg agtttctttc ggagatggta gatggtagga tacttct 47

<210> 9
<211> 10706
<212> DNA
<213> Artificial sequence

<220>
<223>  pRF109

<400>  9
catggacaag aaatactcca tcggcctgga cattggaacc aactctgtcg gctgggctgt      60

catcaccgac gagtacaagg tgccctccaa gaaattcaag gtcctcggaa acaccgatcg     120

acactccatc aagaaaaacc tcattggtgc cctgttgttc gattctggcg agactgccga     180

agctaccaga ctcaagcgaa ctgctcggcg acgttacacc cgacggaaga accgaatctg     240

ctacctgcag gagatctttt ccaacgagat ggccaaggtg gacgattcgt tctttcatcg     300

actggaggaa tccttcctcg tcgaggaaga caagaaacac gagcgtcatc ccatctttgg     360

caacattgtg gacgaggttg cttaccacga gaagtatcct accatctacc atctccgaaa     420

gaaactcgtc gattccaccg acaaggcgga tctcagactt atctacctcg ctctggcaca     480

catgatcaag tttcgaggtc atttcctcat cgagggcgat ctcaatcccg acaacagcga     540

tgtggacaag ctgttcattc agctcgttca gacctacaac cagctgttcg aggaaaaccc     600

catcaatgcc tccggagtcg atgcaaaggc catcttgtct gctcgactct cgaagagcag     660

acgactggag aacctcattg cccaacttcc tggcgagaaa aagaacggac tgtttggcaa     720

cctcattgcc ctttctcttg gtctcacacc caacttcaag tccaacttcg atctggcgga     780

ggacgccaag ctccagctgt ccaaggacac ctacgacgat gacctcgaca acctgcttgc     840

acagattggc gatcagtacg ccgacctgtt tctcgctgcc aagaaccttt cggatgctat     900

tctcttgtct gacattctgc gagtcaacac cgagatcaca aaggctcccc tttctgcctc     960

catgatcaag cgatacgacg agcaccatca ggatctcaca ctgctcaagg ctcttgtccg    1020

acagcaactg cccgagaagt acaaggagat ctttttcgat cagtcgaaga acggctacgc    1080

tggatacatc gacggcggag cctctcagga agagttctac aagttcatca agccaattct    1140

cgagaagatg gacggaaccg aggaactgct tgtcaagctc aatcgagagg atctgcttcg    1200

gaagcaacga accttcgaca acggcagcat tcctcatcag atccacctcg gtgagctgca    1260

cgccattctt cgacgtcagg aagacttcta cccctttctc aaggacaacc gagagaagat    1320

cgagaagatt cttacctttc gaatccccta ctatgttggt cctcttgcca gaggaaactc    1380

tcgatttgct tggatgactc gaaagtccga ggaaaccatc actccctgga acttcgagga    1440

agtcgtggac aagggtgcct ctgcacagtc cttcatcgag cgaatgacca acttcgacaa    1500

gaatctgccc aacgagaagg ttcttcccaa gcattcgctg ctctacgagt actttacagt    1560

ctacaacgaa ctcaccaaag tcaagtacgt taccgaggga atgcgaaagc ctgccttctt    1620

gtctggcgaa cagaagaaag ccattgtcga tctcctgttc aagaccaacc gaaaggtcac    1680

tgttaagcag ctcaaggagg actacttcaa gaaaatcgag tgtttcgaca gcgtcgagat    1740

ttccggagtt gaggaccgat tcaacgcctc tttgggcacc tatcacgatc tgctcaagat 1800

tatcaaggac aaggattttc tcgacaacga ggaaaacgag gacattctgg aggacatcgt 1860

gctcactctt accctgttcg aagatcggga gatgatcgag gaacgactca agacatacgc 1920

tcacctgttc gacgacaagg tcatgaaaca actcaagcga cgtagataca ccggctgggg 1980

aagactttcg cgaaagctca tcaacggcat cagagacaag cagtccggaa agaccattct 2040

ggactttctc aagtccgatg gctttgccaa ccgaaacttc atgcagctca ttcacgacga 2100

ttctcttacc ttcaaggagg acatccagaa ggcacaagtg tccggtcagg gcgacagctt 2160

gcacgaacat attgccaacc tggctggttc gccagccatc aagaaaggca ttctccagac 2220

tgtcaaggtt gtcgacgagc tggtgaaggt catgggacgt cacaagcccg agaacattgt 2280

gatcgagatg gccagagaga accagacaac tcaaaagggt cagaaaaact cgcgagagcg 2340

gatgaagcga atcgaggaag gcatcaagga gctgggatcc cagattctca aggagcatcc 2400

cgtcgagaac actcaactgc agaacgagaa gctgtatctc tactatctgc agaatggtcg 2460

agacatgtac gtggatcagg aactggacat caatcgtctc agcgactacg atgtggacca 2520

cattgtccct caatcctttc tcaaggacga ttctatcgac aacaaggtcc ttacacgatc 2580

cgacaagaac agaggcaagt cggacaacgt tcccagcgaa gaggtggtca aaaagatgaa 2640

gaactactgg cgacagctgc tcaacgccaa gctcattacc cagcgaaagt tcgacaatct 2700

taccaaggcc gagcgaggcg gtctgtccga gctcgacaag gctggcttca tcaagcgtca 2760

actcgtcgag accagacaga tcacaaagca cgtcgcacag attctcgatt ctcggatgaa 2820

caccaagtac gacgagaacg acaagctcat ccgagaggtc aaggtgatta ctctcaagtc 2880

caaactggtc tccgatttcc gaaaggactt tcagttctac aaggtgcgag agatcaacaa 2940

ttaccaccat gcccacgatg cttacctcaa cgccgtcgtt ggcactgcgc tcatcaagaa 3000

ataccccaag ctcgaaagcg agttcgttta cggcgattac aaggtctacg acgttcgaaa 3060

gatgattgcc aagtccgaac aggagattgg caaggctact gccaagtact tcttttactc 3120

caacatcatg aactttttca agaccgagat caccttggcc aacggagaga ttcgaaagag 3180

accacttatc gagaccaacg gcgaaactgg agagatcgtg tgggacaagg gtcgagactt 3240

tgcaaccgtg cgaaaggttc tgtcgatgcc tcaggtcaac atcgtcaaga aaaccgaggt 3300

tcagactggc ggattctcca aggagtcgat tctgcccaag cgaaactccg acaagctcat 3360

cgctcgaaag aaagactggg atcccaagaa atacggtggc ttcgattctc ctaccgtcgc 3420

ctattccgtg cttgtcgttg cgaaggtcga gaagggcaag tccaaaaagc tcaagtccgt 3480

caaggagctg ctcggaatta ccatcatgga gcgatcgagc ttcgagaaga atcccatcga 3540

cttcttggaa gccaagggtt acaaggaggt caagaaagac ctcattatca agctgcccaa 3600

gtactctctg ttcgaactgg agaacggtcg aaagcgtatg ctcgcctccg ctggcgagct 3660

gcagaaggga aacgagcttg ccttgccttc gaagtacgtc aactttctct atctggcttc 3720

tcactacgag aagctcaagg gttctcccga ggacaacgaa cagaagcaac tcttcgttga 3780

gcagcacaaa cattacctcg acgagattat cgagcagatt tccgagtttt cgaagcgagt 3840

catcctggct gatgccaact tggacaaggt gctctctgcc tacaacaagc atcgggacaa 3900

acccattcga gaacaggcgg agaacatcat tcacctgttt actcttacca acctgggtgc 3960

tcctgcagct ttcaagtact tcgataccac tatcgaccga aagcggtaca catccaccaa 4020

ggaggttctc gatgccaccc tgattcacca gtccatcact ggcctgtacg agacccgaat 4080

cgacctgtct cagcttggtg gcgactccag agccgatccc aagaaaaagc gaaaggtcta 4140

agcggccgca agtgtggatg gggaagtgag tgcccggttc tgtgtgcaca attggcaatc 4200

caagatggat ggattcaaca caggatatag cgagctacgt tggtggtgcg aggatatagc 4260

aacggatatt tatgtttgac acttgagaat gtacgataca agcactgtcc aagtacaata 4320

ctaaacatac tgtacatact catactcgta cccgggcaac ggtttcactt gagtgcagtg 4380

gctagtgctc ttactcgtac agtgtgcaat actgcgtatc atagtctttg atgtatatcg 4440

tattcattca tgttagttgc gtacgagccg gaagcataaa gtgtaaagcc tggggtgcct 4500

aatgagtgag ctaactcaca ttaattgcgt tgcgctcact gcccgctttc cagtcgggaa 4560

acctgtcgtg ccagctgcat taatgaatcg gccaacgcgc ggggagaggc ggtttgcgta 4620

ttgggcgctc ttccgcttcc tcgctcactg actcgctgcg ctcggtcgtt cggctgcggc 4680

gagcggtatc agctcactca aaggcggtaa tacggttatc cacagaatca ggggataacg 4740

caggaaagaa catgtgagca aaaggccagc aaaaggccag gaaccgtaaa aaggccgcgt 4800

tgctggcgtt tttccatagg ctccgccccc ctgacgagca tcacaaaaat cgacgctcaa 4860

gtcagaggtg gcgaaacccg acaggactat aaagatacca ggcgtttccc cctggaagct 4920

ccctcgtgcg ctctcctgtt ccgaccctgc cgcttaccgg atacctgtcc gcctttctcc 4980

cttcgggaag cgtggcgctt tctcatagct cacgctgtag gtatctcagt tcggtgtagg 5040

tcgttcgctc caagctgggc tgtgtgcacg aaccccccgt tcagcccgac cgctgcgcct 5100

tatccggtaa ctatcgtctt gagtccaacc cggtaagaca cgacttatcg ccactggcag 5160

cagccactgg taacaggatt agcagagcga ggtatgtagg cggtgctaca gagttcttga 5220

agtggtggcc taactacggc tacactagaa ggacagtatt tggtatctgc gctctgctga 5280

agccagttac cttcggaaaa agagttggta gctcttgatc cggcaaacaa accaccgctg 5340

gtagcggtgg tttttttgtt tgcaagcagc agattacgcg cagaaaaaaa ggatctcaag 5400

aagatccttt gatcttttct acggggtctg acgctcagtg gaacgaaaac tcacgttaag 5460

ggattttggt catgagatta tcaaaaagga tcttcaccta gatcctttta aattaaaaat 5520

gaagttttaa atcaatctaa agtatatatg agtaaacttg gtctgacagt taccaatgct 5580

taatcagtga ggcacctatc tcagcgatct gtctatttcg ttcatccata gttgcctgac 5640

tccccgtcgt gtagataact acgatacggg agggcttacc atctggcccc agtgctgcaa 5700

tgataccgcg agacccacgc tcaccggctc cagatttatc agcaataaac cagccagccg 5760

gaagggccga gcgcagaagt ggtcctgcaa ctttatccgc ctccatccag tctattaatt 5820

gttgccggga agctagagta agtagttcgc cagttaatag tttgcgcaac gttgttgcca 5880

ttgctacagg catcgtggtg tcacgctcgt cgtttggtat ggcttcattc agctccggtt 5940

cccaacgatc aaggcgagtt acatgatccc ccatgttgtg caaaaaagcg gttagctcct 6000

tcggtcctcc gatcgttgtc agaagtaagt tggccgcagt gttatcactc atggttatgg 6060

cagcactgca taattctctt actgtcatgc catccgtaag atgcttttct gtgactggtg 6120

agtactcaac caagtcattc tgagaatagt gtatgcggcg accgagttgc tcttgcccgg 6180

cgtcaatacg ggataatacc gcgccacata gcagaacttt aaaagtgctc atcattggaa 6240

aacgttcttc ggggcgaaaa ctctcaagga tcttaccgct gttgagatcc agttcgatgt 6300

aacccactcg tgcacccaac tgatcttcag catcttttac tttcaccagc gtttctgggt 6360

gagcaaaaac aggaaggcaa aatgccgcaa aaaagggaat aagggcgaca cggaaatgtt 6420

gaatactcat actcttcctt tttcaatatt attgaagcat ttatcagggt tattgtctca 6480

tgagcggata catatttgaa tgtatttaga aaaataaaca aataggggtt ccgcgcacat 6540

ttccccgaaa agtgccacct gacgcgccct gtagcggcgc attaagcgcg gcgggtgtgg 6600

tggttacgcg cagcgtgacc gctacacttg ccagcgccct agcgcccgct cctttcgctt 6660

tcttcccttc ctttctcgcc acgttcgccg gctttccccg tcaagctcta aatcgggggc 6720

tccctttagg gttccgattt agtgctttac ggcacctcga ccccaaaaaa cttgattagg 6780

gtgatggttc acgtagtggg ccatcgccct gatagacggt ttttcgccct ttgacgttgg 6840

agtccacgtt ctttaatagt ggactcttgt tccaaactgg aacaacactc aaccctatct 6900

cggtctattc ttttgattta taagggattt tgccgatttc ggcctattgg ttaaaaaatg 6960

agctgattta acaaaaattt aacgcgaatt ttaacaaaat attaacgctt acaatttcca 7020

ttcgccattc aggctgcgca actgttggga agggcgatcg gtgcgggcct cttcgctatt 7080

acgccagctg gcgaaagggg gatgtgctgc aaggcgatta agttgggtaa cgccagggtt 7140

ttcccagtca cgacgttgta aaacgacggc cagtgaattg taatacgact cactataggg 7200

cgaattgggt accgggcccc ccctcgaggt cgatggtgtc gataagcttg atatcgaatt 7260

catgtcacac aaaccgatct tcgcctcaag gaaacctaat tctacatccg agagactgcc 7320

gagatccagt ctacactgat taattttcgg gccaataatt taaaaaaatc gtgttatata 7380

atattatatg tattatatat atacatcatg atgatactga cagtcatgtc ccattgctaa 7440

atagacagac tccatctgcc gcctccaact gatgttctca atatttaagg ggtcatctcg 7500

cattgtttaa taataaacag actccatcta ccgcctccaa atgatgttct caaaatatat 7560

tgtatgaact tatttttatt acttagtatt attagacaac ttacttgctt tatgaaaaac 7620

acttcctatt taggaaacaa tttataatgg cagttcgttc atttaacaat ttatgtagaa 7680

taaatgttat aaatgcgtat gggaaatctt aaatatggat agcataaatg atatctgcat 7740

tgcctaattc gaaatcaaca gcaacgaaaa aaatcccttg tacaacataa atagtcatcg 7800

agaaatatca actatcaaag aacagctatt cacacgttac tattgagatt attattggac 7860

gagaatcaca cactcaactg tctttctctc ttctagaaat acaggtacaa gtatgtacta 7920

ttctcattgt tcatacttct agtcatttca tcccacatat tccttggatt tctctccaat 7980

gaatgacatt ctatcttgca aattcaacaa ttataataag atataccaaa gtagcggtat 8040

agtggcaatc aaaaagcttc tctggtgtgc ttctcgtatt tatttttatt ctaatgatcc 8100

attaaaggta tatatttatt tcttgttata taatcctttt gtttattaca tgggctggat 8160

acataaaggt attttgattt aattttttgc ttaaattcaa tcccccctcg ttcagtgtca 8220

actgtaatgg taggaaatta ccatactttt gaagaagcaa aaaaaatgaa agaaaaaaaa 8280

aatcgtattt ccaggttaga cgttccgcag aatctagaat gcggtatgcg gtacattgtt 8340

cttcgaacgt aaaagttgcg ctccctgaga tattgtacat ttttgctttt acaagtacaa 8400

gtacatcgta caactatgta ctactgttga tgcatccaca acagtttgtt ttgttttttt 8460

ttgttttttt tttttctaat gattcattac cgctatgtat acctacttgt acttgtagta 8520

agccgggtta ttggcgttca attaatcata gacttatgaa tctgcacggt gtgcgctgcg 8580

agttactttt agcttatgca tgctacttgg gtgtaatatt gggatctgtt cggaaatcaa 8640

cggatgctca atcgatttcg acagtaatta attaagtcat acacaagtca gctttcttcg 8700

agcctcatat aagtataagt agttcaacgt attagcactg tacccagcat ctccgtatcg 8760

agaaacacaa caacatgccc cattggacag atcatgcgga tacacaggtt gtgcagtatc 8820

atacatactc gatcagacag gtcgtctgac catcatacaa gctgaacaag cgctccatac 8880

ttgcacgctc tctatataca cagttaaatt acatatccat agtctaacct ctaacagtta 8940

atcttctggt aagcctccca gccagccttc tggtatcgct tggcctcctc aataggatct 9000

cggttctggc cgtacagacc tcggccgaca attatgatat ccgttccggt agacatgaca 9060

tcctcaacag ttcggtactg ctgtccgaga gcgtctccct tgtcgtcaag acccaccccg 9120

ggggtcagaa taagccagtc ctcagagtcg cccttaggtc ggttctgggc aatgaagcca 9180

accacaaact cggggtcgga tcgggcaagc tcaatggtct gcttggagta ctcgccagtg 9240

gccagagagc ccttgcaaga cagctcggcc agcatgagca gacctctggc cagcttctcg 9300

ttgggagagg ggactaggaa ctccttgtac tgggagttct cgtagtcaga gacgtcctcc 9360

ttcttctgtt cagagacagt ttcctcggca ccagctcgca ggccagcaat gattccggtt 9420

ccgggtacac cgtgggcgtt ggtgatatcg gaccactcgg cgattcggtg acaccggtac 9480

tggtgcttga cagtgttgcc aatatctgcg aactttctgt cctcgaacag gaagaaaccg 9540

tgcttaagag caagttcctt gagggggagc acagtgccgg cgtaggtgaa gtcgtcaatg 9600

atgtcgatat gggttttgat catgcacaca taaggtccga ccttatcggc aagctcaatg 9660

agctccttgg tggtggtaac atccagagaa gcacacaggt tggttttctt ggctgccacg 9720

agcttgagca ctcgagcggc aaaggcggac ttgtggacgt tagctcgagc ttcgtaggag 9780

ggcattttgg tggtgaagag gagactgaaa taaatttagt ctgcagaact ttttatcgga 9840

accttatctg gggcagtgaa gtatatgtta tggtaatagt tacgagttag ttgaacttat 9900

agatagactg gactatacgg ctatcggtcc aaattagaaa gaacgtcaat ggctctctgg 9960

gcgtcgcctt tgccgacaaa aatgtgatca tgatgaaagc cagcaatgac gttgcagctg 10020

atattgttgt cggccaaccg cgccgaaaac gcagctgtca gacccacagc ctccaacgaa 10080

gaatgtatcg tcaaagtgat ccaagcacac tcatagttgg agtcgtactc caaaggcggc 10140

aatgacgagt cagacagata ctcgtcgacg tttaaaccat catctaaggg cctcaaaact 10200

acctcggaac tgctgcgctg atctggacac cacagaggtt ccgagcactt taggttgcac 10260

caaatgtccc accaggtgca ggcagaaaac gctggaacag cgtgtacagt ttgtcttaac 10320

aaaaagtgag ggcgctgagg tcgagcaggg tggtgtgact tgttatagcc tttagagctg 10380

cgaaagcgcg tatggatttg gctcatcagg ccagattgag ggtctgtgga cacatgtcat 10440

gttagtgtac ttcaatcgcc ccctggatat agccccgaca ataggccgtg gcctcatttt 10500

tttgccttcc gcacatttcc attgctcggt acccacacct tgcttctcct gcacttgcca 10560

accttaatac tggtttacat tgaccaacat cttacaagcg gggggcttgt ctagggtata 10620

tataaacagt ggctctccca atcggttgcc agtctctttt ttcctttctt tccccacaga 10680

ttcgaaatct aaactacaca tcacac 10706

<210> 10
<211> 4140
<212> DNA
<213> Artificial sequence

<220>
<223> Aar1- Cas9 ORF

<400> 10
atggacaaga aatactccat cggcctggac attggaacca actctgtcgg ctgggctgtc 60

atcaccgacg agtacaaggt gccctccaag aaattcaagg tcctcggaaa caccgatcga 120

cactccatca agaaaaacct cattggtgcc ctgttgttcg attctggcga gactgccgaa 180

gctaccagac tcaagcgaac tgctcggcga cgttacaccc gacggaagaa ccgaatctgc 240

tacctgcagg agatcttttc caacgagatg gccaaggtgg acgattcgtt ctttcatcga 300

ctggaggaat ccttcctcgt cgaggaagac aagaaacacg agcgtcatcc catctttggc 360

aacattgtgg acgaggttgc ttaccacgag aagtatccta ccatctacca tctccgaaag 420

aaactcgtcg attccaccga caaggcggat ctcagactta tctacctcgc tctggcacac 480

atgatcaagt ttcgaggtca tttcctcatc gagggcgatc tcaatcccga caacagcgat 540

gtggacaagc tgttcattca gctcgttcag acctacaacc agctgttcga ggaaaacccc 600

atcaatgcct ccggagtcga tgcaaaggcc atcttgtctg ctcgactctc gaagagcaga 660

cgactggaga acctcattgc ccaacttcct ggcgagaaaa agaacggact gtttggcaac 720

ctcattgccc tttctcttgg tctcacaccc aacttcaagt ccaacttcga tctggcggag 780

gacgccaagc tccagctgtc caaggacacc tacgacgatg acctcgacaa cctgcttgca 840

cagattggcg atcagtacgc cgacctgttt ctcgctgcca agaacctttc ggatgctatt 900

ctcttgtctg acattctgcg agtcaacacc gagatcacaa aggctcccct ttctgcctcc 960

atgatcaagc gatacgacga gcaccatcag gatctcacac tgctcaaggc tcttgtccga 1020

cagcaactgc ccgagaagta caaggagatc tttttcgatc agtcgaagaa cggctacgct 1080

ggatacatcg acggcggagc ctctcaggaa gagttctaca agttcatcaa gccaattctc 1140

gagaagatgg acggaaccga ggaactgctt gtcaagctca atcgagagga tctgcttcgg 1200

aagcaacgaa ccttcgacaa cggcagcatt cctcatcaga tccacctcgg tgagctgcac 1260

gccattcttc gacgtcagga agacttctac ccctttctca aggacaaccg agagaagatc 1320

gagaagattc ttacctttcg aatcccctac tatgttggtc ctcttgccag aggaaactct 1380

cgatttgctt ggatgactcg aaagtccgag gaaaccatca ctccctggaa cttcgaggaa 1440

gtcgtggaca agggtgcctc tgcacagtcc ttcatcgagc gaatgaccaa cttcgacaag 1500

aatctgccca acgagaaggt tcttcccaag cattcgctgc tctacgagta ctttacagtc 1560

tacaacgaac tcaccaaagt caagtacgtt accgagggaa tgcgaaagcc tgccttcttg 1620

tctggcgaac agaagaaagc cattgtcgat ctcctgttca agaccaaccg aaaggtcact 1680

gttaagcagc tcaaggagga ctacttcaag aaaatcgagt gtttcgacag cgtcgagatt 1740

tccggagttg aggaccgatt caacgcctct ttgggcacct atcacgatct gctcaagatt 1800

atcaaggaca aggattttct cgacaacgag gaaaacgagg acattctgga ggacatcgtg 1860

ctcactctta ccctgttcga agatcgggag atgatcgagg aacgactcaa gacatacgct 1920

cacctgttcg acgacaaggt catgaaacaa ctcaagcgac gtagatacac cggctgggga 1980

agactttcgc gaaagctcat caacggcatc agagacaagc agtccggaaa gaccattctg 2040

gactttctca agtccgatgg ctttgccaac cgaaacttca tgcagctcat tcacgacgat 2100

tctcttacct tcaaggagga catccagaag gcacaagtgt ccggtcaggg cgacagcttg 2160

cacgaacata ttgccaacct ggctggttcg ccagccatca agaaaggcat tctccagact 2220

gtcaaggttg tcgacgagct ggtgaaggtc atgggacgtc acaagcccga gaacattgtg 2280

atcgagatgg ccagagagaa ccagacaact caaaagggtc agaaaaactc gcgagagcgg 2340

atgaagcgaa tcgaggaagg catcaaggag ctgggatccc agattctcaa ggagcatccc 2400

gtcgagaaca ctcaactgca gaacgagaag ctgtatctct actatctgca gaatggtcga 2460

gacatgtacg tggatcagga actggacatc aatcgtctca gcgactacga tgtggaccac 2520

attgtccctc aatcctttct caaggacgat tctatcgaca acaaggtcct tacacgatcc 2580

gacaagaaca gaggcaagtc ggacaacgtt cccagcgaag aggtggtcaa aaagatgaag 2640

aactactggc gacagctgct caacgccaag ctcattaccc agcgaaagtt cgacaatctt 2700

accaaggccg agcgaggcgg tctgtccgag ctcgacaagg ctggcttcat caagcgtcaa 2760

ctcgtcgaga ccagacagat cacaaagcac gtcgcacaga ttctcgattc tcggatgaac 2820

accaagtacg acgagaacga caagctcatc cgagaggtca aggtgattac tctcaagtcc 2880

aaactggtct ccgatttccg aaaggacttt cagttctaca aggtgcgaga gatcaacaat 2940

taccaccatg cccacgatgc ttacctcaac gccgtcgttg gcactgcgct catcaagaaa 3000

taccccaagc tcgaaagcga gttcgtttac ggcgattaca aggtctacga cgttcgaaag 3060

atgattgcca agtccgaaca ggagattggc aaggctactg ccaagtactt cttttactcc 3120

aacatcatga actttttcaa gaccgagatc accttggcca acggagagat tcgaaagaga 3180

ccacttatcg agaccaacgg cgaaactgga gagatcgtgt gggacaaggg tcgagacttt 3240

gcaaccgtgc gaaaggttct gtcgatgcct caggtcaaca tcgtcaagaa aaccgaggtt 3300

cagactggcg gattctccaa ggagtcgatt ctgcccaagc gaaactccga caagctcatc 3360

gctcgaaaga aagactggga tcccaagaaa tacggtggct tcgattctcc taccgtcgcc 3420

tattccgtgc ttgtcgttgc gaaggtcgag aagggcaagt ccaaaaagct caagtccgtc 3480

aaggagctgc tcggaattac catcatggag cgatcgagct tcgagaagaa tcccatcgac 3540

ttcttggaag ccaaggttta caaggaggtc aagaaagacc tcattatcaa gctgcccaag 3600

tactctctgt tcgaactgga gaacggtcga aagcgtatgc tcgcctccgc tggcgagctg 3660

cagaagggaa acgagcttgc cttgccttcg aagtacgtca actttctcta tctggcttct 3720

cactacgaga agctcaaggg ttctcccgag gacaacgaac agaagcaact cttcgttgag 3780

cagcacaaac attacctcga cgagattatc gagcagattt ccgagttttc gaagcgagtc 3840

atcctggctg atgccaactt ggacaaggtg ctctctgcct acaacaagca tcgggacaaa 3900

cccattcgag aacaggcgga gaacatcatt cacctgttta ctcttaccaa cctgggtgct 3960

cctgcagctt tcaagtactt cgataccact atcgaccgaa agcggtacac atccaccaag 4020

gaggttctcg atgccaccct gattcaccag tccatcactg gcctgtacga gacccgaatc 4080

gacctgtctc agcttggtgg cgactccaga gccgatccca agaaaaagcg aaaggtctaa 4140

<210> 11
<211> 10706
<212> DNA
<213> Artificial sequence

<220>
<223> pRF141

<400> 11
catggacaag aaatactcca tcggcctgga cattggaacc aactctgtcg gctgggctgt 60

catcaccgac gagtacaagg tgccctccaa gaaattcaag gtcctcggaa acaccgatcg 120

acactccatc aagaaaaacc tcattggtgc cctgttgttc gattctggcg agactgccga 180

agctaccaga ctcaagcgaa ctgctcggcg acgttacacc cgacggaaga accgaatctg 240

ctacctgcag gagatctttt ccaacgagat ggccaaggtg gacgattcgt tctttcatcg 300

actggaggaa tccttcctcg tcgaggaaga caagaaacac gagcgtcatc ccatctttgg 360

caacattgtg gacgaggttg cttaccacga gaagtatcct accatctacc atctccgaaa 420

gaaactcgtc gattccaccg acaaggcgga tctcagactt atctacctcg ctctggcaca 480

catgatcaag tttcgaggtc atttcctcat cgagggcgat ctcaatcccg acaacagcga 540

tgtggacaag ctgttcattc agctcgttca gacctacaac cagctgttcg aggaaaaccc 600

catcaatgcc tccggagtcg atgcaaaggc catcttgtct gctcgactct cgaagagcag 660

acgactggag aacctcattg cccaacttcc tggcgagaaa aagaacggac tgtttggcaa 720

cctcattgcc ctttctcttg gtctcacacc caacttcaag tccaacttcg atctggcgga 780

ggacgccaag ctccagctgt ccaaggacac ctacgacgat gacctcgaca acctgcttgc 840

acagattggc gatcagtacg ccgacctgtt tctcgctgcc aagaaccttt cggatgctat 900

tctcttgtct gacattctgc gagtcaacac cgagatcaca aaggctcccc tttctgcctc 960

catgatcaag cgatacgacg agcaccatca ggatctcaca ctgctcaagg ctcttgtccg 1020

acagcaactg cccgagaagt acaaggagat ctttttcgat cagtcgaaga acggctacgc 1080

tggatacatc gacggcggag cctctcagga agagttctac aagttcatca agccaattct 1140

cgagaagatg gacggaaccg aggaactgct tgtcaagctc aatcgagagg atctgcttcg 1200

gaagcaacga accttcgaca acggcagcat tcctcatcag atccacctcg gtgagctgca 1260

cgccattctt cgacgtcagg aagacttcta cccctttctc aaggacaacc gagagaagat 1320

cgagaagatt cttacctttc gaatccccta ctatgttggt cctcttgcca gaggaaactc 1380

tcgatttgct tggatgactc gaaagtccga ggaaaccatc actccctgga acttcgagga 1440

agtcgtggac aagggtgcct ctgcacagtc cttcatcgag cgaatgacca acttcgacaa 1500

gaatctgccc aacgagaagg ttcttcccaa gcattcgctg ctctacgagt actttacagt 1560

ctacaacgaa ctcaccaaag tcaagtacgt taccgaggga atgcgaaagc ctgccttctt 1620

gtctggcgaa cagaagaaag ccattgtcga tctcctgttc aagaccaacc gaaaggtcac 1680

tgttaagcag ctcaaggagg actacttcaa gaaaatcgag tgtttcgaca gcgtcgagat 1740

ttccggagtt gaggaccgat tcaacgcctc tttgggcacc tatcacgatc tgctcaagat 1800

tatcaaggac aaggattttc tcgacaacga ggaaaacgag gacattctgg aggacatcgt 1860

gctcactctt accctgttcg aagatcggga gatgatcgag gaacgactca agacatacgc 1920

tcacctgttc gacgacaagg tcatgaaaca actcaagcga cgtagataca ccggctgggg 1980

aagactttcg cgaaagctca tcaacggcat cagagacaag cagtccggaa agaccattct 2040

ggactttctc aagtccgatg gctttgccaa ccgaaacttc atgcagctca ttcacgacga 2100

ttctcttacc ttcaaggagg acatccagaa ggcacaagtg tccggtcagg gcgacagctt 2160

gcacgaacat attgccaacc tggctggttc gccagccatc aagaaaggca ttctccagac 2220

tgtcaaggtt gtcgacgagc tggtgaaggt catgggacgt cacaagcccg agaacattgt 2280

gatcgagatg gccagagaga accagacaac tcaaaagggt cagaaaaact cgcgagagcg 2340

gatgaagcga atcgaggaag gcatcaagga gctgggatcc cagattctca aggagcatcc 2400

cgtcgagaac actcaactgc agaacgagaa gctgtatctc tactatctgc agaatggtcg 2460

agacatgtac gtggatcagg aactggacat caatcgtctc agcgactacg atgtggacca 2520

cattgtccct caatcctttc tcaaggacga ttctatcgac aacaaggtcc ttacacgatc 2580

cgacaagaac agaggcaagt cggacaacgt tcccagcgaa gaggtggtca aaaagatgaa 2640

gaactactgg cgacagctgc tcaacgccaa gctcattacc cagcgaaagt tcgacaatct 2700

taccaaggcc gagcgaggcg gtctgtccga gctcgacaag gctggcttca tcaagcgtca 2760

actcgtcgag accagacaga tcacaaagca cgtcgcacag attctcgatt ctcggatgaa 2820

caccaagtac gacgagaacg acaagctcat ccgagaggtc aaggtgatta ctctcaagtc 2880

caaactggtc tccgatttcc gaaaggactt tcagttctac aaggtgcgag agatcaacaa 2940

ttaccaccat gcccacgatg cttacctcaa cgccgtcgtt ggcactgcgc tcatcaagaa 3000

ataccccaag ctcgaaagcg agttcgttta cggcgattac aaggtctacg acgttcgaaa 3060

gatgattgcc aagtccgaac aggagattgg caaggctact gccaagtact tcttttactc 3120

caacatcatg aactttttca agaccgagat caccttggcc aacggagaga ttcgaaagag 3180

accacttatc gagaccaacg gcgaaactgg agagatcgtg tgggacaagg gtcgagactt 3240

tgcaaccgtg cgaaaggttc tgtcgatgcc tcaggtcaac atcgtcaaga aaaccgaggt 3300

tcagactggc ggattctcca aggagtcgat tctgcccaag cgaaactccg acaagctcat 3360

cgctcgaaag aaagactggg atcccaagaa atacggtggc ttcgattctc ctaccgtcgc 3420

ctattccgtg cttgtcgttg cgaaggtcga gaagggcaag tccaaaaagc tcaagtccgt 3480

caaggagctg ctcggaatta ccatcatgga gcgatcgagc ttcgagaaga atcccatcga 3540

cttcttggaa gccaagggtt acaaggaggt caagaaagac ctcattatca agctgcccaa 3600

gtactctctg ttcgaactgg agaacggtcg aaagcgtatg ctcgcctccg ctggcgagct 3660

gcagaaggga aacgagcttg ccttgccttc gaagtacgtc aactttctct atctggcttc 3720

tcactacgag aagctcaagg gttctcccga ggacaacgaa cagaagcaac tcttcgttga 3780

gcagcacaaa cattacctcg acgagattat cgagcagatt tccgagtttt cgaagcgagt 3840

catcctggct gatgccaact tggacaaggt gctctctgcc tacaacaagc atcgggacaa 3900

acccattcga gaacaggcgg agaacatcat tcacctgttt actcttacca acctgggtgc 3960

tcctgcagct ttcaagtact tcgataccac tatcgaccga aagcggtaca catccaccaa 4020

ggaggttctc gatgccaccc tgattcacca gtccatcact ggcctgtacg agacccgaat 4080

cgacctgtct cagcttggtg gcgactccag agccgatccc aagaaaaagc gaaaggtcta 4140

agcggccgca agtgtggatg gggaagtgag tgcccggttc tgtgtgcaca attggcaatc 4200

caagatggat ggattcaaca cagggatata gcgagctacg tggtggtgcg aggatatagc 4260

aacggatatt tatgtttgac acttgagaat gtacgataca agcactgtcc aagtacaata 4320

ctaaacatac tgtacatact catactcgta cccgggcaac ggtttcactt gagtgcagtg 4380

gctagtgctc ttactcgtac agtgtgcaat actgcgtatc atagtctttg atgtatatcg 4440

tattcattca tgttagttgc gtacgagccg gaagcataaa gtgtaaagcc tggggtgcct 4500

aatgagtgag ctaactcaca ttaattgcgt tgcgctcact gcccgctttc cagtcgggaa 4560

acctgtcgtg ccagctgcat taatgaatcg gccaacgcgc ggggagaggc ggtttgcgta 4620

ttgggcgctc ttccgcttcc tcgctcactg actcgctgcg ctcggtcgtt cggctgcggc 4680

gagcggtatc agctcactca aaggcggtaa tacggttatc cacagaatca ggggataacg 4740

caggaaagaa catgtgagca aaaggccagc aaaaggccag gaaccgtaaa aaggccgcgt 4800

tgctggcgtt tttccatagg ctccgccccc ctgacgagca tcacaaaaat cgacgctcaa 4860

gtcagaggtg gcgaaacccg acaggactat aaagatacca ggcgtttccc cctggaagct 4920

ccctcgtgcg ctctcctgtt ccgaccctgc cgcttaccgg atacctgtcc gcctttctcc 4980

cttcgggaag cgtggcgctt tctcatagct cacgctgtag gtatctcagt tcggtgtagg 5040

tcgttcgctc caagctgggc tgtgtgcacg aaccccccgt tcagcccgac cgctgcgcct 5100

tatccggtaa ctatcgtctt gagtccaacc cggtaagaca cgacttatcg ccactggcag 5160

cagccactgg taacaggatt agcagagcga ggtatgtagg cggtgctaca gagttcttga 5220

agtggtggcc taactacggc tacactagaa ggacagtatt tggtatctgc gctctgctga 5280

agccagttac cttcggaaaa agagttggta gctcttgatc cggcaaacaa accaccgctg 5340

gtagcggtgg tttttttgtt tgcaagcagc agattacgcg cagaaaaaaa ggatctcaag 5400

aagatccttt gatcttttct acggggtctg acgctcagtg gaacgaaaac tcacgttaag 5460

ggattttggt catgagatta tcaaaaagga tcttcaccta gatcctttta aattaaaaat 5520

gaagttttaa atcaatctaa agtatatatg agtaaacttg gtctgacagt taccaatgct 5580

taatcagtga ggcacctatc tcagcgatct gtctatttcg ttcatccata gttgcctgac 5640

tccccgtcgt gtagataact acgatacggg agggcttacc atctggcccc agtgctgcaa 5700

tgataccgcg agacccacgc tcaccggctc cagatttatc agcaataaac cagccagccg 5760

gaagggccga gcgcagaagt ggtcctgcaa ctttatccgc ctccatccag tctattaatt 5820

gttgccggga agctagagta agtagttcgc cagttaatag tttgcgcaac gttgttgcca 5880

ttgctacagg catcgtggtg tcacgctcgt cgtttggtat ggcttcattc agctccggtt 5940

cccaacgatc aaggcgagtt acatgatccc ccatgttgtg caaaaaagcg gttagctcct 6000

tcggtcctcc gatcgttgtc agaagtaagt tggccgcagt gttatcactc atggttatgg 6060

cagcactgca taattctctt actgtcatgc catccgtaag atgcttttct gtgactggtg 6120

agtactcaac caagtcattc tgagaatagt gtatgcggcg accgagttgc tcttgcccgg 6180

cgtcaatacg ggataatacc gcgccacata gcagaacttt aaaagtgctc atcattggaa 6240

aacgttcttc ggggcgaaaa ctctcaagga tcttaccgct gttgagatcc agttcgatgt 6300

aacccactcg tgcacccaac tgatcttcag catcttttac tttcaccagc gtttctgggt 6360

gagcaaaaac aggaaggcaa aatgccgcaa aaaagggaat aagggcgaca cggaaatgtt 6420

gaatactcat actcttcctt tttcaatatt attgaagcat ttatcagggt tattgtctca 6480

tgagcggata catatttgaa tgtatttaga aaaataaaca aataggggtt ccgcgcacat 6540

ttccccgaaa agtgccacct gacgcgccct gtagcggcgc attaagcgcg gcgggtgtgg 6600

tggttacgcg cagcgtgacc gctacacttg ccagcgccct agcgcccgct cctttcgctt 6660

tcttcccttc ctttctcgcc acgttcgccg gctttccccg tcaagctcta aatcgggggc 6720

tccctttagg gttccgattt agtgctttac ggcacctcga ccccaaaaaa cttgattagg 6780

gtgatggttc acgtagtggg ccatcgccct gatagacggt ttttcgccct ttgacgttgg 6840

agtccacgtt ctttaatagt ggactcttgt tccaaactgg aacaacactc aaccctatct 6900

cggtctattc ttttgattta taagggattt tgccgatttc ggcctattgg ttaaaaaatg 6960

agctgattta acaaaaattt aacgcgaatt ttaacaaaat attaacgctt acaatttcca 7020

ttcgccattc aggctgcgca actgttggga agggcgatcg gtgcgggcct cttcgctatt 7080

acgccagctg gcgaaagggg gatgtgctgc aaggcgatta agttgggtaa cgccagggtt 7140

ttcccagtca cgacgttgta aaacgacggc cagtgaattg taatacgact cactataggg 7200

cgaattgggt accgggcccc ccctcgaggt cgatggtgtc gataagcttg atatcgaatt 7260

catgtcacac aaaccgatct tcgcctcaag gaaacctaat tctacatccg agagactgcc 7320

gagatccagt ctacactgat taattttcgg gccaataatt taaaaaaatc gtgttatata 7380

atattatatg tattatatat atacatcatg atgatactga cagtcatgtc ccattgctaa 7440

atagacagac tccatctgcc gcctccaact gatgttctca atatttaagg ggtcatctcg 7500

cattgtttaa taataaacag actccatcta ccgcctccaa atgatgttct caaaatatat 7560

tgtatgaact tatttttatt acttagtatt attagacaac ttacttgctt tatgaaaaac 7620

acttcctatt taggaaacaa tttataatgg cagttcgttc atttaacaat ttatgtagaa 7680

taaatgttat aaatgcgtat gggaaatctt aaatatggat agcataaatg atatctgcat 7740

tgcctaattc gaaatcaaca gcaacgaaaa aaatcccttg tacaacataa atagtcatcg 7800

agaaatatca actatcaaag aacagctatt cacacgttac tattgagatt attattggac 7860

gagaatcaca cactcaactg tctttctctc ttctagaaat acaggtacaa gtatgtacta 7920

ttctcattgt tcatacttct agtcatttca tcccacatat tccttggatt tctctccaat 7980

gaatgacatt ctatcttgca aattcaacaa ttataataag atataccaaa gtagcggtat 8040

agtggcaatc aaaaagcttc tctggtgtgc ttctcgtatt tatttttatt ctaatgatcc 8100

attaaaggta tatatttatt tcttgttata taatcctttt gtttattaca tgggctggat 8160

acataaaggt attttgattt aattttttgc ttaaattcaa tcccccctcg ttcagtgtca 8220

actgtaatgg taggaaatta ccatactttt gaagaagcaa aaaaaatgaa agaaaaaaaa 8280

aatcgtattt ccaggttaga cgttccgcag aatctagaat gcggtatgcg gtacattgtt 8340

cttcgaacgt aaaagttgcg ctccctgaga tattgtacat ttttgctttt acaagtacaa 8400

gtacatcgta caactatgta ctactgttga tgcatccaca acagtttgtt ttgttttttt 8460

ttgttttttt tttttctaat gattcattac cgctatgtat acctacttgt acttgtagta 8520

agccgggtta ttggcgttca attaatcata gacttatgaa tctgcacggt gtgcgctgcg 8580

agttactttt agcttatgca tgctacttgg gtgtaatatt gggatctgtt cggaaatcaa 8640

cggatgctca atcgatttcg acagtaatta attaagtcat acacaagtca gctttcttcg 8700

agcctcatat aagtataagt agttcaacgt attagcactg tacccagcat ctccgtatcg 8760

agaaacacaa caacatgccc cattggacag atcatgcgga tacacaggtt gtgcagtatc 8820

atacatactc gatcagacag gtcgtctgac catcatacaa gctgaacaag cgctccatac 8880

ttgcacgctc tctatataca cagttaaatt acatatccat agtctaacct ctaacagtta 8940

atcttctggt aagcctccca gccagccttc tggtatcgct tggcctcctc aataggatct 9000

cggttctggc cgtacagacc tcggccgaca attatgatat ccgttccggt agacatgaca 9060

tcctcaacag ttcggtactg ctgtccgaga gcgtctccct tgtcgtcaag acccaccccg 9120

ggggtcagaa taagccagtc ctcagagtcg cccttaggtc ggttctgggc aatgaagcca 9180

accacaaact cggggtcgga tcgggcaagc tcaatggtct gcttggagta ctcgccagtg 9240

gccagagagc ccttgcaaga cagctcggcc agcatgagca gacctctggc cagcttctcg 9300

ttgggagagg ggactaggaa ctccttgtac tgggagttct cgtagtcaga gacgtcctcc 9360

ttcttctgtt cagagacagt ttcctcggca ccagctcgca ggccagcaat gattccggtt 9420

ccgggtacac cgtgggcgtt ggtgatatcg gaccactcgg cgattcggtg acaccggtac 9480

tggtgcttga cagtgttgcc aatatctgcg aactttctgt cctcgaacag gaagaaaccg 9540

tgcttaagag caagttcctt gagggggagc acagtgccgg cgtaggtgaa gtcgtcaatg 9600

atgtcgatat gggttttgat catgcacaca taaggtccga ccttatcggc aagctcaatg 9660

agctccttgg tggtggtaac atccagagaa gcacacaggt tggttttctt ggctgccacg 9720

agcttgagca ctcgagcggc aaaggcggac ttgtggacgt tagctcgagc ttcgtaggag 9780

ggcattttgg tggtgaagag gagactgaaa taaatttagt ctgcagaact ttttatcgga 9840

accttatctg gggcagtgaa gtatatgtta tggtaatagt tacgagttag ttgaacttat 9900

agatagactg gactatacgg ctatcggtcc aaattagaaa gaacgtcaat ggctctctgg 9960

gcgtcgcctt tgccgacaaa aatgtgatca tgatgaaagc cagcaatgac gttgcagctg 10020

atattgttgt cggccaaccg cgccgaaaac gcagctgtca gacccacagc ctccaacgaa 10080

gaatgtatcg tcaaagtgat ccaagcacac tcatagttgg agtcgtactc caaaggcggc 10140

aatgacgagt cagacagata ctcgtcgacg tttaaaccat catctaaggg cctcaaaact 10200

acctcggaac tgctgcgctg atctggacac cacagaggtt ccgagcactt taggttgcac 10260

caaatgtccc accaggtgca ggcagaaaac gctggaacag cgtgtacagt ttgtcttaac 10320

aaaaagtgag ggcgctgagg tcgagcaggg tggtgtgact tgttatagcc tttagagctg 10380

cgaaagcgcg tatggatttg gctcatcagg ccagattgag ggtctgtgga cacatgtcat 10440

gttagtgtac ttcaatcgcc ccctggatat agccccgaca ataggccgtg gcctcatttt 10500

tttgccttcc gcacatttcc attgctcggt acccacacct tgcttctcct gcacttgcca 10560

accttaatac tggtttacat tgaccaacat cttacaagcg gggggcttgt ctagggtata 10620

tataaacagt ggctctccca atcggttgcc agtctctttt ttcctttctt tccccacaga 10680

ttcgaaatct aaactacaca tcacac 10706

<210> 12
<211> 1048

<212>  DNA
<213>  Artificial sequence

<220>
<223>  high-throughput cloning cassette

<400>  12
gcgcacgtta attaaatttt ttttgatttt cttttttgac cccgtcttca attacacttc      60

ccaactggga acacccctct ttatcgaccc attttaggta atttacccta gcccattgtc     120

tccataagga atattaccct aacccacagt ccagggtgcc caggtccttc tttggccaaa     180

ttttaacttc ggtcctatgg cacagcggta gcgcgtgaga ttgcaaatct taaggtcccg     240

agttcgaatc tcggtgggac ctagttattt ttgatagata atttcgtgat gattagaaac     300

ttaacgcaaa ataatggccg gcatggtccc agcctcctcg ctggcgccgg ctgggcaaca     360

tgcttcggca tggcgaatgg gacgcaggtg atggcgggat cgttgtatat ttcttgacac     420

cttttcggca tcgccctaaa ttcggcgtcc tcatattgtg tgaggacgtt ttattacgtg     480

tttacgaagc aaaagctaaa accaggagct atttaatggc aacagttaac cagctggtac     540

gcaaaccacg tgctcgcaaa gttgcgaaaa gcaacgtgcc tgcgctggaa gcatgcccgc     600

aaaaacgtgg cgtatgtact cgtgtatata ctaccactcc taaaaaaccg aactccgcgc     660

tgcgtaaagt atgccgtgtt cgtctgacta acggtttcga agtgacttcc tacatcggtg     720

gtgaaggtca caacctgcag gagcactccg tgatcctgat ccgtggcggt cgtgttaaag     780

acctcccggg tgttcgttac cacaccgtac gtggtgcgct tgactgctcc ggcgttaaag     840

accgtaagca ggctcgttcc aagtatggcg tgaagcgtcc taaggcttag gttaataaca     900

ggcctgctgg taatcgcagg cctttttatt tttacacctg cgttttagag ctagaaatag     960

caagttaaaa taaggctagt ccgttatcaa cttgaaaaag tggcaccgag tcggtgcttt    1020

tttttttgtt ttttatcgat gcgcgcac                                       1048


<210>  13
<211>  300
<212>  DNA
<213>  Yarrowia lipolytica

<400>  13
attttttttg attttctttt ttgaccccgt cttcaattac acttcccaac tgggaacacc      60

cctctttatc gacccatttt aggtaattta ccctagccca ttgtctccat aaggaatatt     120

accctaaccc acagtccagg gtgcccaggt ccttctttgg ccaaatttta acttcggtcc     180

tatggcacag cggtagcgcg tgagattgca aatcttaagg tcccgagttc gaatctcggt     240

gggacctagt tatttttgat agataatttc gtgatgatta gaaacttaac gcaaaataat     300


<210>  14
<211>  68

<212>  DNA
<213>  Herpes Delta virus

<400>  14
ggccggcatg gtcccagcct cctcgctggc gccggctggg caacatgctt cggcatggcg      60

aatgggac                                                                68


<210>  15
<211>  544
<212>  DNA
<213>  Escherischia coli

<400>  15
atggcgggat cgttgtatat ttcttgacac cttttcggca tcgccctaaa ttcggcgtcc      60

tcatattgtg tgaggacgtt ttattacgtg tttacgaagc aaaagctaaa accaggagct     120

atttaatggc aacagttaac cagctggtac gcaaaccacg tgctcgcaaa gttgcgaaaa     180

gcaacgtgcc tgcgctggaa gcatgcccgc aaaaacgtgg cgtatgtact cgtgtatata     240

ctaccactcc taaaaaaccg aactccgcgc tgcgtaaagt atgccgtgtt cgtctgacta     300

acggtttcga agtgacttcc tacatcggtg gtgaaggtca caacctgcag gagcactccg     360

tgatcctgat ccgtggcggt cgtgttaaag acctcccggg tgttcgttac cacaccgtac     420

gtggtgcgct tgactgctcc ggcgttaaag accgtaagca ggctcgttcc aagtatggcg     480

tgaagcgtcc taaggcttag gttaataaca ggcctgctgg taatcgcagg cctttttatt     540

ttta                                                                   544


<210>  16
<211>  80
<212>  DNA
<213>  Artificial sequence

<220>
<223>  DNA encoding Cas9 CER domain

<400>  16
gttttagagc tagaaatagc aagttaaaat aaggctagtc cgttatcaac ttgaaaaagt      60

ggcaccgagt cggtgctttt                                                   80


<210>  17
<211>  14
<212>  DNA
<213>  Saccharomyces cerevisiae

<400>  17
tttttttgtt tttt                                                         14


<210>  18
<211>  11714
<212>  DNA
<213>  Artificial sequence

<220>
<223>  pRF291

<400>  18
   cgataaaaaa caaaaaaaaa agcaccgact cggtgccact ttttcaagtt gataacggac      60
   tagccttatt ttaacttgct atttctagct ctaaaacgca ggtgtaaaaa taaaaaggcc     120
   tgcgattacc agcaggcctg ttattaacct aagccttagg acgcttcacg ccatacttgg     180
   aacgagcctg cttacggtct ttaacgccgg agcagtcaag cgcaccacgt acggtgtggt     240
   aacgaacacc cgggaggtct ttaacacgac cgccacggat caggatcacg gagtgctcct     300
   gcaggttgtg accttcacca ccgatgtagg aagtcacttc gaaaccgtta gtcagacgaa     360
   cacggcatac tttacgcagc gcggagttcg gttttttagg agtggtagta tatacacgag     420
   tacatacgcc acgtttttgc gggcatgctt ccagcgcagg cacgttgctt ttcgcaactt     480
   tgcgagcacg tggtttgcgt accagctggt taactgttgc cattaaatag ctcctggttt     540
   tagcttttgc ttcgtaaaca cgtaataaaa cgtcctcaca caatatgagg acgccgaatt     600
   tagggcgatg ccgaaaaggt gtcaagaaat atacaacgat cccgccatca cctgcgtccc     660
   attcgccatg ccgaagcatg ttgcccagcc ggcgccagcg aggaggctgg gaccatgccg     720
   gccattattt tgcgttaagt ttctaatcat cacgaaatta tctatcaaaa ataactaggt     780
   cccaccgaga ttcgaactcg ggaccttaag atttgcaatc tcacgcgcta ccgctgtgcc     840
   ataggaccga agttaaaatt tggccaaaga aggacctggg caccctggac tgtgggttag     900
   ggtaatattc cttatggaga caatgggcta gggtaaatta cctaaaatgg gtcgataaag     960
   aggggtgttc ccagttggga agtgtaattg aagacggggt caaaaaagaa aatcaaaaaa    1020
   aatttaatta agtcatacac aagtcagctt tcttcgagcc tcatataagt ataagtagtt    1080
   caacgtatta gcactgtacc cagcatctcc gtatcgagaa acacaacaac atgccccatt    1140
   ggacagatca tgcggataca caggttgtgc agtatcatac atactcgatc agacaggtcg    1200
   tctgaccatc atacaagctg aacaagcgct ccatacttgc acgctctcta tatacacagt    1260
   taaattacat atccatagtc taacctctaa cagttaatct tctggtaagc ctcccagcca    1320
   gccttctggt atcgcttggc ctcctcaata ggatctcggt tctggccgta cagacctcgg    1380
   ccgacaatta tgatatccgt tccggtagac atgacatcct caacagttcg gtactgctgt    1440
   ccgagagcgt ctcccttgtc gtcaagaccc accccggggg tcagaataag ccagtcctca    1500
   gagtcgccct taggtcggtt ctgggcaatg aagccaacca caaactcggg gtcggatcgg    1560
   gcaagctcaa tggtctgctt ggagtactcg ccagtggcca gagagccctt gcaagacagc    1620
   tcggccagca tgagcagacc tctggccagc ttctcgttgg gagaggggac taggaactcc    1680
   ttgtactggg agttctcgta gtcagagacg tcctccttct tctgttcaga gacagtttcc    1740
   tcggcaccag ctcgcaggcc agcaatgatt ccggttccgg gtacaccgtg ggcgttggtg 1800
   atatcggacc actcggcgat tcggtgacac cggtactggt gcttgacagt gttgccaata 1860
   tctgcgaact ttctgtcctc gaacaggaag aaaccgtgct taagagcaag ttccttgagg 1920
   gggagcacag tgccggcgta ggtgaagtcg tcaatgatgt cgatatgggt tttgatcatg 1980
   cacacataag gtccgacctt atcggcaagc tcaatgagct ccttggtggt ggtaacatcc 2040
   agagaagcac acaggttggt tttcttggct gccacgagct tgagcactcg agcggcaaag 2100
   gcggacttgt ggacgttagc tcgagcttcg taggagggca ttttggtggt gaagaggaga 2160
   ctgaaataaa tttagtctgc agaacttttt atcggaacct tatctggggc agtgaagtat 2220
   atgttatggt aatagttacg agttagttga acttatagat agactggact atacggctat 2280
   cggtccaaat tagaaagaac gtcaatggct ctctgggcgt cgcctttgcc gacaaaaatg 2340
   tgatcatgat gaaagccagc aatgacgttg cagctgatat tgttgtcggc caaccgcgcc 2400
   gaaaacgcag ctgtcagacc cacagcctcc aacgaagaat gtatcgtcaa agtgatccaa 2460
   gcacactcat agttggagtc gtactccaaa ggcggcaatg acgagtcaga cagatactcg 2520
   tcgacgttta aaccatcatc taagggcctc aaaactacct cggaactgct gcgctgatct 2580
   ggacaccaca gaggttccga gcactttagg ttgcaccaaa tgtcccacca ggtgcaggca 2640
   gaaaacgctg gaacagcgtg tacagtttgt cttaacaaaa agtgagggcg ctgaggtcga 2700
   gcagggtggt gtgacttgtt atagccttta gagctgcgaa agcgcgtatg gatttggctc 2760
   atcaggccag attgagggtc tgtggacaca tgtcatgtta gtgtacttca atcgccccct 2820
   ggatatagcc ccgacaatag gccgtggcct catttttttg ccttccgcac atttccattg 2880
   ctcggtaccc acaccttgct tctcctgcac ttgccaacct taatactggt ttacattgac 2940
   caacatctta caagcggggg gcttgtctag ggtatatata aacagtggct ctcccaatcg 3000
   gttgccagtc tcttttttcc tttctttccc cacagattcg aaatctaaac tacacatcac 3060
   accatggaca agaaatactc catcggcctg gacattggaa ccaactctgt cggctgggct 3120
   gtcatcaccg acgagtacaa ggtgccctcc aagaaattca aggtcctcgg aaacaccgat 3180
   cgacactcca tcaagaaaaa cctcattggt gccctgttgt tcgattctgg cgagactgcc 3240
   gaagctacca gactcaagcg aactgctcgg cgacgttaca cccgacggaa gaaccgaatc 3300
   tgctacctgc aggagatctt ttccaacgag atggccaagg tggacgattc gttctttcat 3360
   cgactggagg aatccttcct cgtcgaggaa gacaagaaac acgagcgtca tcccatcttt 3420
   ggcaacattg tggacgaggt tgcttaccac gagaagtatc ctaccatcta ccatctccga 3480
   aagaaactcg tcgattccac cgacaaggcg gatctcagac ttatctacct cgctctggca 3540
   cacatgatca agtttcgagg tcatttcctc atcgagggcg atctcaatcc cgacaacagc 3600
   gatgtggaca agctgttcat tcagctcgtt cagacctaca accagctgtt cgaggaaaac 3660
   cccatcaatg cctccggagt cgatgcaaag gccatcttgt ctgctcgact ctcgaagagc 3720
   agacgactgg agaacctcat tgcccaactt cctggcgaga aaaagaacgg actgtttggc 3780
   aacctcattg ccctttctct tggtctcaca cccaacttca agtccaactt cgatctggcg 3840
   gaggacgcca agctccagct gtccaaggac acctacgacg atgacctcga caacctgctt 3900
   gcacagattg gcgatcagta cgccgacctg tttctcgctg ccaagaacct ttcggatgct 3960
   attctcttgt ctgacattct gcgagtcaac accgagatca caaaggctcc cctttctgcc 4020
   tccatgatca agcgatacga cgagcaccat caggatctca cactgctcaa ggctcttgtc 4080
   cgacagcaac tgcccgagaa gtacaaggag atctttttcg atcagtcgaa gaacggctac 4140
   gctggataca tcgacggcgg agcctctcag gaagagttct acaagttcat caagccaatt 4200
   ctcgagaaga tggacggaac cgaggaactg cttgtcaagc tcaatcgaga ggatctgctt 4260
   cggaagcaac gaaccttcga caacggcagc attcctcatc agatccacct cggtgagctg 4320
   cacgccattc ttcgacgtca ggaagacttc tacccctttc tcaaggacaa ccgagagaag 4380
   atcgagaaga ttcttacctt tcgaatcccc tactatgttg gtcctcttgc cagaggaaac 4440
   tctcgatttg cttggatgac tcgaaagtcc gaggaaacca tcactccctg gaacttcgag 4500
   gaagtcgtgg acaagggtgc ctctgcacag tccttcatcg agcgaatgac caacttcgac 4560
   aagaatctgc ccaacgagaa ggttcttccc aagcattcgc tgctctacga gtactttaca 4620
   gtctacaacg aactcaccaa agtcaagtac gttaccgagg gaatgcgaaa gcctgccttc 4680
   ttgtctggcg aacagaagaa agccattgtc gatctcctgt tcaagaccaa ccgaaaggtc 4740
   actgttaagc agctcaagga ggactacttc aagaaaatcg agtgtttcga cagcgtcgag 4800
   atttccggag ttgaggaccg attcaacgcc tctttgggca cctatcacga tctgctcaag 4860
   attatcaagg acaaggattt tctcgacaac gaggaaaacg aggacattct ggaggacatc 4920
   gtgctcactc ttaccctgtt cgaagatcgg gagatgatcg aggaacgact caagacatac 4980
   gctcacctgt tcgacgacaa ggtcatgaaa caactcaagc gacgtagata caccggctgg 5040
   ggaagacttt cgcgaaagct catcaacggc atcagagaca agcagtccgg aaagaccatt 5100
   ctggactttc tcaagtccga tggctttgcc aaccgaaact tcatgcagct cattcacgac 5160
   gattctctta ccttcaagga ggacatccag aaggcacaag tgtccggtca gggcgacagc 5220
   ttgcacgaac atattgccaa cctggctggt tcgccagcca tcaagaaagg cattctccag 5280
   actgtcaagg ttgtcgacga gctggtgaag gtcatgggac gtcacaagcc cgagaacatt 5340
   gtgatcgaga tggccagaga gaaccagaca actcaaaagg gtcagaaaaa ctcgcgagag 5400
   cggatgaagc gaatcgagga aggcatcaag gagctgggat cccagattct caaggagcat 5460
   cccgtcgaga acactcaact gcagaacgag aagctgtatc tctactatct gcagaatggt 5520
   cgagacatgt acgtggatca ggaactggac atcaatcgtc tcagcgacta cgatgtggac 5580
   cacattgtcc ctcaatcctt tctcaaggac gattctatcg acaacaaggt ccttacacga 5640
   tccgacaaga acagaggcaa gtcggacaac gttcccagcg aagaggtggt caaaaagatg 5700
   aagaactact ggcgacagct gctcaacgcc aagctcatta cccagcgaaa gttcgacaat 5760
   cttaccaagg ccgagcgagg cggtctgtcc gagctcgaca aggctggctt catcaagcgt 5820
   caactcgtcg agaccagaca gatcacaaag cacgtcgcac agattctcga ttctcggatg 5880
   aacaccaagt acgacgagaa cgacaagctc atccgagagg tcaaggtgat tactctcaag 5940
   tccaaactgg tctccgattt ccgaaaggac tttcagttct acaaggtgcg agagatcaac 6000
   aattaccacc atgcccacga tgcttacctc aacgccgtcg ttggcactgc gctcatcaag 6060
   aaatacccca agctcgaaag cgagttcgtt tacggcgatt acaaggtcta cgacgttcga 6120
   aagatgattg ccaagtccga acaggagatt ggcaaggcta ctgccaagta cttcttttac 6180
   tccaacatca tgaacttttt caagaccgag atcaccttgg ccaacggaga gattcgaaag 6240
   agaccactta tcgagaccaa cggcgaaact ggagagatcg tgtgggacaa gggtcgagac 6300
   tttgcaaccg tgcgaaaggt tctgtcgatg cctcaggtca acatcgtcaa gaaaaccgag 6360
   gttcagactg gcggattctc caaggagtcg attctgccca agcgaaactc cgacaagctc 6420
   atcgctcgaa agaaagactg ggatcccaag aaatacggtg gcttcgattc tcctaccgtc 6480
   gcctattccg tgcttgtcgt tgcgaaggtc gagaagggca agtccaaaaa gctcaagtcc 6540
   gtcaaggagc tgctcggaat taccatcatg gagcgatcga gcttcgagaa gaatcccatc 6600
   gacttcttgg aagccaaggg ttacaaggag gtcaagaaag acctcattat caagctgccc 6660
   aagtactctc tgttcgaact ggagaacggt cgaaagcgta tgctcgcctc cgctggcgag 6720
   ctgcagaagg gaaacgagct tgccttgcct tcgaagtacg tcaactttct ctatctggct 6780
   tctcactacg agaagctcaa gggttctccc gaggacaacg aacagaagca actcttcgtt 6840
   gagcagcaca aacattacct cgacgagatt atcgagcaga tttccgagtt ttcgaagcga 6900
   gtcatcctgg ctgatgccaa cttggacaag gtgctctctg cctacaacaa gcatcgggac 6960
   aaacccattc gagaacaggc ggagaacatc attcacctgt ttactcttac caacctgggt 7020
   gctcctgcag ctttcaagta cttcgatacc actatcgacc gaaagcggta cacatccacc 7080
   aaggaggttc tcgatgccac cctgattcac cagtccatca ctggcctgta cgagacccga 7140
   atcgacctgt ctcagcttgg tggcgactcc agagccgatc ccaagaaaaa gcgaaaggtc 7200
   taagcggccg caagtgtgga tggggaagtg agtgcccggt tctgtgtgca caattggcaa 7260
   tccaagatgg atggattcaa cacagggata tagcgagcta cgtggtggtg cgaggatata 7320
   gcaacggata tttatgtttg acacttgaga atgtacgata caagcactgt ccaagtacaa 7380
   tactaaacat actgtacata ctcatactcg tacccgggca acggtttcac ttgagtgcag 7440
   tggctagtgc tcttactcgt acagtgtgca atactgcgta tcatagtctt tgatgtatat 7500
   cgtattcatt catgttagtt gcgtacgagc cggaagcata aagtgtaaag cctggggtgc 7560
   ctaatgagtg agctaactca cattaattgc gttgcgctca ctgcccgctt tccagtcggg 7620
   aaacctgtcg tgccagctgc attaatgaat cggccaacgc gcggggagag gcggtttgcg 7680
   tattgggcgc tcttccgctt cctcgctcac tgactcgctg cgctcggtcg ttcggctgcg 7740
   gcgagcggta tcagctcact caaaggcggt aatacggtta tccacagaat caggggataa 7800
   cgcaggaaag aacatgtgag caaaaggcca gcaaaaggcc aggaaccgta aaaaggccgc 7860
   gttgctggcg tttttccata ggctccgccc ccctgacgag catcacaaaa atcgacgctc 7920
   aagtcagagg tggcgaaacc cgacaggact ataaagatac caggcgtttc cccctggaag 7980
   ctccctcgtg cgctctcctg ttccgaccct gccgcttacc ggatacctgt ccgcctttct 8040
   cccttcggga agcgtggcgc tttctcatag ctcacgctgt aggtatctca gttcggtgta 8100
   ggtcgttcgc tccaagctgg gctgtgtgca cgaacccccc gttcagcccg accgctgcgc 8160
   cttatccggt aactatcgtc ttgagtccaa cccggtaaga cacgacttat cgccactggc 8220
   agcagccact ggtaacagga ttagcagagc gaggtatgta ggcggtgcta cagagttctt 8280
   gaagtggtgg cctaactacg gctacactag aaggacagta tttggtatct gcgctctgct 8340
   gaagccagtt accttcggaa aaagagttgg tagctcttga tccggcaaac aaaccaccgc 8400
   tggtagcggt ggtttttttg tttgcaagca gcagattacg cgcagaaaaa aaggatctca 8460
   agaagatcct ttgatctttt ctacggggtc tgacgctcag tggaacgaaa actcacgtta 8520
   agggattttg gtcatgagat tatcaaaaag gatcttcacc tagatccttt taaattaaaa 8580
   atgaagtttt aaatcaatct aaagtatata tgagtaaact tggtctgaca gttaccaatg 8640
   cttaatcagt gaggcaccta tctcagcgat ctgtctattt cgttcatcca tagttgcctg 8700
   actccccgtc gtgtagataa ctacgatacg ggagggctta ccatctggcc ccagtgctgc 8760
   aatgataccg cgagacccac gctcaccggc tccagattta tcagcaataa accagccagc 8820
   cggaagggcc gagcgcagaa gtggtcctgc aactttatcc gcctccatcc agtctattaa 8880
   ttgttgccgg gaagctagag taagtagttc gccagttaat agtttgcgca acgttgttgc 8940
   cattgctaca ggcatcgtgg tgtcacgctc gtcgtttggt atggcttcat tcagctccgg 9000
   ttcccaacga tcaaggcgag ttacatgatc ccccatgttg tgcaaaaaag cggttagctc 9060
   cttcggtcct ccgatcgttg tcagaagtaa gttggccgca gtgttatcac tcatggttat 9120
   ggcagcactg cataattctc ttactgtcat gccatccgta agatgctttt ctgtgactgg 9180
   tgagtactca accaagtcat tctgagaata gtgtatgcgg cgaccgagtt gctcttgccc 9240
   ggcgtcaata cgggataata ccgcgccaca tagcagaact ttaaaagtgc tcatcattgg 9300
   aaaacgttct tcggggcgaa aactctcaag gatcttaccg ctgttgagat ccagttcgat 9360
   gtaacccact cgtgcaccca actgatcttc agcatctttt actttcacca gcgtttctgg 9420
   gtgagcaaaa acaggaaggc aaaatgccgc aaaaaaggga ataagggcga cacggaaatg 9480
   ttgaatactc atactcttcc tttttcaata ttattgaagc atttatcagg gttattgtct 9540
   catgagcgga tacatatttg aatgtattta gaaaaataaa caaatagggg ttccgcgcac 9600
   atttccccga aaagtgccac ctgacgcgcc ctgtagcggc gcattaagcg cggcgggtgt 9660
   ggtggttacg cgcagcgtga ccgctacact tgccagcgcc ctagcgcccg ctcctttcgc 9720
   tttcttccct tcctttctcg ccacgttcgc cggctttccc cgtcaagctc taaatcgggg 9780
   gctcccttta gggttccgat ttagtgcttt acggcacctc gaccccaaaa aacttgatta 9840
   gggtgatggt tcacgtagtg ggccatcgcc ctgatagacg gtttttcgcc ctttgacgtt 9900
   ggagtccacg ttctttaata gtggactctt gttccaaact ggaacaacac tcaaccctat 9960
   ctcggtctat tcttttgatt tataagggat tttgccgatt tcggcctatt ggttaaaaaa 10020
   tgagctgatt taacaaaaat ttaacgcgaa ttttaacaaa atattaacgc ttacaatttc 10080
   cattcgccat tcaggctgcg caactgttgg gaagggcgat cggtgcgggc ctcttcgcta 10140
   ttacgccagc tggcgaaagg gggatgtgct gcaaggcgat taagttgggt aacgccaggg 10200
   ttttcccagt cacgacgttg taaaacgacg gccagtgaat tgtaatacga ctcactatag 10260
   ggcgaattgg gtaccgggcc ccccctcgag gtcgatggtg tcgataagct tgatatcgaa 10320
   ttcatgtcac acaaaccgat cttcgcctca aggaaaccta attctacatc cgagagactg 10380
   ccgagatcca gtctacactg attaattttc gggccaataa tttaaaaaaa tcgtgttata 10440
   taatattata tgtattatat atatacatca tgatgatact gacagtcatg tcccattgct 10500
   aaatagacag actccatctg ccgcctccaa ctgatgttct caatatttaa ggggtcatct 10560
   cgcattgttt aataataaac agactccatc taccgcctcc aaatgatgtt ctcaaaatat 10620
   attgtatgaa cttattttta ttacttagta ttattagaca acttacttgc tttatgaaaa 10680
   acacttccta tttaggaaac aatttataat ggcagttcgt tcatttaaca atttatgtag 10740
   aataaatgtt ataaatgcgt atgggaaatc ttaaatatgg atagcataaa tgatatctgc 10800
   attgcctaat tcgaaatcaa cagcaacgaa aaaaatccct tgtacaacat aaatagtcat 10860
   cgagaaatat caactatcaa agaacagcta ttcacacgtt actattgaga ttattattgg 10920
   acgagaatca cacactcaac tgtctttctc tcttctagaa atacaggtac aagtatgtac 10980
   tattctcatt gttcatactt ctagtcattt catcccacat attccttgga tttctctcca 11040
   atgaatgaca ttctatcttg caaattcaac aattataata agatatacca aagtagcggt 11100
   atagtggcaa tcaaaaagct tctctggtgt gcttctcgta tttattttta ttctaatgat 11160
   ccattaaagg tatatattta tttcttgtta tataatcctt ttgtttatta catgggctgg 11220
   atacataaag gtattttgat ttaatttttt gcttaaattc aatcccccct cgttcagtgt     11280
   caactgtaat ggtaggaaat taccatactt ttgaagaagc aaaaaaaatg aaagaaaaaa     11340
   aaaatcgtat ttccaggtta gacgttccgc agaatctaga atgcggtatg cggtacattg     11400
   ttcttcgaac gtaaaagttg cgctccctga gatattgtac atttttgctt ttacaagtac     11460
   aagtacatcg tacaactatg tactactgtt gatgcatcca caacagtttg ttttgttttt     11520
   ttttgttttt tttttttcta atgattcatt accgctatgt atacctactt gtacttgtag     11580
   taagccgggt tattggcgtt caattaatca tagacttatg aatctgcacg gtgtgcgctg     11640
   cgagttactt ttagcttatg catgctactt gggtgtaata ttgggatctg ttcggaaatc     11700
   aacggatgct caat                                                       11714

<210>  19
<211>  32
<212>  DNA
<213>  Artificial sequence

<220>
<223>   Can1-1F

<400>  19
aatgggactc aaacgattac ccaccctcgt tt                                   32

<210>  20
<211>  32
<212>  DNA
<213>  Artificial sequence

<220>
<223>  Can1-1R

<400>  20
tctaaaacga gggtgggtaa tcgtttgagt cc                                   32

<210>  21
<211>  20
<212>  DNA
<213>  Artificial sequence

<220>
<223>  DNA encoding Can1-1 VT domain

<400>  21
tcaaacgatt acccaccctc                                                 20

<210>  22
<211>  23
<212>  DNA
<213>  Yarrowia lipolytica

<400>  22
tcaaacgatt acccaccctc cgg                                             23

<210>  23
<211>  1719
<212>  DNA
<213>  Yarrowia lipolytica

<400>  23
   atggaaaaga cattttcaaa cgattaccca ccctccggga ctgaggccca catccacatc        60
   aaccacacgg cccactcgga tgactcagag gaggtgccct cgcacaagga aaattacaac       120
   accagtggcc acgacctgga ggagtccgac ccggataacc atgtcggtga gaccctcgag       180
   gtcaagcgag gtctcaagat gcgacacatc tccatgatct cgcttggagg aaccattggt       240
   accggtctct tcattggtac cggaggagct ctccagcagg ccggtccctg tggcgccctc       300
   gtcgcctacg tgttcatggc caccattgtc tactctgttg ccgagtctct tggagaactg       360
   gctacgtaca ttcccatcac cggctccttt gccgtcttta ctacccgata tctgtcacag       420
   tcgtttggtg cctccatggg ctggctatac tggttctcgt gggcgatcac cttcgccatc       480
   gagctcaaca ccattggtcc cgtgattgag tactggactg acgccgttcc tactgctgcc       540
   tggattgcca tcttcttcgt catcctcact accatcaact tcttccccgt gggcttctat       600
   ggcgaagtcg agttctgggt ggcctccgtg aaggtcattg ccatcattgg atggctcatc       660
   tacgcgctct gcatgacgtg tggagcaggt gtaacaggtc ctgtgggatt cagatactgg       720
   aaccaccccg gacccatggg agacggaatc tggaccgacg gcgtgcccat tgtgcgaaac       780
   gcgcccggtc gacgattcat gggatggctc aattcgctcg ttaacgccgc cttcacctac       840
   cagggctgtg agctggtcgg agtcactgcc ggtgaggccc agaaccccag aaagtccgtc       900
   cctcgagcca tcaaccgagt ctttgctcga atttgcatct tctacattgg ctctatcttc       960
   ttcatgggca tgctcgtgcc ctttaacgac cccaagctga ccgatgactc ctccgtcatc      1020
   gcctcctctc cttttgttat tgccattatc aactctggca ccaaggtgct ccctcacatt      1080
   ttcaacgccg tcattctcat caccctgatt tcggcaggaa actccaacgt ctacattggc      1140
   tcgcgagtgg tctacgccct ggctgactcc ggaaccgcac caaagttctt caagcgaacc      1200
   accaagaagg gagtgccgta cgtggcagtc tgcttcacct cggcgtttgg tctgctggcc      1260
   ttcatgtctg tgtccgagtc gtcgtccact gtcttcgact ggttcatcaa catctccgct      1320
   gtggccggcc tcatctgttg ggccttcatc tctgcctccc acatccgatt catgcaagtg      1380
   cttaagcaca gagggatctc cagagatacg ctgcccttca aggcacgatg gcagccattc      1440
   tactcatggt acgcgctcgt ctccatcatc ttcatcactc tcatccaggg cttcacgtcc      1500
   ttctggcact ttaccgccgc caagttcatg actgcataca tctccgtcat tgtctgggtc      1560
   ggtttgtaca ttatcttcca gtgtctgttc cgatgcaagt tccttatccc tattgaggat      1620
   gtggacattg acaccggccg acgagagatt gacgacgatg tgtgggagga gaagatcccc      1680
   acaaagtggt acgagaagtt ttggaatatt attgcataa 1719

<210> 24
<211> 12167
<212> DNA
<213> Artificial sequence

<220>
<223> Artificial Sequence

<400> 24
cgataaaaaa caaaaaaaaa agcaccgact cggtgccact ttttcaagtt gataacggac 60

tagccttatt ttaacttgct atttctagct ctaaaacgca ggtgtaaaaa taaaaaggcc 120

tgcgattacc agcaggcctg ttattaacct aagccttagg acgcttcacg ccatacttgg 180

aacgagcctg cttacggtct ttaacgccgg agcagtcaag cgcaccacgt acggtgtggt 240

aacgaacacc cgggaggtct ttaacacgac cgccacggat caggatcacg gagtgctcct 300

gcaggttgtg accttcacca ccgatgtagg aagtcacttc gaaaccgtta gtcagacgaa 360

cacggcatac tttacgcagc gcggagttcg gttttttagg agtggtagta tatacacgag 420

tacatacgcc acgtttttgc gggcatgctt ccagcgcagg cacgttgctt ttcgcaactt 480

tgcgagcacg tggtttgcgt accagctggt taactgttgc cattaaatag ctcctggttt 540

tagcttttgc ttcgtaaaca cgtaataaaa cgtcctcaca caatatgagg acgccgaatt 600

tagggcgatg ccgaaaaggt gtcaagaaat atacaacgat cccgccatca cctgcgtccc 660

attcgccatg ccgaagcatg ttgcccagcc ggcgccagcg aggaggctgg gaccatgccg 720

gccattattt tgcgttaagt ttctaatcat cacgaaatta tctatcaaaa ataactaggt 780

cccaccgaga ttcgaactcg ggaccttaag atttgcaatc tcacgcgcta ccgctgtgcc 840

ataggaccga agttaaaatt tggccaaaga aggacctggg caccctggac tgtgggttag 900

ggtaatattc cttatggaga caatgggcta gggtaaatta cctaaaatgg gtcgataaag 960

aggggtgttc ccagttggga agtgtaattg aagacggggt caaaaaagaa aatcaaaaaa 1020

aatttaatta agactatgat aacttcgtat aatgtatgct atacgaacgg tagcacactg 1080

tacgagtaag agcactagcc actgcactca agtgaaaccg ttgcccgggt acgagtatga 1140

gtatgtacag tatgtttagt attgtacttg gacagtgctt gtatcgtaca ttctcaagtg 1200

tcaaacataa atatccgttg ctatatcctc gcaccaccac gtagctcgct atatccctgt 1260

gttgaatcca tccatcttgg attgccaatt gtgcacacag aaccgggcac tcacttcccc 1320

atccacactt gcggccgcta ttcctttgcc ctcggacgag tgctggggcg tcggtttcca 1380

ctatcggcga gtacttctac acagccatcg gtccagacgg ccgcgcttct gcgggcgatt 1440

tgtgtacgcc cgacagtccc ggctccggat cggacgattg cgtcgcatcg accctgcgcc 1500

caagctgcat catcgaaatt gccgtcaacc aagctctgat agagttggtc aagaccaatg 1560

cggagcatat acgcccggag ccgcggcgat cctgcaagct ccggatgcct ccgctcgaag 1620

tagcgcgtct gctgctccat acaagccaac cacggcctcc agaagaagat gttggcgacc 1680

tcgtattggg aatccccgaa catcgcctcg ctccagtcaa tgaccgctgt tatgcggcca 1740

ttgtccgtca ggacattgtt ggagccgaaa tccgcgtgca cgaggtgccg gacttcgggg 1800

cagtcctcgg cccaaagcat cagctcatcg agagcctgcg cgacggacgc actgacggtg 1860

tcgtccatca cagtttgcca gtgatacaca tggggatcag caatcgcgca tatgaaatca 1920

cgccatgtag tgtattgacc gattccttgc ggtccgaatg ggccgaaccc gctcgtctgg 1980

ctaagatcgg ccgcagcgat cgcatccata gcctccgcga ccggctgcag aacagcgggc 2040

agttcggttt caggcaggtc ttgcaacgtg acaccctgtg cacggcggga gatgcaatag 2100

gtcaggctct cgctgaactc cccaatgtca agcacttccg gaatcgggag cgcggccgat 2160

gcaaagtgcc gataaacata acgatctttg tagaaaccat cggcgcagct atttacccgc 2220

aggacatatc cacgccctcc tacatcgaag ctgaaagcac gagattcttc gccctccgag 2280

agctgcatca ggtcggagac gctgtcgaac ttttcgatca gaaacttctc gacagacgtc 2340

gcggtgagtt caggcttttt ggccatggtt gatgtgtgtt taattcaaga atgaatatag 2400

agaagagaag aagaaaaaag attcaattga gccggcgatg cagaccctta tataaatgtt 2460

gccttggaca gacggagcaa gcccgcccaa acctacgttc ggtataatat gttaagcttt 2520

ttaacacaaa ggtttggctt ggggtaacct gatgtggtgc aaaagaccgg gcgttggcga 2580

gccattgcgc gggcgaatgg ggccgtgact cgtctcaaat tcgagggcgt gcctcaattc 2640

gtgcccccgt ggctttttcc cgccgtttcc gccccgtttg caccactgca gccgcttctt 2700

tggttcggac accttgctgc gagctaggtg ccttgtgcta cttaaaaagt ggcctcccaa 2760

caccaacatg acatgagtgc gtgggccaag acacgttggc ggggtcgcag tcggctcaat 2820

ggcccggaaa aaacgctgct ggagctggtt cggacgcagt ccgccgcggc gtatggatat 2880

ccgcaaggtt ccatagcgcc attgccctcc gtcggcgtct atcccgcaac ctaccgttcg 2940

tataatgtat gctatacgaa gttatgagcg ggcttaaggt ttaaaccatc atctaagggc 3000

ctcaaaacta cctcggaact gctgcgctga tctggacacc acagaggttc cgagcacttt 3060

aggttgcacc aaatgtccca ccaggtgcag gcagaaaacg ctggaacagc gtgtacagtt 3120

tgtcttaaca aaaagtgagg gcgctgaggt cgagcagggt ggtgtgactt gttatagcct 3180

ttagagctgc gaaagcgcgt atggatttgg ctcatcaggc cagattgagg gtctgtggac 3240

acatgtcatg ttagtgtact tcaatcgccc cctggatata gccccgacaa taggccgtgg 3300

cctcattttt ttgccttccg cacatttcca ttgctcggta cccacacctt gcttctcctg 3360

cacttgccaa ccttaatact ggtttacatt gaccaacatc ttacaagcgg ggggcttgtc 3420

tagggtatat ataaacagtg gctctcccaa tcggttgcca gtctcttttt tcctttcttt 3480

ccccacagat tcgaaatcta aactacacat cacaccatgg acaagaaata ctccatcggc 3540

ctggacattg gaaccaactc tgtcggctgg gctgtcatca ccgacgagta caaggtgccc 3600

tccaagaaat tcaaggtcct cggaaacacc gatcgacact ccatcaagaa aaacctcatt 3660

ggtgccctgt tgttcgattc tggcgagact gccgaagcta ccagactcaa gcgaactgct 3720

cggcgacgtt acacccgacg gaagaaccga atctgctacc tgcaggagat cttttccaac 3780

gagatggcca aggtggacga ttcgttcttt catcgactgg aggaatcctt cctcgtcgag 3840

gaagacaaga aacacgagcg tcatcccatc tttggcaaca ttgtggacga ggttgcttac 3900

cacgagaagt atcctaccat ctaccatctc cgaaagaaac tcgtcgattc caccgacaag 3960

gcggatctca gacttatcta cctcgctctg gcacacatga tcaagtttcg aggtcatttc 4020

ctcatcgagg gcgatctcaa tcccgacaac agcgatgtgg acaagctgtt cattcagctc 4080

gttcagacct acaaccagct gttcgaggaa aaccccatca atgcctccgg agtcgatgca 4140

aaggccatct tgtctgctcg actctcgaag agcagacgac tggagaacct cattgcccaa 4200

cttcctggcg agaaaaagaa cggactgttt ggcaacctca ttgccctttc tcttggtctc 4260

acacccaact tcaagtccaa cttcgatctg gcggaggacg ccaagctcca gctgtccaag 4320

gacacctacg acgatgacct cgacaacctg cttgcacaga ttggcgatca gtacgccgac 4380

ctgtttctcg ctgccaagaa cctttcggat gctattctct tgtctgacat tctgcgagtc 4440

aacaccgaga tcacaaaggc tcccctttct gcctccatga tcaagcgata cgacgagcac 4500

catcaggatc tcacactgct caaggctctt gtccgacagc aactgcccga gaagtacaag 4560

gagatctttt tcgatcagtc gaagaacggc tacgctggat acatcgacgg cggagcctct 4620

caggaagagt tctacaagtt catcaagcca attctcgaga agatggacgg aaccgaggaa 4680

ctgcttgtca agctcaatcg agaggatctg cttcggaagc aacgaacctt cgacaacggc 4740

agcattcctc atcagatcca cctcggtgag ctgcacgcca ttcttcgacg tcaggaagac 4800

ttctacccct ttctcaagga caaccgagag aagatcgaga agattcttac ctttcgaatc 4860

ccctactatg ttggtcctct tgccagagga aactctcgat ttgcttggat gactcgaaag 4920

tccgaggaaa ccatcactcc ctggaacttc gaggaagtcg tggacaaggg tgcctctgca 4980

cagtccttca tcgagcgaat gaccaacttc gacaagaatc tgcccaacga gaaggttctt 5040

cccaagcatt cgctgctcta cgagtacttt acagtctaca acgaactcac caaagtcaag 5100

tacgttaccg agggaatgcg aaagcctgcc ttcttgtctg gcgaacagaa gaaagccatt 5160

gtcgatctcc tgttcaagac caaccgaaag gtcactgtta agcagctcaa ggaggactac 5220

ttcaagaaaa tcgagtgttt cgacagcgtc gagatttccg gagttgagga ccgattcaac 5280

gcctctttgg gcacctatca cgatctgctc aagattatca aggacaagga ttttctcgac 5340

aacgaggaaa acgaggacat tctggaggac atcgtgctca ctcttaccct gttcgaagat 5400

cgggagatga tcgaggaacg actcaagaca tacgctcacc tgttcgacga caaggtcatg 5460

aaacaactca agcgacgtag atacaccggc tggggaagac tttcgcgaaa gctcatcaac 5520

ggcatcagag acaagcagtc cggaaagacc attctggact ttctcaagtc cgatggcttt 5580

gccaaccgaa acttcatgca gctcattcac gacgattctc ttaccttcaa ggaggacatc 5640

cagaaggcac aagtgtccgg tcagggcgac agcttgcacg aacatattgc caacctggct 5700

ggttcgccag ccatcaagaa aggcattctc cagactgtca aggttgtcga cgagctggtg 5760

aaggtcatgg gacgtcacaa gcccgagaac attgtgatcg agatggccag agagaaccag 5820

acaactcaaa agggtcagaa aaactcgcga gagcggatga agcgaatcga ggaaggcatc 5880

aaggagctgg gatcccagat tctcaaggag catcccgtcg agaacactca actgcagaac 5940

gagaagctgt atctctacta tctgcagaat ggtcgagaca tgtacgtgga tcaggaactg 6000

gacatcaatc gtctcagcga ctacgatgtg gaccacattg tccctcaatc ctttctcaag 6060

gacgattcta tcgacaacaa ggtccttaca cgatccgaca agaacagagg caagtcggac 6120

aacgttccca gcgaagaggt ggtcaaaaag atgaagaact actggcgaca gctgctcaac 6180

gccaagctca ttacccagcg aaagttcgac aatcttacca aggccgagcg aggcggtctg 6240

tccgagctcg acaaggctgg cttcatcaag cgtcaactcg tcgagaccag acagatcaca 6300

aagcacgtcg cacagattct cgattctcgg atgaacacca agtacgacga gaacgacaag 6360

ctcatccgag aggtcaaggt gattactctc aagtccaaac tggtctccga tttccgaaag 6420

gactttcagt tctacaaggt gcgagagatc aacaattacc accatgccca cgatgcttac 6480

ctcaacgccg tcgttggcac tgcgctcatc aagaaatacc ccaagctcga aagcgagttc 6540

gtttacggcg attacaaggt ctacgacgtt cgaaagatga ttgccaagtc cgaacaggag 6600

attggcaagg ctactgccaa gtacttcttt tactccaaca tcatgaactt tttcaagacc 6660

gagatcacct tggccaacgg agagattcga aagagaccac ttatcgagac caacggcgaa 6720

actggagaga tcgtgtggga caagggtcga gactttgcaa ccgtgcgaaa ggttctgtcg 6780

atgcctcagg tcaacatcgt caagaaaacc gaggttcaga ctggcggatt ctccaaggag 6840

tcgattctgc ccaagcgaaa ctccgacaag ctcatcgctc gaaagaaaga ctgggatccc 6900

aagaaatacg gtggcttcga ttctcctacc gtcgcctatt ccgtgcttgt cgttgcgaag 6960

gtcgagaagg gcaagtccaa aaagctcaag tccgtcaagg agctgctcgg aattaccatc 7020

atggagcgat cgagcttcga gaagaatccc atcgacttct tggaagccaa gggttacaag 7080

gaggtcaaga aagacctcat tatcaagctg cccaagtact ctctgttcga actggagaac 7140

ggtcgaaagc gtatgctcgc ctccgctggc gagctgcaga agggaaacga gcttgccttg 7200

ccttcgaagt acgtcaactt tctctatctg gcttctcact acgagaagct caagggttct 7260

cccgaggaca acgaacagaa gcaactcttc gttgagcagc acaaacatta cctcgacgag 7320

attatcgagc agatttccga gttttcgaag cgagtcatcc tggctgatgc caacttggac 7380

aaggtgctct ctgcctacaa caagcatcgg gacaaaccca ttcgagaaca ggcggagaac 7440

atcattcacc tgtttactct taccaacctg ggtgctcctg cagctttcaa gtacttcgat 7500

accactatcg accgaaagcg gtacacatcc accaaggagg ttctcgatgc caccctgatt 7560

caccagtcca tcactggcct gtacgagacc cgaatcgacc tgtctcagct tggtggcgac 7620

tccagagccg atcccaagaa aaagcgaaag gtctaagcgg ccgcaagtgt ggatggggaa 7680

gtgagtgccc ggttctgtgt gcacaattgg caatccaaga tggatggatt caacacaggg 7740

atatagcgag ctacgtggtg gtgcgaggat atagcaacgg atatttatgt ttgacacttg 7800

agaatgtacg atacaagcac tgtccaagta caatactaaa catactgtac atactcatac 7860

tcgtacccgg gcaacggttt cacttgagtg cagtggctag tgctcttact cgtacagtgt 7920

gcaatactgc gtatcatagt ctttgatgta tatcgtattc attcatgtta gttgcgtacg 7980

agccggaagc ataaagtgta aagcctgggg tgcctaatga gtgagctaac tcacattaat 8040

tgcgttgcgc tcactgcccg ctttccagtc gggaaacctg tcgtgccagc tgcattaatg 8100

aatcggccaa cgcgcgggga gaggcggttt gcgtattggg cgctcttccg cttcctcgct 8160

cactgactcg ctgcgctcgg tcgttcggct gcggcgagcg gtatcagctc actcaaaggc 8220

ggtaatacgg ttatccacag aatcagggga taacgcagga aagaacatgt gagcaaaagg 8280

ccagcaaaag gccaggaacc gtaaaaaggc cgcgttgctg gcgtttttcc ataggctccg 8340

cccccctgac gagcatcaca aaaatcgacg ctcaagtcag aggtggcgaa acccgacagg 8400

actataaaga taccaggcgt ttccccctgg aagctccctc gtgcgctctc ctgttccgac 8460

cctgccgctt accggatacc tgtccgcctt tctcccttcg ggaagcgtgg cgctttctca 8520

tagctcacgc tgtaggtatc tcagttcggt gtaggtcgtt cgctccaagc tgggctgtgt 8580

gcacgaaccc cccgttcagc ccgaccgctg cgccttatcc ggtaactatc gtcttgagtc 8640

caacccggta agacacgact tatcgccact ggcagcagcc actggtaaca ggattagcag 8700

agcgaggtat gtaggcggtg ctacagagtt cttgaagtgg tggcctaact acggctacac 8760

tagaaggaca gtatttggta tctgcgctct gctgaagcca gttaccttcg gaaaaagagt 8820

tggtagctct tgatccggca aacaaaccac cgctggtagc ggtggttttt ttgtttgcaa 8880

gcagcagatt acgcgcagaa aaaaaggatc tcaagaagat cctttgatct tttctacggg 8940

gtctgacgct cagtggaacg aaaactcacg ttaagggatt ttggtcatga gattatcaaa 9000

aaggatcttc acctagatcc ttttaaatta aaaatgaagt tttaaatcaa tctaaagtat 9060

atatgagtaa acttggtctg acagttacca atgcttaatc agtgaggcac ctatctcagc 9120

gatctgtcta tttcgttcat ccatagttgc ctgactcccc gtcgtgtaga taactacgat 9180

acgggagggc ttaccatctg gccccagtgc tgcaatgata ccgcgagacc cacgctcacc 9240

ggctccagat ttatcagcaa taaaccagcc agccggaagg gccgagcgca gaagtggtcc 9300

tgcaacttta tccgcctcca tccagtctat taattgttgc cgggaagcta gagtaagtag 9360

ttcgccagtt aatagtttgc gcaacgttgt tgccattgct acaggcatcg tggtgtcacg 9420

ctcgtcgttt ggtatggctt cattcagctc cggttcccaa cgatcaaggc gagttacatg 9480

atcccccatg ttgtgcaaaa aagcggttag ctccttcggt cctccgatcg ttgtcagaag 9540

taagttggcc gcagtgttat cactcatggt tatggcagca ctgcataatt ctcttactgt 9600

catgccatcc gtaagatgct tttctgtgac tggtgagtac tcaaccaagt cattctgaga 9660

atagtgtatg cggcgaccga gttgctcttg cccggcgtca atacgggata ataccgcgcc 9720

acatagcaga actttaaaag tgctcatcat tggaaaacgt tcttcggggc gaaaactctc 9780

aaggatctta ccgctgttga gatccagttc gatgtaaccc actcgtgcac ccaactgatc 9840

ttcagcatct tttactttca ccagcgtttc tgggtgagca aaaacaggaa ggcaaaatgc 9900

cgcaaaaaag ggaataaggg cgacacggaa atgttgaata ctcatactct tcctttttca 9960

atattattga agcatttatc agggttattg tctcatgagc ggatacatat ttgaatgtat 10020

ttagaaaaat aaacaaatag gggttccgcg cacatttccc cgaaaagtgc cacctgacgc 10080

gccctgtagc ggcgcattaa gcgcggcggg tgtggtggtt acgcgcagcg tgaccgctac 10140

acttgccagc gccctagcgc ccgctccttt cgctttcttc ccttcctttc tcgccacgtt 10200

cgccggcttt ccccgtcaag ctctaaatcg ggggctccct ttagggttcc gatttagtgc 10260

tttacggcac ctcgacccca aaaaacttga ttagggtgat ggttcacgta gtgggccatc 10320

gccctgatag acggtttttc gccctttgac gttggagtcc acgttcttta atagtggact 10380

cttgttccaa actggaacaa cactcaaccc tatctcggtc tattcttttg atttataagg 10440

gattttgccg atttcggcct attggttaaa aaatgagctg atttaacaaa aatttaacgc 10500

gaattttaac aaaatattaa cgcttacaat ttccattcgc cattcaggct gcgcaactgt 10560

tgggaagggc gatcggtgcg ggcctcttcg ctattacgcc agctggcgaa agggggatgt 10620

gctgcaaggc gattaagttg ggtaacgcca gggttttccc agtcacgacg ttgtaaaacg 10680

acggccagtg aattgtaata cgactcacta tagggcgaat tgggtaccgg gccccccctc 10740

gaggtcgatg gtgtcgataa gcttgatatc gaattcatgt cacacaaacc gatcttcgcc 10800

tcaaggaaac ctaattctac atccgagaga ctgccgagat ccagtctaca ctgattaatt 10860

ttcgggccaa taatttaaaa aaatcgtgtt atataatatt atatgtatta tatatataca 10920

tcatgatgat actgacagtc atgtcccatt gctaaataga cagactccat ctgccgcctc 10980

caactgatgt tctcaatatt taaggggtca tctcgcattg tttaataata aacagactcc 11040

atctaccgcc tccaaatgat gttctcaaaa tatattgtat gaacttattt ttattactta 11100

gtattattag acaacttact tgctttatga aaaacacttc ctatttagga aacaatttat 11160

aatggcagtt cgttcattta acaatttatg tagaataaat gttataaatg cgtatgggaa 11220

atcttaaata tggatagcat aaatgatatc tgcattgcct aattcgaaat caacagcaac 11280

gaaaaaaatc ccttgtacaa cataaatagt catcgagaaa tatcaactat caaagaacag 11340

ctattcacac gttactattg agattattat tggacgagaa tcacacactc aactgtcttt 11400

ctctcttcta gaaatacagg tacaagtatg tactattctc attgttcata cttctagtca 11460

tttcatccca catattcctt ggatttctct ccaatgaatg acattctatc ttgcaaattc 11520

aacaattata ataagatata ccaaagtagc ggtatagtgg caatcaaaaa gcttctctgg 11580

tgtgcttctc gtatttattt ttattctaat gatccattaa aggtatatat ttatttcttg 11640

ttatataatc cttttgttta ttacatgggc tggatacata aaggtatttt gatttaattt 11700

tttgcttaaa ttcaatcccc cctcgttcag tgtcaactgt aatggtagga aattaccata 11760

cttttgaaga agcaaaaaaa atgaaagaaa aaaaaaatcg tatttccagg ttagacgttc 11820

cgcagaatct agaatgcggt atgcggtaca ttgttcttcg aacgtaaaag ttgcgctccc 11880

tgagatattg tacatttttg cttttacaag tacaagtaca tcgtacaact atgtactact 11940

gttgatgcat ccacaacagt ttgttttgtt tttttttgtt tttttttttt ctaatgattc 12000

attaccgcta tgtataccta cttgtacttg tagtaagccg ggttattggc gttcaattaa 12060

tcatagactt atgaatctgc acggtgtgcg ctgcgagtta cttttagctt atgcatgcta 12120

cttgggtgta atattgggat ctgttcggaa atcaacggat gctcaat 12167

<210> 25
<211> 1963
<212> DNA
<213> Artificial sequence

<220>
<223> Artificial Sequence

<400> 25
gtttaaacct taagcccgct cataacttcg tatagcatac attatacgaa cggtaggttg 60

cgggatagac gccgacggag ggcaatggcg ctatggaacc ttgcggatat ccatacgccg 120

cggcggactg cgtccgaacc agctccagca gcgttttttc cgggccattg agccgactgc 180

gaccccgcca acgtgtcttg gcccacgcac tcatgtcatg ttggtgttgg gaggccactt 240

tttaagtagc acaaggcacc tagctcgcag caaggtgtcc gaaccaaaga agcggctgca 300

gtggtgcaaa cggggcggaa acggcgggaa aaagccacgg gggcacgaat tgaggcacgc 360

cctcgaattt gagacgagtc acggccccat tcgcccgcgc aatggctcgc caacgcccgg 420

tcttttgcac cacatcaggt taccccaagc caaacctttg tgttaaaaag cttaacatat 480

tataccgaac gtaggtttgg gcgggcttgc tccgtctgtc caaggcaaca tttatataag 540

ggtctgcatc gccggctcaa ttgaatcttt tttcttcttc tcttctctat attcattctt 600

gaattaaaca cacatcaacc atggccaaaa agcctgaact caccgcgacg tctgtcgaga 660

agtttctgat cgaaaagttc gacagcgtct ccgacctgat gcagctctcg gagggcgaag 720

aatctcgtgc tttcagcttc gatgtaggag ggcgtggata tgtcctgcgg gtaaatagct 780

gcgccgatgg tttctacaaa gatcgttatg tttatcggca ctttgcatcg gccgcgctcc 840

cgattccgga agtgcttgac attggggagt tcagcgagag cctgacctat tgcatctccc 900

gccgtgcaca gggtgtcacg ttgcaagacc tgcctgaaac cgaactgccc gctgttctgc 960

agccggtcgc ggaggctatg gatgcgatcg ctgcggccga tcttagccag acgagcgggt 1020

tcggcccatt cggaccgcaa ggaatcggtc aatacactac atggcgtgat ttcatatgcg 1080

cgattgctga tccccatgtg tatcactggc aaactgtgat ggacgacacc gtcagtgcgt 1140

ccgtcgcgca ggctctcgat gagctgatgc tttgggccga ggactgcccc gaagtccggc 1200

acctcgtgca cgcggatttc ggctccaaca atgtcctgac ggacaatggc cgcataacag 1260

cggtcattga ctggagcgag gcgatgttcg gggattccca atacgaggtc gccaacatct 1320

tcttctggag gccgtggttg gcttgtatgg agcagcagac gcgctacttc gagcggaggc 1380

atccggagct tgcaggatcg ccgcggctcc gggcgtatat gctccgcatt ggtcttgacc 1440

aactctatca gagcttggtt gacggcaatt tcgatgatgc agcttgggcg cagggtcgat 1500

gcgacgcaat cgtccgatcc ggagccggga ctgtcgggcg tacacaaatc gcccgcagaa 1560

gcgcggccgt ctggaccgat ggctgtgtag aagtactcgc cgatagtgga aaccgacgcc 1620

ccagcactcg tccgagggca aaggaatagc ggccgcaagt gtggatgggg aagtgagtgc 1680

ccggttctgt gtgcacaatt ggcaatccaa gatggatgga ttcaacacag ggatatagcg 1740

agctacgtgg tggtgcgagg atatagcaac ggatatttat gtttgacact tgagaatgta 1800

cgatacaagc actgtccaag tacaatacta aacatactgt acatactcat actcgtaccc 1860

gggcaacggt ttcacttgag tgcagtggct agtgctctta ctcgtacagt gtgctaccgt 1920

tcgtatagca tacattatac gaagttatca tagtcttaat taa 1963

<210> 26
<211> 23
<212> DNA
<213> Yarrowia lipolytica

<400> 26
cgctcgagtg ctcaagctcg tgg 23

<210>  27
<211>  604
<212>  DNA
<213>  Artificial Sequence

<220>
<223>  Artificial Sequence

<400>  27
ttaattaaat tttttttgat tttctttttt gaccccgtct tcaattacac ttcccaactg        60

ggaacacccc tctttatcga cccattttag gtaatttacc ctagcccatt gtctccataa       120

ggaatattac cctaacccac agtccagggt gcccaggtcc ttctttggcc aaattttaac       180

ttcggtccta tggcacagcg gtagcgcgtg agattgcaaa tcttaaggtc ccgagttcga       240

atctcggtgg gacctagtta tttttgatag ataatttcgt gatgattaga aacttaacgc       300

aaaataatgc ctggctagct caatcggtag agcgtgagac tcttatacaa gaaatctcaa       360

ggctgtgggt tcaagcccca cgtcgggcta gccgctcgag tgctcaagct cggttttaga       420

gctagaaata gcaagttaaa ataaggctag tccgttatca acttgaaaaa gtggcaccga       480

gtcggtgctc cgatatagtg taggggctat cacatcacgc tctcatcaag aagtcttctt       540

gagaaccgtg gagaccgggg ttcgattccc cgtatcggag tttttttttt tgttttttat       600

cgat                                                                    604


<210>  28
<211>  81
<212>  DNA
<213>  Artificial Sequence

<220>
<223>  Artificial Sequence

<400>  28
gcctggctag ctcaatcggt agagcgtgag actcttatac aagaaatctc aaggctgtgg        60

gttcaagccc cacgtcgggc t                                                  81


<210>  29
<211>  92
<212>  DNA
<213>  Artificial Sequence

<220>
<223>  Artificial Sequence

<400>  29
tccgatatag tgtaggggct atcacatcac gctctcatca agaagtcttc ttgagaaccg        60

tggagaccgg ggttcgattc cccgtatcgg ag                                      92


<210>  30
<211>  11738

<212>  DNA
<213>  Artificial Sequence

<220>
<223>  Artificial Sequence

<400>  30
attgagcatc cgttgatttc cgaacagatc ccaatattac acccaagtag catgcataag      60

ctaaaagtaa ctcgcagcgc acaccgtgca gattcataag tctatgatta attgaacgcc     120

aataacccgg cttactacaa gtacaagtag gtatacatag cggtaatgaa tcattagaaa     180

aaaaaaaaac aaaaaaaaac aaaacaaact gttgtggatg catcaacagt agtacatagt     240

tgtacgatgt acttgtactt gtaaaagcaa aaatgtacaa tatctcaggg agcgcaactt     300

ttacgttcga agaacaatgt accgcatacc gcattctaga ttctgcggaa cgtctaacct     360

ggaaatacga tttttttttt ctttcatttt ttttgcttct tcaaaagtat ggtaatttcc     420

taccattaca gttgacactg aacgaggggg gattgaattt aagcaaaaaa ttaaatcaaa     480

atacctttat gtatccagcc catgtaataa acaaaaggat tatataacaa gaaataaata     540

tataccttta atggatcatt agaataaaaa taaatacgag aagcacacca gagaagcttt     600

ttgattgcca ctataccgct actttggtat atcttattat aattgttgaa tttgcaagat     660

agaatgtcat tcattggaga gaaatccaag gaatatgtgg gatgaaatga ctagaagtat     720

gaacaatgag aatagtacat acttgtacct gtatttctag aagagagaaa gacagttgag     780

tgtgtgattc tcgtccaata ataatctcaa tagtaacgtg tgaatagctg ttctttgata     840

gttgatattt ctcgatgact atttatgttg tacaagggat ttttttcgtt gctgttgatt     900

tcgaattagg caatgcagat atcatttatg ctatccatat ttaagatttc ccatacgcat     960

ttataacatt tattctacat aaattgttaa atgaacgaac tgccattata aattgtttcc    1020

taaataggaa gtgtttttca taaagcaagt aagttgtcta ataatactaa gtaataaaaa    1080

taagttcata caatatattt tgagaacatc atttggaggc ggtagatgga gtctgtttat    1140

tattaaacaa tgcgagatga ccccttaaat attgagaaca tcagttggag gcggcagatg    1200

gagtctgtct atttagcaat gggacatgac tgtcagtatc atcatgatgt atatatataa    1260

tacatataat attatataac acgatttttt taaattattg gcccgaaaat taatcagtgt    1320

agactggatc tcggcagtct ctcggatgta gaattaggtt tccttgaggc gaagatcggt    1380

ttgtgtgaca tgaattcgat atcaagctta tcgacaccat cgacctcgag ggggggcccg    1440

gtacccaatt cgccctatag tgagtcgtat tacaattcac tggccgtcgt tttacaacgt    1500

cgtgactggg aaaaccctgg cgttacccaa cttaatcgcc ttgcagcaca tccccctttc    1560

gccagctggc gtaatagcga agaggcccgc accgatcgcc cttcccaaca gttgcgcagc    1620

ctgaatggcg aatggaaatt gtaagcgtta atattttgtt aaaattcgcg ttaaattttt    1680

gttaaatcag ctcatttttt aaccaatagg ccgaaatcgg caaaatccct tataaatcaa 1740

aagaatagac cgagataggg ttgagtgttg ttccagtttg gaacaagagt ccactattaa 1800

agaacgtgga ctccaacgtc aaagggcgaa aaaccgtcta tcagggcgat ggcccactac 1860

gtgaaccatc accctaatca agttttttgg ggtcgaggtg ccgtaaagca ctaaatcgga 1920

accctaaagg gagcccccga tttagagctt gacggggaaa gccggcgaac gtggcgagaa 1980

aggaagggaa gaaagcgaaa ggagcgggcg ctagggcgct ggcaagtgta gcggtcacgc 2040

tgcgcgtaac caccacaccc gccgcgctta atgcgccgct acagggcgcg tcaggtggca 2100

cttttcgggg aaatgtgcgc ggaaccccta tttgtttatt tttctaaata cattcaaata 2160

tgtatccgct catgagacaa taaccctgat aaatgcttca ataatattga aaaaggaaga 2220

gtatgagtat tcaacatttc cgtgtcgccc ttattccctt ttttgcggca ttttgccttc 2280

ctgtttttgc tcacccagaa acgctggtga aagtaaaaga tgctgaagat cagttgggtg 2340

cacgagtggg ttacatcgaa ctggatctca acagcggtaa gatccttgag agttttcgcc 2400

ccgaagaacg ttttccaatg atgagcactt ttaaagttct gctatgtggc gcggtattat 2460

cccgtattga cgccgggcaa gagcaactcg gtcgccgcat acactattct cagaatgact 2520

tggttgagta ctcaccagtc acagaaaagc atcttacgga tggcatgaca gtaagagaat 2580

tatgcagtgc tgccataacc atgagtgata acactgcggc caacttactt ctgacaacga 2640

tcggaggacc gaaggagcta accgcttttt tgcacaacat gggggatcat gtaactcgcc 2700

ttgatcgttg ggaaccggag ctgaatgaag ccataccaaa cgacgagcgt gacaccacga 2760

tgcctgtagc aatggcaaca acgttgcgca aactattaac tggcgaacta cttactctag 2820

cttcccggca acaattaata gactggatgg aggcggataa agttgcagga ccacttctgc 2880

gctcggccct tccggctggc tggtttattg ctgataaatc tggagccggt gagcgtgggt 2940

ctcgcggtat cattgcagca ctggggccag atggtaagcc ctcccgtatc gtagttatct 3000

acacgacggg gagtcaggca actatggatg aacgaaatag acagatcgct gagataggtg 3060

cctcactgat taagcattgg taactgtcag accaagttta ctcatatata ctttagattg 3120

atttaaaact tcatttttaa tttaaaagga tctaggtgaa gatccttttt gataatctca 3180

tgaccaaaat cccttaacgt gagttttcgt tccactgagc gtcagacccc gtagaaaaga 3240

tcaaaggatc ttcttgagat cctttttttc tgcgcgtaat ctgctgcttg caaacaaaaa 3300

aaccaccgct accagcggtg gtttgtttgc cggatcaaga gctaccaact ctttttccga 3360

aggtaactgg cttcagcaga gcgcagatac caaatactgt ccttctagtg tagccgtagt 3420

taggccacca cttcaagaac tctgtagcac cgcctacata cctcgctctg ctaatcctgt 3480

taccagtggc tgctgccagt ggcgataagt cgtgtcttac cgggttggac tcaagacgat 3540

agttaccgga taaggcgcag cggtcgggct gaacgggggg ttcgtgcaca cagcccagct 3600

tggagcgaac gacctacacc gaactgagat acctacagcg tgagctatga gaaagcgcca 3660

cgcttcccga agggagaaag gcggacaggt atccggtaag cggcagggtc ggaacaggag 3720

agcgcacgag ggagcttcca gggggaaacg cctggtatct ttatagtcct gtcgggtttc 3780

gccacctctg acttgagcgt cgatttttgt gatgctcgtc aggggggcgg agcctatgga 3840

aaaacgccag caacgcggcc tttttacggt tcctggcctt ttgctggcct tttgctcaca 3900

tgttctttcc tgcgttatcc cctgattctg tggataaccg tattaccgcc tttgagtgag 3960

ctgataccgc tcgccgcagc cgaacgaccg agcgcagcga gtcagtgagc gaggaagcgg 4020

aagagcgccc aatacgcaaa ccgcctctcc ccgcgcgttg gccgattcat taatgcagct 4080

ggcacgacag gtttcccgac tggaaagcgg gcagtgagcg caacgcaatt aatgtgagtt 4140

agctcactca ttaggcaccc caggctttac actttatgct tccggctcgt acgcaactaa 4200

catgaatgaa tacgatatac atcaaagact atgatacgca gtattgcaca ctgtacgagt 4260

aagagcacta gccactgcac tcaagtgaaa ccgttgcccg ggtacgagta tgagtatgta 4320

cagtatgttt agtattgtac ttggacagtg cttgtatcgt acattctcaa gtgtcaaaca 4380

taaatatccg ttgctatatc ctcgcaccac cacgtagctc gctatatccc tgtgttgaat 4440

ccatccatct tggattgcca attgtgcaca cagaaccggg cactcacttc cccatccaca 4500

cttgcggccg cttagacctt tcgctttttc ttgggatcgg ctctggagtc gccaccaagc 4560

tgagacaggt cgattcgggt ctcgtacagg ccagtgatgg actggtgaat cagggtggca 4620

tcgagaacct ccttggtgga tgtgtaccgc tttcggtcga tagtggtatc gaagtacttg 4680

aaagctgcag gagcacccag gttggtaaga gtaaacaggt gaatgatgtt ctccgcctgt 4740

tctcgaatgg gtttgtcccg atgcttgttg taggcagaga gcaccttgtc caagttggca 4800

tcagccagga tgactcgctt cgaaaactcg gaaatctgct cgataatctc gtcgaggtaa 4860

tgtttgtgct gctcaacgaa gagttgcttc tgttcgttgt cctcgggaga acccttgagc 4920

ttctcgtagt gagaagccag atagagaaag ttgacgtact tcgaaggcaa ggcaagctcg 4980

tttcccttct gcagctcgcc agcggaggcg agcatacgct ttcgaccgtt ctccagttcg 5040

aacagagagt acttgggcag cttgataatg aggtctttct tgacctcctt gtaacccttg 5100

gcttccaaga agtcgatggg attcttctcg aagctcgatc gctccatgat ggtaattccg 5160

agcagctcct tgacggactt gagctttttg gacttgccct tctcgacctt cgcaacgaca 5220

agcacggaat aggcgacggt aggagaatcg aagccaccgt atttcttggg atcccagtct 5280

ttctttcgag cgatgagctt gtcggagttt cgcttgggca gaatcgactc cttggagaat 5340

ccgccagtct gaacctcggt tttcttgacg atgttgacct gaggcatcga cagaaccttt 5400

cgcacggttg caaagtctcg acccttgtcc cacacgatct ctccagtttc gccgttggtc 5460

tcgataagtg gtctctttcg aatctctccg ttggccaagg tgatctcggt cttgaaaaag 5520

ttcatgatgt tggagtaaaa gaagtacttg gcagtagcct tgccaatctc ctgttcggac 5580

ttggcaatca tctttcgaac gtcgtagacc ttgtaatcgc cgtaaacgaa ctcgctttcg 5640

agcttggggt atttcttgat gagcgcagtg ccaacgacgg cgttgaggta agcatcgtgg 5700

gcatggtggt aattgttgat ctctcgcacc ttgtagaact gaaagtcctt tcggaaatcg 5760

gagaccagtt tggacttgag agtaatcacc ttgacctctc ggatgagctt gtcgttctcg 5820

tcgtacttgg tgttcatccg agaatcgaga atctgtgcga cgtgctttgt gatctgtctg 5880

gtctcgacga gttgacgctt gatgaagcca gccttgtcga gctcggacag accgcctcgc 5940

tcggccttgg taagattgtc gaactttcgc tgggtaatga gcttggcgtt gagcagctgt 6000

cgccagtagt tcttcatctt tttgaccacc tcttcgctgg gaacgttgtc cgacttgcct 6060

ctgttcttgt cggatcgtgt aaggaccttg ttgtcgatag aatcgtcctt gagaaaggat 6120

tgagggacaa tgtggtccac atcgtagtcg ctgagacgat tgatgtccag ttcctgatcc 6180

acgtacatgt ctcgaccatt ctgcagatag tagagataca gcttctcgtt ctgcagttga 6240

gtgttctcga cgggatgctc cttgagaatc tgggatccca gctccttgat gccttcctcg 6300

attcgcttca tccgctctcg cgagtttttc tgaccctttt gagttgtctg gttctctctg 6360

gccatctcga tcacaatgtt ctcgggcttg tgacgtccca tgaccttcac cagctcgtcg 6420

acaaccttga cagtctggag aatgcctttc ttgatggctg gcgaaccagc caggttggca 6480

atatgttcgt gcaagctgtc gccctgaccg gacacttgtg ccttctggat gtcctccttg 6540

aaggtaagag aatcgtcgtg aatgagctgc atgaagtttc ggttggcaaa gccatcggac 6600

ttgagaaagt ccagaatggt ctttccggac tgcttgtctc tgatgccgtt gatgagcttt 6660

cgcgaaagtc ttccccagcc ggtgtatcta cgtcgcttga gttgtttcat gaccttgtcg 6720

tcgaacaggt gagcgtatgt cttgagtcgt tcctcgatca tctcccgatc ttcgaacagg 6780

gtaagagtga gcacgatgtc ctccagaatg tcctcgtttt cctcgttgtc gagaaaatcc 6840

ttgtccttga taatcttgag cagatcgtga taggtgccca aagaggcgtt gaatcggtcc 6900

tcaactccgg aaatctcgac gctgtcgaaa cactcgattt tcttgaagta gtcctccttg 6960

agctgcttaa cagtgacctt tcggttggtc ttgaacagga gatcgacaat ggctttcttc 7020

tgttcgccag acaagaaggc aggctttcgc attccctcgg taacgtactt gactttggtg 7080

agttcgttgt agactgtaaa gtactcgtag agcagcgaat gcttgggaag aaccttctcg 7140

ttgggcagat tcttgtcgaa gttggtcatt cgctcgatga aggactgtgc agaggcaccc 7200

ttgtccacga cttcctcgaa gttccaggga gtgatggttt cctcggactt tcgagtcatc 7260

caagcaaatc gagagtttcc tctggcaaga ggaccaacat agtaggggat tcgaaaggta 7320

agaatcttct cgatcttctc tcggttgtcc ttgagaaagg ggtagaagtc ttcctgacgt 7380

cgaagaatgg cgtgcagctc accgaggtgg atctgatgag gaatgctgcc gttgtcgaag 7440

gttcgttgct tccgaagcag atcctctcga ttgagcttga caagcagttc ctcggttccg 7500

tccatcttct cgagaattgg cttgatgaac ttgtagaact cttcctgaga ggctccgccg 7560

tcgatgtatc cagcgtagcc gttcttcgac tgatcgaaaa agatctcctt gtacttctcg 7620

ggcagttgct gtcggacaag agccttgagc agtgtgagat cctgatggtg ctcgtcgtat 7680

cgcttgatca tggaggcaga aaggggagcc tttgtgatct cggtgttgac tcgcagaatg 7740

tcagacaaga gaatagcatc cgaaaggttc ttggcagcga gaaacaggtc ggcgtactga 7800

tcgccaatct gtgcaagcag gttgtcgagg tcatcgtcgt aggtgtcctt ggacagctgg 7860

agcttggcgt cctccgccag atcgaagttg gacttgaagt tgggtgtgag accaagagaa 7920

agggcaatga ggttgccaaa cagtccgttc tttttctcgc caggaagttg ggcaatgagg 7980

ttctccagtc gtctgctctt cgagagtcga gcagacaaga tggcctttgc atcgactccg 8040

gaggcattga tggggttttc ctcgaacagc tggttgtagg tctgaacgag ctgaatgaac 8100

agcttgtcca catcgctgtt gtcgggattg agatcgccct cgatgaggaa atgacctcga 8160

aacttgatca tgtgtgccag agcgaggtag ataagtctga gatccgcctt gtcggtggaa 8220

tcgacgagtt tctttcggag atggtagatg gtaggatact tctcgtggta agcaacctcg 8280

tccacaatgt tgccaaagat gggatgacgc tcgtgtttct tgtcttcctc gacgaggaag 8340

gattcctcca gtcgatgaaa gaacgaatcg tccaccttgg ccatctcgtt ggaaaagatc 8400

tcctgcaggt agcagattcg gttcttccgt cgggtgtaac gtcgccgagc agttcgcttg 8460

agtctggtag cttcggcagt ctcgccagaa tcgaacaaca gggcaccaat gaggtttttc 8520

ttgatggagt gtcgatcggt gtttccgagg accttgaatt tcttggaggg caccttgtac 8580

tcgtcggtga tgacagccca gccgacagag ttggttccaa tgtccaggcc gatggagtat 8640

ttcttgtcca tggtgtgatg tgtagtttag atttcgaatc tgtggggaaa gaaaggaaaa 8700

aagagactgg caaccgattg ggagagccac tgtttatata taccctagac aagccccccg 8760

cttgtaagat gttggtcaat gtaaaccagt attaaggttg gcaagtgcag gagaagcaag 8820

gtgtgggtac cgagcaatgg aaatgtgcgg aaggcaaaaa aatgaggcca cggcctattg 8880

tcggggctat atccaggggg cgattgaagt acactaacat gacatgtgtc cacagaccct 8940

caatctggcc tgatgagcca aatccatacg cgctttcgca gctctaaagg ctataacaag 9000

tcacaccacc ctgctcgacc tcagcgccct cactttttgt taagacaaac tgtacacgct 9060

gttccagcgt tttctgcctg cacctggtgg gacatttggt gcaacctaaa gtgctcggaa 9120

cctctgtggt gtccagatca gcgcagcagt tccgaggtag ttttgaggcc cttagatgat 9180

ggtttaaacc ttaagcccgc tcataacttc gtatagcata cattatacga acggtaggtt 9240

gcgggataga cgccgacgga gggcaatggc gctatggaac cttgcggata tccatacgcc 9300

gcggcggact gcgtccgaac cagctccagc agcgtttttt ccgggccatt gagccgactg 9360

cgaccccgcc aacgtgtctt ggcccacgca ctcatgtcat gttggtgttg ggaggccact 9420

ttttaagtag cacaaggcac ctagctcgca gcaaggtgtc cgaaccaaag aagcggctgc 9480

agtggtgcaa acggggcgga aacggcggga aaaagccacg ggggcacgaa ttgaggcacg 9540

ccctcgaatt tgagacgagt cacggcccca ttcgcccgcg caatggctcg ccaacgcccg 9600

gtcttttgca ccacatcagg ttaccccaag ccaaaccttt gtgttaaaaa gcttaacata 9660

ttataccgaa cgtaggtttg ggcgggcttg ctccgtctgt ccaaggcaac atttatataa 9720

gggtctgcat cgccggctca attgaatctt ttttcttctt ctcttctcta tattcattct 9780

tgaattaaac acacatcaac catggccaaa aagcctgaac tcaccgcgac gtctgtcgag 9840

aagtttctga tcgaaaagtt cgacagcgtc tccgacctga tgcagctctc ggagggcgaa 9900

gaatctcgtg ctttcagctt cgatgtagga gggcgtggat atgtcctgcg ggtaaatagc 9960

tgcgccgatg gtttctacaa agatcgttat gtttatcggc actttgcatc ggccgcgctc 10020

ccgattccgg aagtgcttga cattggggag ttcagcgaga gcctgaccta ttgcatctcc 10080

cgccgtgcac agggtgtcac gttgcaagac ctgcctgaaa ccgaactgcc cgctgttctg 10140

cagccggtcg cggaggctat ggatgcgatc gctgcggccg atcttagcca gacgagcggg 10200

ttcggcccat tcggaccgca aggaatcggt caatacacta catggcgtga tttcatatgc 10260

gcgattgctg atccccatgt gtatcactgg caaactgtga tggacgacac cgtcagtgcg 10320

tccgtcgcgc aggctctcga tgagctgatg ctttgggccg aggactgccc cgaagtccgg 10380

cacctcgtgc acgcggattt cggctccaac aatgtcctga cggacaatgg ccgcataaca 10440

gcggtcattg actggagcga ggcgatgttc ggggattccc aatacgaggt cgccaacatc 10500

ttcttctgga ggccgtggtt ggcttgtatg gagcagcaga cgcgctactt cgagcggagg 10560

catccggagc ttgcaggatc gccgcggctc cgggcgtata tgctccgcat tggtcttgac 10620

caactctatc agagcttggt tgacggcaat ttcgatgatg cagcttgggc gcagggtcga 10680

tgcgacgcaa tcgtccgatc cggagccggg actgtcgggc gtacacaaat cgcccgcaga 10740

agcgcggccg tctggaccga tggctgtgta gaagtactcg ccgatagtgg aaaccgacgc 10800

cccagcactc gtccgagggc aaaggaatag cggccgcaag tgtggatggg gaagtgagtg 10860

cccggttctg tgtgcacaat tggcaatcca agatggatgg attcaacaca gggatatagc 10920

gagctacgtg gtggtgcgag gatatagcaa cggatattta tgtttgacac ttgagaatgt 10980

acgatacaag cactgtccaa gtacaatact aaacatactg tacatactca tactcgtacc 11040

cgggcaacgg tttcacttga gtgcagtggc tagtgctctt actcgtacag tgtgctaccg 11100

ttcgtatagc atacattata cgaagttatc atagtcttaa ttaaattttt tttgattttc 11160

ttttttgacc ccgtcttcaa ttacacttcc caactgggaa cacccctctt tatcgaccca 11220

ttttaggtaa tttaccctag cccattgtct ccataaggaa tattacccta acccacagtc 11280

cagggtgccc aggtccttct ttggccaaat tttaacttcg gtcctatggc acagcggtag 11340

cgcgtgagat tgcaaatctt aaggtcccga gttcgaatct cggtgggacc tagttatttt 11400

tgatagataa tttcgtgatg attagaaact taacgcaaaa taatgcctgg ctagctcaat 11460

cggtagagcg tgagactctt atacaagaaa tctcaaggct gtgggttcaa gccccacgtc 11520

gggctagccg ctcgagtgct caagctcggt tttagagcta gaaatagcaa gttaaaataa 11580

ggctagtccg ttatcaactt gaaaaagtgg caccgagtcg gtgctccgat atagtgtagg 11640

ggctatcaca tcacgctctc atcaagaagt cttcttgaga accgtggaga ccggggttcg 11700

attccccgta tcggagtttt tttttttgtt ttttatcg 11738

<210> 31
<211> 569
<212> DNA
<213> Artificial Sequence

<220>
<223> Artificial Sequence

<400> 31
   ttaattaaag ttattggttg acattgtttt ttccattctg tttttttttt tttttttttt 60
   ttttttttta attctaatga ttcattacag ctatatacct agtaagccgg gttattggcg 120
   ttcaataaat catacacttc tgaatctttg cgatgtgcgc tgcgaggcgc tgtcggttgg 180
   tttgtcgacg cttcgtctag ttgatcacat tgtcgtggtc tcgaggtagt gaatcatggc 240
   taacagatcg agaaatcata tcaatcgttt cttgtcttat ccagactcga ggttggttta 300
   gtttgttctg attacataat cagaaaacaa cggctctggt acaaaaccac tttgtgttca 360
   gcgtccgcct ggctagctca atcggtagag cgtgagactc ttatacaaga aatctcaagg 420
   ctgtgggttc aagccccacg tcgggctagc cgctcgagtg ctcaagctcg gttttagagc 480
   tagaaatagc aagttaaaat aaggctagtc cgttatcaac ttgaaaaagt ggcaccgagt 540
   cggtgctttt ttttttgttt tttatcgat 569

<210> 32
<211> 366
<212> DNA
<213> Artificial Sequence

<220>
<223> Artificial Sequence

<400> 32
ttaattaaag ttattggttg acattgtttt ttccattctg tttttttttt tttttttttt 60

ttttttttta attctaatga ttcattacag ctatatacct agtaagccgg gttattggcg 120

ttcaataaat catacacttc tgaatctttg cgatgtgcgc tgcgaggcgc tgtcggttgg 180

tttgtcgacg cttcgtctag ttgatcacat tgtcgtggtc tcgaggtagt gaatcatggc 240

taacagatcg agaaatcata tcaatcgttt cttgtcttat ccagactcga ggttggttta 300

gtttgttctg attacataat cagaaaacaa cggctctggt acaaaaccac tttgtgttca 360

gcgtcc 366

<210> 33
<211> 11703
<212> DNA
<213> Artificial Sequence

<220>
<223> Artificial Sequence

<400> 33
attgagcatc cgttgatttc cgaacagatc ccaatattac acccaagtag catgcataag 60

ctaaaagtaa ctcgcagcgc acaccgtgca gattcataag tctatgatta attgaacgcc 120

aataacccgg cttactacaa gtacaagtag gtatacatag cggtaatgaa tcattagaaa 180

aaaaaaaaac aaaaaaaaac aaaacaaact gttgtggatg catcaacagt agtacatagt 240

tgtacgatgt acttgtactt gtaaaagcaa aaatgtacaa tatctcaggg agcgcaactt 300

ttacgttcga agaacaatgt accgcatacc gcattctaga ttctgcggaa cgtctaacct 360

ggaaatacga tttttttttt ctttcatttt ttttgcttct tcaaaagtat ggtaatttcc 420

taccattaca gttgacactg aacgaggggg gattgaattt aagcaaaaaa ttaaatcaaa 480

atacctttat gtatccagcc catgtaataa acaaaaggat tatataacaa gaaataaata 540

tataccttta atggatcatt agaataaaaa taaatacgag aagcacacca gagaagcttt 600

ttgattgcca ctataccgct actttggtat atcttattat aattgttgaa tttgcaagat 660

agaatgtcat tcattggaga gaaatccaag gaatatgtgg gatgaaatga ctagaagtat 720

gaacaatgag aatagtacat acttgtacct gtatttctag aagagagaaa gacagttgag 780

tgtgtgattc tcgtccaata ataatctcaa tagtaacgtg tgaatagctg ttctttgata 840

gttgatattt ctcgatgact atttatgttg tacaagggat ttttttcgtt gctgttgatt 900

tcgaattagg caatgcagat atcatttatg ctatccatat ttaagatttc ccatacgcat 960

ttataacatt tattctacat aaattgttaa atgaacgaac tgccattata aattgtttcc 1020

taaataggaa gtgtttttca taaagcaagt aagttgtcta ataatactaa gtaataaaaa 1080

taagttcata caatatattt tgagaacatc atttggaggc ggtagatgga gtctgtttat 1140

tattaaacaa tgcgagatga ccccttaaat attgagaaca tcagttggag gcggcagatg 1200

gagtctgtct atttagcaat gggacatgac tgtcagtatc atcatgatgt atatatataa 1260

tacatataat attatataac acgatttttt taaattattg gcccgaaaat taatcagtgt 1320

agactggatc tcggcagtct ctcggatgta gaattaggtt tccttgaggc gaagatcggt 1380

ttgtgtgaca tgaattcgat atcaagctta tcgacaccat cgacctcgag ggggggcccg 1440

gtacccaatt cgccctatag tgagtcgtat tacaattcac tggccgtcgt tttacaacgt 1500

cgtgactggg aaaaccctgg cgttacccaa cttaatcgcc ttgcagcaca tccccctttc 1560

gccagctggc gtaatagcga agaggcccgc accgatcgcc cttcccaaca gttgcgcagc 1620

ctgaatggcg aatggaaatt gtaagcgtta atattttgtt aaaattcgcg ttaaattttt 1680

gttaaatcag ctcatttttt aaccaatagg ccgaaatcgg caaaatccct tataaatcaa 1740

aagaatagac cgagataggg ttgagtgttg ttccagtttg gaacaagagt ccactattaa 1800

agaacgtgga ctccaacgtc aaagggcgaa aaaccgtcta tcagggcgat ggcccactac 1860

gtgaaccatc accctaatca agttttttgg ggtcgaggtg ccgtaaagca ctaaatcgga 1920

accctaaagg gagcccccga tttagagctt gacggggaaa gccggcgaac gtggcgagaa 1980

aggaagggaa gaaagcgaaa ggagcgggcg ctagggcgct ggcaagtgta gcggtcacgc 2040

tgcgcgtaac caccacaccc gccgcgctta atgcgccgct acagggcgcg tcaggtggca 2100

cttttcgggg aaatgtgcgc ggaaccccta tttgtttatt tttctaaata cattcaaata 2160

tgtatccgct catgagacaa taaccctgat aaatgcttca ataatattga aaaaggaaga 2220

gtatgagtat tcaacatttc cgtgtcgccc ttattccctt ttttgcggca ttttgccttc 2280

ctgtttttgc tcacccagaa acgctggtga aagtaaaaga tgctgaagat cagttgggtg 2340

cacgagtggg ttacatcgaa ctggatctca acagcggtaa gatccttgag agttttcgcc 2400

ccgaagaacg ttttccaatg atgagcactt ttaaagttct gctatgtggc gcggtattat 2460

cccgtattga cgccgggcaa gagcaactcg gtcgccgcat acactattct cagaatgact 2520

tggttgagta ctcaccagtc acagaaaagc atcttacgga tggcatgaca gtaagagaat 2580

tatgcagtgc tgccataacc atgagtgata acactgcggc caacttactt ctgacaacga 2640

tcggaggacc gaaggagcta accgcttttt tgcacaacat gggggatcat gtaactcgcc 2700

ttgatcgttg ggaaccggag ctgaatgaag ccataccaaa cgacgagcgt gacaccacga 2760

tgcctgtagc aatggcaaca acgttgcgca aactattaac tggcgaacta cttactctag 2820

cttcccggca acaattaata gactggatgg aggcggataa agttgcagga ccacttctgc 2880

gctcggccct tccggctggc tggtttattg ctgataaatc tggagccggt gagcgtgggt 2940

ctcgcggtat cattgcagca ctggggccag atggtaagcc ctcccgtatc gtagttatct 3000

acacgacggg gagtcaggca actatggatg aacgaaatag acagatcgct gagataggtg 3060

cctcactgat taagcattgg taactgtcag accaagttta ctcatatata ctttagattg 3120

atttaaaact tcatttttaa tttaaaagga tctaggtgaa gatccttttt gataatctca 3180

tgaccaaaat cccttaacgt gagttttcgt tccactgagc gtcagacccc gtagaaaaga 3240

tcaaaggatc ttcttgagat cctttttttc tgcgcgtaat ctgctgcttg caaacaaaaa 3300

aaccaccgct accagcggtg gtttgtttgc cggatcaaga gctaccaact ctttttccga 3360

aggtaactgg cttcagcaga gcgcagatac caaatactgt ccttctagtg tagccgtagt 3420

taggccacca cttcaagaac tctgtagcac cgcctacata cctcgctctg ctaatcctgt 3480

taccagtggc tgctgccagt ggcgataagt cgtgtcttac cgggttggac tcaagacgat 3540

agttaccgga taaggcgcag cggtcgggct gaacgggggg ttcgtgcaca cagcccagct 3600

tggagcgaac gacctacacc gaactgagat acctacagcg tgagctatga gaaagcgcca 3660

cgcttcccga agggagaaag gcggacaggt atccggtaag cggcagggtc ggaacaggag 3720

agcgcacgag ggagcttcca gggggaaacg cctggtatct ttatagtcct gtcgggtttc 3780

gccacctctg acttgagcgt cgatttttgt gatgctcgtc aggggggcgg agcctatgga 3840

aaaacgccag caacgcggcc tttttacggt tcctggcctt ttgctggcct tttgctcaca 3900

tgttctttcc tgcgttatcc cctgattctg tggataaccg tattaccgcc tttgagtgag 3960

ctgataccgc tcgccgcagc cgaacgaccg agcgcagcga gtcagtgagc gaggaagcgg 4020

aagagcgccc aatacgcaaa ccgcctctcc ccgcgcgttg gccgattcat taatgcagct 4080

ggcacgacag gtttcccgac tggaaagcgg gcagtgagcg caacgcaatt aatgtgagtt 4140

agctcactca ttaggcaccc caggctttac actttatgct tccggctcgt acgcaactaa 4200

catgaatgaa tacgatatac atcaaagact atgatacgca gtattgcaca ctgtacgagt 4260

aagagcacta gccactgcac tcaagtgaaa ccgttgcccg ggtacgagta tgagtatgta 4320

cagtatgttt agtattgtac ttggacagtg cttgtatcgt acattctcaa gtgtcaaaca 4380

taaatatccg ttgctatatc ctcgcaccac cacgtagctc gctatatccc tgtgttgaat 4440

ccatccatct tggattgcca attgtgcaca cagaaccggg cactcacttc cccatccaca 4500

cttgcggccg cttagacctt tcgctttttc ttgggatcgg ctctggagtc gccaccaagc 4560

tgagacaggt cgattcgggt ctcgtacagg ccagtgatgg actggtgaat cagggtggca 4620

tcgagaacct ccttggtgga tgtgtaccgc tttcggtcga tagtggtatc gaagtacttg 4680

aaagctgcag gagcacccag gttggtaaga gtaaacaggt gaatgatgtt ctccgcctgt 4740

tctcgaatgg gtttgtcccg atgcttgttg taggcagaga gcaccttgtc caagttggca 4800

tcagccagga tgactcgctt cgaaaactcg gaaatctgct cgataatctc gtcgaggtaa 4860

tgtttgtgct gctcaacgaa gagttgcttc tgttcgttgt cctcgggaga acccttgagc 4920

ttctcgtagt gagaagccag atagagaaag ttgacgtact tcgaaggcaa ggcaagctcg 4980

tttcccttct gcagctcgcc agcggaggcg agcatacgct ttcgaccgtt ctccagttcg 5040

aacagagagt acttgggcag cttgataatg aggtctttct tgacctcctt gtaacccttg 5100

gcttccaaga agtcgatggg attcttctcg aagctcgatc gctccatgat ggtaattccg 5160

agcagctcct tgacggactt gagctttttg gacttgccct tctcgacctt cgcaacgaca 5220

agcacggaat aggcgacggt aggagaatcg aagccaccgt atttcttggg atcccagtct 5280

ttctttcgag cgatgagctt gtcggagttt cgcttgggca gaatcgactc cttggagaat 5340

ccgccagtct gaacctcggt tttcttgacg atgttgacct gaggcatcga cagaaccttt 5400

cgcacggttg caaagtctcg acccttgtcc cacacgatct ctccagtttc gccgttggtc 5460

tcgataagtg gtctctttcg aatctctccg ttggccaagg tgatctcggt cttgaaaaag 5520

ttcatgatgt tggagtaaaa gaagtacttg gcagtagcct tgccaatctc ctgttcggac 5580

ttggcaatca tctttcgaac gtcgtagacc ttgtaatcgc cgtaaacgaa ctcgctttcg 5640

agcttggggt atttcttgat gagcgcagtg ccaacgacgg cgttgaggta agcatcgtgg 5700

gcatggtggt aattgttgat ctctcgcacc ttgtagaact gaaagtcctt tcggaaatcg 5760

gagaccagtt tggacttgag agtaatcacc ttgacctctc ggatgagctt gtcgttctcg 5820

tcgtacttgg tgttcatccg agaatcgaga atctgtgcga cgtgctttgt gatctgtctg 5880

gtctcgacga gttgacgctt gatgaagcca gccttgtcga gctcggacag accgcctcgc 5940

tcggccttgg taagattgtc gaactttcgc tgggtaatga gcttggcgtt gagcagctgt 6000

cgccagtagt tcttcatctt tttgaccacc tcttcgctgg gaacgttgtc cgacttgcct 6060

ctgttcttgt cggatcgtgt aaggaccttg ttgtcgatag aatcgtcctt gagaaaggat 6120

tgagggacaa tgtggtccac atcgtagtcg ctgagacgat tgatgtccag ttcctgatcc 6180

acgtacatgt ctcgaccatt ctgcagatag tagagataca gcttctcgtt ctgcagttga 6240

gtgttctcga cgggatgctc cttgagaatc tgggatccca gctccttgat gccttcctcg 6300

attcgcttca tccgctctcg cgagtttttc tgaccctttt gagttgtctg gttctctctg 6360

gccatctcga tcacaatgtt ctcgggcttg tgacgtccca tgaccttcac cagctcgtcg 6420

acaaccttga cagtctggag aatgcctttc ttgatggctg gcgaaccagc caggttggca 6480

atatgttcgt gcaagctgtc gccctgaccg gacacttgtg ccttctggat gtcctccttg 6540

aaggtaagag aatcgtcgtg aatgagctgc atgaagtttc ggttggcaaa gccatcggac 6600

ttgagaaagt ccagaatggt ctttccggac tgcttgtctc tgatgccgtt gatgagcttt 6660

cgcgaaagtc ttccccagcc ggtgtatcta cgtcgcttga gttgtttcat gaccttgtcg 6720

tcgaacaggt gagcgtatgt cttgagtcgt tcctcgatca tctcccgatc ttcgaacagg 6780

gtaagagtga gcacgatgtc ctccagaatg tcctcgtttt cctcgttgtc gagaaaatcc 6840

ttgtccttga taatcttgag cagatcgtga taggtgccca aagaggcgtt gaatcggtcc 6900

tcaactccgg aaatctcgac gctgtcgaaa cactcgattt tcttgaagta gtcctccttg 6960

agctgcttaa cagtgacctt tcggttggtc ttgaacagga gatcgacaat ggctttcttc 7020

tgttcgccag acaagaaggc aggctttcgc attccctcgg taacgtactt gactttggtg 7080

agttcgttgt agactgtaaa gtactcgtag agcagcgaat gcttgggaag aaccttctcg 7140

ttgggcagat tcttgtcgaa gttggtcatt cgctcgatga aggactgtgc agaggcaccc 7200

ttgtccacga cttcctcgaa gttccaggga gtgatggttt cctcggactt tcgagtcatc 7260

caagcaaatc gagagtttcc tctggcaaga ggaccaacat agtaggggat tcgaaaggta 7320

agaatcttct cgatcttctc tcggttgtcc ttgagaaagg ggtagaagtc ttcctgacgt 7380

cgaagaatgg cgtgcagctc accgaggtgg atctgatgag gaatgctgcc gttgtcgaag 7440

gttcgttgct tccgaagcag atcctctcga ttgagcttga caagcagttc ctcggttccg 7500

tccatcttct cgagaattgg cttgatgaac ttgtagaact cttcctgaga ggctccgccg 7560

tcgatgtatc cagcgtagcc gttcttcgac tgatcgaaaa agatctcctt gtacttctcg 7620

ggcagttgct gtcggacaag agccttgagc agtgtgagat cctgatggtg ctcgtcgtat 7680

cgcttgatca tggaggcaga aaggggagcc tttgtgatct cggtgttgac tcgcagaatg 7740

tcagacaaga gaatagcatc cgaaaggttc ttggcagcga gaaacaggtc ggcgtactga 7800

tcgccaatct gtgcaagcag gttgtcgagg tcatcgtcgt aggtgtcctt ggacagctgg 7860

agcttggcgt cctccgccag atcgaagttg gacttgaagt tgggtgtgag accaagagaa 7920

agggcaatga ggttgccaaa cagtccgttc tttttctcgc caggaagttg ggcaatgagg 7980

ttctccagtc gtctgctctt cgagagtcga gcagacaaga tggcctttgc atcgactccg 8040

gaggcattga tggggttttc ctcgaacagc tggttgtagg tctgaacgag ctgaatgaac 8100

agcttgtcca catcgctgtt gtcgggattg agatcgccct cgatgaggaa atgacctcga 8160

aacttgatca tgtgtgccag agcgaggtag ataagtctga gatccgcctt gtcggtggaa 8220

tcgacgagtt tctttcggag atggtagatg gtaggatact tctcgtggta agcaacctcg 8280

tccacaatgt tgccaaagat gggatgacgc tcgtgtttct tgtcttcctc gacgaggaag 8340

gattcctcca gtcgatgaaa gaacgaatcg tccaccttgg ccatctcgtt ggaaaagatc 8400

tcctgcaggt agcagattcg gttcttccgt cgggtgtaac gtcgccgagc agttcgcttg 8460

agtctggtag cttcggcagt ctcgccagaa tcgaacaaca gggcaccaat gaggtttttc 8520

ttgatggagt gtcgatcggt gtttccgagg accttgaatt tcttggaggg caccttgtac 8580

tcgtcggtga tgacagccca gccgacagag ttggttccaa tgtccaggcc gatggagtat 8640

ttcttgtcca tggtgtgatg tgtagtttag atttcgaatc tgtggggaaa gaaaggaaaa 8700

aagagactgg caaccgattg ggagagccac tgtttatata taccctagac aagccccccg 8760

cttgtaagat gttggtcaat gtaaaccagt attaaggttg gcaagtgcag gagaagcaag 8820

gtgtgggtac cgagcaatgg aaatgtgcgg aaggcaaaaa aatgaggcca cggcctattg 8880

tcggggctat atccaggggg cgattgaagt acactaacat gacatgtgtc cacagaccct 8940

caatctggcc tgatgagcca aatccatacg cgctttcgca gctctaaagg ctataacaag 9000

tcacaccacc ctgctcgacc tcagcgccct cactttttgt taagacaaac tgtacacgct 9060

gttccagcgt tttctgcctg cacctggtgg gacatttggt gcaacctaaa gtgctcggaa 9120

cctctgtggt gtccagatca gcgcagcagt tccgaggtag ttttgaggcc cttagatgat 9180

ggtttaaacc ttaagcccgc tcataacttc gtatagcata cattatacga acggtaggtt 9240

gcgggataga cgccgacgga gggcaatggc gctatggaac cttgcggata tccatacgcc 9300

gcggcggact gcgtccgaac cagctccagc agcgtttttt ccgggccatt gagccgactg 9360

cgaccccgcc aacgtgtctt ggcccacgca ctcatgtcat gttggtgttg ggaggccact 9420

ttttaagtag cacaaggcac ctagctcgca gcaaggtgtc cgaaccaaag aagcggctgc 9480

agtggtgcaa acggggcgga aacggcggga aaaagccacg ggggcacgaa ttgaggcacg 9540

ccctcgaatt tgagacgagt cacggcccca ttcgcccgcg caatggctcg ccaacgcccg 9600

gtcttttgca ccacatcagg ttaccccaag ccaaaccttt gtgttaaaaa gcttaacata 9660

ttataccgaa cgtaggtttg ggcgggcttg ctccgtctgt ccaaggcaac atttatataa 9720

gggtctgcat cgccggctca attgaatctt ttttcttctt ctcttctcta tattcattct 9780

tgaattaaac acacatcaac catggccaaa aagcctgaac tcaccgcgac gtctgtcgag 9840

aagtttctga tcgaaaagtt cgacagcgtc tccgacctga tgcagctctc ggagggcgaa 9900

gaatctcgtg ctttcagctt cgatgtagga gggcgtggat atgtcctgcg ggtaaatagc 9960

tgcgccgatg gtttctacaa agatcgttat gtttatcggc actttgcatc ggccgcgctc 10020

ccgattccgg aagtgcttga cattggggag ttcagcgaga gcctgaccta ttgcatctcc 10080

cgccgtgcac agggtgtcac gttgcaagac ctgcctgaaa ccgaactgcc cgctgttctg 10140

cagccggtcg cggaggctat ggatgcgatc gctgcggccg atcttagcca gacgagcggg 10200

ttcggcccat tcggaccgca aggaatcggt caatacacta catggcgtga tttcatatgc 10260

gcgattgctg atccccatgt gtatcactgg caaactgtga tggacgacac cgtcagtgcg 10320

tccgtcgcgc aggctctcga tgagctgatg ctttgggccg aggactgccc cgaagtccgg 10380

cacctcgtgc acgcggattt cggctccaac aatgtcctga cggacaatgg ccgcataaca 10440

gcggtcattg actggagcga ggcgatgttc ggggattccc aatacgaggt cgccaacatc 10500

ttcttctgga ggccgtggtt ggcttgtatg gagcagcaga cgcgctactt cgagcggagg 10560

catccggagc ttgcaggatc gccgcggctc cgggcgtata tgctccgcat tggtcttgac 10620

caactctatc agagcttggt tgacggcaat ttcgatgatg cagcttgggc gcagggtcga 10680

tgcgacgcaa tcgtccgatc cggagccggg actgtcgggc gtacacaaat cgcccgcaga 10740

agcgcggccg tctggaccga tggctgtgta gaagtactcg ccgatagtgg aaaccgacgc 10800

cccagcactc gtccgagggc aaaggaatag cggccgcaag tgtggatggg gaagtgagtg 10860

cccggttctg tgtgcacaat tggcaatcca agatggatgg attcaacaca gggatatagc 10920

gagctacgtg gtggtgcgag gatatagcaa cggatattta tgtttgacac ttgagaatgt 10980

acgatacaag cactgtccaa gtacaatact aaacatactg tacatactca tactcgtacc 11040

cgggcaacgg tttcacttga gtgcagtggc tagtgctctt actcgtacag tgtgctaccg 11100

ttcgtatagc atacattata cgaagttatc atagtcttaa ttaaagttat tggttgacat 11160

tgttttttcc attctgtttt tttttttttt tttttttttt tttttaattc taatgattca 11220

ttacagctat atacctagta agccgggtta ttggcgttca ataaatcata cacttctgaa 11280

tctttgcgat gtgcgctgcg aggcgctgtc ggttggtttg tcgacgcttc gtctagttga 11340

tcacattgtc gtggtctcga ggtagtgaat catggctaac agatcgagaa atcatatcaa 11400

tcgtttcttg tcttatccag actcgaggtt ggtttagttt gttctgatta cataatcaga 11460

aaacaacggc tctggtacaa aaccactttg tgttcagcgt ccgcctggct agctcaatcg 11520

gtagagcgtg agactcttat acaagaaatc tcaaggctgt gggttcaagc cccacgtcgg 11580

gctagccgct cgagtgctca agctcggttt tagagctaga aatagcaagt taaaataagg 11640

ctagtccgtt atcaacttga aaaagtggca ccgagtcggt gctttttttt ttgtttttta 11700

tcg 11703

<210> 34
<211> 209
<212> DNA
<213> Artificial Sequence

<220>
<223> Artificial Sequence

<400> 34
ttaattaagc ctggctagct caatcggtag agcgtgagac tcttatacaa gaaatctcaa 60

ggctgtgggt tcaagcccca cgtcgggcta gtctcaccga catggttatc gttttagagc 120

tagaaatagc aagttaaaat aaggctagtc cgttatcaac ttgaaaaagt ggcaccgagt 180

cggtgctttt tttttgtttt tttatcgat 209

<210> 35
<211> 20
<212> DNA
<213> Artificial Sequence

<220>
<223> Artificial Sequence

<400>  35
gtctcaccga catggttatc                                                    20


<210>  36
<211>  11343
<212>  DNA
<213>  Artificial Sequence

<220>
<223>  Artificial Sequence

<400>  36
attgagcatc cgttgatttc cgaacagatc ccaatattac acccaagtag catgcataag        60

ctaaaagtaa ctcgcagcgc acaccgtgca gattcataag tctatgatta attgaacgcc       120

aataacccgg cttactacaa gtacaagtag gtatacatag cggtaatgaa tcattagaaa       180

aaaaaaaaac aaaaaaaaac aaaacaaact gttgtggatg catcaacagt agtacatagt       240

tgtacgatgt acttgtactt gtaaaagcaa aaatgtacaa tatctcaggg agcgcaactt       300

ttacgttcga agaacaatgt accgcatacc gcattctaga ttctgcggaa cgtctaacct       360

ggaaatacga tttttttttt ctttcatttt ttttgcttct tcaaaagtat ggtaatttcc       420

taccattaca gttgacactg aacgaggggg gattgaattt aagcaaaaaa ttaaatcaaa       480

atacctttat gtatccagcc catgtaataa acaaaaggat tatataacaa gaaataaata       540

tataccttta atggatcatt agaataaaaa taaatacgag aagcacacca gagaagcttt       600

ttgattgcca ctataccgct actttggtat atcttattat aattgttgaa tttgcaagat       660

agaatgtcat tcattggaga gaaatccaag gaatatgtgg gatgaaatga ctagaagtat       720

gaacaatgag aatagtacat acttgtacct gtatttctag aagagagaaa gacagttgag       780

tgtgtgattc tcgtccaata ataatctcaa tagtaacgtg tgaatagctg ttctttgata       840

gttgatattt ctcgatgact atttatgttg tacaagggat ttttttcgtt gctgttgatt       900

tcgaattagg caatgcagat atcatttatg ctatccatat ttaagatttc ccatacgcat       960

ttataacatt tattctacat aaattgttaa atgaacgaac tgccattata aattgtttcc      1020

taaataggaa gtgtttttca taaagcaagt aagttgtcta ataatactaa gtaataaaaa      1080

taagttcata caatatattt tgagaacatc atttggaggc ggtagatgga gtctgtttat      1140

tattaaacaa tgcgagatga ccccttaaat attgagaaca tcagttggag gcggcagatg      1200

gagtctgtct atttagcaat gggacatgac tgtcagtatc atcatgatgt atatatataa      1260

tacatataat attatataac acgatttttt taaattattg gcccgaaaat taatcagtgt      1320

agactggatc tcggcagtct ctcggatgta gaattaggtt tccttgaggc gaagatcggt      1380

ttgtgtgaca tgaattcgat atcaagctta tcgacaccat cgacctcgag ggggggcccg       1440

gtacccaatt cgccctatag tgagtcgtat tacaattcac tggccgtcgt tttacaacgt      1500

cgtgactggg aaaaccctgg cgttacccaa cttaatcgcc ttgcagcaca tccccctttc 1560

gccagctggc gtaatagcga agaggcccgc accgatcgcc cttcccaaca gttgcgcagc 1620

ctgaatggcg aatggaaatt gtaagcgtta atattttgtt aaaattcgcg ttaaattttt 1680

gttaaatcag ctcatttttt aaccaatagg ccgaaatcgg caaaatccct tataaatcaa 1740

aagaatagac cgagataggg ttgagtgttg ttccagtttg gaacaagagt ccactattaa 1800

agaacgtgga ctccaacgtc aaagggcgaa aaaccgtcta tcagggcgat ggcccactac 1860

gtgaaccatc accctaatca agttttttgg ggtcgaggtg ccgtaaagca ctaaatcgga 1920

accctaaagg gagcccccga tttagagctt gacggggaaa gccggcgaac gtggcgagaa 1980

aggaagggaa gaaagcgaaa ggagcgggcg ctagggcgct ggcaagtgta gcggtcacgc 2040

tgcgcgtaac caccacaccc gccgcgctta atgcgccgct acagggcgcg tcaggtggca 2100

cttttcgggg aaatgtgcgc ggaaccccta tttgtttatt tttctaaata cattcaaata 2160

tgtatccgct catgagacaa taaccctgat aaatgcttca ataatattga aaaaggaaga 2220

gtatgagtat tcaacatttc cgtgtcgccc ttattccctt ttttgcggca ttttgccttc 2280

ctgtttttgc tcacccagaa acgctggtga aagtaaaaga tgctgaagat cagttgggtg 2340

cacgagtggg ttacatcgaa ctggatctca acagcggtaa gatccttgag agttttcgcc 2400

ccgaagaacg ttttccaatg atgagcactt ttaaagttct gctatgtggc gcggtattat 2460

cccgtattga cgccgggcaa gagcaactcg gtcgccgcat acactattct cagaatgact 2520

tggttgagta ctcaccagtc acagaaaagc atcttacgga tggcatgaca gtaagagaat 2580

tatgcagtgc tgccataacc atgagtgata acactgcggc caacttactt ctgacaacga 2640

tcggaggacc gaaggagcta accgcttttt tgcacaacat gggggatcat gtaactcgcc 2700

ttgatcgttg ggaaccggag ctgaatgaag ccataccaaa cgacgagcgt gacaccacga 2760

tgcctgtagc aatggcaaca acgttgcgca aactattaac tggcgaacta cttactctag 2820

cttcccggca acaattaata gactggatgg aggcggataa agttgcagga ccacttctgc 2880

gctcggccct tccggctggc tggtttattg ctgataaatc tggagccggt gagcgtgggt 2940

ctcgcggtat cattgcagca ctggggccag atggtaagcc ctcccgtatc gtagttatct 3000

acacgacggg gagtcaggca actatggatg aacgaaatag acagatcgct gagataggtg 3060

cctcactgat taagcattgg taactgtcag accaagttta ctcatatata ctttagattg 3120

atttaaaact tcatttttaa tttaaaagga tctaggtgaa gatccttttt gataatctca 3180

tgaccaaaat cccttaacgt gagttttcgt tccactgagc gtcagacccc gtagaaaaga 3240

tcaaaggatc ttcttgagat cctttttttc tgcgcgtaat ctgctgcttg caaacaaaaa 3300

aaccaccgct accagcggtg gtttgtttgc cggatcaaga gctaccaact ctttttccga 3360

aggtaactgg cttcagcaga gcgcagatac caaatactgt ccttctagtg tagccgtagt 3420

taggccacca cttcaagaac tctgtagcac cgcctacata cctcgctctg ctaatcctgt 3480

taccagtggc tgctgccagt ggcgataagt cgtgtcttac cgggttggac tcaagacgat 3540

agttaccgga taaggcgcag cggtcgggct gaacgggggg ttcgtgcaca cagcccagct 3600

tggagcgaac gacctacacc gaactgagat acctacagcg tgagctatga gaaagcgcca 3660

cgcttcccga agggagaaag gcggacaggt atccggtaag cggcagggtc ggaacaggag 3720

agcgcacgag ggagcttcca gggggaaacg cctggtatct ttatagtcct gtcgggtttc 3780

gccacctctg acttgagcgt cgatttttgt gatgctcgtc aggggggcgg agcctatgga 3840

aaaacgccag caacgcggcc tttttacggt tcctggcctt ttgctggcct tttgctcaca 3900

tgttctttcc tgcgttatcc cctgattctg tggataaccg tattaccgcc tttgagtgag 3960

ctgataccgc tcgccgcagc cgaacgaccg agcgcagcga gtcagtgagc gaggaagcgg 4020

aagagcgccc aatacgcaaa ccgcctctcc ccgcgcgttg gccgattcat taatgcagct 4080

ggcacgacag gtttcccgac tggaaagcgg gcagtgagcg caacgcaatt aatgtgagtt 4140

agctcactca ttaggcaccc caggctttac actttatgct tccggctcgt acgcaactaa 4200

catgaatgaa tacgatatac atcaaagact atgatacgca gtattgcaca ctgtacgagt 4260

aagagcacta gccactgcac tcaagtgaaa ccgttgcccg ggtacgagta tgagtatgta 4320

cagtatgttt agtattgtac ttggacagtg cttgtatcgt acattctcaa gtgtcaaaca 4380

taaatatccg ttgctatatc ctcgcaccac cacgtagctc gctatatccc tgtgttgaat 4440

ccatccatct tggattgcca attgtgcaca cagaaccggg cactcacttc cccatccaca 4500

cttgcggccg cttagacctt tcgctttttc ttgggatcgg ctctggagtc gccaccaagc 4560

tgagacaggt cgattcgggt ctcgtacagg ccagtgatgg actggtgaat cagggtggca 4620

tcgagaacct ccttggtgga tgtgtaccgc tttcggtcga tagtggtatc gaagtacttg 4680

aaagctgcag gagcacccag gttggtaaga gtaaacaggt gaatgatgtt ctccgcctgt 4740

tctcgaatgg gtttgtcccg atgcttgttg taggcagaga gcaccttgtc caagttggca 4800

tcagccagga tgactcgctt cgaaaactcg gaaatctgct cgataatctc gtcgaggtaa 4860

tgtttgtgct gctcaacgaa gagttgcttc tgttcgttgt cctcgggaga acccttgagc 4920

ttctcgtagt gagaagccag atagagaaag ttgacgtact tcgaaggcaa ggcaagctcg 4980

tttcccttct gcagctcgcc agcggaggcg agcatacgct ttcgaccgtt ctccagttcg 5040

aacagagagt acttgggcag cttgataatg aggtctttct tgacctcctt gtaacccttg 5100

gcttccaaga agtcgatggg attcttctcg aagctcgatc gctccatgat ggtaattccg 5160

agcagctcct tgacggactt gagctttttg gacttgccct tctcgacctt cgcaacgaca 5220

agcacggaat aggcgacggt aggagaatcg aagccaccgt atttcttggg atcccagtct 5280

ttctttcgag cgatgagctt gtcggagttt cgcttgggca gaatcgactc cttggagaat 5340

ccgccagtct gaacctcggt tttcttgacg atgttgacct gaggcatcga cagaaccttt 5400

cgcacggttg caaagtctcg acccttgtcc cacacgatct ctccagtttc gccgttggtc 5460

tcgataagtg gtctctttcg aatctctccg ttggccaagg tgatctcggt cttgaaaaag 5520

ttcatgatgt tggagtaaaa gaagtacttg gcagtagcct tgccaatctc ctgttcggac 5580

ttggcaatca tctttcgaac gtcgtagacc ttgtaatcgc cgtaaacgaa ctcgctttcg 5640

agcttggggt atttcttgat gagcgcagtg ccaacgacgg cgttgaggta agcatcgtgg 5700

gcatggtggt aattgttgat ctctcgcacc ttgtagaact gaaagtcctt tcggaaatcg 5760

gagaccagtt tggacttgag agtaatcacc ttgacctctc ggatgagctt gtcgttctcg 5820

tcgtacttgg tgttcatccg agaatcgaga atctgtgcga cgtgctttgt gatctgtctg 5880

gtctcgacga gttgacgctt gatgaagcca gccttgtcga gctcggacag accgcctcgc 5940

tcggccttgg taagattgtc gaactttcgc tgggtaatga gcttggcgtt gagcagctgt 6000

cgccagtagt tcttcatctt tttgaccacc tcttcgctgg gaacgttgtc cgacttgcct 6060

ctgttcttgt cggatcgtgt aaggaccttg ttgtcgatag aatcgtcctt gagaaaggat 6120

tgagggacaa tgtggtccac atcgtagtcg ctgagacgat tgatgtccag ttcctgatcc 6180

acgtacatgt ctcgaccatt ctgcagatag tagagataca gcttctcgtt ctgcagttga 6240

gtgttctcga cgggatgctc cttgagaatc tgggatccca gctccttgat gccttcctcg 6300

attcgcttca tccgctctcg cgagtttttc tgaccctttt gagttgtctg gttctctctg 6360

gccatctcga tcacaatgtt ctcgggcttg tgacgtccca tgaccttcac cagctcgtcg 6420

acaaccttga cagtctggag aatgcctttc ttgatggctg gcgaaccagc caggttggca 6480

atatgttcgt gcaagctgtc gccctgaccg gacacttgtg ccttctggat gtcctccttg 6540

aaggtaagag aatcgtcgtg aatgagctgc atgaagtttc ggttggcaaa gccatcggac 6600

ttgagaaagt ccagaatggt ctttccggac tgcttgtctc tgatgccgtt gatgagcttt 6660

cgcgaaagtc ttccccagcc ggtgtatcta cgtcgcttga gttgtttcat gaccttgtcg 6720

tcgaacaggt gagcgtatgt cttgagtcgt tcctcgatca tctcccgatc ttcgaacagg 6780

gtaagagtga gcacgatgtc ctccagaatg tcctcgtttt cctcgttgtc gagaaaatcc 6840

ttgtccttga taatcttgag cagatcgtga taggtgccca aagaggcgtt gaatcggtcc 6900

tcaactccgg aaatctcgac gctgtcgaaa cactcgattt tcttgaagta gtcctccttg 6960

agctgcttaa cagtgacctt tcggttggtc ttgaacagga gatcgacaat ggctttcttc 7020

tgttcgccag acaagaaggc aggctttcgc attccctcgg taacgtactt gactttggtg 7080

agttcgttgt agactgtaaa gtactcgtag agcagcgaat gcttgggaag aaccttctcg 7140

ttgggcagat tcttgtcgaa gttggtcatt cgctcgatga aggactgtgc agaggcaccc 7200

ttgtccacga cttcctcgaa gttccaggga gtgatggttt cctcggactt tcgagtcatc 7260

caagcaaatc gagagtttcc tctggcaaga ggaccaacat agtaggggat tcgaaaggta 7320

agaatcttct cgatcttctc tcggttgtcc ttgagaaagg ggtagaagtc ttcctgacgt 7380

cgaagaatgg cgtgcagctc accgaggtgg atctgatgag gaatgctgcc gttgtcgaag 7440

gttcgttgct tccgaagcag atcctctcga ttgagcttga caagcagttc ctcggttccg 7500

tccatcttct cgagaattgg cttgatgaac ttgtagaact cttcctgaga ggctccgccg 7560

tcgatgtatc cagcgtagcc gttcttcgac tgatcgaaaa agatctcctt gtacttctcg 7620

ggcagttgct gtcggacaag agccttgagc agtgtgagat cctgatggtg ctcgtcgtat 7680

cgcttgatca tggaggcaga aaggggagcc tttgtgatct cggtgttgac tcgcagaatg 7740

tcagacaaga gaatagcatc cgaaaggttc ttggcagcga gaaacaggtc ggcgtactga 7800

tcgccaatct gtgcaagcag gttgtcgagg tcatcgtcgt aggtgtcctt ggacagctgg 7860

agcttggcgt cctccgccag atcgaagttg gacttgaagt tgggtgtgag accaagagaa 7920

agggcaatga ggttgccaaa cagtccgttc tttttctcgc caggaagttg ggcaatgagg 7980

ttctccagtc gtctgctctt cgagagtcga gcagacaaga tggcctttgc atcgactccg 8040

gaggcattga tggggttttc ctcgaacagc tggttgtagg tctgaacgag ctgaatgaac 8100

agcttgtcca catcgctgtt gtcgggattg agatcgccct cgatgaggaa atgacctcga 8160

aacttgatca tgtgtgccag agcgaggtag ataagtctga gatccgcctt gtcggtggaa 8220

tcgacgagtt tctttcggag atggtagatg gtaggatact tctcgtggta agcaacctcg 8280

tccacaatgt tgccaaagat gggatgacgc tcgtgtttct tgtcttcctc gacgaggaag 8340

gattcctcca gtcgatgaaa gaacgaatcg tccaccttgg ccatctcgtt ggaaaagatc 8400

tcctgcaggt agcagattcg gttcttccgt cgggtgtaac gtcgccgagc agttcgcttg 8460

agtctggtag cttcggcagt ctcgccagaa tcgaacaaca gggcaccaat gaggtttttc 8520

ttgatggagt gtcgatcggt gtttccgagg accttgaatt tcttggaggg caccttgtac 8580

tcgtcggtga tgacagccca gccgacagag ttggttccaa tgtccaggcc gatggagtat 8640

ttcttgtcca tggtgtgatg tgtagtttag atttcgaatc tgtggggaaa gaaaggaaaa 8700

aagagactgg caaccgattg ggagagccac tgtttatata taccctagac aagccccccg 8760

cttgtaagat gttggtcaat gtaaaccagt attaaggttg gcaagtgcag gagaagcaag 8820

gtgtgggtac cgagcaatgg aaatgtgcgg aaggcaaaaa aatgaggcca cggcctattg 8880

tcggggctat atccaggggg cgattgaagt acactaacat gacatgtgtc cacagaccct 8940

caatctggcc tgatgagcca aatccatacg cgctttcgca gctctaaagg ctataacaag 9000

tcacaccacc ctgctcgacc tcagcgccct cactttttgt taagacaaac tgtacacgct 9060

gttccagcgt tttctgcctg cacctggtgg gacatttggt gcaacctaaa gtgctcggaa 9120

cctctgtggt gtccagatca gcgcagcagt tccgaggtag ttttgaggcc cttagatgat 9180

ggtttaaacc ttaagcccgc tcataacttc gtatagcata cattatacga acggtaggtt 9240

gcgggataga cgccgacgga gggcaatggc gctatggaac cttgcggata tccatacgcc 9300

gcggcggact gcgtccgaac cagctccagc agcgtttttt ccgggccatt gagccgactg 9360

cgaccccgcc aacgtgtctt ggcccacgca ctcatgtcat gttggtgttg ggaggccact 9420

ttttaagtag cacaaggcac ctagctcgca gcaaggtgtc cgaaccaaag aagcggctgc 9480

agtggtgcaa acggggcgga aacggcggga aaaagccacg ggggcacgaa ttgaggcacg 9540

ccctcgaatt tgagacgagt cacggcccca ttcgcccgcg caatggctcg ccaacgcccg 9600

gtcttttgca ccacatcagg ttaccccaag ccaaaccttt gtgttaaaaa gcttaacata 9660

ttataccgaa cgtaggtttg ggcgggcttg ctccgtctgt ccaaggcaac atttatataa 9720

gggtctgcat cgccggctca attgaatctt ttttcttctt ctcttctcta tattcattct 9780

tgaattaaac acacatcaac catggccaaa aagcctgaac tcaccgcgac gtctgtcgag 9840

aagtttctga tcgaaaagtt cgacagcgtc tccgacctga tgcagctctc ggagggcgaa 9900

gaatctcgtg ctttcagctt cgatgtagga gggcgtggat atgtcctgcg ggtaaatagc 9960

tgcgccgatg gtttctacaa agatcgttat gtttatcggc actttgcatc ggccgcgctc 10020

ccgattccgg aagtgcttga cattggggag ttcagcgaga gcctgaccta ttgcatctcc 10080

cgccgtgcac agggtgtcac gttgcaagac ctgcctgaaa ccgaactgcc cgctgttctg 10140

cagccggtcg cggaggctat ggatgcgatc gctgcggccg atcttagcca gacgagcggg 10200

ttcggcccat tcggaccgca aggaatcggt caatacacta catggcgtga tttcatatgc 10260

gcgattgctg atccccatgt gtatcactgg caaactgtga tggacgacac cgtcagtgcg 10320

tccgtcgcgc aggctctcga tgagctgatg ctttgggccg aggactgccc cgaagtccgg 10380

cacctcgtgc acgcggattt cggctccaac aatgtcctga cggacaatgg ccgcataaca 10440

gcggtcattg actggagcga ggcgatgttc ggggattccc aatacgaggt cgccaacatc 10500

ttcttctgga ggccgtggtt ggcttgtatg gagcagcaga cgcgctactt cgagcggagg 10560

catccggagc ttgcaggatc gccgcggctc cgggcgtata tgctccgcat tggtcttgac 10620

caactctatc agagcttggt tgacggcaat ttcgatgatg cagcttgggc gcagggtcga 10680

tgcgacgcaa tcgtccgatc cggagccggg actgtcgggc gtacacaaat cgcccgcaga 10740

agcgcggccg tctggaccga tggctgtgta gaagtactcg ccgatagtgg aaaccgacgc 10800

cccagcactc gtccgagggc aaaggaatag cggccgcaag tgtggatggg gaagtgagtg 10860

cccggttctg tgtgcacaat tggcaatcca agatggatgg attcaacaca gggatatagc 10920

gagctacgtg gtggtgcgag gatatagcaa cggatattta tgtttgacac ttgagaatgt 10980

acgatacaag cactgtccaa gtacaatact aaacatactg tacatactca tactcgtacc 11040

cgggcaacgg tttcacttga gtgcagtggc tagtgctctt actcgtacag tgtgctaccg 11100

ttcgtatagc atacattata cgaagttatc atagtcttaa ttaagcctgg ctagctcaat 11160

cggtagagcg tgagactctt atacaagaaa tctcaaggct gtgggttcaa gccccacgtc 11220

gggctagtct caccgacatg gttatcgttt tagagctaga aatagcaagt taaaataagg 11280

ctagtccgtt atcaacttga aaaagtggca ccgagtcggt gctttttttt ttgtttttta 11340

tcg 11343

<210> 37
<211> 201
<212> DNA
<213> Artificial Sequence

<220>
<223> Artificial Sequence

<400> 37
   ttaattaagg tcgtgtggtg taatggttat cacgtcccgc tcacaccggg gaggctccga 60
   gttcgatcct cggcatgatc aagtctcacc gacatggtta tcgttttaga gctagaaata 120
   gcaagttaaa ataaggctag tccgttatca acttgaaaaa gtggcaccga gtcggtgctt 180
   ttttttttgt tttttatcga t 201

<210> 38
<211> 73
<212> DNA
<213> Artificial Sequence

<220>
<223> Artificial Sequence

<400> 38
ggtcgtgtgg tgtaatggtt atcacgtccc gctcacaccg gggaggctcc gagttcgatc 60

ctcggcatga tca 73

<210> 39
<211> 11335
<212> DNA
<213> Artificial Sequence

<220>
<223> Artificial Sequence

<400> 39
attgagcatc cgttgatttc cgaacagatc ccaatattac acccaagtag catgcataag 60

ctaaaagtaa ctcgcagcgc acaccgtgca gattcataag tctatgatta attgaacgcc 120

aataacccgg cttactacaa gtacaagtag gtatacatag cggtaatgaa tcattagaaa 180

aaaaaaaaac aaaaaaaaac aaaacaaact gttgtggatg catcaacagt agtacatagt 240

tgtacgatgt acttgtactt gtaaaagcaa aaatgtacaa tatctcaggg agcgcaactt 300

ttacgttcga agaacaatgt accgcatacc gcattctaga ttctgcggaa cgtctaacct 360

ggaaatacga tttttttttt ctttcatttt ttttgcttct tcaaaagtat ggtaatttcc 420

taccattaca gttgacactg aacgaggggg gattgaattt aagcaaaaaa ttaaatcaaa 480

atacctttat gtatccagcc catgtaataa acaaaaggat tatataacaa gaaataaata 540

tataccttta atggatcatt agaataaaaa taaatacgag aagcacacca gagaagcttt 600

ttgattgcca ctataccgct actttggtat atcttattat aattgttgaa tttgcaagat 660

agaatgtcat tcattggaga gaaatccaag gaatatgtgg gatgaaatga ctagaagtat 720

gaacaatgag aatagtacat acttgtacct gtatttctag aagagagaaa gacagttgag 780

tgtgtgattc tcgtccaata ataatctcaa tagtaacgtg tgaatagctg ttctttgata 840

gttgatattt ctcgatgact atttatgttg tacaagggat ttttttcgtt gctgttgatt 900

tcgaattagg caatgcagat atcatttatg ctatccatat ttaagatttc ccatacgcat 960

ttataacatt tattctacat aaattgttaa atgaacgaac tgccattata aattgtttcc 1020

taaataggaa gtgtttttca taaagcaagt aagttgtcta ataatactaa gtaataaaaa 1080

taagttcata caatatattt tgagaacatc atttggaggc ggtagatgga gtctgtttat 1140

tattaaacaa tgcgagatga ccccttaaat attgagaaca tcagttggag gcggcagatg 1200

gagtctgtct atttagcaat gggacatgac tgtcagtatc atcatgatgt atatatataa 1260

tacatataat attatataac acgatttttt taaattattg gcccgaaaat taatcagtgt 1320

agactggatc tcggcagtct ctcggatgta gaattaggtt tccttgaggc gaagatcggt 1380

ttgtgtgaca tgaattcgat atcaagctta tcgacaccat cgacctcgag ggggggcccg 1440

gtacccaatt cgccctatag tgagtcgtat tacaattcac tggccgtcgt tttacaacgt 1500

cgtgactggg aaaaccctgg cgttacccaa cttaatcgcc ttgcagcaca tccccctttc 1560

gccagctggc gtaatagcga agaggcccgc accgatcgcc cttcccaaca gttgcgcagc 1620

ctgaatggcg aatggaaatt gtaagcgtta atattttgtt aaaattcgcg ttaaattttt 1680

gttaaatcag ctcatttttt aaccaatagg ccgaaatcgg caaaatccct tataaatcaa 1740

aagaatagac cgagataggg ttgagtgttg ttccagtttg gaacaagagt ccactattaa 1800

agaacgtgga ctccaacgtc aaagggcgaa aaaccgtcta tcagggcgat ggcccactac 1860

gtgaaccatc accctaatca agttttttgg ggtcgaggtg ccgtaaagca ctaaatcgga 1920

accctaaagg gagcccccga tttagagctt gacggggaaa gccggcgaac gtggcgagaa 1980

aggaagggaa gaaagcgaaa ggagcgggcg ctagggcgct ggcaagtgta gcggtcacgc 2040

tgcgcgtaac caccacaccc gccgcgctta atgcgccgct acagggcgcg tcaggtggca 2100

cttttcgggg aaatgtgcgc ggaaccccta tttgtttatt tttctaaata cattcaaata 2160

tgtatccgct catgagacaa taaccctgat aaatgcttca ataatattga aaaaggaaga 2220

gtatgagtat tcaacatttc cgtgtcgccc ttattccctt ttttgcggca ttttgccttc 2280

ctgtttttgc tcacccagaa acgctggtga aagtaaaaga tgctgaagat cagttgggtg 2340

cacgagtggg ttacatcgaa ctggatctca acagcggtaa gatccttgag agttttcgcc 2400

ccgaagaacg ttttccaatg atgagcactt ttaaagttct gctatgtggc gcggtattat 2460

cccgtattga cgccgggcaa gagcaactcg gtcgccgcat acactattct cagaatgact 2520

tggttgagta ctcaccagtc acagaaaagc atcttacgga tggcatgaca gtaagagaat 2580

tatgcagtgc tgccataacc atgagtgata acactgcggc caacttactt ctgacaacga 2640

tcggaggacc gaaggagcta accgcttttt tgcacaacat gggggatcat gtaactcgcc 2700

ttgatcgttg ggaaccggag ctgaatgaag ccataccaaa cgacgagcgt gacaccacga 2760

tgcctgtagc aatggcaaca acgttgcgca aactattaac tggcgaacta cttactctag 2820

cttcccggca acaattaata gactggatgg aggcggataa agttgcagga ccacttctgc 2880

gctcggccct tccggctggc tggtttattg ctgataaatc tggagccggt gagcgtgggt 2940

ctcgcggtat cattgcagca ctggggccag atggtaagcc ctcccgtatc gtagttatct 3000

acacgacggg gagtcaggca actatggatg aacgaaatag acagatcgct gagataggtg 3060

cctcactgat taagcattgg taactgtcag accaagttta ctcatatata ctttagattg 3120

atttaaaact tcatttttaa tttaaaagga tctaggtgaa gatccttttt gataatctca 3180

tgaccaaaat cccttaacgt gagttttcgt tccactgagc gtcagacccc gtagaaaaga 3240

tcaaaggatc ttcttgagat cctttttttc tgcgcgtaat ctgctgcttg caaacaaaaa 3300

aaccaccgct accagcggtg gtttgtttgc cggatcaaga gctaccaact ctttttccga 3360

aggtaactgg cttcagcaga gcgcagatac caaatactgt ccttctagtg tagccgtagt 3420

taggccacca cttcaagaac tctgtagcac cgcctacata cctcgctctg ctaatcctgt 3480

taccagtggc tgctgccagt ggcgataagt cgtgtcttac cgggttggac tcaagacgat 3540

agttaccgga taaggcgcag cggtcgggct gaacgggggg ttcgtgcaca cagcccagct 3600

tggagcgaac gacctacacc gaactgagat acctacagcg tgagctatga gaaagcgcca 3660

cgcttcccga agggagaaag gcggacaggt atccggtaag cggcagggtc ggaacaggag 3720

agcgcacgag ggagcttcca gggggaaacg cctggtatct ttatagtcct gtcgggtttc 3780

gccacctctg acttgagcgt cgatttttgt gatgctcgtc aggggggcgg agcctatgga 3840

aaaacgccag caacgcggcc tttttacggt tcctggcctt ttgctggcct tttgctcaca 3900

tgttctttcc tgcgttatcc cctgattctg tggataaccg tattaccgcc tttgagtgag 3960

ctgataccgc tcgccgcagc cgaacgaccg agcgcagcga gtcagtgagc gaggaagcgg 4020

aagagcgccc aatacgcaaa ccgcctctcc ccgcgcgttg gccgattcat taatgcagct 4080

ggcacgacag gtttcccgac tggaaagcgg gcagtgagcg caacgcaatt aatgtgagtt 4140

agctcactca ttaggcaccc caggctttac actttatgct tccggctcgt acgcaactaa 4200

catgaatgaa tacgatatac atcaaagact atgatacgca gtattgcaca ctgtacgagt 4260

aagagcacta gccactgcac tcaagtgaaa ccgttgcccg ggtacgagta tgagtatgta 4320

cagtatgttt agtattgtac ttggacagtg cttgtatcgt acattctcaa gtgtcaaaca 4380

taaatatccg ttgctatatc ctcgcaccac cacgtagctc gctatatccc tgtgttgaat 4440

ccatccatct tggattgcca attgtgcaca cagaaccggg cactcacttc cccatccaca 4500

cttgcggccg cttagacctt tcgctttttc ttgggatcgg ctctggagtc gccaccaagc 4560

tgagacaggt cgattcgggt ctcgtacagg ccagtgatgg actggtgaat cagggtggca 4620

tcgagaacct ccttggtgga tgtgtaccgc tttcggtcga tagtggtatc gaagtacttg 4680

aaagctgcag gagcacccag gttggtaaga gtaaacaggt gaatgatgtt ctccgcctgt 4740

tctcgaatgg gtttgtcccg atgcttgttg taggcagaga gcaccttgtc caagttggca 4800

tcagccagga tgactcgctt cgaaaactcg gaaatctgct cgataatctc gtcgaggtaa 4860

tgtttgtgct gctcaacgaa gagttgcttc tgttcgttgt cctcgggaga acccttgagc 4920

ttctcgtagt gagaagccag atagagaaag ttgacgtact tcgaaggcaa ggcaagctcg 4980

tttcccttct gcagctcgcc agcggaggcg agcatacgct ttcgaccgtt ctccagttcg 5040

aacagagagt acttgggcag cttgataatg aggtctttct tgacctcctt gtaacccttg 5100

gcttccaaga agtcgatggg attcttctcg aagctcgatc gctccatgat ggtaattccg 5160

agcagctcct tgacggactt gagctttttg gacttgccct tctcgacctt cgcaacgaca 5220

agcacggaat aggcgacggt aggagaatcg aagccaccgt atttcttggg atcccagtct 5280

ttctttcgag cgatgagctt gtcggagttt cgcttgggca gaatcgactc cttggagaat 5340

ccgccagtct gaacctcggt tttcttgacg atgttgacct gaggcatcga cagaaccttt 5400

cgcacggttg caaagtctcg acccttgtcc cacacgatct ctccagtttc gccgttggtc 5460

tcgataagtg gtctctttcg aatctctccg ttggccaagg tgatctcggt cttgaaaaag 5520

ttcatgatgt tggagtaaaa gaagtacttg gcagtagcct tgccaatctc ctgttcggac 5580

ttggcaatca tctttcgaac gtcgtagacc ttgtaatcgc cgtaaacgaa ctcgctttcg 5640

agcttggggt atttcttgat gagcgcagtg ccaacgacgg cgttgaggta agcatcgtgg 5700

gcatggtggt aattgttgat ctctcgcacc ttgtagaact gaaagtcctt tcggaaatcg 5760

gagaccagtt tggacttgag agtaatcacc ttgacctctc ggatgagctt gtcgttctcg 5820

tcgtacttgg tgttcatccg agaatcgaga atctgtgcga cgtgctttgt gatctgtctg 5880

gtctcgacga gttgacgctt gatgaagcca gccttgtcga gctcggacag accgcctcgc 5940

tcggccttgg taagattgtc gaactttcgc tgggtaatga gcttggcgtt gagcagctgt 6000

cgccagtagt tcttcatctt tttgaccacc tcttcgctgg gaacgttgtc cgacttgcct 6060

ctgttcttgt cggatcgtgt aaggaccttg ttgtcgatag aatcgtcctt gagaaaggat 6120

tgagggacaa tgtggtccac atcgtagtcg ctgagacgat tgatgtccag ttcctgatcc 6180

acgtacatgt ctcgaccatt ctgcagatag tagagataca gcttctcgtt ctgcagttga 6240

gtgttctcga cgggatgctc cttgagaatc tgggatccca gctccttgat gccttcctcg 6300

attcgcttca tccgctctcg cgagtttttc tgaccctttt gagttgtctg gttctctctg 6360

gccatctcga tcacaatgtt ctcgggcttg tgacgtccca tgaccttcac cagctcgtcg 6420

acaaccttga cagtctggag aatgcctttc ttgatggctg gcgaaccagc caggttggca 6480

atatgttcgt gcaagctgtc gccctgaccg gacacttgtg ccttctggat gtcctccttg 6540

aaggtaagag aatcgtcgtg aatgagctgc atgaagtttc ggttggcaaa gccatcggac 6600

ttgagaaagt ccagaatggt ctttccggac tgcttgtctc tgatgccgtt gatgagcttt 6660

cgcgaaagtc ttccccagcc ggtgtatcta cgtcgcttga gttgtttcat gaccttgtcg 6720

tcgaacaggt gagcgtatgt cttgagtcgt tcctcgatca tctcccgatc ttcgaacagg 6780

gtaagagtga gcacgatgtc ctccagaatg tcctcgtttt cctcgttgtc gagaaaatcc 6840

ttgtccttga taatcttgag cagatcgtga taggtgccca aagaggcgtt gaatcggtcc 6900

tcaactccgg aaatctcgac gctgtcgaaa cactcgattt tcttgaagta gtcctccttg 6960

agctgcttaa cagtgacctt tcggttggtc ttgaacagga gatcgacaat ggctttcttc 7020

tgttcgccag acaagaaggc aggctttcgc attccctcgg taacgtactt gactttggtg 7080

agttcgttgt agactgtaaa gtactcgtag agcagcgaat gcttgggaag aaccttctcg 7140

ttgggcagat tcttgtcgaa gttggtcatt cgctcgatga aggactgtgc agaggcaccc 7200

ttgtccacga cttcctcgaa gttccaggga gtgatggttt cctcggactt tcgagtcatc 7260

caagcaaatc gagagtttcc tctggcaaga ggaccaacat agtaggggat tcgaaaggta 7320

agaatcttct cgatcttctc tcggttgtcc ttgagaaagg ggtagaagtc ttcctgacgt 7380

cgaagaatgg cgtgcagctc accgaggtgg atctgatgag gaatgctgcc gttgtcgaag 7440

gttcgttgct tccgaagcag atcctctcga ttgagcttga caagcagttc ctcggttccg 7500

tccatcttct cgagaattgg cttgatgaac ttgtagaact cttcctgaga ggctccgccg 7560

tcgatgtatc cagcgtagcc gttcttcgac tgatcgaaaa agatctcctt gtacttctcg 7620

ggcagttgct gtcggacaag agccttgagc agtgtgagat cctgatggtg ctcgtcgtat 7680

cgcttgatca tggaggcaga aaggggagcc tttgtgatct cggtgttgac tcgcagaatg 7740

tcagacaaga gaatagcatc cgaaaggttc ttggcagcga gaaacaggtc ggcgtactga 7800

tcgccaatct gtgcaagcag gttgtcgagg tcatcgtcgt aggtgtcctt ggacagctgg 7860

agcttggcgt cctccgccag atcgaagttg gacttgaagt tgggtgtgag accaagagaa 7920

agggcaatga ggttgccaaa cagtccgttc tttttctcgc caggaagttg ggcaatgagg 7980

ttctccagtc gtctgctctt cgagagtcga gcagacaaga tggcctttgc atcgactccg 8040

gaggcattga tggggttttc ctcgaacagc tggttgtagg tctgaacgag ctgaatgaac 8100

agcttgtcca catcgctgtt gtcgggattg agatcgccct cgatgaggaa atgacctcga 8160

aacttgatca tgtgtgccag agcgaggtag ataagtctga gatccgcctt gtcggtggaa 8220

tcgacgagtt tctttcggag atggtagatg gtaggatact tctcgtggta agcaacctcg 8280

tccacaatgt tgccaaagat gggatgacgc tcgtgtttct tgtcttcctc gacgaggaag 8340

gattcctcca gtcgatgaaa gaacgaatcg tccaccttgg ccatctcgtt ggaaaagatc 8400

tcctgcaggt agcagattcg gttcttccgt cgggtgtaac gtcgccgagc agttcgcttg 8460

agtctggtag cttcggcagt ctcgccagaa tcgaacaaca gggcaccaat gaggtttttc 8520

ttgatggagt gtcgatcggt gtttccgagg accttgaatt tcttggaggg caccttgtac 8580

tcgtcggtga tgacagccca gccgacagag ttggttccaa tgtccaggcc gatggagtat 8640

ttcttgtcca tggtgtgatg tgtagtttag atttcgaatc tgtggggaaa gaaaggaaaa 8700

aagagactgg caaccgattg ggagagccac tgtttatata taccctagac aagccccccg 8760

cttgtaagat gttggtcaat gtaaaccagt attaaggttg gcaagtgcag gagaagcaag 8820

gtgtgggtac cgagcaatgg aaatgtgcgg aaggcaaaaa aatgaggcca cggcctattg 8880

tcggggctat atccaggggg cgattgaagt acactaacat gacatgtgtc cacagaccct 8940

caatctggcc tgatgagcca aatccatacg cgctttcgca gctctaaagg ctataacaag 9000

tcacaccacc ctgctcgacc tcagcgccct cactttttgt taagacaaac tgtacacgct 9060

gttccagcgt tttctgcctg cacctggtgg gacatttggt gcaacctaaa gtgctcggaa 9120

cctctgtggt gtccagatca gcgcagcagt tccgaggtag ttttgaggcc cttagatgat 9180

ggtttaaacc ttaagcccgc tcataacttc gtatagcata cattatacga acggtaggtt 9240

gcgggataga cgccgacgga gggcaatggc gctatggaac cttgcggata tccatacgcc 9300

gcggcggact gcgtccgaac cagctccagc agcgtttttt ccgggccatt gagccgactg 9360

cgaccccgcc aacgtgtctt ggcccacgca ctcatgtcat gttggtgttg ggaggccact 9420

ttttaagtag cacaaggcac ctagctcgca gcaaggtgtc cgaaccaaag aagcggctgc 9480

agtggtgcaa acggggcgga aacggcggga aaaagccacg ggggcacgaa ttgaggcacg 9540

ccctcgaatt tgagacgagt cacggcccca ttcgcccgcg caatggctcg ccaacgcccg 9600

gtcttttgca ccacatcagg ttaccccaag ccaaaccttt gtgttaaaaa gcttaacata 9660

ttataccgaa cgtaggtttg ggcgggcttg ctccgtctgt ccaaggcaac atttatataa 9720

gggtctgcat cgccggctca attgaatctt ttttcttctt ctcttctcta tattcattct 9780

tgaattaaac acacatcaac catggccaaa aagcctgaac tcaccgcgac gtctgtcgag 9840

aagtttctga tcgaaaagtt cgacagcgtc tccgacctga tgcagctctc ggagggcgaa 9900

gaatctcgtg ctttcagctt cgatgtagga gggcgtggat atgtcctgcg ggtaaatagc 9960

tgcgccgatg gtttctacaa agatcgttat gtttatcggc actttgcatc ggccgcgctc 10020

ccgattccgg aagtgcttga cattggggag ttcagcgaga gcctgaccta ttgcatctcc 10080

cgccgtgcac agggtgtcac gttgcaagac ctgcctgaaa ccgaactgcc cgctgttctg 10140

cagccggtcg cggaggctat ggatgcgatc gctgcggccg atcttagcca gacgagcggg 10200

ttcggcccat tcggaccgca aggaatcggt caatacacta catggcgtga tttcatatgc 10260

gcgattgctg atccccatgt gtatcactgg caaactgtga tggacgacac cgtcagtgcg 10320

tccgtcgcgc aggctctcga tgagctgatg ctttgggccg aggactgccc cgaagtccgg 10380

cacctcgtgc acgcggattt cggctccaac aatgtcctga cggacaatgg ccgcataaca 10440

gcggtcattg actggagcga ggcgatgttc ggggattccc aatacgaggt cgccaacatc 10500

ttcttctgga ggccgtggtt ggcttgtatg gagcagcaga cgcgctactt cgagcggagg 10560

catccggagc ttgcaggatc gccgcggctc cgggcgtata tgctccgcat tggtcttgac 10620

caactctatc agagcttggt tgacggcaat ttcgatgatg cagcttgggc gcagggtcga 10680

tgcgacgcaa tcgtccgatc cggagccggg actgtcgggc gtacacaaat cgcccgcaga 10740

agcgcggccg tctggaccga tggctgtgta gaagtactcg ccgatagtgg aaaccgacgc 10800

cccagcactc gtccgagggc aaaggaatag cggccgcaag tgtggatggg gaagtgagtg 10860

cccggttctg tgtgcacaat tggcaatcca agatggatgg attcaacaca gggatatagc 10920

gagctacgtg gtggtgcgag gatatagcaa cggatattta tgtttgacac ttgagaatgt 10980

acgatacaag cactgtccaa gtacaatact aaacatactg tacatactca tactcgtacc 11040

cgggcaacgg tttcacttga gtgcagtggc tagtgctctt actcgtacag tgtgctaccg 11100

ttcgtatagc atacattata cgaagttatc atagtcttaa ttaaggtcgt gtggtgtaat 11160

ggttatcacg tcccgctcac accgggaggc ctccgagttc gatcctcggc atgatcaagt 11220

ctcaccgaca tggttatcgt tttagagcta gaaatagcaa gttaaaataa ggctagtccg 11280

ttatcaactt gaaaaagtgg caccgagtcg gtgctttttt ttttgttttt tatcg 11335

<210> 40
<211> 1047
<212> DNA
<213> Artificial Sequence

<220>
<223>  Artificial Sequence

<400>  40
ttaattaaat tttttttgat tttctttttt gaccccgtct tcaattacac ttcccaactg      60

ggaacacccc tctttatcga cccattttag gtaatttacc ctagcccatt gtctccataa     120

ggaatattac cctaacccac agtccagggt gcccaggtcc ttctttggcc aaattttaac     180

ttcggtccta tggcacagcg gtagcgcgtg agattgcaaa tcttaaggtc ccgagttcga     240

atctcggtgg gacctagtta tttttgatag ataatttcgt gatgattaga aacttaacgc     300

aaaataatgc ctggctagct caatcggtag agcgtgagac tcttatacaa gaaatctcaa     360

ggctgtgggt tcaagcccca cgtcgggcta gcaggtgatg gcgggatcgt tgtatatttc     420

ttgacacctt ttcggcatcg ccctaaattc ggcgtcctca tattgtgtga ggacgtttta     480

ttacgtgttt acgaagcaaa agctaaaacc aggagctatt taatggcaac agttaaccag     540

ctggtacgca aaccacgtgc tcgcaaagtt gcgaaaagca acgtgcctgc gctggaagca     600

tgcccgcaaa aacgtggcgt atgtactcgt gtatatacta ccactcctaa aaaaccgaac     660

tccgcgctgc gtaaagtatg ccgtgttcgt ctgactaacg gtttcgaagt gacttcctac     720

atcggtggtg aaggtcacaa cctgcaggag cactccgtga tcctgatccg tggcggtcgt     780

gttaaagacc tcccgggtgt tcgttaccac accgtacgtg gtgcgcttga ctgctccggc     840

gttaaagacc gtaagcaggc tcgttccaag tatggcgtga agcgtcctaa ggcttaggtt     900

aataacaggc ctgctggtaa tcgcaggcct ttttattttt acacctgcgt tttagagcta     960

gaaatagcaa gttaaaataa ggctagtccg ttatcaactt gaaaaagtgg caccgagtcg    1020

gtgctttttt ttttgttttt tatcgat                                        1047


<210>  41
<211>  12181
<212>  DNA
<213>  Artificial Sequence

<220>
<223>  Artificial Sequence

<400>  41
attgagcatc cgttgatttc cgaacagatc ccaatattac acccaagtag catgcataag      60

ctaaaagtaa ctcgcagcgc acaccgtgca gattcataag tctatgatta attgaacgcc     120

aataacccgg cttactacaa gtacaagtag gtatacatag cggtaatgaa tcattagaaa     180

aaaaaaaaac aaaaaaaaac aaaacaaact gttgtggatg catcaacagt agtacatagt     240

tgtacgatgt acttgtactt gtaaaagcaa aaatgtacaa tatctcaggg agcgcaactt     300

ttacgttcga agaacaatgt accgcatacc gcattctaga ttctgcggaa cgtctaacct     360

ggaaatacga tttttttttt ctttcatttt ttttgcttct tcaaaagtat ggtaatttcc     420

taccattaca gttgacactg aacgaggggg gattgaattt aagcaaaaaa ttaaatcaaa 480

atacctttat gtatccagcc catgtaataa acaaaaggat tatataacaa gaaataaata 540

tataccttta atggatcatt agaataaaaa taaatacgag aagcacacca gagaagcttt 600

ttgattgcca ctataccgct actttggtat atcttattat aattgttgaa tttgcaagat 660

agaatgtcat tcattggaga gaaatccaag gaatatgtgg gatgaaatga ctagaagtat 720

gaacaatgag aatagtacat acttgtacct gtatttctag aagagagaaa gacagttgag 780

tgtgtgattc tcgtccaata ataatctcaa tagtaacgtg tgaatagctg ttctttgata 840

gttgatattt ctcgatgact atttatgttg tacaaggatt ttttttcgtt gctgttgatt 900

tcgaattagg caatgcagat atcatttatg ctatccatat ttaagatttc ccatacgcat 960

ttataacatt tattctacat aaattgttaa atgaacgaac tgccattata aattgtttcc 1020

taaataggaa gtgtttttca taaagcaagt aagttgtcta ataatactaa gtaataaaaa 1080

taagttcata caatatattt tgagaacatc atttggaggc ggtagatgga gtctgtttat 1140

tattaaacaa tgcgagatga ccccttaaat attgagaaca tcagttggag gcggcagatg 1200

gagtctgtct atttagcaat gggacatgac tgtcagtatc atcatgatgt atatatataa 1260

tacatataat attatataac acgatttttt taaattattg gcccgaaaat taatcagtgt 1320

agactggatc tcggcagtct ctcggatgta gaattaggtt tccttgaggc gaagatcggt 1380

ttgtgtgaca tgaattcgat atcaagctta tcgacaccat cgacctcgag ggggggcccg 1440

gtacccaatt cgccctatag tgagtcgtat tacaattcac tggccgtcgt tttacaacgt 1500

cgtgactggg aaaaccctgg cgttacccaa cttaatcgcc ttgcagcaca tccccctttc 1560

gccagctggc gtaatagcga agaggcccgc accgatcgcc cttcccaaca gttgcgcagc 1620

ctgaatggcg aatggaaatt gtaagcgtta atattttgtt aaaattcgcg ttaaattttt 1680

gttaaatcag ctcatttttt aaccaatagg ccgaaatcgg caaaatccct tataaatcaa 1740

aagaatagac cgagataggg ttgagtgttg ttccagtttg gaacaagagt ccactattaa 1800

agaacgtgga ctccaacgtc aaagggcgaa aaaccgtcta tcagggcgat ggcccactac 1860

gtgaaccatc accctaatca agttttttgg ggtcgaggtg ccgtaaagca ctaaatcgga 1920

accctaaagg gagcccccga tttagagctt gacggggaaa gccggcgaac gtggcgagaa 1980

aggaagggaa gaaagcgaaa ggagcgggcg ctagggcgct ggcaagtgta gcggtcacgc 2040

tgcgcgtaac caccacaccc gccgcgctta atgcgccgct acagggcgcg tcaggtggca 2100

cttttcgggg aaatgtgcgc ggaaccccta tttgtttatt tttctaaata cattcaaata 2160

tgtatccgct catgagacaa taaccctgat aaatgcttca ataatattga aaaaggaaga 2220

gtatgagtat tcaacatttc cgtgtcgccc ttattccctt ttttgcggca ttttgccttc 2280

ctgtttttgc tcacccagaa acgctggtga aagtaaaaga tgctgaagat cagttgggtg 2340

cacgagtggg ttacatcgaa ctggatctca acagcggtaa gatccttgag agttttcgcc 2400

ccgaagaacg ttttccaatg atgagcactt ttaaagttct gctatgtggc gcggtattat 2460

cccgtattga cgccgggcaa gagcaactcg gtcgccgcat acactattct cagaatgact 2520

tggttgagta ctcaccagtc acagaaaagc atcttacgga tggcatgaca gtaagagaat 2580

tatgcagtgc tgccataacc atgagtgata acactgcggc caacttactt ctgacaacga 2640

tcggaggacc gaaggagcta accgcttttt tgcacaacat gggggatcat gtaactcgcc 2700

ttgatcgttg ggaaccggag ctgaatgaag ccataccaaa cgacgagcgt gacaccacga 2760

tgcctgtagc aatggcaaca acgttgcgca aactattaac tggcgaacta cttactctag 2820

cttcccggca acaattaata gactggatgg aggcggataa agttgcagga ccacttctgc 2880

gctcggccct tccggctggc tggtttattg ctgataaatc tggagccggt gagcgtgggt 2940

ctcgcggtat cattgcagca ctggggccag atggtaagcc ctcccgtatc gtagttatct 3000

acacgacggg gagtcaggca actatggatg aacgaaatag acagatcgct gagataggtg 3060

cctcactgat taagcattgg taactgtcag accaagttta ctcatatata ctttagattg 3120

atttaaaact tcatttttaa tttaaaagga tctaggtgaa gatccttttt gataatctca 3180

tgaccaaaat cccttaacgt gagttttcgt tccactgagc gtcagacccc gtagaaaaga 3240

tcaaaggatc ttcttgagat cctttttttc tgcgcgtaat ctgctgcttg caaacaaaaa 3300

aaccaccgct accagcggtg gtttgtttgc cggatcaaga gctaccaact ctttttccga 3360

aggtaactgg cttcagcaga gcgcagatac caaatactgt ccttctagtg tagccgtagt 3420

taggccacca cttcaagaac tctgtagcac cgcctacata cctcgctctg ctaatcctgt 3480

taccagtggc tgctgccagt ggcgataagt cgtgtcttac cgggttggac tcaagacgat 3540

agttaccgga taaggcgcag cggtcgggct gaacgggggg ttcgtgcaca cagcccagct 3600

tggagcgaac gacctacacc gaactgagat acctacagcg tgagctatga gaaagcgcca 3660

cgcttcccga agggagaaag gcggacaggt atccggtaag cggcagggtc ggaacaggag 3720

agcgcacgag ggagcttcca gggggaaacg cctggtatct ttatagtcct gtcgggtttc 3780

gccacctctg acttgagcgt cgatttttgt gatgctcgtc aggggggcgg agcctatgga 3840

aaaacgccag caacgcggcc tttttacggt tcctggcctt ttgctggcct tttgctcaca 3900

tgttctttcc tgcgttatcc cctgattctg tggataaccg tattaccgcc tttgagtgag 3960

ctgataccgc tcgccgcagc cgaacgaccg agcgcagcga gtcagtgagc gaggaagcgg 4020

aagagcgccc aatacgcaaa ccgcctctcc ccgcgcgttg gccgattcat taatgcagct 4080

ggcacgacag gtttcccgac tggaaagcgg gcagtgagcg caacgcaatt aatgtgagtt 4140

agctcactca ttaggcaccc caggctttac actttatgct tccggctcgt acgcaactaa 4200

catgaatgaa tacgatatac atcaaagact atgatacgca gtattgcaca ctgtacgagt 4260

aagagcacta gccactgcac tcaagtgaaa ccgttgcccg ggtacgagta tgagtatgta 4320

cagtatgttt agtattgtac ttggacagtg cttgtatcgt acattctcaa gtgtcaaaca 4380

taaatatccg ttgctatatc ctcgcaccac cacgtagctc gctatatccc tgtgttgaat 4440

ccatccatct tggattgcca attgtgcaca cagaaccggg cactcacttc cccatccaca 4500

cttgcggccg cttagacctt tcgctttttc ttgggatcgg ctctggagtc gccaccaagc 4560

tgagacaggt cgattcgggt ctcgtacagg ccagtgatgg actggtgaat cagggtggca 4620

tcgagaacct ccttggtgga tgtgtaccgc tttcggtcga tagtggtatc gaagtacttg 4680

aaagctgcag gagcacccag gttggtaaga gtaaacaggt gaatgatgtt ctccgcctgt 4740

tctcgaatgg gtttgtcccg atgcttgttg taggcagaga gcaccttgtc caagttggca 4800

tcagccagga tgactcgctt cgaaaactcg gaaatctgct cgataatctc gtcgaggtaa 4860

tgtttgtgct gctcaacgaa gagttgcttc tgttcgttgt cctcgggaga acccttgagc 4920

ttctcgtagt gagaagccag atagagaaag ttgacgtact tcgaaggcaa ggcaagctcg 4980

tttcccttct gcagctcgcc agcggaggcg agcatacgct ttcgaccgtt ctccagttcg 5040

aacagagagt acttgggcag cttgataatg aggtctttct tgacctcctt gtaacccttg 5100

gcttccaaga agtcgatggg attcttctcg aagctcgatc gctccatgat ggtaattccg 5160

agcagctcct tgacggactt gagctttttg gacttgccct tctcgacctt cgcaacgaca 5220

agcacggaat aggcgacggt aggagaatcg aagccaccgt atttcttggg atcccagtct 5280

ttctttcgag cgatgagctt gtcggagttt cgcttgggca gaatcgactc cttggagaat 5340

ccgccagtct gaacctcggt tttcttgacg atgttgacct gaggcatcga cagaaccttt 5400

cgcacggttg caaagtctcg acccttgtcc cacacgatct ctccagtttc gccgttggtc 5460

tcgataagtg gtctctttcg aatctctccg ttggccaagg tgatctcggt cttgaaaaag 5520

ttcatgatgt tggagtaaaa gaagtacttg gcagtagcct tgccaatctc ctgttcggac 5580

ttggcaatca tctttcgaac gtcgtagacc ttgtaatcgc cgtaaacgaa ctcgctttcg 5640

agcttggggt atttcttgat gagcgcagtg ccaacgacgg cgttgaggta agcatcgtgg 5700

gcatggtggt aattgttgat ctctcgcacc ttgtagaact gaaagtcctt tcggaaatcg 5760

gagaccagtt tggacttgag agtaatcacc ttgacctctc ggatgagctt gtcgttctcg 5820

tcgtacttgg tgttcatccg agaatcgaga atctgtgcga cgtgctttgt gatctgtctg 5880

gtctcgacga gttgacgctt gatgaagcca gccttgtcga gctcggacag accgcctcgc 5940

tcggccttgg taagattgtc gaactttcgc tgggtaatga gcttggcgtt gagcagctgt 6000

cgccagtagt tcttcatctt tttgaccacc tcttcgctgg gaacgttgtc cgacttgcct 6060

ctgttcttgt cggatcgtgt aaggaccttg ttgtcgatag aatcgtcctt gagaaaggat 6120

tgagggacaa tgtggtccac atcgtagtcg ctgagacgat tgatgtccag ttcctgatcc 6180

acgtacatgt ctcgaccatt ctgcagatag tagagataca gcttctcgtt ctgcagttga 6240

gtgttctcga cgggatgctc cttgagaatc tgggatccca gctccttgat gccttcctcg 6300

attcgcttca tccgctctcg cgagtttttc tgaccctttt gagttgtctg gttctctctg 6360

gccatctcga tcacaatgtt ctcgggcttg tgacgtccca tgaccttcac cagctcgtcg 6420

acaaccttga cagtctggag aatgcctttc ttgatggctg gcgaaccagc caggttggca 6480

atatgttcgt gcaagctgtc gccctgaccg gacacttgtg ccttctggat gtcctccttg 6540

aaggtaagag aatcgtcgtg aatgagctgc atgaagtttc ggttggcaaa gccatcggac 6600

ttgagaaagt ccagaatggt ctttccggac tgcttgtctc tgatgccgtt gatgagcttt 6660

cgcgaaagtc ttccccagcc ggtgtatcta cgtcgcttga gttgtttcat gaccttgtcg 6720

tcgaacaggt gagcgtatgt cttgagtcgt tcctcgatca tctcccgatc ttcgaacagg 6780

gtaagagtga gcacgatgtc ctccagaatg tcctcgtttt cctcgttgtc gagaaaatcc 6840

ttgtccttga taatcttgag cagatcgtga taggtgccca aagaggcgtt gaatcggtcc 6900

tcaactccgg aaatctcgac gctgtcgaaa cactcgattt tcttgaagta gtcctccttg 6960

agctgcttaa cagtgacctt tcggttggtc ttgaacagga gatcgacaat ggctttcttc 7020

tgttcgccag acaagaaggc aggctttcgc attccctcgg taacgtactt gactttggtg 7080

agttcgttgt agactgtaaa gtactcgtag agcagcgaat gcttgggaag aaccttctcg 7140

ttgggcagat tcttgtcgaa gttggtcatt cgctcgatga aggactgtgc agaggcaccc 7200

ttgtccacga cttcctcgaa gttccaggga gtgatggttt cctcggactt tcgagtcatc 7260

caagcaaatc gagagtttcc tctggcaaga ggaccaacat agtaggggat tcgaaaggta 7320

agaatcttct cgatcttctc tcggttgtcc ttgagaaagg ggtagaagtc ttcctgacgt 7380

cgaagaatgg cgtgcagctc accgaggtgg atctgatgag gaatgctgcc gttgtcgaag 7440

gttcgttgct tccgaagcag atcctctcga ttgagcttga caagcagttc ctcggttccg 7500

tccatcttct cgagaattgg cttgatgaac ttgtagaact cttcctgaga ggctccgccg 7560

tcgatgtatc cagcgtagcc gttcttcgac tgatcgaaaa agatctcctt gtacttctcg 7620

ggcagttgct gtcggacaag agccttgagc agtgtgagat cctgatggtg ctcgtcgtat 7680

cgcttgatca tggaggcaga aaggggagcc tttgtgatct cggtgttgac tcgcagaatg 7740

tcagacaaga gaatagcatc cgaaaggttc ttggcagcga gaaacaggtc ggcgtactga 7800

tcgccaatct gtgcaagcag gttgtcgagg tcatcgtcgt aggtgtcctt ggacagctgg 7860

agcttggcgt cctccgccag atcgaagttg gacttgaagt tgggtgtgag accaagagaa 7920

agggcaatga ggttgccaaa cagtccgttc tttttctcgc caggaagttg ggcaatgagg 7980

ttctccagtc gtctgctctt cgagagtcga gcagacaaga tggcctttgc atcgactccg 8040

gaggcattga tggggttttc ctcgaacagc tggttgtagg tctgaacgag ctgaatgaac 8100

agcttgtcca catcgctgtt gtcgggattg agatcgccct cgatgaggaa atgacctcga 8160

aacttgatca tgtgtgccag agcgaggtag ataagtctga gatccgcctt gtcggtggaa 8220

tcgacgagtt tctttcggag atggtagatg gtaggatact tctcgtggta agcaacctcg 8280

tccacaatgt tgccaaagat gggatgacgc tcgtgtttct tgtcttcctc gacgaggaag 8340

gattcctcca gtcgatgaaa gaacgaatcg tccaccttgg ccatctcgtt ggaaaagatc 8400

tcctgcaggt agcagattcg gttcttccgt cgggtgtaac gtcgccgagc agttcgcttg 8460

agtctggtag cttcggcagt ctcgccagaa tcgaacaaca gggcaccaat gaggtttttc 8520

ttgatggagt gtcgatcggt gtttccgagg accttgaatt tcttggaggg caccttgtac 8580

tcgtcggtga tgacagccca gccgacagag ttggttccaa tgtccaggcc gatggagtat 8640

ttcttgtcca tggtgtgatg tgtagtttag atttcgaatc tgtggggaaa gaaaggaaaa 8700

aagagactgg caaccgattg ggagagccac tgtttatata taccctagac aagccccccg 8760

cttgtaagat gttggtcaat gtaaaccagt attaaggttg gcaagtgcag gagaagcaag 8820

gtgtgggtac cgagcaatgg aaatgtgcgg aaggcaaaaa aatgaggcca cggcctattg 8880

tcggggctat atccaggggg cgattgaagt acactaacat gacatgtgtc cacagaccct 8940

caatctggcc tgatgagcca aatccatacg cgctttcgca gctctaaagg ctataacaag 9000

tcacaccacc ctgctcgacc tcagcgccct cactttttgt taagacaaac tgtacacgct 9060

gttccagcgt tttctgcctg cacctggtgg gacatttggt gcaacctaaa gtgctcggaa 9120

cctctgtggt gtccagatca gcgcagcagt tccgaggtag ttttgaggcc cttagatgat 9180

ggtttaaacc ttaagcccgc tcataacttc gtatagcata cattatacga acggtaggtt 9240

gcgggataga cgccgacgga gggcaatggc gctatggaac cttgcggata tccatacgcc 9300

gcggcggact gcgtccgaac cagctccagc agcgtttttt ccgggccatt gagccgactg 9360

cgaccccgcc aacgtgtctt ggcccacgca ctcatgtcat gttggtgttg ggaggccact 9420

ttttaagtag cacaaggcac ctagctcgca gcaaggtgtc cgaaccaaag aagcggctgc 9480

agtggtgcaa acggggcgga aacggcggga aaaagccacg ggggcacgaa ttgaggcacg 9540

ccctcgaatt tgagacgagt cacggcccca ttcgcccgcg caatggctcg ccaacgcccg 9600

gtcttttgca ccacatcagg ttaccccaag ccaaaccttt gtgttaaaaa gcttaacata 9660

ttataccgaa cgtaggtttg ggcgggcttg ctccgtctgt ccaaggcaac atttatataa 9720

gggtctgcat cgccggctca attgaatctt ttttcttctt ctcttctcta tattcattct 9780

tgaattaaac acacatcaac catggccaaa aagcctgaac tcaccgcgac gtctgtcgag 9840

aagtttctga tcgaaaagtt cgacagcgtc tccgacctga tgcagctctc ggagggcgaa 9900

gaatctcgtg ctttcagctt cgatgtagga gggcgtggat atgtcctgcg ggtaaatagc 9960

tgcgccgatg gtttctacaa agatcgttat gtttatcggc actttgcatc ggccgcgctc 10020

ccgattccgg aagtgcttga cattggggag ttcagcgaga gcctgaccta ttgcatctcc 10080

cgccgtgcac agggtgtcac gttgcaagac ctgcctgaaa ccgaactgcc cgctgttctg 10140

cagccggtcg cggaggctat ggatgcgatc gctgcggccg atcttagcca gacgagcggg 10200

ttcggcccat tcggaccgca aggaatcggt caatacacta catggcgtga tttcatatgc 10260

gcgattgctg atccccatgt gtatcactgg caaactgtga tggacgacac cgtcagtgcg 10320

tccgtcgcgc aggctctcga tgagctgatg ctttgggccg aggactgccc cgaagtccgg 10380

cacctcgtgc acgcggattt cggctccaac aatgtcctga cggacaatgg ccgcataaca 10440

gcggtcattg actggagcga ggcgatgttc ggggattccc aatacgaggt cgccaacatc 10500

ttcttctgga ggccgtggtt ggcttgtatg gagcagcaga cgcgctactt cgagcggagg 10560

catccggagc ttgcaggatc gccgcggctc cgggcgtata tgctccgcat tggtcttgac 10620

caactctatc agagcttggt tgacggcaat ttcgatgatg cagcttgggc gcagggtcga 10680

tgcgacgcaa tcgtccgatc cggagccggg actgtcgggc gtacacaaat cgcccgcaga 10740

agcgcggccg tctggaccga tggctgtgta gaagtactcg ccgatagtgg aaaccgacgc 10800

cccagcactc gtccgagggc aaaggaatag cggccgcaag tgtggatggg gaagtgagtg 10860

cccggttctg tgtgcacaat tggcaatcca agatggatgg attcaacaca gggatatagc 10920

gagctacgtg gtggtgcgag gatatagcaa cggatattta tgtttgacac ttgagaatgt 10980

acgatacaag cactgtccaa gtacaatact aaacatactg tacatactca tactcgtacc 11040

cgggcaacgg tttcacttga gtgcagtggc tagtgctctt actcgtacag tgtgctaccg 11100

ttcgtatagc atacattata cgaagttatc atagtcttaa ttaaattttt tttgattttc 11160

ttttttgacc ccgtcttcaa ttacacttcc caactgggaa cacccctctt tatcgaccca 11220

ttttaggtaa tttaccctag cccattgtct ccataaggaa tattacccta acccacagtc 11280

cagggtgccc aggtccttct ttggccaaat tttaacttcg gtcctatggc acagcggtag 11340

cgcgtgagat tgcaaatctt aaggtcccga gttcgaatct cggtgggacc tagttatttt 11400

tgatagataa tttcgtgatg attagaaact taacgcaaaa taatgcctgg ctagctcaat 11460

cggtagagcg tgagactctt atacaagaaa tctcaaggct gtgggttcaa gccccacgtc 11520

gggctagcag gtgatggcgg gatcgttgta tatttcttga caccttttcg gcatcgccct 11580

aaattcggcg tcctcatatt gtgtgaggac gttttattac gtgtttacga agcaaaagct 11640

aaaaccagga gctatttaat ggcaacagtt aaccagctgg tacgcaaacc acgtgctcgc 11700

aaagttgcga aaagcaacgt gcctgcgctg gaagcatgcc cgcaaaaacg tggcgtatgt 11760

actcgtgtat atactaccac tcctaaaaaa ccgaactccg cgctgcgtaa agtatgccgt     11820

gttcgtctga ctaacggttt cgaagtgact tcctacatcg gtggtgaagg tcacaacctg     11880

caggagcact ccgtgatcct gatccgtggc ggtcgtgtta aagacctccc gggtgttcgt     11940

taccacaccg tacgtggtgc gcttgactgc tccggcgtta aagaccgtaa gcaggctcgt     12000

tccaagtatg gcgtgaagcg tcctaaggct taggttaata acaggcctgc tggtaatcgc     12060

aggccttttt atttttacac ctgcgtttta gagctagaaa tagcaagtta aaataaggct     12120

agtccgttat caacttgaaa aagtggcacc gagtcggtgc tttttttttt gttttttatc     12180

g                                                                     12181

<210>  42
<211>  32
<212>  DNA
<213>  Artificial Sequence

<220>
<223>  Artificial Sequence

<400>  42
tcgggctatc aaacgattac ccaccctcgt tt                                   32

<210>  43
<211>  32
<212>  DNA
<213>  Artificial Sequence

<220>
<223>  Artificial Sequence

<400>  43
tctaaaacga gggtgggtaa tcgtttgata gc                                   32

<210>  44
<211>  89
<212>  DNA
<213>  Artificial Sequence

<220>
<223>  Artificial Sequence

<400>  44
tcgggctagg tcgtgtggtg taatggttat cacgaggctc cgagttcgat cctcggcatg     60

atcaagtctc accgacatgg ttatcgttt                                       89

<210>  45
<211>  89
<212>  DNA
<213>  Artificial Sequence

<220>
<223>  Artificial Sequence

<400>  45
tctaaaacga taaccatgtc ggtgagactt gatcatgccg aggatcgaac tcggagcctc      60

gtgataacca ttacaccaca cgacctagc                                        89


<210>  46
<211>  56
<212>  DNA
<213>  Artificial Sequence

<220>
<223>  Artificial Sequence

<400>  46
ggtcgtgtgg tgtaatggtt atcacgaggc tccgagttcg atcctcggca tgatca          56


<210>  47
<211>  11700
<212>  DNA
<213>  Artificial Sequence

<220>
<223>  Artificial Sequence

<400>  47
aacgtcgtga ctgggaaaac cctggcgtta cccaacttaa tcgccttgca gcacatcccc      60

ctttcgccag ctggcgtaat agcgaagagg cccgcaccga tcgcccttcc caacagttgc     120

gcagcctgaa tggcgaatgg aaattgtaag cgttaatatt ttgttaaaat tcgcgttaaa     180

tttttgttaa atcagctcat tttttaacca ataggccgaa atcggcaaaa tcccttataa     240

atcaaaagaa tagaccgaga tagggttgag tgttgttcca gtttggaaca agagtccact     300

attaaagaac gtggactcca acgtcaaagg gcgaaaaacc gtctatcagg gcgatggccc     360

actacgtgaa ccatcaccct aatcaagttt tttggggtcg aggtgccgta aagcactaaa     420

tcggaaccct aaagggagcc cccgatttag agcttgacgg ggaaagccgg cgaacgtggc     480

gagaaaggaa gggaagaaag cgaaaggagc gggcgctagg gcgctggcaa gtgtagcggt     540

cacgctgcgc gtaaccacca cacccgccgc gcttaatgcg ccgctacagg gcgcgtcagg     600

tggcactttt cggggaaatg tgcgcggaac ccctatttgt ttatttttct aaatacattc     660

aaatatgtat ccgctcatga gacaataacc ctgataaatg cttcaataat attgaaaaag     720

gaagagtatg agtattcaac atttccgtgt cgcccttatt cccttttttg cggcattttg     780

ccttcctgtt tttgctcacc cagaaacgct ggtgaaagta aaagatgctg aagatcagtt     840

gggtgcacga gtgggttaca tcgaactgga tctcaacagc ggtaagatcc ttgagagttt     900

tcgccccgaa gaacgttttc caatgatgag cacttttaaa gttctgctat gtggcgcggt     960

attatcccgt attgacgccg ggcaagagca actcggtcgc cgcatacact attctcagaa    1020

tgacttggtt gagtactcac cagtcacaga aaagcatctt acggatggca tgacagtaag    1080

agaattatgc agtgctgcca taaccatgag tgataacact gcggccaact tacttctgac 1140

aacgatcgga ggaccgaagg agctaaccgc ttttttgcac aacatggggg atcatgtaac 1200

tcgccttgat cgttgggaac cggagctgaa tgaagccata ccaaacgacg agcgtgacac 1260

cacgatgcct gtagcaatgg caacaacgtt gcgcaaacta ttaactggcg aactacttac 1320

tctagcttcc cggcaacaat taatagactg gatggaggcg gataaagttg caggaccact 1380

tctgcgctcg gcccttccgg ctggctggtt tattgctgat aaatctggag ccggtgagcg 1440

tgggtctcgc ggtatcattg cagcactggg gccagatggt aagccctccc gtatcgtagt 1500

tatctacacg acggggagtc aggcaactat ggatgaacga aatagacaga tcgctgagat 1560

aggtgcctca ctgattaagc attggtaact gtcagaccaa gtttactcat atatacttta 1620

gattgattta aaacttcatt tttaatttaa aaggatctag gtgaagatcc tttttgataa 1680

tctcatgacc aaaatccctt aacgtgagtt ttcgttccac tgagcgtcag accccgtaga 1740

aaagatcaaa ggatcttctt gagatccttt ttttctgcgc gtaatctgct gcttgcaaac 1800

aaaaaaacca ccgctaccag cggtggtttg tttgccggat caagagctac caactctttt 1860

tccgaaggta actggcttca gcagagcgca gataccaaat actgtccttc tagtgtagcc 1920

gtagttaggc caccacttca agaactctgt agcaccgcct acatacctcg ctctgctaat 1980

cctgttacca gtggctgctg ccagtggcga taagtcgtgt cttaccgggt tggactcaag 2040

acgatagtta ccggataagg cgcagcggtc gggctgaacg gggggttcgt gcacacagcc 2100

cagcttggag cgaacgacct acaccgaact gagataccta cagcgtgagc tatgagaaag 2160

cgccacgctt cccgaaggga gaaaggcgga caggtatccg gtaagcggca gggtcggaac 2220

aggagagcgc acgagggagc ttccaggggg aaacgcctgg tatctttata gtcctgtcgg 2280

gtttcgccac ctctgacttg agcgtcgatt tttgtgatgc tcgtcagggg ggcggagcct 2340

atggaaaaac gccagcaacg cggccttttt acggttcctg gccttttgct ggccttttgc 2400

tcacatgttc tttcctgcgt tatcccctga ttctgtggat aaccgtatta ccgcctttga 2460

gtgagctgat accgctcgcc gcagccgaac gaccgagcgc agcgagtcag tgagcgagga 2520

agcggaagag cgcccaatac gcaaaccgcc tctccccgcg cgttggccga ttcattaatg 2580

cagctggcac gacaggtttc ccgactggaa agcgggcagt gagcgcaacg caattaatgt 2640

gagttagctc actcattagg caccccaggc tttacacttt atgcttccgg ctcgtacgca 2700

actaacatga atgaatacga tatacatcaa agactatgat acgcagtatt gcacactgta 2760

cgagtaagag cactagccac tgcactcaag tgaaaccgtt gcccgggtac gagtatgagt 2820

atgtacagta tgtttagtat tgtacttgga cagtgcttgt atcgtacatt ctcaagtgtc 2880

aaacataaat atccgttgct atatcctcgc accaccacgt agctcgctat atccctgtgt 2940

tgaatccatc catcttggat tgccaattgt gcacacagaa ccgggcactc acttccccat 3000

ccacacttgc ggccgcttag acctttcgct ttttcttggg atcggctctg gagtcgccac 3060

caagctgaga caggtcgatt cgggtctcgt acaggccagt gatggactgg tgaatcaggg 3120

tggcatcgag aacctccttg gtggatgtgt accgctttcg gtcgatagtg gtatcgaagt 3180

acttgaaagc tgcaggagca cccaggttgg taagagtaaa caggtgaatg atgttctccg 3240

cctgttctcg aatgggtttg tcccgatgct tgttgtaggc agagagcacc ttgtccaagt 3300

tggcatcagc caggatgact cgcttcgaaa actcggaaat ctgctcgata atctcgtcga 3360

ggtaatgttt gtgctgctca acgaagagtt gcttctgttc gttgtcctcg ggagaaccct 3420

tgagcttctc gtagtgagaa gccagataga gaaagttgac gtacttcgaa ggcaaggcaa 3480

gctcgtttcc cttctgcagc tcgccagcgg aggcgagcat acgctttcga ccgttctcca 3540

gttcgaacag agagtacttg ggcagcttga taatgaggtc tttcttgacc tccttgtaac 3600

ccttggcttc caagaagtcg atgggattct tctcgaagct cgatcgctcc atgatggtaa 3660

ttccgagcag ctccttgacg gacttgagct ttttggactt gcccttctcg accttcgcaa 3720

cgacaagcac ggaataggcg acggtaggag aatcgaagcc accgtatttc ttgggatccc 3780

agtctttctt tcgagcgatg agcttgtcgg agtttcgctt gggcagaatc gactccttgg 3840

agaatccgcc agtctgaacc tcggttttct tgacgatgtt gacctgaggc atcgacagaa 3900

cctttcgcac ggttgcaaag tctcgaccct tgtcccacac gatctctcca gtttcgccgt 3960

tggtctcgat aagtggtctc tttcgaatct ctccgttggc caaggtgatc tcggtcttga 4020

aaaagttcat gatgttggag taaaagaagt acttggcagt agccttgcca atctcctgtt 4080

cggacttggc aatcatcttt cgaacgtcgt agaccttgta atcgccgtaa acgaactcgc 4140

tttcgagctt ggggtatttc ttgatgagcg cagtgccaac gacggcgttg aggtaagcat 4200

cgtgggcatg gtggtaattg ttgatctctc gcaccttgta gaactgaaag tcctttcgga 4260

aatcggagac cagtttggac ttgagagtaa tcaccttgac ctctcggatg agcttgtcgt 4320

tctcgtcgta cttggtgttc atccgagaat cgagaatctg tgcgacgtgc tttgtgatct 4380

gtctggtctc gacgagttga cgcttgatga agccagcctt gtcgagctcg gacagaccgc 4440

ctcgctcggc cttggtaaga ttgtcgaact ttcgctgggt aatgagcttg gcgttgagca 4500

gctgtcgcca gtagttcttc atctttttga ccacctcttc gctgggaacg ttgtccgact 4560

tgcctctgtt cttgtcggat cgtgtaagga ccttgttgtc gatagaatcg tccttgagaa 4620

aggattgagg gacaatgtgg tccacatcgt agtcgctgag acgattgatg tccagttcct 4680

gatccacgta catgtctcga ccattctgca gatagtagag atacagcttc tcgttctgca 4740

gttgagtgtt ctcgacggga tgctccttga gaatctggga tcccagctcc ttgatgcctt 4800

cctcgattcg cttcatccgc tctcgcgagt tttctgaccc cttttgagtt gtctggttct 4860

ctctggccat ctcgatcaca atgttctcgg gcttgtgacg tcccatgacc ttcaccagct 4920

cgtcgacaac cttgacagtc tggagaatgc ctttcttgat ggctggcgaa ccagccaggt 4980

tggcaatatg ttcgtgcaag ctgtcgccct gaccggacac ttgtgccttc tggatgtcct 5040

ccttgaaggt aagagaatcg tcgtgaatga gctgcatgaa gtttcggttg gcaaagccat 5100

cggacttgag aaagtccaga atggtctttc cggactgctt gtctctgatg ccgttgatga 5160

gctttcgcga aagtcttccc cagccggtgt atctacgtcg cttgagttgt ttcatgacct 5220

tgtcgtcgaa caggtgagcg tatgtcttga gtcgttcctc gatcatctcc cgatcttcga 5280

acagggtaag agtgagcacg atgtcctcca gaatgtcctc gttttcctcg ttgtcgagaa 5340

aatccttgtc cttgataatc ttgagcagat cgtgataggt gcccaaagag gcgttgaatc 5400

ggtcctcaac tccggaaatc tcgacgctgt cgaaacactc gattttcttg aagtagtcct 5460

ccttgagctg cttaacagtg acctttcggt tggtcttgaa caggagatcg acaatggctt 5520

tcttctgttc gccagacaag aaggcaggct ttcgcattcc ctcggtaacg tacttgactt 5580

tggtgagttc gttgtagact gtaaagtact cgtagagcag cgaatgcttg ggaagaacct 5640

tctcgttggg cagattcttg tcgaagttgg tcattcgctc gatgaaggac tgtgcagagg 5700

cacccttgtc cacgacttcc tcgaagttcc agggagtgat ggtttcctcg gactttcgag 5760

tcatccaagc aaatcgagag tttcctctgg caagaggacc aacatagtag gggattcgaa 5820

aggtaagaat cttctcgatc ttctctcggt tgtccttgag aaaggggtag aagtcttcct 5880

gacgtcgaag aatggcgtgc agctcaccga ggtggatctg atgaggaatg ctgccgttgt 5940

cgaaggttcg ttgcttccga agcagatcct ctcgattgag cttgacaagc agttcctcgg 6000

ttccgtccat cttctcgaga attggcttga tgaacttgta gaactcttcc tgagaggctc 6060

cgccgtcgat gtatccagcg tagccgttct tcgactgatc gaaaaagatc tccttgtact 6120

tctcgggcag ttgctgtcgg acaagagcct tgagcagtgt gagatcctga tggtgctcgt 6180

cgtatcgctt gatcatggag gcagaaaggg gagcctttgt gatctcggtg ttgactcgca 6240

gaatgtcaga caagagaata gcatccgaaa ggttcttggc agcgagaaac aggtcggcgt 6300

actgatcgcc aatctgtgca agcaggttgt cgaggtcatc gtcgtaggtg tccttggaca 6360

gctggagctt ggcgtcctcc gccagatcga agttggactt gaagttgggt gtgagaccaa 6420

gagaaagggc aatgaggttg ccaaacagtc cgttcttttt ctcgccagga agttgggcaa 6480

tgaggttctc cagtcgtctg ctcttcgaga gtcgagcaga caagatggcc tttgcatcga 6540

ctccggaggc attgatgggg ttttcctcga acagctggtt gtaggtctga acgagctgaa 6600

tgaacagctt gtccacatcg ctgttgtcgg gattgagatc gccctcgatg aggaaatgac 6660

ctcgaaactt gatcatgtgt gccagagcga ggtagataag tctgagatcc gccttgtcgg 6720

tggaatcgac gagtttcttt cggagatggt agatggtagg atacttctcg tggtaagcaa 6780

cctcgtccac aatgttgcca aagatgggat gacgctcgtg tttcttgtct tcctcgacga 6840

ggaaggattc ctccagtcga tgaaagaacg aatcgtccac cttggccatc tcgttggaaa 6900

agatctcctg caggtagcag attcggttct tccgtcgggt gtaacgtcgc cgagcagttc 6960

gcttgagtct ggtagcttcg gcagtctcgc cagaatcgaa caacagggca ccaatgaggt 7020

ttttcttgat ggagtgtcga tcggtgtttc cgaggacctt gaatttcttg gagggcacct 7080

tgtactcgtc ggtgatgaca gcccagccga cagagttggt tccaatgtcc aggccgatgg 7140

agtatttctt gtccatggtg tgatgtgtag tttagatttc gaatctgtgg ggaaagaaag 7200

gaaaaaagag actggcaacc gattgggaga gccactgttt atatataccc tagacaagcc 7260

ccccgcttgt aagatgttgg tcaatgtaaa ccagtattaa ggttggcaag tgcaggagaa 7320

gcaaggtgtg ggtaccgagc aatggaaatg tgcggaaggc aaaaaaatga ggccacggcc 7380

tattgtcggg gctatatcca gggggcgatt gaagtacact aacatgacat gtgtccacag 7440

accctcaatc tggcctgatg agccaaatcc atacgcgctt tcgcagctct aaaggctata 7500

acaagtcaca ccaccctgct cgacctcagc gccctcactt tttgttaaga caaactgtac 7560

acgctgttcc agcgttttct gcctgcacct ggtgggacat ttggtgcaac ctaaagtgct 7620

cggaacctct gtggtgtcca gatcagcgca gcagttccga ggtagttttg aggcccttag 7680

atgatggttt aaaccttaag cccgctcata acttcgtata gcatacatta tacgaacggt 7740

aggttgcggg atagacgccg acggagggca atggcgctat ggaaccttgc ggatatccat 7800

acgccgcggc ggactgcgtc cgaaccagct ccagcagcgt tttttccggg ccattgagcc 7860

gactgcgacc ccgccaacgt gtcttggccc acgcactcat gtcatgttgg tgttgggagg 7920

ccacttttta agtagcacaa ggcacctagc tcgcagcaag gtgtccgaac caaagaagcg 7980

gctgcagtgg tgcaaacggg gcggaaacgg cgggaaaaag ccacgggggc acgaattgag 8040

gcacgccctc gaatttgaga cgagtcacgg ccccattcgc ccgcgcaatg gctcgccaac 8100

gcccggtctt ttgcaccaca tcaggttacc ccaagccaaa cctttgtgtt aaaaagctta 8160

acatattata ccgaacgtag gtttgggcgg gcttgctccg tctgtccaag gcaacattta 8220

tataagggtc tgcatcgccg gctcaattga atcttttttc ttcttctctt ctctatattc 8280

attcttgaat taaacacaca tcaaccatgg ccaaaaagcc tgaactcacc gcgacgtctg 8340

tcgagaagtt tctgatcgaa aagttcgaca gcgtctccga cctgatgcag ctctcggagg 8400

gcgaagaatc tcgtgctttc agcttcgatg taggagggcg tggatatgtc ctgcgggtaa 8460

atagctgcgc cgatggtttc tacaaagatc gttatgttta tcggcacttt gcatcggccg 8520

cgctcccgat tccggaagtg cttgacattg gggagttcag cgagagcctg acctattgca 8580

tctcccgccg tgcacagggt gtcacgttgc aagacctgcc tgaaaccgaa ctgcccgctg 8640

ttctgcagcc ggtcgcggag gctatggatg cgatcgctgc ggccgatctt agccagacga 8700

gcgggttcgg cccattcgga ccgcaaggaa tcggtcaata cactacatgg cgtgatttca 8760

tatgcgcgat tgctgatccc catgtgtatc actggcaaac tgtgatggac gacaccgtca 8820

gtgcgtccgt cgcgcaggct ctcgatgagc tgatgctttg ggccgaggac tgccccgaag 8880

tccggcacct cgtgcacgcg gatttcggct ccaacaatgt cctgacggac aatggccgca 8940

taacagcggt cattgactgg agcgaggcga tgttcgggga ttcccaatac gaggtcgcca 9000

acatcttctt ctggaggccg tggttggctt gtatggagca gcagacgcgc tacttcgagc 9060

ggaggcatcc ggagcttgca ggatcgccgc ggctccgggc gtatatgctc cgcattggtc 9120

ttgaccaact ctatcagagc ttggttgacg gcaatttcga tgatgcagct tgggcgcagg 9180

gtcgatgcga cgcaatcgtc cgatccggag ccgggactgt cgggcgtaca caaatcgccc 9240

gcagaagcgc ggccgtctgg accgatggct gtgtagaagt actcgccgat agtggaaacc 9300

gacgccccag cactcgtccg agggcaaagg aatagcggcc gcaagtgtgg atggggaagt 9360

gagtgcccgg ttctgtgtgc acaattggca atccaagatg gatggattca acacagggat 9420

atagcgagct acgtggtggt gcgaggatat agcaacggat atttatgttt gacacttgag 9480

aatgtacgat acaagcactg tccaagtaca atactaaaca tactgtacat actcatactc 9540

gtacccgggc aacggtttca cttgagtgca gtggctagtg ctcttactcg tacagtgtgc 9600

taccgttcgt atagcataca ttatacgaag ttatcatagt cttaattaaa ttttttttga 9660

ttttcttttt tgaccccgtc ttcaattaca cttcccaact gggaacaccc ctctttatcg 9720

acccatttta ggtaatttac cctagcccat tgtctccata aggaatatta ccctaaccca 9780

cagtccaggg tgcccaggtc cttctttggc caaattttaa cttcggtcct atggcacagc 9840

ggtagcgcgt gagattgcaa atcttaaggt cccgagttcg aatctcggtg ggacctagtt 9900

atttttgata gataatttcg tgatgattag aaacttaacg caaaataatg cctggctagc 9960

tcaatcggta gagcgtgaga ctcttataca agaaatctca aggctgtggg ttcaagcccc 10020

acgtcgggct aggtcgtgtg gtgtaatggt tatcacgagg ctccgagttc gatcctcggc 10080

atgatcaagt ctcaccgaca tggttatcgt tttagagcta gaaatagcaa gttaaaataa 10140

ggctagtccg ttatcaactt gaaaaagtgg caccgagtcg gtgctttttt ttttgttttt 10200

tatcgattga gcatccgttg atttccgaac agatcccaat attacaccca agtagcatgc 10260

ataagctaaa agtaactcgc agcgcacacc gtgcagattc ataagtctat gattaattga 10320

acgccaataa cccggcttac tacaagtaca agtaggtata catagcggta atgaatcatt 10380

agaaaaaaaa aaaacaaaaa aaaacaaaac aaactgttgt ggatgcatca acagtagtac 10440

atagttgtac gatgtacttg tacttgtaaa agcaaaaatg tacaatatct cagggagcgc 10500

aacttttacg ttcgaagaac aatgtaccgc ataccgcatt ctagattctg cggaacgtct     10560

aacctggaaa tacgattttt tttttctttc attttttttg cttcttcaaa agtatggtaa     10620

tttcctacca ttacagttga cactgaacga ggggggattg aatttaagca aaaaattaaa     10680

tcaaaatacc tttatgtatc cagcccatgt aataaacaaa aggattatat aacaagaaat     10740

aaatatatac ctttaatgga tcattagaat aaaaataaat acgagaagca caccagagaa     10800

gctttttgat tgccactata ccgctacttt ggtatatctt attataattg ttgaatttgc     10860

aagatagaat gtcattcatt ggagagaaat ccaaggaata tgtgggatga aatgactaga     10920

agtatgaaca atgagaatag tacatacttg tacctgtatt tctagaagag agaaagacag     10980

ttgagtgtgt gattctcgtc caataataat ctcaatagta acgtgtgaat agctgttctt     11040

tgatagttga tatttctcga tgactattta tgttgtacaa gggatttttt tcgttgctgt     11100

tgatttcgaa ttaggcaatg cagatatcat ttatgctatc catatttaag atttcccata     11160

cgcatttata acatttattc tacataaatt gttaaatgaa cgaactgcca ttataaattg     11220

tttcctaaat aggaagtgtt tttcataaag caagtaagtt gtctaataat actaagtaat     11280

aaaaataagt tcatacaata tattttgaga acatcatttg gaggcggtag atggagtctg     11340

tttattatta aacaatgcga gatgacccct taaatattga gaacatcagt tggaggcggc     11400

agatggagtc tgtctattta gcaatgggac atgactgtca gtatcatcat gatgtatata     11460

tataatacat ataatattat ataacacgat ttttttaaat tattggcccg aaaattaatc     11520

agtgtagact ggatctcggc agtctctcgg atgtagaatt aggtttcctt gaggcgaaga     11580

tcggtttgtg tgacatgaat tcgatatcaa gcttatcgac accatcgacc tcgagggggg     11640

gcccggtacc caattcgccc tatagtgagt cgtattacaa ttcactggcc gtcgttttac     11700

<210>  48
<211>  83
<212>  DNA
<213>  Artificial Sequence

<220>
<223>  Artificial Sequence

<400>  48
tcgggctagg tcgtgtggtg taatggttat cacccgagtt cgatcctcgg catgatcaag     60

tctcaccgac atggttatcg ttt                                             83

<210>  49
<211>  83
<212>  DNA
<213>  Artificial Sequence

<220>
<223>  Artificial Sequence

<400>  49
tctaaaacga taaccatgtc ggtgagactt gatcatgccg aggatcgaac tcgggtgata      60

accattacac cacacgacct agc                                              83


<210>  50
<211>  50
<212>  DNA
<213>  Artificial Sequence

<220>
<223>  Artificial Sequence

<400>  50
ggtcgtgtgg tgtaatggtt atcacccgag ttcgatcctc ggcatgatca                 50


<210>  51
<211>  11694
<212>  DNA
<213>  Artificial Sequence

<220>
<223>  Artificial Sequence

<400>  51
gccgtcgttt tacaacgtcg tgactgggaa aaccctggcg ttacccaact taatcgcctt      60

gcagcacatc cccctttcgc cagctggcgt aatagcgaag aggcccgcac cgatcgccct     120

tcccaacagt tgcgcagcct gaatggcgaa tggaaattgt aagcgttaat attttgttaa     180

aattcgcgtt aaatttttgt taaatcagct cattttttaa ccaataggcc gaaatcggca     240

aaatccctta taaatcaaaa gaatagaccg agatagggtt gagtgttgtt ccagtttgga     300

acaagagtcc actattaaag aacgtggact ccaacgtcaa agggcgaaaa accgtctatc     360

agggcgatgg cccactacgt gaaccatcac cctaatcaag ttttttgggg tcgaggtgcc     420

gtaaagcact aaatcggaac cctaaaggga gcccccgatt tagagcttga cggggaaagc     480

cggcgaacgt ggcgagaaag gaagggaaga aagcgaaagg agcgggcgct agggcgctgg     540

caagtgtagc ggtcacgctg cgcgtaacca ccacacccgc cgcgcttaat gcgccgctac     600

agggcgcgtc aggtggcact tttcggggaa atgtgcgcgg aacccctatt tgtttatttt     660

tctaaataca ttcaaatatg tatccgctca tgagacaata accctgataa atgcttcaat     720

aatattgaaa aaggaagagt atgagtattc aacatttccg tgtcgccctt attccctttt     780

ttgcggcatt ttgccttcct gtttttgctc acccagaaac gctggtgaaa gtaaaagatg     840

ctgaagatca gttgggtgca cgagtgggtt acatcgaact ggatctcaac agcggtaaga     900

tccttgagag ttttcgcccc gaagaacgtt ttccaatgat gagcactttt aaagttctgc     960

tatgtggcgc ggtattatcc cgtattgacg ccgggcaaga gcaactcggt cgccgcatac    1020

actattctca gaatgacttg gttgagtact caccagtcac agaaaagcat cttacggatg    1080

gcatgacagt aagagaatta tgcagtgctg ccataaccat gagtgataac actgcggcca 1140

acttacttct gacaacgatc ggaggaccga aggagctaac cgcttttttg cacaacatgg 1200

gggatcatgt aactcgcctt gatcgttggg aaccggagct gaatgaagcc ataccaaacg 1260

acgagcgtga caccacgatg cctgtagcaa tggcaacaac gttgcgcaaa ctattaactg 1320

gcgaactact tactctagct tcccggcaac aattaataga ctggatggag gcggataaag 1380

ttgcaggacc acttctgcgc tcggcccttc cggctggctg gtttattgct gataaatctg 1440

gagccggtga gcgtgggtct cgcggtatca ttgcagcact ggggccagat ggtaagccct 1500

cccgtatcgt agttatctac acgacgggga gtcaggcaac tatggatgaa cgaaatagac 1560

agatcgctga gataggtgcc tcactgatta agcattggta actgtcagac caagtttact 1620

catatatact ttagattgat ttaaaacttc atttttaatt taaaaggatc taggtgaaga 1680

tcctttttga taatctcatg accaaaatcc cttaacgtga gttttcgttc cactgagcgt 1740

cagaccccgt agaaaagatc aaaggatctt cttgagatcc tttttttctg cgcgtaatct 1800

gctgcttgca aacaaaaaaa ccaccgctac cagcggtggt ttgtttgccg gatcaagagc 1860

taccaactct ttttccgaag gtaactggct tcagcagagc gcagatacca aatactgtcc 1920

ttctagtgta gccgtagtta ggccaccact tcaagaactc tgtagcaccg cctacatacc 1980

tcgctctgct aatcctgtta ccagtggctg ctgccagtgg cgataagtcg tgtcttaccg 2040

ggttggactc aagacgatag ttaccggata aggcgcagcg gtcgggctga acggggggtt 2100

cgtgcacaca gcccagcttg gagcgaacga cctacaccga actgagatac ctacagcgtg 2160

agctatgaga aagcgccacg cttcccgaag ggagaaaggc ggacaggtat ccggtaagcg 2220

gcagggtcgg aacaggagag cgcacgaggg agcttccagg gggaaacgcc tggtatcttt 2280

atagtcctgt cgggtttcgc cacctctgac ttgagcgtcg atttttgtga tgctcgtcag 2340

gggggcggag cctatggaaa aacgccagca acgcggcctt tttacggttc ctggcctttt 2400

gctggccttt tgctcacatg ttctttcctg cgttatcccc tgattctgtg gataaccgta 2460

ttaccgcctt tgagtgagct gataccgctc gccgcagccg aacgaccgag cgcagcgagt 2520

cagtgagcga ggaagcggaa gagcgcccaa tacgcaaacc gcctctcccc gcgcgttggc 2580

cgattcatta atgcagctgg cacgacaggt ttcccgactg gaaagcgggc agtgagcgca 2640

acgcaattaa tgtgagttag ctcactcatt aggcacccca ggctttacac tttatgcttc 2700

cggctcgtac gcaactaaca tgaatgaata cgatatacat caaagactat gatacgcagt 2760

attgcacact gtacgagtaa gagcactagc cactgcactc aagtgaaacc gttgcccggg 2820

tacgagtatg agtatgtaca gtatgtttag tattgtactt ggacagtgct tgtatcgtac 2880

attctcaagt gtcaaacata aatatccgtt gctatatcct cgcaccacca cgtagctcgc 2940

tatatccctg tgttgaatcc atccatcttg gattgccaat tgtgcacaca gaaccgggca 3000

ctcacttccc catccacact tgcggccgct tagacctttc gctttttctt gggatcggct 3060

ctggagtcgc caccaagctg agacaggtcg attcgggtct cgtacaggcc agtgatggac 3120

tggtgaatca gggtggcatc gagaacctcc ttggtggatg tgtaccgctt tcggtcgata 3180

gtggtatcga agtacttgaa agctgcagga gcacccaggt tggtaagagt aaacaggtga 3240

atgatgttct ccgcctgttc tcgaatgggt ttgtcccgat gcttgttgta ggcagagagc 3300

accttgtcca agttggcatc agccaggatg actcgcttcg aaaactcgga aatctgctcg 3360

ataatctcgt cgaggtaatg tttgtgctgc tcaacgaaga gttgcttctg ttcgttgtcc 3420

tcgggagaac ccttgagctt ctcgtagtga gaagccagat agagaaagtt gacgtacttc 3480

gaaggcaagg caagctcgtt tcccttctgc agctcgccag cggaggcgag catacgcttt 3540

cgaccgttct ccagttcgaa cagagagtac ttgggcagct tgataatgag gtctttcttg 3600

acctccttgt aacccttggc ttccaagaag tcgatgggat tcttctcgaa gctcgatcgc 3660

tccatgatgg taattccgag cagctccttg acggacttga gctttttgga cttgcccttc 3720

tcgaccttcg caacgacaag cacggaatag gcgacggtag gagaatcgaa gccaccgtat 3780

ttcttgggat cccagtcttt ctttcgagcg atgagcttgt cggagtttcg cttgggcaga 3840

atcgactcct tggagaatcc gccagtctga acctcggttt tcttgacgat gttgacctga 3900

ggcatcgaca gaacctttcg cacggttgca aagtctcgac ccttgtccca cacgatctct 3960

ccagtttcgc cgttggtctc gataagtggt ctctttcgaa tctctccgtt ggccaaggtg 4020

atctcggtct tgaaaaagtt catgatgttg gagtaaaaga agtacttggc agtagccttg 4080

ccaatctcct gttcggactt ggcaatcatc tttcgaacgt cgtagacctt gtaatcgccg 4140

taaacgaact cgctttcgag cttggggtat ttcttgatga gcgcagtgcc aacgacggcg 4200

ttgaggtaag catcgtgggc atggtggtaa ttgttgatct ctcgcacctt gtagaactga 4260

aagtcctttc ggaaatcgga gaccagtttg gacttgagag taatcacctt gacctctcgg 4320

atgagcttgt cgttctcgtc gtacttggtg ttcatccgag aatcgagaat ctgtgcgacg 4380

tgctttgtga tctgtctggt ctcgacgagt tgacgcttga tgaagccagc cttgtcgagc 4440

tcggacagac cgcctcgctc ggccttggta agattgtcga actttcgctg ggtaatgagc 4500

ttggcgttga gcagctgtcg ccagtagttc ttcatctttt tgaccacctc ttcgctggga 4560

acgttgtccg acttgcctct gttcttgtcg gatcgtgtaa ggaccttgtt gtcgatagaa 4620

tcgtccttga gaaaggattg agggacaatg tggtccacat cgtagtcgct gagacgattg 4680

atgtccagtt cctgatccac gtacatgtct cgaccattct gcagatagta gagatacagc 4740

ttctcgttct gcagttgagt gttctcgacg ggatgctcct tgagaatctg ggatcccagc 4800

tccttgatgc cttcctcgat tcgcttcatc cgctctcgcg agtttttctg acccttttga 4860

gttgtctggt tctctctggc catctcgatc acaatgttct cgggcttgtg acgtcccatg 4920
accttcacca gctcgtcgac aaccttgaca gtctggagaa tgcctttctt gatggctggc 4980
gaaccagcca ggttggcaat atgttcgtgc aagctgtcgc cctgaccgga cacttgtgcc 5040
ttctggatgt cctccttgaa ggtaagagaa tcgtcgtgaa tgagctgcat gaagtttcgg 5100
ttggcaaagc catcggactt gagaaagtcc agaatggtct ttccggactg cttgtctctg 5160
atgccgttga tgagctttcg cgaaagtctt ccccagccgg tgtatctacg tcgcttgagt 5220
tgtttcatga ccttgtcgtc gaacaggtga gcgtatgtct tgagtcgttc ctcgatcatc 5280
tcccgatctt cgaacagggt aagagtgagc acgatgtcct ccagaatgtc ctcgttttcc 5340
tcgttgtcga gaaaatcctt gtccttgata atcttgagca gatcgtgata ggtgcccaaa 5400
gaggcgttga atcggtcctc aactccggaa atctcgacgc tgtcgaaaca ctcgattttc 5460
ttgaagtagt cctccttgag ctgcttaaca gtgacctttc ggttggtctt gaacaggaga 5520
tcgacaatgg ctttcttctg ttcgccagac aagaaggcag gctttcgcat tccctcggta 5580
acgtacttga ctttggtgag ttcgttgtag actgtaaagt actcgtagag cagcgaatgc 5640
ttgggaagaa ccttctcgtt gggcagattc ttgtcgaagt tggtcattcg ctcgatgaag 5700
gactgtgcag aggcaccctt gtccacgact tcctcgaagt tccagggagt gatggtttcc 5760
tcggactttc gagtcatcca agcaaatcga gagtttcctc tggcaagagg accaacatag 5820
taggggattc gaaaggtaag aatcttctcg atcttctctc ggttgtcctt gagaaagggg 5880
tagaagtctt cctgacgtcg aagaatggcg tgcagctcac cgaggtggat ctgatgagga 5940
atgctgccgt tgtcgaaggt tcgttgcttc cgaagcagat cctctcgatt gagcttgaca 6000
agcagttcct cggttccgtc catcttctcg agaattggct tgatgaactt gtagaactct 6060
tcctgagagg ctccgccgtc gatgtatcca gcgtagccgt tcttcgactg atcgaaaaag 6120
atctccttgt acttctcggg cagttgctgt cggacaagag ccttgagcag tgtgagatcc 6180
tgatggtgct cgtcgtatcg cttgatcatg gaggcagaaa ggggagcctt tgtgatctcg 6240
gtgttgactc gcagaatgtc agacaagaga atagcatccg aaaggttctt ggcagcgaga 6300
aacaggtcgg cgtactgatc gccaatctgt gcaagcaggt tgtcgaggtc atcgtcgtag 6360
gtgtccttgg acagctggag cttggcgtcc tccgccagat cgaagttgga cttgaagttg 6420
ggtgtgagac caagagaaag ggcaatgagg ttgccaaaca gtccgttctt tttctcgcca 6480
ggaagttggg caatgaggtt ctccagtcgt ctgctcttcg agagtcgagc agacaagatg 6540
gcctttgcat cgactccgga ggcattgatg gggttttcct cgaacagctg gttgtaggtc 6600
tgaacgagct gaatgaacag cttgtccaca tcgctgttgt cgggattgag atcgccctcg 6660
atgaggaaat gacctcgaaa cttgatcatg tgtgccagag cgaggtagat aagtctgaga 6720
tccgccttgt cggtggaatc gacgagtttc tttcggagat ggtagatggt aggatacttc 6780

tcgtggtaag caacctcgtc cacaatgttg ccaaagatgg gatgacgctc gtgtttcttg 6840

tcttcctcga cgaggaagga ttcctccagt cgatgaaaga acgaatcgtc caccttggcc 6900

atctcgttgg aaaagatctc ctgcaggtag cagattcggt tcttccgtcg ggtgtaacgt 6960

cgccgagcag ttcgcttgag tctggtagct tcggcagtct cgccagaatc gaacaacagg 7020

gcaccaatga ggtttttctt gatggagtgt cgatcggtgt ttccgaggac cttgaatttc 7080

ttggagggca ccttgtactc gtcggtgatg acagcccagc cgacagagtt ggttccaatg 7140

tccaggccga tggagtattt cttgtccatg gtgtgatgtg tagtttagat ttcgaatctg 7200

tggggaaaga aaggaaaaaa gagactggca accgattggg agagccactg tttatatata 7260

ccctagacaa gccccccgct tgtaagatgt tggtcaatgt aaaccagtat taaggttggc 7320

aagtgcagga gaagcaaggt gtgggtaccg agcaatggaa atgtgcggaa ggcaaaaaaa 7380

tgaggccacg gcctattgtc ggggctatat ccagggggcg attgaagtac actaacatga 7440

catgtgtcca cagaccctca atctggcctg atgagccaaa tccatacgcg ctttcgcagc 7500

tctaaaggct ataacaagtc acaccaccct gctcgacctc agcgccctca ctttttgtta 7560

agacaaactg tacacgctgt tccagcgttt tctgcctgca cctggtggga catttggtgc 7620

aacctaaagt gctcggaacc tctgtggtgt ccagatcagc gcagcagttc cgaggtagtt 7680

ttgaggccct tagatgatgg tttaaacctt aagcccgctc ataacttcgt atagcataca 7740

ttatacgaac ggtaggttgc gggatagacg ccgacggagg gcaatggcgc tatggaacct 7800

tgcggatatc catacgccgc ggcggactgc gtccgaacca gctccagcag cgttttttcc 7860

gggccattga gccgactgcg accccgccaa cgtgtcttgg cccacgcact catgtcatgt 7920

tggtgttggg aggccacttt ttaagtagca caaggcacct agctcgcagc aaggtgtccg 7980

aaccaaagaa gcggctgcag tggtgcaaac ggggcggaaa cggcgggaaa aagccacggg 8040

ggcacgaatt gaggcacgcc ctcgaatttg agacgagtca cggccccatt cgcccgcgca 8100

atggctcgcc aacgcccggt cttttgcacc acatcaggtt accccaagcc aaacctttgt 8160

gttaaaaagc ttaacatatt ataccgaacg taggtttggg cgggcttgct ccgtctgtcc 8220

aaggcaacat ttatataagg gtctgcatcg ccggctcaat tgaatctttt ttcttcttct 8280

cttctctata ttcattcttg aattaaacac acatcaacca tggccaaaaa gcctgaactc 8340

accgcgacgt ctgtcgagaa gtttctgatc gaaaagttcg acagcgtctc cgacctgatg 8400

cagctctcgg agggcgaaga atctcgtgct ttcagcttcg atgtaggagg gcgtggatat 8460

gtcctgcggg taaatagctg cgccgatggt ttctacaaag atcgttatgt ttatcggcac 8520

tttgcatcgg ccgcgctccc gattccggaa gtgcttgaca ttggggagtt cagcgagagc 8580

ctgacctatt gcatctcccg ccgtgcacag ggtgtcacgt tgcaagacct gcctgaaacc 8640

gaactgcccg ctgttctgca gccggtcgcg gaggctatgg atgcgatcgc tgcggccgat 8700

cttagccaga cgagcgggtt cggcccattc ggaccgcaag gaatcggtca atacactaca 8760

tggcgtgatt tcatatgcgc gattgctgat ccccatgtgt atcactggca aactgtgatg 8820

gacgacaccg tcagtgcgtc cgtcgcgcag gctctcgatg agctgatgct ttgggccgag 8880

gactgccccg aagtccggca cctcgtgcac gcggatttcg gctccaacaa tgtcctgacg 8940

gacaatggcc gcataacagc ggtcattgac tggagcgagg cgatgttcgg ggattcccaa 9000

tacgaggtcg ccaacatctt cttctggagg ccgtggttgg cttgtatgga gcagcagacg 9060

cgctacttcg agcggaggca tccggagctt gcaggatcgc cgcggctccg ggcgtatatg 9120

ctccgcattg gtcttgacca actctatcag agcttggttg acggcaattt cgatgatgca 9180

gcttgggcgc agggtcgatg cgacgcaatc gtccgatccg gagccgggac tgtcgggcgt 9240

acacaaatcg cccgcagaag cgcggccgtc tggaccgatg gctgtgtaga agtactcgcc 9300

gatagtggaa accgacgccc cagcactcgt ccgagggcaa aggaatagcg gccgcaagtg 9360

tggatgggga agtgagtgcc cggttctgtg tgcacaattg gcaatccaag atggatggat 9420

tcaacacagg gatatagcga gctacgtggt ggtgcgagga tatagcaacg gatatttatg 9480

tttgacactt gagaatgtac gatacaagca ctgtccaagt acaatactaa acatactgta 9540

catactcata ctcgtacccg ggcaacggtt tcacttgagt gcagtggcta gtgctcttac 9600

tcgtacagtg tgctaccgtt cgtatagcat acattatacg aagttatcat agtcttaatt 9660

aaattttttt tgatttcttt ttttgacccc gtcttcaatt acacttccca actgggaaca 9720

cccctcttta tcgacccatt ttaggtaatt taccctagcc cattgtctcc ataaggaata 9780

ttaccctaac ccacagtcca gggtgcccag gtccttcttt ggccaaattt taacttcggt 9840

cctatggcac agcggtagcg cgtgagattg caaatcttaa ggtcccgagt tcgaatctcg 9900

gtgggaccta gttatttttg atagataatt tcgtgatgat tagaaactta acgcaaaata 9960

atgcctggct agctcaatcg gtagagcgtg agactcttat acaagaaatc tcaaggctgt 10020

gggttcaagc cccacgtcgg gctaggtcgt gtggtgtaat ggttatcacc cgagttcgat 10080

cctcggcatg atcaagtctc accgacatgg ttatcgtttt agagctagaa atagcaagtt 10140

aaaataaggc tagtccgtta tcaacttgaa aaagtggcac cgagtcggtg cttttttttt 10200

tgttttttat cgattgagca tccgttgatt tccgaacaga tcccaatatt acacccaagt 10260

agcatgcata agctaaaagt aactcgcagc gcacaccgtg cagattcata agtctatgat 10320

taattgaacg ccaataaccc ggcttactac aagtacaagt aggtatacat agcggtaatg 10380

aatcattaga aaaaaaaaaa acaaaaaaaa acaaaacaaa ctgttgtgga tgcatcaaca 10440

gtagtacata gttgtacgat gtacttgtac ttgtaaaagc aaaaatgtac aatatctcag 10500

ggagcgcaac ttttacgttc gaagaacaat gtaccgcata ccgcattcta gattctgcgg 10560

aacgtctaac ctggaaatac gatttttttt ttctttcatt ttttttgctt cttcaaaagt 10620

atggtaattt cctaccatta cagttgacac tgaacgaggg gggattgaat ttaagcaaaa 10680

aattaaatca aaataccttt atgtatccag cccatgtaat aaacaaaagg attatataac 10740

aagaaataaa tatatacctt taatggatca ttagaataaa aataaatacg agaagcacac 10800

cagagaagct ttttgattgc cactataccg ctactttggt atatcttatt ataattgttg 10860

aatttgcaag atagaatgtc attcattgga gagaaatcca aggaatatgt gggatgaaat 10920

gactagaagt atgaacaatg agaatagtac atacttgtac ctgtatttct agaagagaga 10980

aagacagttg agtgtgtgat tctcgtccaa taataatctc aatagtaacg tgtgaatagc 11040

tgttctttga tagttgatat ttctcgatga ctatttatgt tgtacaaggg atttttttcg 11100

ttgctgttga tttcgaatta ggcaatgcag atatcattta tgctatccat atttaagatt 11160

tcccatacgc atttataaca tttattctac ataaattgtt aaatgaacga actgccatta 11220

taaattgttt cctaaatagg aagtgttttt cataaagcaa gtaagttgtc taataatact 11280

aagtaataaa aataagttca tacaatatat tttgagaaca tcatttggag gcggtagatg 11340

gagtctgttt attattaaac aatgcgagat gaccccttaa atattgagaa catcagttgg 11400

aggcggcaga tggagtctgt ctatttagca atgggacatg actgtcagta tcatcatgat 11460

gtatatatat aatacatata atattatata acacgatttt tttaaattat tggcccgaaa 11520

attaatcagt gtagactgga tctcggcagt ctctcggatg tagaattagg tttccttgag 11580

gcgaagatcg gtttgtgtga catgaattcg atatcaagct tatcgacacc atcgacctcg 11640

agggggggcc cggtacccaa ttcgccctat agtgagtcgt attacaattc actg 11694

<210> 52
<211> 69
<212> DNA
<213> Artificial Sequence

<220>
<223> Artificial Sequence

<400> 52
tcgggctagg tcgtgtggtc cgagttcgat cctcggcatg atcaagtctc accgacatgg 60

ttatcgttt 69

<210> 53
<211> 69
<212> DNA
<213> Artificial Sequence

<220>
<223> Artificial Sequence

<400> 53

tctaaaacga taaccatgtc ggtgagactt gatcatgccg aggatcgaac tcggaccaca 60

cgacctagc 69

<210> 54
<211> 36
<212> DNA
<213> Artificial Sequence

<220>
<223> Artificial Sequence

<400> 54
ggtcgtgtgg tccgagttcg atcctcggca tgatca 36

<210> 55
<211> 11680
<212> DNA
<213> Artificial Sequence

<220>
<223> Artificial Sequence

<400> 55
   gtcgttttac aacgtcgtga ctgggaaaac cctggcgtta cccaacttaa tcgccttgca 60
   gcacatcccc ctttcgccag ctggcgtaat agcgaagagg cccgcaccga tcgcccttcc 120
   caacagttgc gcagcctgaa tggcgaatgg aaattgtaag cgttaatatt ttgttaaaat 180
   tcgcgttaaa tttttgttaa atcagctcat tttttaacca ataggccgaa atcggcaaaa 240
   tcccttataa atcaaaagaa tagaccgaga tagggttgag tgttgttcca gtttggaaca 300
   agagtccact attaaagaac gtggactcca acgtcaaagg gcgaaaaacc gtctatcagg 360
   gcgatggccc actacgtgaa ccatcaccct aatcaagttt tttggggtcg aggtgccgta 420
   aagcactaaa tcggaaccct aaagggagcc cccgatttag agcttgacgg ggaaagccgg 480
   cgaacgtggc gagaaaggaa gggaagaaag cgaaaggagc gggcgctagg gcgctggcaa 540
   gtgtagcggt cacgctgcgc gtaaccacca cacccgccgc gcttaatgcg ccgctacagg 600
   gcgcgtcagg tggcactttt cggggaaatg tgcgcggaac ccctatttgt ttatttttct 660
   aaatacattc aaatatgtat ccgctcatga gacaataacc ctgataaatg cttcaataat 720
   attgaaaaag gaagagtatg agtattcaac atttccgtgt cgcccttatt cccttttttg 780
   cggcattttg ccttcctgtt tttgctcacc cagaaacgct ggtgaaagta aaagatgctg 840
   aagatcagtt gggtgcacga gtgggttaca tcgaactgga tctcaacagc ggtaagatcc 900
   ttgagagttt tcgccccgaa gaacgttttc caatgatgag cacttttaaa gttctgctat 960
   gtggcgcggt attatcccgt attgacgccg ggcaagagca actcggtcgc cgcatacact 1020
   attctcagaa tgacttggtt gagtactcac cagtcacaga aaagcatctt acggatggca 1080
   tgacagtaag agaattatgc agtgctgcca taaccatgag tgataacact gcggccaact 1140
   tacttctgac aacgatcgga ggaccgaagg agctaaccgc ttttttgcac aacatggggg 1200
   atcatgtaac tcgccttgat cgttgggaac cggagctgaa tgaagccata ccaaacgacg 1260
   agcgtgacac cacgatgcct gtagcaatgg caacaacgtt gcgcaaacta ttaactggcg 1320
   aactacttac tctagcttcc cggcaacaat taatagactg gatggaggcg gataaagttg 1380
   caggaccact tctgcgctcg gcccttccgg ctggctggtt tattgctgat aaatctggag 1440
   ccggtgagcg tgggtctcgc ggtatcattg cagcactggg gccagatggt aagccctccc 1500
   gtatcgtagt tatctacacg acggggagtc aggcaactat ggatgaacga aatagacaga 1560
   tcgctgagat aggtgcctca ctgattaagc attggtaact gtcagaccaa gtttactcat 1620
   atatacttta gattgattta aaacttcatt tttaatttaa aaggatctag gtgaagatcc 1680
   tttttgataa tctcatgacc aaaatccctt aacgtgagtt ttcgttccac tgagcgtcag 1740
   accccgtaga aaagatcaaa ggatcttctt gagatccttt ttttctgcgc gtaatctgct 1800
   gcttgcaaac aaaaaaacca ccgctaccag cggtggtttg tttgccggat caagagctac 1860
   caactctttt tccgaaggta actggcttca gcagagcgca gataccaaat actgtccttc 1920
   tagtgtagcc gtagttaggc caccacttca agaactctgt agcaccgcct acatacctcg 1980
   ctctgctaat cctgttacca gtggctgctg ccagtggcga taagtcgtgt cttaccgggt 2040
   tggactcaag acgatagtta ccggataagg cgcagcggtc gggctgaacg gggggttcgt 2100
   gcacacagcc cagcttggag cgaacgacct acaccgaact gagataccta cagcgtgagc 2160
   tatgagaaag cgccacgctt cccgaaggga gaaaggcgga caggtatccg gtaagcggca 2220
   gggtcggaac aggagagcgc acgagggagc ttccaggggg aaacgcctgg tatctttata 2280
   gtcctgtcgg gtttcgccac ctctgacttg agcgtcgatt tttgtgatgc tcgtcagggg 2340
   ggcggagcct atggaaaaac gccagcaacg cggccttttt acggttcctg gccttttgct 2400
   ggccttttgc tcacatgttc tttcctgcgt tatcccctga ttctgtggat aaccgtatta 2460
   ccgcctttga gtgagctgat accgctcgcc gcagccgaac gaccgagcgc agcgagtcag 2520
   tgagcgagga agcggaagag cgcccaatac gcaaaccgcc tctccccgcg cgttggccga 2580
   ttcattaatg cagctggcac gacaggtttc ccgactggaa agcgggcagt gagcgcaacg 2640
   caattaatgt gagttagctc actcattagg caccccaggc tttacacttt atgcttccgg 2700
   ctcgtacgca actaacatga atgaatacga tatacatcaa agactatgat acgcagtatt 2760
   gcacactgta cgagtaagag cactagccac tgcactcaag tgaaaccgtt gcccgggtac 2820
   gagtatgagt atgtacagta tgtttagtat tgtacttgga cagtgcttgt atcgtacatt 2880
   ctcaagtgtc aaacataaat atccgttgct atatcctcgc accaccacgt agctcgctat 2940
   atccctgtgt tgaatccatc catcttggat tgccaattgt gcacacagaa ccgggcactc 3000
   acttccccat ccacacttgc ggccgcttag acctttcgct ttttcttggg atcggctctg 3060
   gagtcgccac caagctgaga caggtcgatt cgggtctcgt acaggccagt gatggactgg 3120
   tgaatcaggg tggcatcgag aacctccttg gtggatgtgt accgctttcg gtcgatagtg 3180
   gtatcgaagt acttgaaagc tgcaggagca cccaggttgg taagagtaaa caggtgaatg 3240
   atgttctccg cctgttctcg aatgggtttg tcccgatgct tgttgtaggc agagagcacc 3300
   ttgtccaagt tggcatcagc caggatgact cgcttcgaaa actcggaaat ctgctcgata 3360
   atctcgtcga ggtaatgttt gtgctgctca acgaagagtt gcttctgttc gttgtcctcg 3420
   ggagaaccct tgagcttctc gtagtgagaa gccagataga gaaagttgac gtacttcgaa 3480
   ggcaaggcaa gctcgtttcc cttctgcagc tcgccagcgg aggcgagcat acgctttcga 3540
   ccgttctcca gttcgaacag agagtacttg ggcagcttga taatgaggtc tttcttgacc 3600
   tccttgtaac ccttggcttc caagaagtcg atgggattct tctcgaagct cgatcgctcc 3660
   atgatggtaa ttccgagcag ctccttgacg gacttgagct tttggacttg gcccttctcg 3720
   accttcgcaa cgacaagcac ggaataggcg acggtaggag aatcgaagcc accgtatttc 3780
   ttgggatccc agtctttctt tcgagcgatg agcttgtcgg agtttcgctt gggcagaatc 3840
   gactccttgg agaatccgcc agtctgaacc tcggttttct tgacgatgtt gacctgaggc 3900
   atcgacagaa cctttcgcac ggttgcaaag tctcgaccct tgtcccacac gatctctcca 3960
   gtttcgccgt tggtctcgat aagtggtctc tttcgaatct ctccgttggc caaggtgatc 4020
   tcggtcttga aaaagttcat gatgttggag taaaagaagt acttggcagt agccttgcca 4080
   atctcctgtt cggacttggc aatcatcttt cgaacgtcgt agaccttgta atcgccgtaa 4140
   acgaactcgc tttcgagctt ggggtatttc ttgatgagcg cagtgccaac gacggcgttg 4200
   aggtaagcat cgtgggcatg gtggtaattg ttgatctctc gcaccttgta gaactgaaag 4260
   tcctttcgga aatcggagac cagtttggac ttgagagtaa tcaccttgac ctctcggatg 4320
   agcttgtcgt tctcgtcgta cttggtgttc atccgagaat cgagaatctg tgcgacgtgc 4380
   tttgtgatct gtctggtctc gacgagttga cgcttgatga agccagcctt gtcgagctcg 4440
   gacagaccgc ctcgctcggc cttggtaaga ttgtcgaact ttcgctgggt aatgagcttg 4500
   gcgttgagca gctgtcgcca gtagttcttc atctttttga ccacctcttc gctgggaacg 4560
   ttgtccgact tgcctctgtt cttgtcggat cgtgtaagga ccttgttgtc gatagaatcg 4620
   tccttgagaa aggattgagg gacaatgtgg tccacatcgt agtcgctgag acgattgatg 4680
   tccagttcct gatccacgta catgtctcga ccattctgca gatagtagag atacagcttc 4740
   tcgttctgca gttgagtgtt ctcgacggga tgctccttga gaatctggga tcccagctcc 4800
   ttgatgcctt cctcgattcg cttcatccgc tctcgcgagt ttttctgacc cttttgagtt 4860
   gtctggttct ctctggccat ctcgatcaca atgttctcgg gcttgtgacg tcccatgacc 4920
   ttcaccagct cgtcgacaac cttgacagtc tggagaatgc ctttcttgat ggctggcgaa 4980
   ccagccaggt tggcaatatg ttcgtgcaag ctgtcgccct gaccggacac ttgtgccttc 5040
   tggatgtcct ccttgaaggt aagagaatcg tcgtgaatga gctgcatgaa gtttcggttg 5100
   gcaaagccat cggacttgag aaagtccaga atggtctttc cggactgctt gtctctgatg 5160
   ccgttgatga gctttcgcga aagtcttccc cagccggtgt atctacgtcg cttgagttgt 5220
   ttcatgacct tgtcgtcgaa caggtgagcg tatgtcttga gtcgttcctc gatcatctcc 5280
   cgatcttcga acagggtaag agtgagcacg atgtcctcca gaatgtcctc gttttcctcg 5340
   ttgtcgagaa aatccttgtc cttgataatc ttgagcagat cgtgataggt gcccaaagag 5400
   gcgttgaatc ggtcctcaac tccggaaatc tcgacgctgt cgaaacactc gattttcttg 5460
   aagtagtcct ccttgagctg cttaacagtg acctttcggt tggtcttgaa caggagatcg 5520
   acaatggctt tcttctgttc gccagacaag aaggcaggct ttcgcattcc ctcggtaacg 5580
   tacttgactt tggtgagttc gttgtagact gtaaagtact cgtagagcag cgaatgcttg 5640
   ggaagaacct tctcgttggg cagattcttg tcgaagttgg tcattcgctc gatgaaggac 5700
   tgtgcagagg cacccttgtc cacgacttcc tcgaagttcc agggagtgat ggtttcctcg 5760
   gactttcgag tcatccaagc aaatcgagag tttcctctgg caagaggacc aacatagtag 5820
   gggattcgaa aggtaagaat cttctcgatc ttctctcggt tgtccttgag aaaggggtag 5880
   aagtcttcct gacgtcgaag aatggcgtgc agctcaccga ggtggatctg atgaggaatg 5940
   ctgccgttgt cgaaggttcg ttgcttccga agcagatcct ctcgattgag cttgacaagc 6000
   agttcctcgg ttccgtccat cttctcgaga attggcttga tgaacttgta gaactcttcc 6060
   tgagaggctc cgccgtcgat gtatccagcg tagccgttct tcgactgatc gaaaaagatc 6120
   tccttgtact tctcgggcag ttgctgtcgg acaagagcct tgagcagtgt gagatcctga 6180
   tggtgctcgt cgtatcgctt gatcatggag gcagaaaggg gagcctttgt gatctcggtg 6240
   ttgactcgca gaatgtcaga caagagaata gcatccgaaa ggttcttggc agcgagaaac 6300
   aggtcggcgt actgatcgcc aatctgtgca agcaggttgt cgaggtcatc gtcgtaggtg 6360
   tccttggaca gctggagctt ggcgtcctcc gccagatcga agttggactt gaagttgggt 6420
   gtgagaccaa gagaaagggc aatgaggttg ccaaacagtc cgttcttttt ctcgccagga 6480
   agttgggcaa tgaggttctc cagtcgtctg ctcttcgaga gtcgagcaga caagatggcc 6540
   tttgcatcga ctccggaggc attgatgggg ttttcctcga acagctggtt gtaggtctga 6600
   acgagctgaa tgaacagctt gtccacatcg ctgttgtcgg gattgagatc gccctcgatg 6660
   aggaaatgac ctcgaaactt gatcatgtgt gccagagcga ggtagataag tctgagatcc 6720
   gccttgtcgg tggaatcgac gagtttcttt cggagatggt agatggtagg atacttctcg 6780
   tggtaagcaa cctcgtccac aatgttgcca aagatgggat gacgctcgtg tttcttgtct 6840
   tcctcgacga ggaaggattc ctccagtcga tgaaagaacg aatcgtccac cttggccatc 6900
   tcgttggaaa agatctcctg caggtagcag attcggttct tccgtcgggt gtaacgtcgc 6960
   cgagcagttc gcttgagtct ggtagcttcg gcagtctcgc cagaatcgaa caacagggca 7020
   ccaatgaggt ttttcttgat ggagtgtcga tcggtgtttc cgaggacctt gaatttcttg 7080
   gagggcacct tgtactcgtc ggtgatgaca gcccagccga cagagttggt tccaatgtcc 7140
   aggccgatgg agtatttctt gtccatggtg tgatgtgtag tttagatttc gaatctgtgg 7200
   ggaaagaaag gaaaaaagag actggcaacc gattgggaga gccactgttt atatataccc 7260
   tagacaagcc ccccgcttgt aagatgttgg tcaatgtaaa ccagtattaa ggttggcaag 7320
   tgcaggagaa gcaaggtgtg ggtaccgagc aatggaaatg tgcggaaggc aaaaaaatga 7380
   ggccacggcc tattgtcggg gctatatcca gggggcgatt gaagtacact aacatgacat 7440
   gtgtccacag accctcaatc tggcctgatg agccaaatcc atacgcgctt tcgcagctct 7500
   aaaggctata acaagtcaca ccaccctgct cgacctcagc gccctcactt tttgttaaga 7560
   caaactgtac acgctgttcc agcgttttct gcctgcacct ggtgggacat ttggtgcaac 7620
   ctaaagtgct cggaacctct gtggtgtcca gatcagcgca gcagttccga ggtagttttg 7680
   aggcccttag atgatggttt aaaccttaag cccgctcata acttcgtata gcatacatta 7740
   tacgaacggt aggttgcggg atagacgccg acggagggca atggcgctat ggaaccttgc 7800
   ggatatccat acgccgcggc ggactgcgtc cgaaccagct ccagcagcgt tttttccggg 7860
   ccattgagcc gactgcgacc ccgccaacgt gtcttggccc acgcactcat gtcatgttgg 7920
   tgttgggagg ccacttttta agtagcacaa ggcacctagc tcgcagcaag gtgtccgaac 7980
   caaagaagcg gctgcagtgg tgcaaacggg gcggaaacgg cgggaaaaag ccacgggggc 8040
   acgaattgag gcacgccctc gaatttgaga cgagtcacgg ccccattcgc ccgcgcaatg 8100
   gctcgccaac gcccggtctt ttgcaccaca tcaggttacc ccaagccaaa cctttgtgtt 8160
   aaaaagctta acatattata ccgaacgtag gtttgggcgg gcttgctccg tctgtccaag 8220
   gcaacattta tataagggtc tgcatcgccg gctcaattga atcttttttc ttcttctctt 8280
   ctctatattc attcttgaat taaacacaca tcaaccatgg ccaaaaagcc tgaactcacc 8340
   gcgacgtctg tcgagaagtt tctgatcgaa aagttcgaca gcgtctccga cctgatgcag 8400
   ctctcggagg gcgaagaatc tcgtgctttc agcttcgatg taggagggcg tggatatgtc 8460
   ctgcgggtaa atagctgcgc cgatggtttc tacaaagatc gttatgttta tcggcacttt 8520
   gcatcggccg cgctcccgat tccggaagtg cttgacattg gggagttcag cgagagcctg 8580
   acctattgca tctcccgccg tgcacagggt gtcacgttgc aagacctgcc tgaaaccgaa 8640
   ctgcccgctg ttctgcagcc ggtcgcggag gctatggatg cgatcgctgc ggccgatctt 8700
   agccagacga gcgggttcgg cccattcgga ccgcaaggaa tcggtcaata cactacatgg 8760
   cgtgatttca tatgcgcgat tgctgatccc catgtgtatc actggcaaac tgtgatggac 8820
   gacaccgtca gtgcgtccgt cgcgcaggct ctcgatgagc tgatgctttg ggccgaggac 8880
   tgccccgaag tccggcacct cgtgcacgcg gatttcggct ccaacaatgt cctgacggac 8940
   aatggccgca taacagcggt cattgactgg agcgaggcga tgttcgggga ttcccaatac 9000
   gaggtcgcca acatcttctt ctggaggccg tggttggctt gtatggagca gcagacgcgc 9060
   tacttcgagc ggaggcatcc ggagcttgca ggatcgccgc ggctccgggc gtatatgctc 9120
   cgcattggtc ttgaccaact ctatcagagc ttggttgacg gcaatttcga tgatgcagct 9180
   tgggcgcagg gtcgatgcga cgcaatcgtc cgatccggag ccgggactgt cgggcgtaca 9240
   caaatcgccc gcagaagcgc ggccgtctgg accgatggct gtgtagaagt actcgccgat 9300
   agtggaaacc gacgccccag cactcgtccg agggcaaagg aatagcggcc gcaagtgtgg 9360
   atggggaagt gagtgcccgg ttctgtgtgc acaattggca atccaagatg gatggattca 9420
   acacagggat atagcgagct acgtggtggt gcgaggatat agcaacggat atttatgttt 9480
   gacacttgag aatgtacgat acaagcactg tccaagtaca atactaaaca tactgtacat 9540
   actcatactc gtacccgggc aacggtttca cttgagtgca gtggctagtg ctcttactcg 9600
   tacagtgtgc taccgttcgt atagcataca ttatacgaag ttatcatagt cttaattaaa 9660
   ttttttttga ttttcttttt tgaccccgtc ttcaattaca cttcccaact gggaacaccc 9720
   ctctttatcg acccatttta ggtaatttac cctagcccat tgtctccata aggaatatta 9780
   ccctaaccca cagtccaggg tgcccaggtc cttctttggc caaattttaa cttcggtcct 9840
   atggcacagc ggtagcgcgt gagattgcaa atcttaaggt cccgagttcg aatctcggtg 9900
   ggacctagtt atttttgata gataatttcg tgatgattag aaacttaacg caaaataatg 9960
   cctggctagc tcaatcggta gagcgtgaga ctcttataca agaaatctca aggctgtggg 10020
   ttcaagcccc acgtcgggct aggtcgtgtg gtccgagttc gatcctcggc atgatcaagt 10080
   ctcaccgaca tggttatcgt tttagagcta gaaatagcaa gttaaaataa ggctagtccg 10140
   ttatcaactt gaaaaagtgg caccgagtcg gtgctttttt ttttgttttt tatcgattga 10200
   gcatccgttg atttccgaac agatcccaat attacaccca agtagcatgc ataagctaaa 10260
   agtaactcgc agcgcacacc gtgcagattc ataagtctat gattaattga acgccaataa 10320
   cccggcttac tacaagtaca agtaggtata catagcggta atgaatcatt agaaaaaaaa 10380
   aaaacaaaaa aaaacaaaac aaactgttgt ggatgcatca acagtagtac atagttgtac 10440
   gatgtacttg tacttgtaaa agcaaaaatg tacaatatct cagggagcgc aacttttacg 10500
   ttcgaagaac aatgtaccgc ataccgcatt ctagattctg cggaacgtct aacctggaaa 10560
   tacgattttt tttttctttc attttttttg cttcttcaaa agtatggtaa tttcctacca     10620
   ttacagttga cactgaacga ggggggattg aatttaagca aaaaattaaa tcaaaatacc     10680
   tttatgtatc cagcccatgt aataaacaaa aggattatat aacaagaaat aaatatatac     10740
   ctttaatgga tcattagaat aaaaataaat acgagaagca caccagagaa gctttttgat     10800
   tgccactata ccgctacttt ggtatatctt attataattg ttgaatttgc aagatagaat     10860
   gtcattcatt ggagagaaat ccaaggaata tgtgggatga aatgactaga agtatgaaca     10920
   atgagaatag tacatacttg tacctgtatt tctagaagag agaaagacag ttgagtgtgt     10980
   gattctcgtc caataataat ctcaatagta acgtgtgaat agctgttctt tgatagttga     11040
   tatttctcga tgactattta tgttgtacaa gggatttttt tcgttgctgt tgatttcgaa     11100
   ttaggcaatg cagatatcat ttatgctatc catatttaag atttcccata cgcatttata     11160
   acatttattc tacataaatt gttaaatgaa cgaactgcca ttataaattg tttcctaaat     11220
   aggaagtgtt tttcataaag caagtaagtt gtctaataat actaagtaat aaaaataagt     11280
   tcatacaata tattttgaga acatcatttg gaggcggtag atggagtctg tttattatta     11340
   aacaatgcga gatgacccct taaatattga gaacatcagt tggaggcggc agatggagtc     11400
   tgtctattta gcaatgggac atgactgtca gtatcatcat gatgtatata tataatacat     11460
   ataatattat ataacacgat ttttttaaat tattggcccg aaaattaatc agtgtagact     11520
   ggatctcggc agtctctcgg atgtagaatt aggtttcctt gaggcgaaga tcggtttgtg     11580
   tgacatgaat tcgatatcaa gcttatcgac accatcgacc tcgagggggg gcccggtacc     11640
   caattcgccc tatagtgagt cgtattacaa ttcactggcc                           11680

<210>  56
<211>  32
<212>  DNA
<213>  Artificial Sequence

<220>
<223>  Artificial Sequence

<400>  56
aatgggacgt ctcaccgaca tggttatcgt tt                                    32

<210>  57
<211>  32
<212>  DNA
<213>  Artificial Sequence

<220>
<223>  Artificial Sequence

<400>  57
tctaaaacga taaccatgtc ggtgagacgt cc                                    32

<210>  58
<211>  32
<212>  DNA
<213>  Artificial Sequence

<220>
<223>  Artificial Sequence

<400>  58
aatgggacca aacgactgtg acagatatgt tt                                    32


<210>  59
<211>  32
<212>  DNA
<213>  Artificial Sequence

<220>
<223>  Artificial Sequence

<400>  59
tctaaaacat atctgtcaca gtcgtttggt cc                                    32


<210>  60
<211>  32
<212>  DNA
<213>  Artificial Sequence

<220>
<223>  Artificial Sequence

<400>  60
aatgggaccc atgaacacgt aggcgacggt tt                                    32


<210>  61
<211>  32
<212>  DNA
<213>  Artificial Sequence

<220>
<223>  Artificial Sequence

<400>  61
tctaaaaccg tcgcctacgt gttcatgggt cc                                    32


<210>  62
<211>  32
<212>  DNA
<213>  Artificial Sequence

<220>
<223>  Artificial Sequence

<400>  62
aatgggaccg acctggagga gtccgaccgt tt                                    32


<210>  63
<211>  32
<212>  DNA

<213>  Artificial Sequence

<220>
<223>  Artificial Sequence

<400>  63
tctaaaacgg tcggactcct ccaggtcggt cc                                    32


<210>  64
<211>  32
<212>  DNA
<213>  Artificial Sequence

<220>
<223>  Artificial Sequence

<400>  64
aatgggaccc tcgtcgccta cgtgttcagt tt                                    32


<210>  65
<211>  32
<212>  DNA
<213>  Artificial Sequence

<220>
<223>  Artificial Sequence

<400>  65
tctaaaactg aacacgtagg cgacgagggt cc                                    32


<210>  66
<211>  32
<212>  DNA
<213>  Artificial Sequence

<220>
<223>  Artificial Sequence

<400>  66
aatgggactg agacctcgct tgacctcggt tt                                    32


<210>  67
<211>  32
<212>  DNA
<213>  Artificial Sequence

<220>
<223>  Artificial Sequence

<400>  67
tctaaaaccg aggtcaagcg aggtctcagt cc                                    32


<210>  68
<211>  32
<212>  DNA
<213>  Artificial Sequence

<220>

<223>  Artificial Sequence

<400>  68
aatgggacga accagtatag ccagcccagt tt                                    32


<210>  69
<211>  32
<212>  DNA
<213>  Artificial Sequence

<220>
<223>  Artificial Sequence

<400>  69
tctaaaactg ggctggctat actggttcgt cc                                    32


<210>  70
<211>  30
<212>  DNA
<213>  Artificial Sequence

<220>
<223>  Artificial Sequence

<400>  70
aatgggacca acagagtaga caatgggttt                                       30


<210>  71
<211>  30
<212>  DNA
<213>  Artificial Sequence

<220>
<223>  Artificial Sequence

<400>  71
tctaaaaccc attgtctact ctgttggtcc                                       30


<210>  72
<211>  32
<212>  DNA
<213>  Artificial Sequence

<220>
<223>  Artificial Sequence

<400>  72
aatgggaccc atgtcggtga gaccctcggt tt                                    32


<210>  73
<211>  32
<212>  DNA
<213>  Artificial Sequence

<220>
<223>  Artificial Sequence

<400>  73

tctaaaaccg agggtctcac cgacatgggt cc                                    32

<210>  74
<211>  32
<212>  DNA
<213>  Artificial Sequence

<220>
<223>  Artificial Sequence

<400>  74
aatgggacta ctctgttgcc gagtctctgt tt                                    32

<210>  75
<211>  32
<212>  DNA
<213>  Artificial Sequence

<220>
<223>  Artificial Sequence

<400>  75
tctaaaacag agactcggca acagagtagt cc                                    32

<210>  76
<211>  32
<212>  DNA
<213>  Artificial Sequence

<220>
<223>  Artificial Sequence

<400>  76
aatgggacgc catcgagctc aacaccatgt tt                                    32

<210>  77
<211>  32
<212>  DNA
<213>  Artificial Sequence

<220>
<223>  Artificial Sequence

<400>  77
tctaaaacat ggtgttgagc tcgatggcgt cc                                    32

<210>  78
<211>  32
<212>  DNA
<213>  Artificial Sequence

<220>
<223>  Artificial Sequence

<400>  78
tcgggctagt ctcaccgaca tggttatcgt tt                                    32

<210>  79
<211>  32
<212>  DNA
<213>  Artificial Sequence

<220>
<223>  Artificial Sequence

<400>  79
tctaaaacga taaccatgtc ggtgagacta gc                                    32


<210>  80
<211>  32
<212>  DNA
<213>  Artificial Sequence

<220>
<223>  Artificial Sequence

<400>  80
tcgggctaca aacgactgtg acagatatgt tt                                    32


<210>  81
<211>  32
<212>  DNA
<213>  Artificial Sequence

<220>
<223>  Artificial Sequence

<400>  81
tctaaaacat atctgtcaca gtcgtttgta gc                                    32


<210>  82
<211>  32
<212>  DNA
<213>  Artificial Sequence

<220>
<223>  Artificial Sequence

<400>  82
tcgggctacc atgaacacgt aggcgacggt tt                                    32


<210>  83
<211>  32
<212>  DNA
<213>  Artificial Sequence

<220>
<223>  Artificial Sequence

<400>  83
tctaaaaccg tcgcctacgt gttcatggta gc                                    32


<210>  84
<211>  32
<212>  DNA

<213>  Artificial Sequence

<220>
<223>  Artificial Sequence

<400>  84
tcgggctacg acctggagga gtccgaccgt tt                                    32


<210>  85
<211>  32
<212>  DNA
<213>  Artificial Sequence

<220>
<223>  Artificial Sequence

<400>  85
tctaaaacgg tcggactcct ccaggtcgta gc                                    32


<210>  86
<211>  32
<212>  DNA
<213>  Artificial Sequence

<220>
<223>  Artificial Sequence

<400>  86
tcgggctacc tcgtcgccta cgtgttcagt tt                                    32


<210>  87
<211>  32
<212>  DNA
<213>  Artificial Sequence

<220>
<223>  Artificial Sequence

<400>  87
tctaaaactg aacacgtagg cgacgaggta gc                                    32


<210>  88
<211>  32
<212>  DNA
<213>  Artificial Sequence

<220>
<223>  Artificial Sequence

<400>  88
tcgggctatg agacctcgct tgacctcggt tt                                    32


<210>  89
<211>  32
<212>  DNA
<213>  Artificial Sequence

<220>

<223>  Artificial Sequence

<400>  89
tctaaaaccg aggtcaagcg aggtctcata gc                                    32


<210>  90
<211>  32
<212>  DNA
<213>  Artificial Sequence

<220>
<223>  Artificial Sequence

<400>  90
tcgggctaga accagtatag ccagcccagt tt                                    32


<210>  91
<211>  32
<212>  DNA
<213>  Artificial Sequence

<220>
<223>  Artificial Sequence

<400>  91
tctaaaactg ggctggctat actggttcta gc                                    32


<210>  92
<211>  30
<212>  DNA
<213>  Artificial Sequence

<220>
<223>  Artificial Sequence

<400>  92
tcgggctaca acagagtaga caatgggttt                                       30


<210>  93
<211>  30
<212>  DNA
<213>  Artificial Sequence

<220>
<223>  Artificial Sequence

<400>  93
tctaaaaccc attgtctact ctgttgtagc                                       30


<210>  94
<211>  32
<212>  DNA
<213>  Artificial Sequence

<220>
<223>  Artificial Sequence

<400>  94

tcgggctacc atgtcggtga gaccctcggt tt                                    32

<210>  95
<211>  32
<212>  DNA
<213>  Artificial Sequence

<220>
<223>  Artificial Sequence

<400>  95
tctaaaaccg agggtctcac cgacatggta gc                                    32

<210>  96
<211>  32
<212>  DNA
<213>  Artificial Sequence

<220>
<223>  Artificial Sequence

<400>  96
tcgggctata ctctgttgcc gagtctctgt tt                                    32

<210>  97
<211>  32
<212>  DNA
<213>  Artificial Sequence

<220>
<223>  Artificial Sequence

<400>  97
tctaaaacag agactcggca acagagtata gc                                    32

<210>  98
<211>  32
<212>  DNA
<213>  Artificial Sequence

<220>
<223>  Artificial Sequence

<400>  98
tcgggctagc catcgagctc aacaccatgt tt                                    32

<210>  99
<211>  32
<212>  DNA
<213>  Artificial Sequence

<220>
<223>  Artificial Sequence

<400>  99
tctaaaacat ggtgttgagc tcgatggcta gc                                    32

<210>  100
<211>  11643
<212>  DNA
<213>  Artificial Sequence

<220>
<223>  Artificial Sequence

<400>  100
attgagcatc cgttgatttc cgaacagatc ccaatattac acccaagtag catgcataag      60

ctaaaagtaa ctcgcagcgc acaccgtgca gattcataag tctatgatta attgaacgcc     120

aataacccgg cttactacaa gtacaagtag gtatacatag cggtaatgaa tcattagaaa     180

aaaaaaaaac aaaaaaaaac aaaacaaact gttgtggatg catcaacagt agtacatagt     240

tgtacgatgt acttgtactt gtaaaagcaa aaatgtacaa tatctcaggg agcgcaactt     300

ttacgttcga agaacaatgt accgcatacc gcattctaga ttctgcggaa cgtctaacct     360

ggaaatacga tttttttttt ctttcatttt ttttgcttct tcaaaagtat ggtaatttcc     420

taccattaca gttgacactg aacgaggggg gattgaattt aagcaaaaaa ttaaatcaaa     480

atacctttat gtatccagcc catgtaataa acaaaaggat tatataacaa gaaataaata     540

tataccttta atggatcatt agaataaaaa taaatacgag aagcacacca gagaagcttt     600

ttgattgcca ctataccgct actttggtat atcttattat aattgttgaa tttgcaagat     660

agaatgtcat tcattggaga gaaatccaag gaatatgtgg gatgaaatga ctagaagtat     720

gaacaatgag aatagtacat acttgtacct gtatttctag aagagagaaa gacagttgag     780

tgtgtgattc tcgtccaata ataatctcaa tagtaacgtg tgaatagctg ttctttgata     840

gttgatattt ctcgatgact atttatgttg tacaagggat ttttttcgtt gctgttgatt     900

tcgaattagg caatgcagat atcatttatg ctatccatat ttaagatttc ccatacgcat     960

ttataacatt tattctacat aaattgttaa atgaacgaac tgccattata aattgtttcc    1020

taaataggaa gtgtttttca taaagcaagt aagttgtcta ataatactaa gtaataaaaa    1080

taagttcata caatatattt tgagaacatc atttggaggc ggtagatgga gtctgtttat    1140

tattaaacaa tgcgagatga ccccttaaat attgagaaca tcagttggag gcggcagatg    1200

gagtctgtct atttagcaat gggacatgac tgtcagtatc atcatgatgt atatatataa    1260

tacatataat attatataac acgatttttt taaattattg gcccgaaaat taatcagtgt    1320

agactggatc tcggcagtct ctcggatgta gaattaggtt tccttgaggc gaagatcggt    1380

ttgtgtgaca tgaattcgat atcaagctta tcgacaccat cgacctcgag ggggggcccg    1440

gtacccaatt cgccctatag tgagtcgtat tacaattcac tggccgtcgt tttacaacgt    1500

cgtgactggg aaaaccctgg cgttacccaa cttaatcgcc ttgcagcaca tccccctttc    1560

gccagctggc gtaatagcga agaggcccgc accgatcgcc cttcccaaca gttgcgcagc    1620

ctgaatggcg aatggaaatt gtaagcgtta atattttgtt aaaattcgcg ttaaattttt 1680

gttaaatcag ctcatttttt aaccaatagg ccgaaatcgg caaaatccct tataaatcaa 1740

aagaatagac cgagataggg ttgagtgttg ttccagtttg gaacaagagt ccactattaa 1800

agaacgtgga ctccaacgtc aaagggcgaa aaaccgtcta tcagggcgat ggcccactac 1860

gtgaaccatc accctaatca agttttttgg ggtcgaggtg ccgtaaagca ctaaatcgga 1920

accctaaagg gagcccccga tttagagctt gacggggaaa gccggcgaac gtggcgagaa 1980

aggaagggaa gaaagcgaaa ggagcgggcg ctagggcgct ggcaagtgta gcggtcacgc 2040

tgcgcgtaac caccacaccc gccgcgctta atgcgccgct acagggcgcg tcaggtggca 2100

cttttcgggg aaatgtgcgc ggaaccccta tttgtttatt tttctaaata cattcaaata 2160

tgtatccgct catgagacaa taaccctgat aaatgcttca ataatattga aaaaggaaga 2220

gtatgagtat tcaacatttc cgtgtcgccc ttattccctt ttttgcggca ttttgccttc 2280

ctgtttttgc tcacccagaa acgctggtga aagtaaaaga tgctgaagat cagttgggtg 2340

cacgagtggg ttacatcgaa ctggatctca acagcggtaa gatccttgag agttttcgcc 2400

ccgaagaacg ttttccaatg atgagcactt ttaaagttct gctatgtggc gcggtattat 2460

cccgtattga cgccgggcaa gagcaactcg gtcgccgcat acactattct cagaatgact 2520

tggttgagta ctcaccagtc acagaaaagc atcttacgga tggcatgaca gtaagagaat 2580

tatgcagtgc tgccataacc atgagtgata acactgcggc caacttactt ctgacaacga 2640

tcggaggacc gaaggagcta accgcttttt tgcacaacat gggggatcat gtaactcgcc 2700

ttgatcgttg ggaaccggag ctgaatgaag ccataccaaa cgacgagcgt gacaccacga 2760

tgcctgtagc aatggcaaca acgttgcgca aactattaac tggcgaacta cttactctag 2820

cttcccggca acaattaata gactggatgg aggcggataa agttgcagga ccacttctgc 2880

gctcggccct tccggctggc tggtttattg ctgataaatc tggagccggt gagcgtgggt 2940

ctcgcggtat cattgcagca ctggggccag atggtaagcc ctcccgtatc gtagttatct 3000

acacgacggg gagtcaggca actatggatg aacgaaatag acagatcgct gagataggtg 3060

cctcactgat taagcattgg taactgtcag accaagttta ctcatatata ctttagattg 3120

atttaaaact tcatttttaa tttaaaagga tctaggtgaa gatccttttt gataatctca 3180

tgaccaaaat cccttaacgt gagttttcgt tccactgagc gtcagacccc gtagaaaaga 3240

tcaaaggatc ttcttgagat cctttttttc tgcgcgtaat ctgctgcttg caaacaaaaa 3300

aaccaccgct accagcggtg gtttgtttgc cggatcaaga gctaccaact ctttttccga 3360

aggtaactgg cttcagcaga gcgcagatac caaatactgt ccttctagtg tagccgtagt 3420

taggccacca cttcaagaac tctgtagcac cgcctacata cctcgctctg ctaatcctgt 3480

taccagtggc tgctgccagt ggcgataagt cgtgtcttac cgggttggac tcaagacgat 3540

agttaccgga taaggcgcag cggtcgggct gaacgggggg ttcgtgcaca cagcccagct 3600

tggagcgaac gacctacacc gaactgagat acctacagcg tgagctatga gaaagcgcca 3660

cgcttcccga agggagaaag gcggacaggt atccggtaag cggcagggtc ggaacaggag 3720

agcgcacgag ggagcttcca gggggaaacg cctggtatct ttatagtcct gtcgggtttc 3780

gccacctctg acttgagcgt cgatttttgt gatgctcgtc aggggggcgg agcctatgga 3840

aaaacgccag caacgcggcc tttttacggt tcctggcctt ttgctggcct tttgctcaca 3900

tgttctttcc tgcgttatcc cctgattctg tggataaccg tattaccgcc tttgagtgag 3960

ctgataccgc tcgccgcagc cgaacgaccg agcgcagcga gtcagtgagc gaggaagcgg 4020

aagagcgccc aatacgcaaa ccgcctctcc ccgcgcgttg gccgattcat taatgcagct 4080

ggcacgacag gtttcccgac tggaaagcgg gcagtgagcg caacgcaatt aatgtgagtt 4140

agctcactca ttaggcaccc caggctttac actttatgct tccggctcgt acgcaactaa 4200

catgaatgaa tacgatatac atcaaagact atgatacgca gtattgcaca ctgtacgagt 4260

aagagcacta gccactgcac tcaagtgaaa ccgttgcccg ggtacgagta tgagtatgta 4320

cagtatgttt agtattgtac ttggacagtg cttgtatcgt acattctcaa gtgtcaaaca 4380

taaatatccg ttgctatatc ctcgcaccac cacgtagctc gctatatccc tgtgttgaat 4440

ccatccatct tggattgcca attgtgcaca cagaaccggg cactcacttc cccatccaca 4500

cttgcggccg cttagacctt tcgctttttc ttgggatcgg ctctggagtc gccaccaagc 4560

tgagacaggt cgattcgggt ctcgtacagg ccagtgatgg actggtgaat cagggtggca 4620

tcgagaacct ccttggtgga tgtgtaccgc tttcggtcga tagtggtatc gaagtacttg 4680

aaagctgcag gagcacccag gttggtaaga gtaaacaggt gaatgatgtt ctccgcctgt 4740

tctcgaatgg gtttgtcccg atgcttgttg taggcagaga gcaccttgtc caagttggca 4800

tcagccagga tgactcgctt cgaaaactcg gaaatctgct cgataatctc gtcgaggtaa 4860

tgtttgtgct gctcaacgaa gagttgcttc tgttcgttgt cctcgggaga acccttgagc 4920

ttctcgtagt gagaagccag atagagaaag ttgacgtact tcgaaggcaa ggcaagctcg 4980

tttcccttct gcagctcgcc agcggaggcg agcatacgct ttcgaccgtt ctccagttcg 5040

aacagagagt acttgggcag cttgataatg aggtctttct tgacctcctt gtaacccttg 5100

gcttccaaga agtcgatggg attcttctcg aagctcgatc gctccatgat ggtaattccg 5160

agcagctcct tgacggactt gagctttttg gacttgccct tctcgacctt cgcaacgaca 5220

agcacggaat aggcgacggt aggagaatcg aagccaccgt atttcttggg atcccagtct 5280

ttctttcgag cgatgagctt gtcggagttt cgcttgggca gaatcgactc cttggagaat 5340

ccgccagtct gaacctcggt tttcttgacg atgttgacct gaggcatcga cagaaccttt 5400

cgcacggttg caaagtctcg acccttgtcc cacacgatct ctccagtttc gccgttggtc 5460

tcgataagtg gtctctttcg aatctctccg ttggccaagg tgatctcggt cttgaaaaag 5520

ttcatgatgt tggagtaaaa gaagtacttg gcagtagcct tgccaatctc ctgttcggac 5580

ttggcaatca tctttcgaac gtcgtagacc ttgtaatcgc cgtaaacgaa ctcgctttcg 5640

agcttggggt atttcttgat gagcgcagtg ccaacgacgg cgttgaggta agcatcgtgg 5700

gcatggtggt aattgttgat ctctcgcacc ttgtagaact gaaagtcctt tcggaaatcg 5760

gagaccagtt tggacttgag agtaatcacc ttgacctctc ggatgagctt gtcgttctcg 5820

tcgtacttgg tgttcatccg agaatcgaga atctgtgcga cgtgctttgt gatctgtctg 5880

gtctcgacga gttgacgctt gatgaagcca gccttgtcga gctcggacag accgcctcgc 5940

tcggccttgg taagattgtc gaactttcgc tgggtaatga gcttggcgtt gagcagctgt 6000

cgccagtagt tcttcatctt tttgaccacc tcttcgctgg gaacgttgtc cgacttgcct 6060

ctgttcttgt cggatcgtgt aaggaccttg ttgtcgatag aatcgtcctt gagaaaggat 6120

tgagggacaa tgtggtccac atcgtagtcg ctgagacgat tgatgtccag ttcctgatcc 6180

acgtacatgt ctcgaccatt ctgcagatag tagagataca gcttctcgtt ctgcagttga 6240

gtgttctcga cgggatgctc cttgagaatc tgggatccca gctccttgat gccttcctcg 6300

attcgcttca tccgctctcg cgagtttttc tgaccctttt gagttgtctg gttctctctg 6360

gccatctcga tcacaatgtt ctcgggcttg tgacgtccca tgaccttcac cagctcgtcg 6420

acaaccttga cagtctggag aatgcctttc ttgatggctg gcgaaccagc caggttggca 6480

atatgttcgt gcaagctgtc gccctgaccg gacacttgtg ccttctggat gtcctccttg 6540

aaggtaagag aatcgtcgtg aatgagctgc atgaagtttc ggttggcaaa gccatcggac 6600

ttgagaaagt ccagaatggt ctttccggac tgcttgtctc tgatgccgtt gatgagcttt 6660

cgcgaaagtc ttccccagcc ggtgtatcta cgtcgcttga gttgtttcat gaccttgtcg 6720

tcgaacaggt gagcgtatgt cttgagtcgt tcctcgatca tctcccgatc ttcgaacagg 6780

gtaagagtga gcacgatgtc ctccagaatg tcctcgtttt cctcgttgtc gagaaaatcc 6840

ttgtccttga taatcttgag cagatcgtga taggtgccca aagaggcgtt gaatcggtcc 6900

tcaactccgg aaatctcgac gctgtcgaaa cactcgattt tcttgaagta gtcctccttg 6960

agctgcttaa cagtgacctt tcggttggtc ttgaacagga gatcgacaat ggctttcttc 7020

tgttcgccag acaagaaggc aggctttcgc attccctcgg taacgtactt gactttggtg 7080

agttcgttgt agactgtaaa gtactcgtag agcagcgaat gcttgggaag aaccttctcg 7140

ttgggcagat tcttgtcgaa gttggtcatt cgctcgatga aggactgtgc agaggcaccc 7200

ttgtccacga cttcctcgaa gttccaggga gtgatggttt cctcggactt tcgagtcatc 7260

caagcaaatc gagagtttcc tctggcaaga ggaccaacat agtaggggat tcgaaaggta 7320

agaatcttct cgatcttctc tcggttgtcc ttgagaaagg ggtagaagtc ttcctgacgt 7380

cgaagaatgg cgtgcagctc accgaggtgg atctgatgag gaatgctgcc gttgtcgaag 7440

gttcgttgct tccgaagcag atcctctcga ttgagcttga caagcagttc ctcggttccg 7500

tccatcttct cgagaattgg cttgatgaac ttgtagaact cttcctgaga ggctccgccg 7560

tcgatgtatc cagcgtagcc gttcttcgac tgatcgaaaa agatctcctt gtacttctcg 7620

ggcagttgct gtcggacaag agccttgagc agtgtgagat cctgatggtg ctcgtcgtat 7680

cgcttgatca tggaggcaga aaggggagcc tttgtgatct cggtgttgac tcgcagaatg 7740

tcagacaaga gaatagcatc cgaaaggttc ttggcagcga gaaacaggtc ggcgtactga 7800

tcgccaatct gtgcaagcag gttgtcgagg tcatcgtcgt aggtgtcctt ggacagctgg 7860

agcttggcgt cctccgccag atcgaagttg gacttgaagt tgggtgtgag accaagagaa 7920

agggcaatga ggttgccaaa cagtccgttc tttttctcgc caggaagttg ggcaatgagg 7980

ttctccagtc gtctgctctt cgagagtcga gcagacaaga tggcctttgc atcgactccg 8040

gaggcattga tggggttttc ctcgaacagc tggttgtagg tctgaacgag ctgaatgaac 8100

agcttgtcca catcgctgtt gtcgggattg agatcgccct cgatgaggaa atgacctcga 8160

aacttgatca tgtgtgccag agcgaggtag ataagtctga gatccgcctt gtcggtggaa 8220

tcgacgagtt tctttcggag atggtagatg gtaggatact tctcgtggta agcaacctcg 8280

tccacaatgt tgccaaagat gggatgacgc tcgtgtttct tgtcttcctc gacgaggaag 8340

gattcctcca gtcgatgaaa gaacgaatcg tccaccttgg ccatctcgtt ggaaaagatc 8400

tcctgcaggt agcagattcg gttcttccgt cgggtgtaac gtcgccgagc agttcgcttg 8460

agtctggtag cttcggcagt ctcgccagaa tcgaacaaca gggcaccaat gaggtttttc 8520

ttgatggagt gtcgatcggt gtttccgagg accttgaatt tcttggaggg caccttgtac 8580

tcgtcggtga tgacagccca gccgacagag ttggttccaa tgtccaggcc gatggagtat 8640

ttcttgtcca tggtgtgatg tgtagtttag atttcgaatc tgtggggaaa gaaaggaaaa 8700

aagagactgg caaccgattg ggagagccac tgtttatata taccctagac aagccccccg 8760

cttgtaagat gttggtcaat gtaaaccagt attaaggttg gcaagtgcag gagaagcaag 8820

gtgtgggtac cgagcaatgg aaatgtgcgg aaggcaaaaa aatgaggcca cggcctattg 8880

tcggggctat atccaggggg cgattgaagt acactaacat gacatgtgtc cacagaccct 8940

caatctggcc tgatgagcca aatccatacg cgctttcgca gctctaaagg ctataacaag 9000

tcacaccacc ctgctcgacc tcagcgccct cactttttgt taagacaaac tgtacacgct 9060

gttccagcgt tttctgcctg cacctggtgg gacatttggt gcaacctaaa gtgctcggaa 9120

cctctgtggt gtccagatca gcgcagcagt tccgaggtag ttttgaggcc cttagatgat 9180

ggtttaaacc ttaagcccgc tcataacttc gtatagcata cattatacga acggtaggtt 9240

gcgggataga cgccgacgga gggcaatggc gctatggaac cttgcggata tccatacgcc 9300

gcggcggact gcgtccgaac cagctccagc agcgtttttt ccgggccatt gagccgactg 9360

cgaccccgcc aacgtgtctt ggcccacgca ctcatgtcat gttggtgttg ggaggccact 9420

ttttaagtag cacaaggcac ctagctcgca gcaaggtgtc cgaaccaaag aagcggctgc 9480

agtggtgcaa acggggcgga aacggcggga aaaagccacg ggggcacgaa ttgaggcacg 9540

ccctcgaatt tgagacgagt cacggcccca ttcgcccgcg caatggctcg ccaacgcccg 9600

gtcttttgca ccacatcagg ttaccccaag ccaaaccttt gtgttaaaaa gcttaacata 9660

ttataccgaa cgtaggtttg ggcgggcttg ctccgtctgt ccaaggcaac atttatataa 9720

gggtctgcat cgccggctca attgaatctt ttttcttctt ctcttctcta tattcattct 9780

tgaattaaac acacatcaac catggccaaa aagcctgaac tcaccgcgac gtctgtcgag 9840

aagtttctga tcgaaaagtt cgacagcgtc tccgacctga tgcagctctc ggagggcgaa 9900

gaatctcgtg ctttcagctt cgatgtagga gggcgtggat atgtcctgcg ggtaaatagc 9960

tgcgccgatg gtttctacaa agatcgttat gtttatcggc actttgcatc ggccgcgctc 10020

ccgattccgg aagtgcttga cattggggag ttcagcgaga gcctgaccta ttgcatctcc 10080

cgccgtgcac agggtgtcac gttgcaagac ctgcctgaaa ccgaactgcc cgctgttctg 10140

cagccggtcg cggaggctat ggatgcgatc gctgcggccg atcttagcca gacgagcggg 10200

ttcggcccat tcggaccgca aggaatcggt caatacacta catggcgtga tttcatatgc 10260

gcgattgctg atccccatgt gtatcactgg caaactgtga tggacgacac cgtcagtgcg 10320

tccgtcgcgc aggctctcga tgagctgatg ctttgggccg aggactgccc cgaagtccgg 10380

cacctcgtgc acgcggattt cggctccaac aatgtcctga cggacaatgg ccgcataaca 10440

gcggtcattg actggagcga ggcgatgttc ggggattccc aatacgaggt cgccaacatc 10500

ttcttctgga ggccgtggtt ggcttgtatg gagcagcaga cgcgctactt cgagcggagg 10560

catccggagc ttgcaggatc gccgcggctc cgggcgtata tgctccgcat tggtcttgac 10620

caactctatc agagcttggt tgacggcaat ttcgatgatg cagcttgggc gcagggtcga 10680

tgcgacgcaa tcgtccgatc cggagccggg actgtcgggc gtacacaaat cgcccgcaga 10740

agcgcggccg tctggaccga tggctgtgta gaagtactcg ccgatagtgg aaaccgacgc 10800

cccagcactc gtccgagggc aaaggaatag cggccgcaag tgtggatggg gaagtgagtg 10860

cccggttctg tgtgcacaat tggcaatcca agatggatgg attcaacaca gggatatagc 10920

gagctacgtg gtggtgcgag gatatagcaa cggatattta tgtttgacac ttgagaatgt 10980

acgatacaag cactgtccaa gtacaatact aaacatactg tacatactca tactcgtacc 11040

cgggcaacgg tttcacttga gtgcagtggc tagtgctctt actcgtacag tgtgctaccg 11100

ttcgtatagc atacattata cgaagttatc atagtcttaa ttaaattttt tttgattttc 11160

ttttttgacc ccgtcttcaa ttacacttcc caactgggaa cacccctctt tatcgaccca 11220

ttttaggtaa tttaccctag cccattgtct ccataaggaa tattacccta acccacagtc 11280

cagggtgccc aggtccttct ttggccaaat tttaacttcg gtcctatggc acagcggtag 11340

cgcgtgagat tgcaaatctt aaggtcccga gttcgaatct cggtgggacc tagttatttt 11400

tgatagataa tttcgtgatg attagaaact taacgcaaaa taatgcctgg ctagctcaat 11460

cggtagagcg tgagactctt atacaagaaa tctcaaggct gtgggttcaa gccccacgtc 11520

gggctatcaa acgattaccc accctcgttt tagagctaga aatagcaagt taaaataagg 11580

ctagtccgtt atcaacttga aaaagtggca ccgagtcggt gctttttttt ttgtttttta 11640

tcg 11643

<210> 101
<211> 509
<212> DNA
<213> Artificial Sequence

<220>
<223> Artificial Sequence

<400> 101
ttaattaaat tttttttgat tttctttttt gaccccgtct tcaattacac ttcccaactg 60

ggaacacccc tctttatcga cccattttag gtaatttacc ctagcccatt gtctccataa 120

ggaatattac cctaacccac agtccagggt gcccaggtcc ttctttggcc aaattttaac 180

ttcggtccta tggcacagcg gtagcgcgtg agattgcaaa tcttaaggtc ccgagttcga 240

atctcggtgg gacctagtta tttttgatag ataatttcgt gatgattaga aacttaacgc 300

aaaataatgc ctggctagct caatcggtag agcgtgagac tcttatacaa gaaatctcaa 360

ggctgtgggt tcaagcccca cgtcgggcta tcaaacgatt acccaccctc gttttagagc 420

tagaaatagc aagttaaaat aaggctagtc cgttatcaac ttgaaaaagt ggcaccgagt 480

cggtgctttt tttttgttt tttatcgat 509

<210> 102
<211> 170
<212> DNA
<213> Artificial Sequence

<220>
<223> Artificial Sequence

<400> 102
ggtcgtgtgg tgtaatggtt atcacgaggc tccgagttcg atcctcggca tgatcaagtc 60

tcaccgacat ggttatcgtt ttagagctag aaatagcaag ttaaaataag gctagtccgt 120

tatcaacttg aaaaagtggc accgagtcgg tgcttttttt tttgtttttt 170

<210> 103
<211> 164
<212> DNA
<213> Artificial Sequence

<220>
<223> Artificial Sequence

<400> 103
ggtcgtgtgg tgtaatggtt atcacccgag ttcgatcctc ggcatgatca agtctcaccg 60

acatggttat cgttttagag ctagaaatag caagttaaaa taaggctagt ccgttatcaa 120

cttgaaaaag tggcaccgag tcggtgcttt tttttttgtt tttt 164

<210> 104
<211> 150
<212> DNA
<213> Artificial Sequence

<220>
<223> Artificial Sequence

<400> 104
ggtcgtgtgg tccgagttcg atcctcggca tgatcaagtc tcaccgacat ggttatcgtt 60

ttagagctag aaatagcaag ttaaaataag gctagtccgt tatcaacttg aaaaagtggc 120

accgagtcgg tgcttttttt tttgtttttt 150

<210> 105
<211> 212
<212> DNA
<213> Artificial Sequence

<220>
<223> Artificial Sequence

<400> 105
ttaattaagg cagattggcc gagtggttta aggcggcaga cttaagatct gctgaacgca 60

agttctcgtg ggttcgaacc ccacatctgt cattcaaacg attacccacc ctcgttttag 120

agctagaaat agcaagttaa aataaggcta gtccgttatc aacttgaaaa agtggcaccg 180

agtcggtgct tttttttttg tttttatcgg at 212

<210> 106
<211> 84
<212> DNA
<213> Artificial Sequence

<220>
<223> Artificial Sequence

<400> 106
ggcagattgg ccgagtggtt taaggcggca gacttaagat ctgctgaacg caagttctcg 60

tgggttcgaa ccccacatct gtca                                          84

<210>  107
<211>  11346
<212>  DNA
<213>  Artificial Sequence

<220>
<223>  Artificial Sequence

<400>  107
acatccccct ttcgccagct ggcgtaatag cgaagaggcc cgcaccgatc gcccttccca      60

acagttgcgc agcctgaatg gcgaatggaa attgtaagcg ttaatatttt gttaaaattc     120

gcgttaaatt tttgttaaat cagctcattt tttaaccaat aggccgaaat cggcaaaatc     180

ccttataaat caaaagaata gaccgagata gggttgagtg ttgttccagt ttggaacaag     240

agtccactat taaagaacgt ggactccaac gtcaaagggc gaaaaaccgt ctatcagggc     300

gatggcccac tacgtgaacc atcaccctaa tcaagttttt tggggtcgag gtgccgtaaa     360

gcactaaatc ggaaccctaa agggagcccc cgatttagag cttgacgggg aaagccggcg     420

aacgtggcga gaaaggaagg gaagaaagcg aaaggagcgg gcgctagggc gctggcaagt     480

gtagcggtca cgctgcgcgt aaccaccaca cccgccgcgc ttaatgcgcc gctacagggc     540

gcgtcaggtg gcacttttcg gggaaatgtg cgcggaaccc ctatttgttt atttttctaa     600

atacattcaa atatgtatcc gctcatgaga caataaccct gataaatgct tcaataatat     660

tgaaaaagga agagtatgag tattcaacat ttccgtgtcg cccttattcc cttttttgcg     720

gcattttgcc ttcctgtttt tgctcaccca gaaacgctgg tgaaagtaaa agatgctgaa     780

gatcagttgg gtgcacgagt gggttacatc gaactggatc tcaacagcgg taagatcctt     840

gagagttttc gccccgaaga acgttttcca atgatgagca cttttaaagt tctgctatgt     900

ggcgcggtat tatcccgtat tgacgccggg caagagcaac tcggtcgccg catacactat     960

tctcagaatg acttggttga gtactcacca gtcacagaaa agcatcttac ggatggcatg    1020

acagtaagag aattatgcag tgctgccata accatgagtg ataacactgc ggccaactta    1080

cttctgacaa cgatcggagg accgaaggag ctaaccgctt ttttgcacaa catgggggat    1140

catgtaactc gccttgatcg ttgggaaccg gagctgaatg aagccatacc aaacgacgag    1200

cgtgacacca cgatgcctgt agcaatggca acaacgttgc gcaaactatt aactggcgaa    1260

ctacttactc tagcttcccg gcaacaatta atagactgga tggaggcgga taaagttgca    1320

ggaccacttc tgcgctcggc ccttccggct ggctggttta ttgctgataa atctggagcc    1380

ggtgagcgtg ggtctcgcgg tatcattgca gcactggggc cagatggtaa gccctcccgt    1440

atcgtagtta tctacacgac ggggagtcag gcaactatgg atgaacgaaa tagacagatc    1500

gctgagatag gtgcctcact gattaagcat tggtaactgt cagaccaagt ttactcatat 1560

atactttaga ttgatttaaa acttcatttt taatttaaaa ggatctaggt gaagatcctt 1620

tttgataatc tcatgaccaa aatcccttaa cgtgagtttt cgttccactg agcgtcagac 1680

cccgtagaaa agatcaaagg atcttcttga gatccttttt ttctgcgcgt aatctgctgc 1740

ttgcaaacaa aaaaaccacc gctaccagcg gtggtttgtt tgccggatca agagctacca 1800

actctttttc cgaaggtaac tggcttcagc agagcgcaga taccaaatac tgtccttcta 1860

gtgtagccgt agttaggcca ccacttcaag aactctgtag caccgcctac atacctcgct 1920

ctgctaatcc tgttaccagt ggctgctgcc agtggcgata agtcgtgtct taccgggttg 1980

gactcaagac gatagttacc ggataaggcg cagcggtcgg gctgaacggg gggttcgtgc 2040

acacagccca gcttggagcg aacgacctac accgaactga gatacctaca gcgtgagcta 2100

tgagaaagcg ccacgcttcc cgaagggaga aaggcggaca ggtatccggt aagcggcagg 2160

gtcggaacag gagagcgcac gagggagctt ccagggggaa acgcctggta tctttatagt 2220

cctgtcgggt ttcgccacct ctgacttgag cgtcgatttt tgtgatgctc gtcagggggg 2280

cggagcctat ggaaaaacgc cagcaacgcg gcctttttac ggttcctggc cttttgctgg 2340

ccttttgctc acatgttctt tcctgcgtta tcccctgatt ctgtggataa ccgtattacc 2400

gcctttgagt gagctgatac cgctcgccgc agccgaacga ccgagcgcag cgagtcagtg 2460

agcgaggaag cggaagagcg cccaatacgc aaaccgcctc tccccgcgcg ttggccgatt 2520

cattaatgca gctggcacga caggtttccc gactggaaag cgggcagtga gcgcaacgca 2580

attaatgtga gttagctcac tcattaggca ccccaggctt tacactttat gcttccggct 2640

cgtacgcaac taacatgaat gaatacgata tacatcaaag actatgatac gcagtattgc 2700

acactgtacg agtaagagca ctagccactg cactcaagtg aaaccgttgc ccgggtacga 2760

gtatgagtat gtacagtatg tttagtattg tacttggaca gtgcttgtat cgtacattct 2820

caagtgtcaa acataaatat ccgttgctat atcctcgcac caccacgtag ctcgctatat 2880

ccctgtgttg aatccatcca tcttggattg ccaattgtgc acacagaacc gggcactcac 2940

ttccccatcc acacttgcgg ccgcttagac ctttcgcttt ttcttgggat cggctctgga 3000

gtcgccacca agctgagaca ggtcgattcg ggtctcgtac aggccagtga tggactggtg 3060

aatcagggtg gcatcgagaa cctccttggt ggatgtgtac cgctttcggt cgatagtggt 3120

atcgaagtac ttgaaagctg caggagcacc caggttggta agagtaaaca ggtgaatgat 3180

gttctccgcc tgttctcgaa tgggtttgtc ccgatgcttg ttgtaggcag agagcacctt 3240

gtccaagttg gcatcagcca ggatgactcg cttcgaaaac tcggaaatct gctcgataat 3300

ctcgtcgagg taatgtttgt gctgctcaac gaagagttgc ttctgttcgt tgtcctcggg 3360

agaacccttg agcttctcgt agtgagaagc cagatagaga aagttgacgt acttcgaagg 3420

caaggcaagc tcgtttccct tctgcagctc gccagcggag gcgagcatac gctttcgacc 3480

gttctccagt tcgaacagag agtacttggg cagcttgata atgaggtctt tcttgacctc 3540

cttgtaaccc ttggcttcca agaagtcgat gggattcttc tcgaagctcg atcgctccat 3600

gatggtaatt ccgagcagct ccttgacgga cttgagcttt ttggacttgc ccttctcgac 3660

cttcgcaacg acaagcacgg aataggcgac ggtaggagaa tcgaagccac cgtatttctt 3720

gggatcccag tctttctttc gagcgatgag cttgtcggag tttcgcttgg gcagaatcga 3780

ctccttggag aatccgccag tctgaacctc ggttttcttg acgatgttga cctgaggcat 3840

cgacagaacc tttcgcacgg ttgcaaagtc tcgacccttg tcccacacga tctctccagt 3900

ttcgccgttg gtctcgataa gtggtctctt tcgaatctct ccgttggcca aggtgatctc 3960

ggtcttgaaa aagttcatga tgttggagta aaagaagtac ttggcagtag ccttgccaat 4020

ctcctgttcg gacttggcaa tcatctttcg aacgtcgtag accttgtaat cgccgtaaac 4080

gaactcgctt tcgagcttgg ggtatttctt gatgagcgca gtgccaacga cggcgttgag 4140

gtaagcatcg tgggcatggt ggtaattgtt gatctctcgc accttgtaga actgaaagtc 4200

ctttcggaaa tcggagacca gtttggactt gagagtaatc accttgacct ctcggatgag 4260

cttgtcgttc tcgtcgtact tggtgttcat ccgagaatcg agaatctgtg cgacgtgctt 4320

tgtgatctgt ctggtctcga cgagttgacg cttgatgaag ccagccttgt cgagctcgga 4380

cagaccgcct cgctcggcct tggtaagatt gtcgaacttt cgctgggtaa tgagcttggc 4440

gttgagcagc tgtcgccagt agttcttcat ctttttgacc acctcttcgc tgggaacgtt 4500

gtccgacttg cctctgttct tgtcggatcg tgtaaggacc ttgttgtcga tagaatcgtc 4560

cttgagaaag gattgaggga caatgtggtc cacatcgtag tcgctgagac gattgatgtc 4620

cagttcctga tccacgtaca tgtctcgacc attctgcaga tagtagagat acagcttctc 4680

gttctgcagt tgagtgttct cgacgggatg ctccttgaga atctgggatc ccagctcctt 4740

gatgccttcc tcgattcgct tcatccgctc tcgcgagttt ttctgaccct tttgagttgt 4800

ctggttctct ctggccatct cgatcacaat gttctcgggc ttgtgacgtc ccatgacctt 4860

caccagctcg tcgacaacct tgacagtctg gagaatgcct ttcttgatgg ctggcgaacc 4920

agccaggttg gcaatatgtt cgtgcaagct gtcgccctga ccggacactt gtgccttctg 4980

gatgtcctcc ttgaaggtaa gagaatcgtc gtgaatgagc tgcatgaagt ttcggttggc 5040

aaagccatcg gacttgagaa agtccagaat ggtctttccg gactgcttgt ctctgatgcc 5100

gttgatgagc tttcgcgaaa gtcttcccca gccggtgtat ctacgtcgct tgagttgttt 5160

catgaccttg tcgtcgaaca ggtgagcgta tgtcttgagt cgttcctcga tcatctcccg 5220

atcttcgaac agggtaagag tgagcacgat gtcctccaga atgtcctcgt tttcctcgtt 5280

gtcgagaaaa tccttgtcct tgataatctt gagcagatcg tgataggtgc ccaaagaggc 5340

gttgaatcgg tcctcaactc cggaaatctc gacgctgtcg aaacactcga ttttcttgaa 5400

gtagtcctcc ttgagctgct taacagtgac ctttcggttg gtcttgaaca ggagatcgac 5460

aatggctttc ttctgttcgc cagacaagaa ggcaggcttt cgcattccct cggtaacgta 5520

cttgactttg gtgagttcgt tgtagactgt aaagtactcg tagagcagcg aatgcttggg 5580

aagaaccttc tcgttgggca gattcttgtc gaagttggtc attcgctcga tgaaggactg 5640

tgcagaggca cccttgtcca cgacttcctc gaagttccag ggagtgatgg tttcctcgga 5700

ctttcgagtc atccaagcaa atcgagagtt tcctctggca agaggaccaa catagtaggg 5760

gattcgaaag gtaagaatct tctcgatctt ctctcggttg tccttgagaa aggggtagaa 5820

gtcttcctga cgtcgaagaa tggcgtgcag ctcaccgagg tggatctgat gaggaatgct 5880

gccgttgtcg aaggttcgtt gcttccgaag cagatcctct cgattgagct tgacaagcag 5940

ttcctcggtt ccgtccatct tctcgagaat tggcttgatg aacttgtaga actcttcctg 6000

agaggctccg ccgtcgatgt atccagcgta gccgttcttc gactgatcga aaaagatctc 6060

cttgtacttc tcgggcagtt gctgtcggac aagagccttg agcagtgtga gatcctgatg 6120

gtgctcgtcg tatcgcttga tcatggaggc agaaagggga gcctttgtga tctcggtgtt 6180

gactcgcaga atgtcagaca agagaatagc atccgaaagg ttcttggcag cgagaaacag 6240

gtcggcgtac tgatcgccaa tctgtgcaag caggttgtcg aggtcatcgt cgtaggtgtc 6300

cttggacagc tggagcttgg cgtcctccgc cagatcgaag ttggacttga agttgggtgt 6360

gagaccaaga gaaagggcaa tgaggttgcc aaacagtccg ttctttttct cgccaggaag 6420

ttgggcaatg aggttctcca gtcgtctgct cttcgagagt cgagcagaca agatggcctt 6480

tgcatcgact ccggaggcat tgatggggtt ttcctcgaac agctggttgt aggtctgaac 6540

gagctgaatg aacagcttgt ccacatcgct gttgtcggga ttgagatcgc cctcgatgag 6600

gaaatgacct cgaaacttga tcatgtgtgc cagagcgagg tagataagtc tgagatccgc 6660

cttgtcggtg gaatcgacga gtttctttcg gagatggtag atggtaggat acttctcgtg 6720

gtaagcaacc tcgtccacaa tgttgccaaa gatgggatga cgctcgtgtt tcttgtcttc 6780

ctcgacgagg aaggattcct ccagtcgatg aaagaacgaa tcgtccacct tggccatctc 6840

gttggaaaag atctcctgca ggtagcagat tcggttcttc cgtcgggtgt aacgtcgccg 6900

agcagttcgc ttgagtctgg tagcttcggc agtctcgcca gaatcgaaca acagggcacc 6960

aatgaggttt ttcttgatgg agtgtcgatc ggtgtttccg aggaccttga atttcttgga 7020

gggcaccttg tactcgtcgg tgatgacagc ccagccgaca gagttggttc caatgtccag 7080

gccgatggag tatttcttgt ccatggtgtg atgtgtagtt tagatttcga atctgtgggg 7140

aaagaaagga aaaaagagac tggcaaccga ttgggagagc cactgtttat atatacccta 7200

gacaagcccc ccgcttgtaa gatgttggtc aatgtaaacc agtattaagg ttggcaagtg 7260

caggagaagc aaggtgtggg taccgagcaa tggaaatgtg cggaaggcaa aaaatgaggc 7320

ccacggccta ttgtcggggc tatatccagg gggcgattga agtacactaa catgacatgt 7380

gtccacagac cctcaatctg gcctgatgag ccaaatccat acgcgctttc gcagctctaa 7440

aggctataac aagtcacacc accctgctcg acctcagcgc cctcactttt tgttaagaca 7500

aactgtacac gctgttccag cgttttctgc ctgcacctgg tgggacattt ggtgcaacct 7560

aaagtgctcg gaacctctgt ggtgtccaga tcagcgcagc agttccgagg tagttttgag 7620

gcccttagat gatggtttaa accttaagcc cgctcataac ttcgtatagc atacattata 7680

cgaacggtag gttgcgggat agacgccgac ggagggcaat ggcgctatgg aaccttgcgg 7740

atatccatac gccgcggcgg actgcgtccg aaccagctcc agcagcgttt tttccgggcc 7800

attgagccga ctgcgacccc gccaacgtgt cttggcccac gcactcatgt catgttggtg 7860

ttgggaggcc actttttaag tagcacaagg cacctagctc gcagcaaggt gtccgaacca 7920

aagaagcggc tgcagtggtg caaacggggc ggaaacggcg ggaaaaagcc acgggggcac 7980

gaattgaggc acgccctcga atttgagacg agtcacggcc ccattcgccc gcgcaatggc 8040

tcgccaacgc ccggtctttt gcaccacatc aggttacccc aagccaaacc tttgtgttaa 8100

aaagcttaac atattatacc gaacgtaggt ttgggcgggc ttgctccgtc tgtccaaggc 8160

aacatttata taagggtctg catcgccggc tcaattgaat cttttttctt cttctcttct 8220

ctatattcat tcttgaatta aacacacatc aaccatggcc aaaaagcctg aactcaccgc 8280

gacgtctgtc gagaagtttc tgatcgaaaa gttcgacagc gtctccgacc tgatgcagct 8340

ctcggagggc gaagaatctc gtgctttcag cttcgatgta ggagggcgtg gatatgtcct 8400

gcgggtaaat agctgcgccg atggtttcta caaagatcgt tatgtttatc ggcactttgc 8460

atcggccgcg ctcccgattc cggaagtgct tgacattggg gagttcagcg agagcctgac 8520

ctattgcatc tcccgccgtg cacagggtgt cacgttgcaa gacctgcctg aaaccgaact 8580

gcccgctgtt ctgcagccgg tcgcggaggc tatggatgcg atcgctgcgg ccgatcttag 8640

ccagacgagc gggttcggcc cattcggacc gcaaggaatc ggtcaataca ctacatggcg 8700

tgatttcata tgcgcgattg ctgatcccca tgtgtatcac tggcaaactg tgatggacga 8760

caccgtcagt gcgtccgtcg cgcaggctct cgatgagctg atgctttggg ccgaggactg 8820

ccccgaagtc cggcacctcg tgcacgcgga tttcggctcc aacaatgtcc tgacggacaa 8880

tggccgcata acagcggtca ttgactggag cgaggcgatg ttcggggatt cccaatacga 8940

ggtcgccaac atcttcttct ggaggccgtg gttggcttgt atggagcagc agacgcgcta 9000

cttcgagcgg aggcatccgg agcttgcagg atcgccgcgg ctccgggcgt atatgctccg 9060

cattggtctt gaccaactct atcagagctt ggttgacggc aatttcgatg atgcagcttg 9120

ggcgcagggt cgatgcgacg caatcgtccg atccggagcc gggactgtcg ggcgtacaca 9180

aatcgcccgc agaagcgcgg ccgtctggac cgatggctgt gtagaagtac tcgccgatag 9240

tggaaaccga cgccccagca ctcgtccgag ggcaaaggaa tagcggccgc aagtgtggat 9300

ggggaagtga gtgcccggtt ctgtgtgcac aattggcaat ccaagatgga tggattcaac 9360

acagggatat agcgagctac gtggtggtgc gaggatatag caacggatat ttatgtttga 9420

cacttgagaa tgtacgatac aagcactgtc caagtacaat actaaacata ctgtacatac 9480

tcatactcgt acccgggcaa cggtttcact tgagtgcagt ggctagtgct cttactcgta 9540

cagtgtgcta ccgttcgtat agcatacatt atacgaagtt atcatagtct taattaaggc 9600

agattggccg agtggtttaa ggcggcagac ttaagatctg ctgaacgcaa gttctcgtgg 9660

gttcgaaccc cacatctgtc attcaaacga ttacccaccc tcgttttaga gctagaaata 9720

gcaagttaaa ataaggctag tccgttatca acttgaaaaa gtggcaccga gtcggtgctt 9780

ttttttttgt tttttatcga ttgagcatcc gttgatttcc gaacagatcc caatattaca 9840

cccaagtagc atgcataagc taaaagtaac tcgcagcgca caccgtgcag attcataagt 9900

ctatgattaa ttgaacgcca ataacccggc ttactacaag tacaagtagg tatacatagc 9960

ggtaatgaat cattagaaaa aaaaaaaaca aaaaaaaaca aaacaaactg ttgtggatgc 10020

atcaacagta gtacatagtt gtacgatgta cttgtacttg taaaagcaaa aatgtacaat 10080

atctcaggga gcgcaacttt tacgttcgaa gaacaatgta ccgcataccg cattctagat 10140

tctgcggaac gtctaacctg gaaatacgat tttttttttc tttcattttt tttgcttctt 10200

caaaagtatg gtaatttcct accattacag ttgacactga acgagggggg attgaattta 10260

agcaaaaaat taaatcaaaa tacctttatg tatccagccc atgtaataaa caaaaggatt 10320

atataacaag aaataaatat atacctttaa tggatcatta gaataaaaat aaatacgaga 10380

agcacaccag agaagctttt tgattgccac tataccgcta ctttggtata tcttattata 10440

attgttgaat ttgcaagata gaatgtcatt cattggagag aaatccaagg aatatgtggg 10500

atgaaatgac tagaagtatg aacaatgaga atagtacata cttgtacctg tatttctaga 10560

agagagaaag acagttgagt gtgtgattct cgtccaataa taatctcaat agtaacgtgt 10620

gaatagctgt tctttgatag ttgatatttc tcgatgacta tttatgttgt acaagggatt 10680

tttttcgttg ctgttgattt cgaattaggc aatgcagata tcatttatgc tatccatatt 10740

taagatttcc catacgcatt tataacattt attctacata aattgttaaa tgaacgaact 10800

gccattataa attgtttcct aaataggaag tgtttttcat aaagcaagta agttgtctaa 10860

taatactaag taataaaaat aagttcatac aatatatttt gagaacatca tttggaggcg 10920

gtagatggag tctgtttatt attaaacaat gcgagatgac cccttaaata ttgagaacat 10980

cagttggagg cggcagatgg agtctgtcta tttagcaatg ggacatgact gtcagtatca 11040

tcatgatgta tatatataat acatataata ttatataaca cgattttttt aaattattgg 11100

cccgaaaatt aatcagtgta gactggatct cggcagtctc tcggatgtag aattaggttt 11160

ccttgaggcg aagatcggtt tgtgtgacat gaattcgata tcaagcttat cgacaccatc 11220

gacctcgagg gggggcccgg tacccaattc gccctatagt gagtcgtatt acaattcact 11280

ggccgtcgtt ttacaacgtc gtgactggga aaaccctggc gttacccaac ttaatcgcct 11340

tgcagc 11346

<210> 108
<211> 237
<212> DNA
<213> Artificial Sequence

<220>
<223> Artificial Sequence

<400> 108
ttaattaagt caatttggcc gagtggtcta aggcgtccga ctcaagtgta agcttagcaa 60

gagaaatcta gctttcggat ctcgcaagag gcgagggttc gaatccctca gttgacattc 120

aaacgattac ccaccctcgt tttagagcta gaaatagcaa gttaaaataa ggctagtccg 180

ttatcaactt gaaaaagtgg caccgagtcg gtgctttttt ttttgttttt tatcgat 237

<210> 109
<211> 109
<212> DNA
<213> Artificial Sequence

<220>
<223> Artificial Sequence

<400> 109
gtcaatttgg ccgagtggtc taaggcgtcc gactcaagtg taagcttagc aagagaaatc 60

tagctttcgg atctcgcaag aggcgagggt tcgaatccct cagttgaca 109

<210> 110
<211> 11371
<212> DNA
<213> Artificial Sequence

<220>
<223> Artificial Sequence

<400> 110
acatccccct ttcgccagct ggcgtaatag cgaagaggcc cgcaccgatc gcccttccca 60

acagttgcgc agcctgaatg gcgaatggaa attgtaagcg ttaatatttt gttaaaattc 120

gcgttaaatt tttgttaaat cagctcattt tttaaccaat aggccgaaat cggcaaaatc 180

ccttataaat caaaagaata gaccgagata gggttgagtg ttgttccagt ttggaacaag 240

agtccactat taaagaacgt ggactccaac gtcaaagggc gaaaaaccgt ctatcagggc 300

gatggcccac tacgtgaacc atcaccctaa tcaagttttt tggggtcgag gtgccgtaaa 360

gcactaaatc ggaaccctaa agggagcccc cgatttagag cttgacgggg aaagccggcg 420

aacgtggcga gaaaggaagg gaagaaagcg aaaggagcgg gcgctagggc gctggcaagt 480

gtagcggtca cgctgcgcgt aaccaccaca cccgccgcgc ttaatgcgcc gctacagggc 540

gcgtcaggtg gcacttttcg gggaaatgtg cgcggaaccc ctatttgttt atttttctaa 600

atacattcaa atatgtatcc gctcatgaga caataaccct gataaatgct tcaataatat 660

tgaaaaagga agagtatgag tattcaacat ttccgtgtcg cccttattcc cttttttgcg 720

gcattttgcc ttcctgtttt tgctcaccca gaaacgctgg tgaaagtaaa agatgctgaa 780

gatcagttgg gtgcacgagt gggttacatc gaactggatc tcaacagcgg taagatcctt 840

gagagttttc gccccgaaga acgttttcca atgatgagca cttttaaagt tctgctatgt 900

ggcgcggtat tatcccgtat tgacgccggg caagagcaac tcggtcgccg catacactat 960

tctcagaatg acttggttga gtactcacca gtcacagaaa agcatcttac ggatggcatg 1020

acagtaagag aattatgcag tgctgccata accatgagtg ataacactgc ggccaactta 1080

cttctgacaa cgatcggagg accgaaggag ctaaccgctt ttttgcacaa catgggggat 1140

catgtaactc gccttgatcg ttgggaaccg gagctgaatg aagccatacc aaacgacgag 1200

cgtgacacca cgatgcctgt agcaatggca acaacgttgc gcaaactatt aactggcgaa 1260

ctacttactc tagcttcccg gcaacaatta atagactgga tggaggcgga taaagttgca 1320

ggaccacttc tgcgctcggc ccttccggct ggctggttta ttgctgataa atctggagcc 1380

ggtgagcgtg ggtctcgcgg tatcattgca gcactggggc cagatggtaa gccctcccgt 1440

atcgtagtta tctacacgac ggggagtcag gcaactatgg atgaacgaaa tagacagatc 1500

gctgagatag gtgcctcact gattaagcat tggtaactgt cagaccaagt ttactcatat 1560

atactttaga ttgatttaaa acttcatttt taatttaaaa ggatctaggt gaagatcctt 1620

tttgataatc tcatgaccaa aatcccttaa cgtgagtttt cgttccactg agcgtcagac 1680

cccgtagaaa agatcaaagg atcttcttga gatccttttt ttctgcgcgt aatctgctgc 1740

ttgcaaacaa aaaaaccacc gctaccagcg gtggtttgtt tgccggatca agagctacca 1800

actctttttc cgaaggtaac tggcttcagc agagcgcaga taccaaatac tgtccttcta 1860

gtgtagccgt agttaggcca ccacttcaag aactctgtag caccgcctac atacctcgct 1920

ctgctaatcc tgttaccagt ggctgctgcc agtggcgata agtcgtgtct taccgggttg 1980

gactcaagac gatagttacc ggataaggcg cagcggtcgg gctgaacggg gggttcgtgc 2040

acacagccca gcttggagcg aacgacctac accgaactga gatacctaca gcgtgagcta 2100

tgagaaagcg ccacgcttcc cgaagggaga aaggcggaca ggtatccggt aagcggcagg 2160

gtcggaacag gagagcgcac gagggagctt ccagggggaa acgcctggta tctttatagt 2220

cctgtcgggt ttcgccacct ctgacttgag cgtcgatttt tgtgatgctc gtcagggggg 2280

cggagcctat ggaaaaacgc cagcaacgcg gcctttttac ggttcctggc cttttgctgg 2340

ccttttgctc acatgttctt tcctgcgtta tcccctgatt ctgtggataa ccgtattacc 2400

gcctttgagt gagctgatac cgctcgccgc agccgaacga ccgagcgcag cgagtcagtg 2460

agcgaggaag cggaagagcg cccaatacgc aaaccgcctc tccccgcgcg ttggccgatt 2520

cattaatgca gctggcacga caggtttccc gactggaaag cgggcagtga gcgcaacgca 2580

attaatgtga gttagctcac tcattaggca ccccaggctt tacactttat gcttccggct 2640

cgtacgcaac taacatgaat gaatacgata tacatcaaag actatgatac gcagtattgc 2700

acactgtacg agtaagagca ctagccactg cactcaagtg aaaccgttgc ccgggtacga 2760

gtatgagtat gtacagtatg tttagtattg tacttggaca gtgcttgtat cgtacattct 2820

caagtgtcaa acataaatat ccgttgctat atcctcgcac caccacgtag ctcgctatat 2880

ccctgtgttg aatccatcca tcttggattg ccaattgtgc acacagaacc gggcactcac 2940

ttccccatcc acacttgcgg ccgcttagac ctttcgcttt ttcttgggat cggctctgga 3000

gtcgccacca agctgagaca ggtcgattcg ggtctcgtac aggccagtga tggactggtg 3060

aatcagggtg gcatcgagaa cctccttggt ggatgtgtac cgctttcggt cgatagtggt 3120

atcgaagtac ttgaaagctg caggagcacc caggttggta agagtaaaca ggtgaatgat 3180

gttctccgcc tgttctcgaa tgggtttgtc ccgatgcttg ttgtaggcag agagcacctt 3240

gtccaagttg gcatcagcca ggatgactcg cttcgaaaac tcggaaatct gctcgataat 3300

ctcgtcgagg taatgtttgt gctgctcaac gaagagttgc ttctgttcgt tgtcctcggg 3360

agaacccttg agcttctcgt agtgagaagc cagatagaga aagttgacgt acttcgaagg 3420

caaggcaagc tcgtttccct tctgcagctc gccagcggag gcgagcatac gctttcgacc 3480

gttctccagt tcgaacagag agtacttggg cagcttgata atgaggtctt tcttgacctc 3540

cttgtaaccc ttggcttcca agaagtcgat gggattcttc tcgaagctcg atcgctccat 3600

gatggtaatt ccgagcagct ccttgacgga cttgagcttt ttggacttgc ccttctcgac 3660

cttcgcaacg acaagcacgg aataggcgac ggtaggagaa tcgaagccac cgtatttctt 3720

gggatcccag tctttctttc gagcgatgag cttgtcggag tttcgcttgg gcagaatcga 3780

ctccttggag aatccgccag tctgaacctc ggttttcttg acgatgttga cctgaggcat 3840

cgacagaacc tttcgcacgg ttgcaaagtc tcgacccttg tcccacacga tctctccagt 3900

ttcgccgttg gtctcgataa gtggtctctt tcgaatctct ccgttggcca aggtgatctc 3960

ggtcttgaaa aagttcatga tgttggagta aaagaagtac ttggcagtag ccttgccaat 4020

ctcctgttcg gacttggcaa tcatctttcg aacgtcgtag accttgtaat cgccgtaaac 4080

gaactcgctt tcgagcttgg ggtatttctt gatgagcgca gtgccaacga cggcgttgag 4140

gtaagcatcg tgggcatggt ggtaattgtt gatctctcgc accttgtaga actgaaagtc 4200

ctttcggaaa tcggagacca gtttggactt gagagtaatc accttgacct ctcggatgag 4260

cttgtcgttc tcgtcgtact tggtgttcat ccgagaatcg agaatctgtg cgacgtgctt 4320

tgtgatctgt ctggtctcga cgagttgacg cttgatgaag ccagccttgt cgagctcgga 4380

cagaccgcct cgctcggcct tggtaagatt gtcgaacttt cgctgggtaa tgagcttggc 4440

gttgagcagc tgtcgccagt agttcttcat ctttttgacc acctcttcgc tgggaacgtt 4500

gtccgacttg cctctgttct tgtcggatcg tgtaaggacc ttgttgtcga tagaatcgtc 4560

cttgagaaag gattgaggga caatgtggtc cacatcgtag tcgctgagac gattgatgtc 4620

cagttcctga tccacgtaca tgtctcgacc attctgcaga tagtagagat acagcttctc 4680

gttctgcagt tgagtgttct cgacgggatg ctccttgaga atctgggatc ccagctcctt 4740

gatgccttcc tcgattcgct tcatccgctc tcgcgagttt ttctgaccct tttgagttgt 4800

ctggttctct ctggccatct cgatcacaat gttctcgggc ttgtgacgtc ccatgacctt 4860

caccagctcg tcgacaacct tgacagtctg gagaatgcct ttcttgatgg ctggcgaacc 4920

agccaggttg gcaatatgtt cgtgcaagct gtcgccctga ccggacactt gtgccttctg 4980

gatgtcctcc ttgaaggtaa gagaatcgtc gtgaatgagc tgcatgaagt ttcggttggc 5040

aaagccatcg gacttgagaa agtccagaat ggtctttccg gactgcttgt ctctgatgcc 5100

gttgatgagc tttcgcgaaa gtcttcccca gccggtgtat ctacgtcgct tgagttgttt 5160

catgaccttg tcgtcgaaca ggtgagcgta tgtcttgagt cgttcctcga tcatctcccg 5220

atcttcgaac agggtaagag tgagcacgat gtcctccaga atgtcctcgt tttcctcgtt 5280

gtcgagaaaa tccttgtcct tgataatctt gagcagatcg tgataggtgc ccaaagaggc 5340

gttgaatcgg tcctcaactc cggaaatctc gacgctgtcg aaacactcga ttttcttgaa 5400

gtagtcctcc ttgagctgct taacagtgac ctttcggttg gtcttgaaca ggagatcgac 5460

aatggctttc ttctgttcgc cagacaagaa ggcaggcttt cgcattccct cggtaacgta 5520

cttgactttg gtgagttcgt tgtagactgt aaagtactcg tagagcagcg aatgcttggg 5580

aagaaccttc tcgttgggca gattcttgtc gaagttggtc attcgctcga tgaaggactg 5640

tgcagaggca cccttgtcca cgacttcctc gaagttccag ggagtgatgg tttcctcgga 5700

ctttcgagtc atccaagcaa atcgagagtt tcctctggca agaggaccaa catagtaggg 5760

gattcgaaag gtaagaatct tctcgatctt ctctcggttg tccttgagaa aggggtagaa 5820

gtcttcctga cgtcgaagaa tggcgtgcag ctcaccgagg tggatctgat gaggaatgct 5880

gccgttgtcg aaggttcgtt gcttccgaag cagatcctct cgattgagct tgacaagcag 5940

ttcctcggtt ccgtccatct tctcgagaat tggcttgatg aacttgtaga actcttcctg 6000

agaggctccg ccgtcgatgt atccagcgta gccgttcttc gactgatcga aaaagatctc 6060

cttgtacttc tcgggcagtt gctgtcggac aagagccttg agcagtgtga gatcctgatg 6120

gtgctcgtcg tatcgcttga tcatggaggc agaaagggga gcctttgtga tctcggtgtt 6180

gactcgcaga atgtcagaca agagaatagc atccgaaagg ttcttggcag cgagaaacag 6240

gtcggcgtac tgatcgccaa tctgtgcaag caggttgtcg aggtcatcgt cgtaggtgtc 6300

cttggacagc tggagcttgg cgtcctccgc cagatcgaag ttggacttga agttgggtgt 6360

gagaccaaga gaaagggcaa tgaggttgcc aaacagtccg ttctttttct cgccaggaag 6420

ttgggcaatg aggttctcca gtcgtctgct cttcgagagt cgagcagaca agatggcctt 6480

tgcatcgact ccggaggcat tgatggggtt ttcctcgaac agctggttgt aggtctgaac 6540

gagctgaatg aacagcttgt ccacatcgct gttgtcggga ttgagatcgc cctcgatgag 6600

gaaatgacct cgaaacttga tcatgtgtgc cagagcgagg tagataagtc tgagatccgc 6660

cttgtcggtg gaatcgacga gtttctttcg gagatggtag atggtaggat acttctcgtg 6720

gtaagcaacc tcgtccacaa tgttgccaaa gatgggatga cgctcgtgtt tcttgtcttc 6780

ctcgacgagg aaggattcct ccagtcgatg aaagaacgaa tcgtccacct tggccatctc 6840

gttggaaaag atctcctgca ggtagcagat tcggttcttc cgtcgggtgt aacgtcgccg 6900

agcagttcgc ttgagtctgg tagcttcggc agtctcgcca gaatcgaaca acagggcacc 6960

aatgaggttt ttcttgatgg agtgtcgatc ggtgtttccg aggaccttga atttcttgga 7020

gggcaccttg tactcgtcgg tgatgacagc ccagccgaca gagttggttc caatgtccag 7080

gccgatggag tatttcttgt ccatggtgtg atgtgtagtt tagatttcga atctgtgggg 7140

aaagaaagga aaaaagagac tggcaaccga ttgggagagc cactgtttat atatacccta 7200

gacaagcccc ccgcttgtaa gatgttggtc aatgtaaacc agtattaagg ttggcaagtg 7260

caggagaagc aaggtgtggg taccgagcaa tggaaatgtg cggaaggcaa aaaaatgagg 7320

ccacggccta ttgtcggggc tatatccagg gggcgattga agtacactaa catgacatgt 7380

gtccacagac cctcaatctg gcctgatgag ccaaatccat acgcgctttc gcagctctaa 7440

aggctataac aagtcacacc accctgctcg acctcagcgc cctcactttt tgttaagaca 7500

aactgtacac gctgttccag cgttttctgc ctgcacctgg tgggacattt ggtgcaacct 7560

aaagtgctcg gaacctctgt ggtgtccaga tcagcgcagc agttccgagg tagttttgag 7620

gcccttagat gatggtttaa accttaagcc cgctcataac ttcgtatagc atacattata 7680

cgaacggtag gttgcgggat agacgccgac ggagggcaat ggcgctatgg aaccttgcgg 7740

atatccatac gccgcggcgg actgcgtccg aaccagctcc agcagcgttt tttccgggcc 7800

attgagccga ctgcgacccc gccaacgtgt cttggcccac gcactcatgt catgttggtg 7860

ttgggaggcc actttttaag tagcacaagg cacctagctc gcagcaaggt gtccgaacca 7920

aagaagcggc tgcagtggtg caaacggggc ggaaacggcg ggaaaaagcc acgggggcac 7980

gaattgaggc acgccctcga atttgagacg agtcacggcc ccattcgccc gcgcaatggc 8040

tcgccaacgc ccggtctttt gcaccacatc aggttacccc aagccaaacc tttgtgttaa 8100

aaagcttaac atattatacc gaacgtaggt ttgggcgggc ttgctccgtc tgtccaaggc 8160

aacatttata taagggtctg catcgccggc tcaattgaat cttttttctt cttctcttct 8220

ctatattcat tcttgaatta aacacacatc aaccatggcc aaaaagcctg aactcaccgc 8280

gacgtctgtc gagaagtttc tgatcgaaaa gttcgacagc gtctccgacc tgatgcagct 8340

ctcggagggc gaagaatctc gtgctttcag cttcgatgta ggagggcgtg gatatgtcct 8400

gcgggtaaat agctgcgccg atggtttcta caaagatcgt tatgtttatc ggcactttgc 8460

atcggccgcg ctcccgattc cggaagtgct tgacattggg gagttcagcg agagcctgac 8520

ctattgcatc tcccgccgtg cacagggtgt cacgttgcaa gacctgcctg aaaccgaact 8580

gcccgctgtt ctgcagccgg tcgcggaggc tatggatgcg atcgctgcgg ccgatcttag 8640

ccagacgagc gggttcggcc cattcggacc gcaaggaatc ggtcaataca ctacatggcg 8700

tgatttcata tgcgcgattg ctgatcccca tgtgtatcac tggcaaactg tgatggacga 8760

caccgtcagt gcgtccgtcg cgcaggctct cgatgagctg atgctttggg ccgaggactg 8820

ccccgaagtc cggcacctcg tgcacgcgga tttcggctcc aacaatgtcc tgacggacaa 8880

tggccgcata acagcggtca ttgactggag cgaggcgatg ttcggggatt cccaatacga 8940

ggtcgccaac atcttcttct ggaggccgtg gttggcttgt atggagcagc agacgcgcta 9000

cttcgagcgg aggcatccgg agcttgcagg atcgccgcgg ctccgggcgt atatgctccg 9060

cattggtctt gaccaactct atcagagctt ggttgacggc aatttcgatg atgcagcttg 9120

ggcgcagggt cgatgcgacg caatcgtccg atccggagcc gggactgtcg ggcgtacaca 9180

aatcgcccgc agaagcgcgg ccgtctggac cgatggctgt gtagaagtac tcgccgatag 9240

tggaaaccga cgccccagca ctcgtccgag ggcaaaggaa tagcggccgc aagtgtggat 9300

ggggaagtga gtgcccggtt ctgtgtgcac aattggcaat ccaagatgga tggattcaac 9360

acagggatat agcgagctac gtggtggtgc gaggatatag caacggatat ttatgtttga 9420

cacttgagaa tgtacgatac aagcactgtc caagtacaat actaaacata ctgtacatac 9480

tcatactcgt acccgggcaa cggtttcact tgagtgcagt ggctagtgct cttactcgta 9540

cagtgtgcta ccgttcgtat agcatacatt atacgaagtt atcatagtct taattaagtc 9600

aatttggccg agtggtctaa ggcgtccgac tcaagtgtaa gcttagcaag agaaatctag 9660

ctttcggatc tcgcaagagg cgagggttcg aatccctcag ttgacattca aacgattacc 9720

caccctcgtt ttagagctag aaatagcaag ttaaaataag gctagtccgt tatcaacttg 9780

aaaaagtggc accgagtcgg tgcttttttt tttgtttttt atcgattgag catccgttga 9840

tttccgaaca gatcccaata ttacacccaa gtagcatgca taagctaaaa gtaactcgca 9900

gcgcacaccg tgcagattca taagtctatg attaattgaa cgccaataac ccggcttact 9960

acaagtacaa gtaggtatac atagcggtaa tgaatcatta gaaaaaaaaa aaacaaaaaa 10020

aaacaaaaca aactgttgtg gatgcatcaa cagtagtaca tagttgtacg atgtacttgt 10080

acttgtaaaa gcaaaaatgt acaatatctc agggagcgca acttttacgt tcgaagaaca 10140

atgtaccgca taccgcattc tagattctgc ggaacgtcta acctggaaat acgatttttt 10200

ttttctttca ttttttttgc ttcttcaaaa gtatggtaat ttcctaccat tacagttgac 10260

actgaacgag gggggattga atttaagcaa aaaattaaat caaaatacct ttatgtatcc 10320

agcccatgta ataaacaaaa ggattatata acaagaaata aatatatacc tttaatggat 10380

cattagaata aaaataaata cgagaagcac accagagaag ctttttgatt gccactatac 10440

cgctactttg gtatatctta ttataattgt tgaatttgca agatagaatg tcattcattg 10500

gagagaaatc caaggaatat gtgggatgaa atgactagaa gtatgaacaa tgagaatagt 10560

acatacttgt acctgtattt ctagaagaga gaaagacagt tgagtgtgtg attctcgtcc 10620

aataataatc tcaatagtaa cgtgtgaata gctgttcttt gatagttgat atttctcgat 10680

gactatttat gttgtacaag ggattttttt cgttgctgtt gatttcgaat taggcaatgc 10740

agatatcatt tatgctatcc atatttaaga tttcccatac gcatttataa catttattct 10800

acataaattg ttaaatgaac gaactgccat tataaattgt ttcctaaata ggaagtgttt 10860

ttcataaagc aagtaagttg tctaataata ctaagtaata aaaataagtt catacaatat 10920

attttgagaa catcatttgg aggcggtaga tggagtctgt ttattattaa acaatgcgag 10980

atgacccctt aaatattgag aacatcagtt ggaggcggca gatggagtct gtctatttag 11040

caatgggaca tgactgtcag tatcatcatg atgtatatat ataatacata taatattata 11100

taacacgatt tttttaaatt attggcccga aaattaatca gtgtagactg gatctcggca 11160

gtctctcgga tgtagaatta ggtttccttg aggcgaagat cggtttgtgt gacatgaatt 11220

cgatatcaag cttatcgaca ccatcgacct cgaggggggg cccggtaccc aattcgccct 11280

atagtgagtc gtattacaat tcactggccg tcgttttaca acgtcgtgac tgggaaaacc 11340

ctggcgttac ccaacttaat cgccttgcag c 11371

<210> 111
<211> 210
<212> DNA

<213>  Artificial Sequence

<220>
<223>  Artificial Sequence

<400>  111
ttaattaagg gagtttggcc gagtggtcta aggcgctagc ttcaggtgct agtctcgaaa      60

gaggcgtgag ttcgaacctc acagctctca ttcaaacgat tacccaccct cgttttagag     120

ctagaaatag caagttaaaa taaggctagt ccgttatcaa cttgaaaaag tggcaccgag     180

tcggtgcttt tttttttgtt ttttatcgat                                       210


<210>  112
<211>  82
<212>  DNA
<213>  Artificial Sequence

<220>
<223>  Artificial Sequence

<400>  112
gggagtttgg ccgagtggtc taaggcgcta gcttcaggtg ctagtctcga aagaggcgtg      60

agttcgaacc tcacagctct ca                                               82


<210>  113
<211>  11344
<212>  DNA
<213>  Artificial Sequence

<220>
<223>  Artificial Sequence

<400>  113
   acatccccct ttcgccagct ggcgtaatag cgaagaggcc cgcaccgatc gcccttccca      60
   acagttgcgc agcctgaatg gcgaatggaa attgtaagcg ttaatatttt gttaaaattc     120
   gcgttaaatt tttgttaaat cagctcattt tttaaccaat aggccgaaat cggcaaaatc     180
   ccttataaat caaaagaata gaccgagata gggttgagtg ttgttccagt ttggaacaag     240
   agtccactat taaagaacgt ggactccaac gtcaaagggc gaaaaaccgt ctatcagggc     300
   gatggcccac tacgtgaacc atcaccctaa tcaagttttt tggggtcgag gtgccgtaaa     360
   gcactaaatc ggaaccctaa agggagcccc cgatttagag cttgacgggg aaagccggcg     420
   aacgtggcga gaaaggaagg gaagaaagcg aaaggagcgg gcgctagggc gctggcaagt     480
   gtagcggtca cgctgcgcgt aaccaccaca cccgccgcgc ttaatgcgcc gctacagggc     540
   gcgtcaggtg gcacttttcg gggaaatgtg cgcggaaccc ctatttgttt atttttctaa     600
   atacattcaa atatgtatcc gctcatgaga caataaccct gataaatgct tcaataatat     660
   tgaaaaagga agagtatgag tattcaacat ttccgtgtcg cccttattcc cttttttgcg     720
   gcattttgcc ttcctgtttt tgctcaccca gaaacgctgg tgaaagtaaa agatgctgaa     780
   gatcagttgg gtgcacgagt gggttacatc gaactggatc tcaacagcgg taagatcctt 840
   gagagttttc gccccgaaga acgttttcca atgatgagca cttttaaagt tctgctatgt 900
   ggcgcggtat tatcccgtat tgacgccggg caagagcaac tcggtcgccg catacactat 960
   tctcagaatg acttggttga gtactcacca gtcacagaaa agcatcttac ggatggcatg 1020
   acagtaagag aattatgcag tgctgccata accatgagtg ataacactgc ggccaactta 1080
   cttctgacaa cgatcggagg accgaaggag ctaaccgctt ttttgcacaa catgggggat 1140
   catgtaactc gccttgatcg ttgggaaccg gagctgaatg aagccatacc aaacgacgag 1200
   cgtgacacca cgatgcctgt agcaatggca acaacgttgc gcaaactatt aactggcgaa 1260
   ctacttactc tagcttcccg gcaacaatta atagactgga tggaggcgga taaagttgca 1320
   ggaccacttc tgcgctcggc ccttccggct ggctggttta ttgctgataa atctggagcc 1380
   ggtgagcgtg ggtctcgcgg tatcattgca gcactggggc cagatggtaa gccctcccgt 1440
   atcgtagtta tctacacgac ggggagtcag gcaactatgg atgaacgaaa tagacagatc 1500
   gctgagatag gtgcctcact gattaagcat tggtaactgt cagaccaagt ttactcatat 1560
   atactttaga ttgatttaaa acttcatttt taatttaaaa ggatctaggt gaagatcctt 1620
   tttgataatc tcatgaccaa aatcccttaa cgtgagtttt cgttccactg agcgtcagac 1680
   cccgtagaaa agatcaaagg atcttcttga gatccttttt ttctgcgcgt aatctgctgc 1740
   ttgcaaacaa aaaaaccacc gctaccagcg gtggtttgtt tgccggatca agagctacca 1800
   actctttttc cgaaggtaac tggcttcagc agagcgcaga taccaaatac tgtccttcta 1860
   gtgtagccgt agttaggcca ccacttcaag aactctgtag caccgcctac atacctcgct 1920
   ctgctaatcc tgttaccagt ggctgctgcc agtggcgata agtcgtgtct taccgggttg 1980
   gactcaagac gatagttacc ggataaggcg cagcggtcgg gctgaacggg gggttcgtgc 2040
   acacagccca gcttggagcg aacgacctac accgaactga gatacctaca gcgtgagcta 2100
   tgagaaagcg ccacgcttcc cgaagggaga aaggcggaca ggtatccggt aagcggcagg 2160
   gtcggaacag gagagcgcac gagggagctt ccagggggaa acgcctggta tctttatagt 2220
   cctgtcgggt ttcgccacct ctgacttgag cgtcgatttt tgtgatgctc gtcagggggg 2280
   cggagcctat ggaaaaacgc cagcaacgcg gcctttttac ggttcctggc cttttgctgg 2340
   ccttttgctc acatgttctt tcctgcgtta tcccctgatt ctgtggataa ccgtattacc 2400
   gcctttgagt gagctgatac cgctcgccgc agccgaacga ccgagcgcag cgagtcagtg 2460
   agcgaggaag cggaagagcg cccaatacgc aaaccgcctc tccccgcgcg ttggccgatt 2520
   cattaatgca gctggcacga caggtttccc gactggaaag cgggcagtga gcgcaacgca 2580
   attaatgtga gttagctcac tcattaggca ccccaggctt tacactttat gcttccggct 2640
   cgtacgcaac taacatgaat gaatacgata tacatcaaag actatgatac gcagtattgc 2700
   acactgtacg agtaagagca ctagccactg cactcaagtg aaaccgttgc ccgggtacga 2760
   gtatgagtat gtacagtatg tttagtattg tacttggaca gtgcttgtat cgtacattct 2820
   caagtgtcaa acataaatat ccgttgctat atcctcgcac caccacgtag ctcgctatat 2880
   ccctgtgttg aatccatcca tcttggattg ccaattgtgc acacagaacc gggcactcac 2940
   ttccccatcc acacttgcgg ccgcttagac ctttcgcttt ttcttgggat cggctctgga 3000
   gtcgccacca agctgagaca ggtcgattcg ggtctcgtac aggccagtga tggactggtg 3060
   aatcagggtg gcatcgagaa cctccttggt ggatgtgtac cgctttcggt cgatagtggt 3120
   atcgaagtac ttgaaagctg caggagcacc caggttggta agagtaaaca ggtgaatgat 3180
   gttctccgcc tgttctcgaa tgggtttgtc ccgatgcttg ttgtaggcag agagcacctt 3240
   gtccaagttg gcatcagcca ggatgactcg cttcgaaaac tcggaaatct gctcgataat 3300
   ctcgtcgagg taatgtttgt gctgctcaac gaagagttgc ttctgttcgt tgtcctcggg 3360
   agaacccttg agcttctcgt agtgagaagc cagatagaga aagttgacgt acttcgaagg 3420
   caaggcaagc tcgtttccct tctgcagctc gccagcggag gcgagcatac gctttcgacc 3480
   gttctccagt tcgaacagag agtacttggg cagcttgata atgaggtctt tcttgacctc 3540
   cttgtaaccc ttggcttcca agaagtcgat gggattcttc tcgaagctcg atcgctccat 3600
   gatggtaatt ccgagcagct ccttgacgga cttgagcttt ttggacttgc ccttctcgac 3660
   cttcgcaacg acaagcacgg aataggcgac ggtaggagaa tcgaagccac cgtatttctt 3720
   gggatcccag tctttctttc gagcgatgag cttgtcggag tttcgcttgg gcagaatcga 3780
   ctccttggag aatccgccag tctgaacctc ggttttcttg acgatgttga cctgaggcat 3840
   cgacagaacc tttcgcacgg ttgcaaagtc tcgacccttg tcccacacga tctctccagt 3900
   ttcgccgttg gtctcgataa gtggtctctt tcgaatctct ccgttggcca aggtgatctc 3960
   ggtcttgaaa aagttcatga tgttggagta aaagaagtac ttggcagtag ccttgccaat 4020
   ctcctgttcg gacttggcaa tcatctttcg aacgtcgtag accttgtaat cgccgtaaac 4080
   gaactcgctt tcgagcttgg ggtatttctt gatgagcgca gtgccaacga cggcgttgag 4140
   gtaagcatcg tgggcatggt ggtaattgtt gatctctcgc accttgtaga actgaaagtc 4200
   ctttcggaaa tcggagacca gtttggactt gagagtaatc accttgacct ctcggatgag 4260
   cttgtcgttc tcgtcgtact tggtgttcat ccgagaatcg agaatctgtg cgacgtgctt 4320
   tgtgatctgt ctggtctcga cgagttgacg cttgatgaag ccagccttgt cgagctcgga 4380
   cagaccgcct cgctcggcct tggtaagatt gtcgaacttt cgctgggtaa tgagcttggc 4440
   gttgagcagc tgtcgccagt agttcttcat ctttttgacc acctcttcgc tgggaacgtt 4500
   gtccgacttg cctctgttct tgtcggatcg tgtaaggacc ttgttgtcga tagaatcgtc 4560
   cttgagaaag gattgaggga caatgtggtc cacatcgtag tcgctgagac gattgatgtc 4620
   cagttcctga tccacgtaca tgtctcgacc attctgcaga tagtagagat acagcttctc 4680
   gttctgcagt tgagtgttct cgacgggatg ctccttgaga atctgggatc ccagctcctt 4740
   gatgccttcc tcgattcgct tcatccgctc tcgcgagttt ttctgaccct tttgagttgt 4800
   ctggttctct ctggccatct cgatcacaat gttctcgggc ttgtgacgtc ccatgacctt 4860
   caccagctcg tcgacaacct tgacagtctg gagaatgcct ttcttgatgg ctggcgaacc 4920
   agccaggttg gcaatatgtt cgtgcaagct gtcgccctga ccggacactt gtgccttctg 4980
   gatgtcctcc ttgaaggtaa gagaatcgtc gtgaatgagc tgcatgaagt ttcggttggc 5040
   aaagccatcg gacttgagaa agtccagaat ggtctttccg gactgcttgt ctctgatgcc 5100
   gttgatgagc tttcgcgaaa gtcttcccca gccggtgtat ctacgtcgct tgagttgttt 5160
   catgaccttg tcgtcgaaca ggtgagcgta tgtcttgagt cgttcctcga tcatctcccg 5220
   atcttcgaac agggtaagag tgagcacgat gtcctccaga atgtcctcgt tttcctcgtt 5280
   gtcgagaaaa tccttgtcct tgataatctt gagcagatcg tgataggtgc ccaaagaggc 5340
   gttgaatcgg tcctcaactc cggaaatctc gacgctgtcg aaacactcga ttttcttgaa 5400
   gtagtcctcc ttgagctgct taacagtgac ctttcggttg gtcttgaaca ggagatcgac 5460
   aatggctttc ttctgttcgc cagacaagaa ggcaggcttt cgcattccct cggtaacgta 5520
   cttgactttg gtgagttcgt tgtagactgt aaagtactcg tagagcagcg aatgcttggg 5580
   aagaaccttc tcgttgggca gattcttgtc gaagttggtc attcgctcga tgaaggactg 5640
   tgcagaggca cccttgtcca cgacttcctc gaagttccag ggagtgatgg tttcctcgga 5700
   ctttcgagtc atccaagcaa atcgagagtt tcctctggca agaggaccaa catagtaggg 5760
   gattcgaaag gtaagaatct tctcgatctt ctctcggttg tccttgagaa aggggtagaa 5820
   gtcttcctga cgtcgaagaa tggcgtgcag ctcaccgagg tggatctgat gaggaatgct 5880
   gccgttgtcg aaggttcgtt gcttccgaag cagatcctct cgattgagct tgacaagcag 5940
   ttcctcggtt ccgtccatct tctcgagaat tggcttgatg aacttgtaga actcttcctg 6000
   agaggctccg ccgtcgatgt atccagcgta gccgttcttc gactgatcga aaaagatctc 6060
   cttgtacttc tcgggcagtt gctgtcggac aagagccttg agcagtgtga gatcctgatg 6120
   gtgctcgtcg tatcgcttga tcatggaggc agaaagggga gcctttgtga tctcggtgtt 6180
   gactcgcaga atgtcagaca agagaatagc atccgaaagg ttcttggcag cgagaaacag 6240
   gtcggcgtac tgatcgccaa tctgtgcaag caggttgtcg aggtcatcgt cgtaggtgtc 6300
   cttggacagc tggagcttgg cgtcctccgc cagatcgaag ttggacttga agttgggtgt 6360
   gagaccaaga gaaagggcaa tgaggttgcc aaacagtccg ttctttttct cgccaggaag 6420
   ttgggcaatg aggttctcca gtcgtctgct cttcgagagt cgagcagaca agatggcctt 6480
   tgcatcgact ccggaggcat tgatggggtt ttcctcgaac agctggttgt aggtctgaac 6540
   gagctgaatg aacagcttgt ccacatcgct gttgtcggga ttgagatcgc cctcgatgag 6600
   gaaatgacct cgaaacttga tcatgtgtgc cagagcgagg tagataagtc tgagatccgc 6660
   cttgtcggtg gaatcgacga gtttctttcg gagatggtag atggtaggat acttctcgtg 6720
   gtaagcaacc tcgtccacaa tgttgccaaa gatgggatga cgctcgtgtt tcttgtcttc 6780
   ctcgacgagg aaggattcct ccagtcgatg aaagaacgaa tcgtccacct tggccatctc 6840
   gttggaaaag atctcctgca ggtagcagat tcggttcttc cgtcgggtgt aacgtcgccg 6900
   agcagttcgc ttgagtctgg tagcttcggc agtctcgcca gaatcgaaca acagggcacc 6960
   aatgaggttt ttcttgatgg agtgtcgatc ggtgtttccg aggaccttga atttcttgga 7020
   gggcaccttg tactcgtcgg tgatgacagc ccagccgaca gagttggttc caatgtccag 7080
   gccgatggag tatttcttgt ccatggtgtg atgtgtagtt tagatttcga atctgtgggg 7140
   aaagaaagga aaaaagagac tggcaaccga ttgggagagc cactgtttat atatacccta 7200
   gacaagcccc ccgcttgtaa gatgttggtc aatgtaaacc agtattaagg ttggcaagtg 7260
   caggagaagc aaggtgtggg taccgagcaa tggaaatgtg cggaaggcaa aaaaatgagg 7320
   ccacggccta ttgtcggggc tatatccagg gggcgattga agtacactaa catgacatgt 7380
   gtccacagac cctcaatctg gcctgatgag ccaaatccat acgcgctttc gcagctctaa 7440
   aggctataac aagtcacacc accctgctcg acctcagcgc cctcactttt tgttaagaca 7500
   aactgtacac gctgttccag cgttttctgc ctgcacctgg tgggacattt ggtgcaacct 7560
   aaagtgctcg gaacctctgt ggtgtccaga tcagcgcagc agttccgagg tagttttgag 7620
   gcccttagat gatggtttaa accttaagcc cgctcataac ttcgtatagc atacattata 7680
   cgaacggtag gttgcgggat agacgccgac ggagggcaat ggcgctatgg aaccttgcgg 7740
   atatccatac gccgcggcgg actgcgtccg aaccagctcc agcagcgttt tttccgggcc 7800
   attgagccga ctgcgacccc gccaacgtgt cttggcccac gcactcatgt catgttggtg 7860
   ttgggaggcc actttttaag tagcacaagg cacctagctc gcagcaaggt gtccgaacca 7920
   aagaagcggc tgcagtggtg caaacggggc ggaaacggcg ggaaaaagcc acgggggcac 7980
   gaattgaggc acgccctcga atttgagacg agtcacggcc ccattcgccc gcgcaatggc 8040
   tcgccaacgc ccggtctttt gcaccacatc aggttacccc aagccaaacc tttgtgttaa 8100
   aaagcttaac atattatacc gaacgtaggt ttgggcgggc ttgctccgtc tgtccaaggc 8160
   aacatttata taagggtctg catcgccggc tcaattgaat ctttttcttc cttctcttct 8220
   ctatattcat tcttgaatta aacacacatc aaccatggcc aaaaagcctg aactcaccgc 8280
   gacgtctgtc gagaagtttc tgatcgaaaa gttcgacagc gtctccgacc tgatgcagct 8340
   ctcggagggc gaagaatctc gtgctttcag cttcgatgta ggagggcgtg gatatgtcct 8400
   gcgggtaaat agctgcgccg atggtttcta caaagatcgt tatgtttatc ggcactttgc 8460
   atcggccgcg ctcccgattc cggaagtgct tgacattggg gagttcagcg agagcctgac 8520
   ctattgcatc tcccgccgtg cacagggtgt cacgttgcaa gacctgcctg aaaccgaact 8580
   gcccgctgtt ctgcagccgg tcgcggaggc tatggatgcg atcgctgcgg ccgatcttag 8640
   ccagacgagc gggttcggcc cattcggacc gcaaggaatc ggtcaataca ctacatggcg 8700
   tgatttcata tgcgcgattg ctgatcccca tgtgtatcac tggcaaactg tgatggacga 8760
   caccgtcagt gcgtccgtcg cgcaggctct cgatgagctg atgctttggg ccgaggactg 8820
   ccccgaagtc cggcacctcg tgcacgcgga tttcggctcc aacaatgtcc tgacggacaa 8880
   tggccgcata acagcggtca ttgactggag cgaggcgatg ttcggggatt cccaatacga 8940
   ggtcgccaac atcttcttct ggaggccgtg gttggcttgt atggagcagc agacgcgcta 9000
   cttcgagcgg aggcatccgg agcttgcagg atcgccgcgg ctccgggcgt atatgctccg 9060
   cattggtctt gaccaactct atcagagctt ggttgacggc aatttcgatg atgcagcttg 9120
   ggcgcagggt cgatgcgacg caatcgtccg atccggagcc gggactgtcg ggcgtacaca 9180
   aatcgcccgc agaagcgcgg ccgtctggac cgatggctgt gtagaagtac tcgccgatag 9240
   tggaaaccga cgccccagca ctcgtccgag ggcaaaggaa tagcggccgc aagtgtggat 9300
   ggggaagtga gtgcccggtt ctgtgtgcac aattggcaat ccaagatgga tggattcaac 9360
   acagggatat agcgagctac gtggtggtgc gaggatatag caacggatat ttatgtttga 9420
   cacttgagaa tgtacgatac aagcactgtc caagtacaat actaaacata ctgtacatac 9480
   tcatactcgt acccgggcaa cggtttcact tgagtgcagt ggctagtgct cttactcgta 9540
   cagtgtgcta ccgttcgtat agcatacatt atacgaagtt atcatagtct taattaaggg 9600
   agtttggccg agtggtctaa ggcgctagct tcaggtgcta gtctcgaaag aggcgtgagt 9660
   tcgaacctca cagctctcat tcaaacgatt acccaccctc gttttagagc tagaaatagc 9720
   aagttaaaat aaggctagtc cgttatcaac ttgaaaaagt ggcaccgagt cggtgctttt 9780
   tttttgtttt tttatcgatt gagcatccgt tgatttccga acagatccca atattacacc 9840
   caagtagcat gcataagcta aaagtaactc gcagcgcaca ccgtgcagat tcataagtct 9900
   atgattaatt gaacgccaat aacccggctt actacaagta caagtaggta tacatagcgg 9960
   taatgaatca ttagaaaaaa aaaaaacaaa aaaaaacaaa acaaactgtt gtggatgcat 10020
   caacagtagt acatagttgt acgatgtact tgtacttgta aaagcaaaaa tgtacaatat 10080
   ctcaggagcg gcaactttta cgttcgaaga acaatgtacc gcataccgca ttctagattc 10140
   tgcggaacgt ctaacctgga aatacgattt tttttttctt tcattttttt tgcttcttca 10200
   aaagtatggt aatttcctac cattacagtt gacactgaac gaggggggat tgaatttaag 10260
   caaaaaatta aatcaaaata cctttatgta tccagcccat gtaataaaca aaaggattat 10320
   ataacaagaa ataaatatat acctttaatg gatcattaga ataaaaataa atacgagaag 10380
   cacaccagag aagctttttg attgccacta taccgctact ttggtatatc ttattataat 10440
   tgttgaattt gcaagataga atgtcattca ttggagagaa atccaaggaa tatgtgggat 10500
   gaaatgacta gaagtatgaa caatgagaat agtacatact tgtacctgta tttctagaag 10560
   agagaaagac agttgagtgt gtgattctcg tccaataata atctcaatag taacgtgtga 10620
   atagctgttc tttgatagtt gatatttctc gatgactatt tatgttgtac aagggatttt 10680
   tttcgttgct gttgatttcg aattaggcaa tgcagatatc atttatgcta tccatattta 10740
   agatttccca tacgcattta taacatttat tctacataaa ttgttaaatg aacgaactgc 10800
   cattataaat tgtttcctaa ataggaagtg tttttcataa agcaagtaag ttgtctaata 10860
   atactaagta ataaaaataa gttcatacaa tatattttga gaacatcatt tggaggcggt 10920
   agatggagtc tgtttattat taaacaatgc gagatgaccc cttaaatatt gagaacatca 10980
   gttggaggcg gcagatggag tctgtctatt tagcaatggg acatgactgt cagtatcatc 11040
   atgatgtata tatataatac atataatatt atataacacg atttttttaa attattggcc 11100
   cgaaaattaa tcagtgtaga ctggatctcg gcagtctctc ggatgtagaa ttaggtttcc 11160
   ttgaggcgaa gatcggtttg tgtgacatga attcgatatc aagcttatcg acaccatcga 11220
   cctcgagggg gggcccggta cccaattcgc cctatagtga gtcgtattac aattcactgg 11280
   ccgtcgtttt acaacgtcgt gactgggaaa accctggcgt tacccaactt aatcgccttg 11340
   cagc 11344

<210> 114
<211> 220
<212> DNA
<213> Artificial Sequence

<220>
<223> Artificial Sequence

<400> 114
ttaattaagg tcccttggcc cagttggtta aggcgtcgtg ctaataagaa atagcaactt 60

tgcaacgcga agatcagcag ttcgatcctg ctaggaccca ttcaaacgat tacccaccct 120

cgttttagag ctagaaatag caagttaaaa taaggctagt ccgttatcaa cttgaaaaag 180

tggcaccgag tcggtgcttt tttttttgtt ttttatcgat 220

<210> 115
<211> 92
<212> DNA
<213> Artificial Sequence

<220>
<223>  Artificial Sequence

<400>  115
ggtcccttgg cccagttggt taaggcgtcg tgctaataag aaatagcaac tttgcaacgc      60

gaagatcagc agttcgatcc tgctagggac ca                                    92


<210>  116
<211>  11354
<212>  DNA
<213>  Artificial Sequence

<220>
<223>  Artificial Sequence

<400>  116
acatccccct ttcgccagct ggcgtaatag cgaagaggcc cgcaccgatc gcccttccca      60

acagttgcgc agcctgaatg gcgaatggaa attgtaagcg ttaatatttt gttaaaattc     120

gcgttaaatt tttgttaaat cagctcattt tttaaccaat aggccgaaat cggcaaaatc     180

ccttataaat caaaagaata gaccgagata gggttgagtg ttgttccagt ttggaacaag     240

agtccactat taaagaacgt ggactccaac gtcaaagggc gaaaaaccgt ctatcagggc     300

gatggcccac tacgtgaacc atcaccctaa tcaagttttt tggggtcgag gtgccgtaaa     360

gcactaaatc ggaaccctaa agggagcccc cgatttagag cttgacgggg aaagccggcg     420

aacgtggcga gaaaggaagg gaagaaagcg aaaggagcgg gcgctagggc gctggcaagt     480

gtagcggtca cgctgcgcgt aaccaccaca cccgccgcgc ttaatgcgcc gctacagggc     540

gcgtcaggtg gcacttttcg gggaaatgtg cgcggaaccc ctatttgttt atttttctaa     600

atacattcaa atatgtatcc gctcatgaga caataaccct gataaatgct tcaataatat     660

tgaaaaagga agagtatgag tattcaacat ttccgtgtcg cccttattcc cttttttgcg     720

gcattttgcc ttcctgtttt tgctcaccca gaaacgctgg tgaaagtaaa agatgctgaa     780

gatcagttgg gtgcacgagt gggttacatc gaactggatc tcaacagcgg taagatcctt     840

gagagttttc gccccgaaga acgttttcca atgatgagca cttttaaagt tctgctatgt     900

ggcgcggtat tatcccgtat tgacgccggg caagagcaac tcggtcgccg catacactat     960

tctcagaatg acttggttga gtactcacca gtcacagaaa agcatcttac ggatggcatg    1020

acagtaagag aattatgcag tgctgccata accatgagtg ataacactgc ggccaactta    1080

cttctgacaa cgatcggagg accgaaggag ctaaccgctt ttttgcacaa catgggggat    1140

catgtaactc gccttgatcg ttgggaaccg gagctgaatg aagccatacc aaacgacgag    1200

cgtgacacca cgatgcctgt agcaatggca acaacgttgc gcaaactatt aactggcgaa    1260

ctacttactc tagcttcccg gcaacaatta atagactgga tggaggcgga taaagttgca    1320

ggaccacttc tgcgctcggc ccttccggct ggctggttta ttgctgataa atctggagcc 1380

ggtgagcgtg ggtctcgcgg tatcattgca gcactggggc cagatggtaa gccctcccgt 1440

atcgtagtta tctacacgac ggggagtcag gcaactatgg atgaacgaaa tagacagatc 1500

gctgagatag gtgcctcact gattaagcat tggtaactgt cagaccaagt ttactcatat 1560

atactttaga ttgatttaaa acttcatttt taatttaaaa ggatctaggt gaagatcctt 1620

tttgataatc tcatgaccaa aatcccttaa cgtgagtttt cgttccactg agcgtcagac 1680

cccgtagaaa agatcaaagg atcttcttga gatccttttt ttctgcgcgt aatctgctgc 1740

ttgcaaacaa aaaaaccacc gctaccagcg gtggtttgtt tgccggatca agagctacca 1800

actctttttc cgaaggtaac tggcttcagc agagcgcaga taccaaatac tgtccttcta 1860

gtgtagccgt agttaggcca ccacttcaag aactctgtag caccgcctac atacctcgct 1920

ctgctaatcc tgttaccagt ggctgctgcc agtggcgata agtcgtgtct taccgggttg 1980

gactcaagac gatagttacc ggataaggcg cagcggtcgg gctgaacggg gggttcgtgc 2040

acacagccca gcttggagcg aacgacctac accgaactga gatacctaca gcgtgagcta 2100

tgagaaagcg ccacgcttcc cgaagggaga aaggcggaca ggtatccggt aagcggcagg 2160

gtcggaacag gagagcgcac gagggagctt ccagggggaa acgcctggta tctttatagt 2220

cctgtcgggt ttcgccacct ctgacttgag cgtcgatttt tgtgatgctc gtcagggggg 2280

cggagcctat ggaaaaacgc cagcaacgcg gcctttttac ggttcctggc cttttgctgg 2340

ccttttgctc acatgttctt tcctgcgtta tcccctgatt ctgtggataa ccgtattacc 2400

gcctttgagt gagctgatac cgctcgccgc agccgaacga ccgagcgcag cgagtcagtg 2460

agcgaggaag cggaagagcg cccaatacgc aaaccgcctc tccccgcgcg ttggccgatt 2520

cattaatgca gctggcacga caggtttccc gactggaaag cgggcagtga gcgcaacgca 2580

attaatgtga gttagctcac tcattaggca ccccaggctt tacactttat gcttccggct 2640

cgtacgcaac taacatgaat gaatacgata tacatcaaag actatgatac gcagtattgc 2700

acactgtacg agtaagagca ctagccactg cactcaagtg aaaccgttgc ccgggtacga 2760

gtatgagtat gtacagtatg tttagtattg tacttggaca gtgcttgtat cgtacattct 2820

caagtgtcaa acataaatat ccgttgctat atcctcgcac caccacgtag ctcgctatat 2880

ccctgtgttg aatccatcca tcttggattg ccaattgtgc acacagaacc gggcactcac 2940

ttccccatcc acacttgcgg ccgcttagac ctttcgcttt ttcttgggat cggctctgga 3000

gtcgccacca agctgagaca ggtcgattcg ggtctcgtac aggccagtga tggactggtg 3060

aatcagggtg gcatcgagaa cctccttggt ggatgtgtac cgctttcggt cgatagtggt 3120

atcgaagtac ttgaaagctg caggagcacc caggttggta agagtaaaca ggtgaatgat 3180

gttctccgcc tgttctcgaa tgggtttgtc ccgatgcttg ttgtaggcag agagcacctt 3240

gtccaagttg gcatcagcca ggatgactcg cttcgaaaac tcggaaatct gctcgataat 3300

ctcgtcgagg taatgtttgt gctgctcaac gaagagttgc ttctgttcgt tgtcctcggg 3360

agaacccttg agcttctcgt agtgagaagc cagatagaga aagttgacgt acttcgaagg 3420

caaggcaagc tcgtttccct tctgcagctc gccagcggag gcgagcatac gctttcgacc 3480

gttctccagt tcgaacagag agtacttggg cagcttgata atgaggtctt tcttgacctc 3540

cttgtaaccc ttggcttcca agaagtcgat gggattcttc tcgaagctcg atcgctccat 3600

gatggtaatt ccgagcagct ccttgacgga cttgagcttt ttggacttgc ccttctcgac 3660

cttcgcaacg acaagcacgg aataggcgac ggtaggagaa tcgaagccac cgtatttctt 3720

gggatcccag tctttctttc gagcgatgag cttgtcggag tttcgcttgg gcagaatcga 3780

ctccttggag aatccgccag tctgaacctc ggttttcttg acgatgttga cctgaggcat 3840

cgacagaacc tttcgcacgg ttgcaaagtc tcgacccttg tcccacacga tctctccagt 3900

ttcgccgttg gtctcgataa gtggtctctt tcgaatctct ccgttggcca aggtgatctc 3960

ggtcttgaaa aagttcatga tgttggagta aaagaagtac ttggcagtag ccttgccaat 4020

ctcctgttcg gacttggcaa tcatctttcg aacgtcgtag accttgtaat cgccgtaaac 4080

gaactcgctt tcgagcttgg ggtatttctt gatgagcgca gtgccaacga cggcgttgag 4140

gtaagcatcg tgggcatggt ggtaattgtt gatctctcgc accttgtaga actgaaagtc 4200

ctttcggaaa tcggagacca gtttggactt gagagtaatc accttgacct ctcggatgag 4260

cttgtcgttc tcgtcgtact tggtgttcat ccgagaatcg agaatctgtg cgacgtgctt 4320

tgtgatctgt ctggtctcga cgagttgacg cttgatgaag ccagccttgt cgagctcgga 4380

cagaccgcct cgctcggcct tggtaagatt gtcgaacttt cgctgggtaa tgagcttggc 4440

gttgagcagc tgtcgccagt agttcttcat ctttttgacc acctcttcgc tgggaacgtt 4500

gtccgacttg cctctgttct tgtcggatcg tgtaaggacc ttgttgtcga tagaatcgtc 4560

cttgagaaag gattgaggga caatgtggtc cacatcgtag tcgctgagac gattgatgtc 4620

cagttcctga tccacgtaca tgtctcgacc attctgcaga tagtagagat acagcttctc 4680

gttctgcagt tgagtgttct cgacgggatg ctccttgaga atctgggatc ccagctcctt 4740

gatgccttcc tcgattcgct tcatccgctc tcgcgagttt ttctgaccct tttgagttgt 4800

ctggttctct ctggccatct cgatcacaat gttctcgggc ttgtgacgtc ccatgacctt 4860

caccagctcg tcgacaacct tgacagtctg gagaatgcct ttcttgatgg ctggcgaacc 4920

agccaggttg gcaatatgtt cgtgcaagct gtcgccctga ccggacactt gtgccttctg 4980

gatgtcctcc ttgaaggtaa gagaatcgtc gtgaatgagc tgcatgaagt ttcggttggc 5040

aaagccatcg gacttgagaa agtccagaat ggtctttccg gactgcttgt ctctgatgcc 5100

gttgatgagc tttcgcgaaa gtcttcccca gccggtgtat ctacgtcgct tgagttgttt 5160

catgaccttg tcgtcgaaca ggtgagcgta tgtcttgagt cgttcctcga tcatctcccg 5220

atcttcgaac agggtaagag tgagcacgat gtcctccaga atgtcctcgt tttcctcgtt 5280

gtcgagaaaa tccttgtcct tgataatctt gagcagatcg tgataggtgc ccaaagaggc 5340

gttgaatcgg tcctcaactc cggaaatctc gacgctgtcg aaacactcga ttttcttgaa 5400

gtagtcctcc ttgagctgct taacagtgac ctttcggttg gtcttgaaca ggagatcgac 5460

aatggctttc ttctgttcgc cagacaagaa ggcaggcttt cgcattccct cggtaacgta 5520

cttgactttg gtgagttcgt tgtagactgt aaagtactcg tagagcagcg aatgcttggg 5580

aagaaccttc tcgttgggca gattcttgtc gaagttggtc attcgctcga tgaaggactg 5640

tgcagaggca cccttgtcca cgacttcctc gaagttccag ggagtgatgg tttcctcgga 5700

ctttcgagtc atccaagcaa atcgagagtt tcctctggca agaggaccaa catagtaggg 5760

gattcgaaag gtaagaatct tctcgatctt ctctcggttg tccttgagaa aggggtagaa 5820

gtcttcctga cgtcgaagaa tggcgtgcag ctcaccgagg tggatctgat gaggaatgct 5880

gccgttgtcg aaggttcgtt gcttccgaag cagatcctct cgattgagct tgacaagcag 5940

ttcctcggtt ccgtccatct tctcgagaat tggcttgatg aacttgtaga actcttcctg 6000

agaggctccg ccgtcgatgt atccagcgta gccgttcttc gactgatcga aaaagatctc 6060

cttgtacttc tcgggcagtt gctgtcggac aagagccttg agcagtgtga gatcctgatg 6120

gtgctcgtcg tatcgcttga tcatggaggc agaaagggga gcctttgtga tctcggtgtt 6180

gactcgcaga atgtcagaca agagaatagc atccgaaagg ttcttggcag cgagaaacag 6240

gtcggcgtac tgatcgccaa tctgtgcaag caggttgtcg aggtcatcgt cgtaggtgtc 6300

cttggacagc tggagcttgg cgtcctccgc cagatcgaag ttggacttga agttgggtgt 6360

gagaccaaga gaaagggcaa tgaggttgcc aaacagtccg ttctttttct cgccaggaag 6420

ttgggcaatg aggttctcca gtcgtctgct cttcgagagt cgagcagaca agatggcctt 6480

tgcatcgact ccggaggcat tgatggggtt ttcctcgaac agctggttgt aggtctgaac 6540

gagctgaatg aacagcttgt ccacatcgct gttgtcggga ttgagatcgc cctcgatgag 6600

gaaatgacct cgaaacttga tcatgtgtgc cagagcgagg tagataagtc tgagatccgc 6660

cttgtcggtg gaatcgacga gtttctttcg gagatggtag atggtaggat acttctcgtg 6720

gtaagcaacc tcgtccacaa tgttgccaaa gatgggatga cgctcgtgtt tcttgtcttc 6780

ctcgacgagg aaggattcct ccagtcgatg aaagaacgaa tcgtccacct tggccatctc 6840

gttggaaaag atctcctgca ggtagcagat tcggttcttc cgtcgggtgt aacgtcgccg 6900

agcagttcgc ttgagtctgg tagcttcggc agtctcgcca gaatcgaaca acagggcacc 6960

aatgaggttt ttcttgatgg agtgtcgatc ggtgtttccg aggaccttga atttcttgga 7020

gggcaccttg tactcgtcgg tgatgacagc ccagccgaca gagttggttc caatgtccag 7080

gccgatggag tatttcttgt ccatggtgtg atgtgtagtt tagatttcga atctgtgggg 7140

aaagaaagga aaaaagagac tggcaaccga ttgggagagc cactgtttat atatacccta 7200

gacaagcccc ccgcttgtaa gatgttggtc aatgtaaacc agtattaagg ttggcaagtg 7260

caggagaagc aaggtgtggg taccgagcaa tggaaatgtg cggaaggcaa aaaaatgagg 7320

ccacggccta ttgtcggggc tatatccagg gggcgattga agtacactaa catgacatgt 7380

gtccacagac cctcaatctg gcctgatgag ccaaatccat acgcgctttc gcagctctaa 7440

aggctataac aagtcacacc accctgctcg acctcagcgc cctcactttt tgttaagaca 7500

aactgtacac gctgttccag cgttttctgc ctgcacctgg tgggacattt ggtgcaacct 7560

aaagtgctcg gaacctctgt ggtgtccaga tcagcgcagc agttccgagg tagttttgag 7620

gcccttagat gatggtttaa accttaagcc cgctcataac ttcgtatagc atacattata 7680

cgaacggtag gttgcgggat agacgccgac ggagggcaat ggcgctatgg aaccttgcgg 7740

atatccatac gccgcggcgg actgcgtccg aaccagctcc agcagcgttt tttccgggcc 7800

attgagccga ctgcgacccc gccaacgtgt cttggcccac gcactcatgt catgttggtg 7860

ttgggaggcc actttttaag tagcacaagg cacctagctc gcagcaaggt gtccgaacca 7920

aagaagcggc tgcagtggtg caaacggggc ggaaacggcg ggaaaaagcc acgggggcac 7980

gaattgaggc acgccctcga atttgagacg agtcacggcc ccattcgccc gcgcaatggc 8040

tcgccaacgc ccggtctttt gcaccacatc aggttacccc aagccaaacc tttgtgttaa 8100

aaagcttaac atattatacc gaacgtaggt ttgggcgggc ttgctccgtc tgtccaaggc 8160

aacatttata taagggtctg catcgccggc tcaattgaat cttttttctt cttctcttct 8220

ctatattcat tcttgaatta aacacacatc aaccatggcc aaaaagcctg aactcaccgc 8280

gacgtctgtc gagaagtttc tgatcgaaaa gttcgacagc gtctccgacc tgatgcagct 8340

ctcggagggc gaagaatctc gtgctttcag cttcgatgta ggagggcgtg gatatgtcct 8400

gcgggtaaat agctgcgccg atggtttcta caaagatcgt tatgtttatc ggcactttgc 8460

atcggccgcg ctcccgattc cggaagtgct tgacattggg gagttcagcg agagcctgac 8520

ctattgcatc tcccgccgtg cacagggtgt cacgttgcaa gacctgcctg aaaccgaact 8580

gcccgctgtt ctgcagccgg tcgcggaggc tatggatgcg atcgctgcgg ccgatcttag 8640

ccagacgagc gggttcggcc cattcggacc gcaaggaatc ggtcaataca ctacatggcg 8700

tgatttcata tgcgcgattg ctgatcccca tgtgtatcac tggcaaactg tgatggacga 8760

caccgtcagt gcgtccgtcg cgcaggctct cgatgagctg atgctttggg ccgaggactg 8820

ccccgaagtc cggcacctcg tgcacgcgga tttcggctcc aacaatgtcc tgacggacaa 8880

tggccgcata acagcggtca ttgactggag cgaggcgatg ttcggggatt cccaatacga 8940

ggtcgccaac atcttcttct ggaggccgtg gttggcttgt atggagcagc agacgcgcta 9000

cttcgagcgg aggcatccgg agcttgcagg atcgccgcgg ctccgggcgt atatgctccg 9060

cattggtctt gaccaactct atcagagctt ggttgacggc aatttcgatg atgcagcttg 9120

ggcgcagggt cgatgcgacg caatcgtccg atccggagcc gggactgtcg ggcgtacaca 9180

aatcgcccgc agaagcgcgg ccgtctggac cgatggctgt gtagaagtac tcgccgatag 9240

tggaaaccga cgccccagca ctcgtccgag ggcaaaggaa tagcggccgc aagtgtggat 9300

ggggaagtga gtgcccggtt ctgtgtgcac aattggcaat ccaagatgga tggattcaac 9360

acagggatat agcgagctac gtggtggtgc gaggatatag caacggatat ttatgtttga 9420

cacttgagaa tgtacgatac aagcactgtc caagtacaat actaaacata ctgtacatac 9480

tcatactcgt acccgggcaa cggtttcact tgagtgcagt ggctagtgct cttactcgta 9540

cagtgtgcta ccgttcgtat agcatacatt atacgaagtt atcatagtct taattaaggt 9600

cccttggccc agttggttaa ggcgtcgtgc taataagaaa tagcaacttt gcaacgcgaa 9660

gatcagcagt tcgatcctgc tagggaccat tcaaacgatt acccaccctc gttttagagc 9720

tagaaatagc aagttaaaat aaggctagtc cgttatcaac ttgaaaaagt ggcaccgagt 9780

cggtgctttt tttttgtttt tttatcgatt gagcatccgt tgatttccga acagatccca 9840

atattacacc caagtagcat gcataagcta aaagtaactc gcagcgcaca ccgtgcagat 9900

tcataagtct atgattaatt gaacgccaat aacccggctt actacaagta caagtaggta 9960

tacatagcgg taatgaatca ttagaaaaaa aaaaaacaaa aaaaaacaaa acaaactgtt 10020

gtggatgcat caacagtagt acatagttgt acgatgtact tgtacttgta aaagcaaaaa 10080

tgtacaatat ctcagggagc gcaactttta cgttcgaaga acaatgtacc gcataccgca 10140

ttctagattc tgcggaacgt ctaacctgga aatacgattt tttttttctt tcattttttt 10200

tgcttcttca aaagtatggt aatttcctac cattacagtt gacactgaac gaggggggat 10260

tgaatttaag caaaaaatta aatcaaaata cctttatgta tccagcccat gtaataaaca 10320

aaaggattat ataacaagaa ataaatatat acctttaatg gatcattaga ataaaaataa 10380

atacgagaag cacaccagag aagctttttg attgccacta taccgctact ttggtatatc 10440

ttattataat tgttgaattt gcaagataga atgtcattca ttggagagaa atccaaggaa 10500

tatgtgggat gaaatgacta gaagtatgaa caatgagaat agtacatact tgtacctgta 10560

tttctagaag agagaaagac agttgagtgt gtgattctcg tccaataata atctcaatag 10620

taacgtgtga atagctgttc tttgatagtt gatatttctc gatgactatt tatgttgtac 10680

aagggatttt tttcgttgct gttgatttcg aattaggcaa tgcagatatc atttatgcta 10740

tccatattta agatttccca tacgcattta taacatttat tctacataaa ttgttaaatg 10800

aacgaactgc cattataaat tgtttcctaa ataggaagtg tttttcataa agcaagtaag 10860

ttgtctaata atactaagta ataaaaataa gttcatacaa tatattttga gaacatcatt 10920

tggaggcggt agatggagtc tgtttattat taaacaatgc gagatgaccc cttaaatatt 10980

gagaacatca gttggaggcg gcagatggag tctgtctatt tagcaatggg acatgactgt 11040

cagtatcatc atgatgtata tatataatac atataatatt atataacacg atttttttaa 11100

attattggcc cgaaaattaa tcagtgtaga ctggatctcg gcagtctctc ggatgtagaa 11160

ttaggtttcc ttgaggcgaa gatcggtttg tgtgacatga attcgatatc aagcttatcg 11220

acaccatcga cctcgagggg gggcccggta cccaattcgc cctatagtga gtcgtattac 11280

aattcactgg ccgtcgtttt acaacgtcgt gactgggaaa accctggcgt tacccaactt 11340

aatcgccttg cagc 11354

<210> 117
<211> 203
<212> DNA
<213> Artificial Sequence

<220>
<223> Artificial Sequence

<400> 117
ttaattaagg gtttcttggt ctagttggtc atggcatccg ctttacacgc ggaacgtcgg 60

cagttcgatc ctgtcagaaa tcattcaaac gattacccac cctcgtttta gagctagaaa 120

tagcaagtta aaataaggct agtccgttat caacttgaaa aagtggcacc gagtcggtgc 180

tttttttttt gtttttatc gat 203

<210> 118
<211> 75
<212> DNA
<213> Artificial Sequence

<220>
<223> Artificial Sequence

<400> 118
gggtttcttg gtctagttgg tcatggcatc cgctttacac gcggaacgtc ggcagttcga 60

tcctgtcaga aatca 75

<210> 119
<211> 11337
<212> DNA
<213> Artificial Sequence

<220>
<223> Artificial Sequence

<400> 119

acatccccct ttcgccagct ggcgtaatag cgaagaggcc cgcaccgatc gcccttccca 60

acagttgcgc agcctgaatg gcgaatggaa attgtaagcg ttaatatttt gttaaaattc 120

gcgttaaatt tttgttaaat cagctcattt tttaaccaat aggccgaaat cggcaaaatc 180

ccttataaat caaaagaata gaccgagata gggttgagtg ttgttccagt ttggaacaag 240

agtccactat taaagaacgt ggactccaac gtcaaagggc gaaaaaccgt ctatcagggc 300

gatggcccac tacgtgaacc atcaccctaa tcaagttttt tggggtcgag gtgccgtaaa 360

gcactaaatc ggaaccctaa agggagcccc cgatttagag cttgacgggg aaagccggcg 420

aacgtggcga gaaaggaagg gaagaaagcg aaaggagcgg gcgctagggc gctggcaagt 480

gtagcggtca cgctgcgcgt aaccaccaca cccgccgcgc ttaatgcgcc gctacagggc 540

gcgtcaggtg gcacttttcg gggaaatgtg cgcggaaccc ctatttgttt atttttctaa 600

atacattcaa atatgtatcc gctcatgaga caataaccct gataaatgct tcaataatat 660

tgaaaaagga agagtatgag tattcaacat ttccgtgtcg cccttattcc cttttttgcg 720

gcattttgcc ttcctgtttt tgctcaccca gaaacgctgg tgaaagtaaa agatgctgaa 780

gatcagttgg gtgcacgagt gggttacatc gaactggatc tcaacagcgg taagatcctt 840

gagagttttc gccccgaaga acgttttcca atgatgagca cttttaaagt tctgctatgt 900

ggcgcggtat tatcccgtat tgacgccggg caagagcaac tcggtcgccg catacactat 960

tctcagaatg acttggttga gtactcacca gtcacagaaa agcatcttac ggatggcatg 1020

acagtaagag aattatgcag tgctgccata accatgagtg ataacactgc ggccaactta 1080

cttctgacaa cgatcggagg accgaaggag ctaaccgctt ttttgcacaa catgggggat 1140

catgtaactc gccttgatcg ttgggaaccg gagctgaatg aagccatacc aaacgacgag 1200

cgtgacacca cgatgcctgt agcaatggca acaacgttgc gcaaactatt aactggcgaa 1260

ctacttactc tagcttcccg gcaacaatta atagactgga tggaggcgga taaagttgca 1320

ggaccacttc tgcgctcggc ccttccggct ggctggttta ttgctgataa atctggagcc 1380

ggtgagcgtg ggtctcgcgg tatcattgca gcactggggc cagatggtaa gccctcccgt 1440

atcgtagtta tctacacgac ggggagtcag gcaactatgg atgaacgaaa tagacagatc 1500

gctgagatag gtgcctcact gattaagcat tggtaactgt cagaccaagt ttactcatat 1560

atactttaga ttgatttaaa acttcatttt taatttaaaa ggatctaggt gaagatcctt 1620

tttgataatc tcatgaccaa aatcccttaa cgtgagtttt cgttccactg agcgtcagac 1680

cccgtagaaa agatcaaagg atcttcttga gatccttttt ttctgcgcgt aatctgctgc 1740

ttgcaaacaa aaaaaccacc gctaccagcg gtggtttgtt tgccggatca agagctacca 1800

actctttttc cgaaggtaac tggcttcagc agagcgcaga taccaaatac tgtccttcta 1860

gtgtagccgt agttaggcca ccacttcaag aactctgtag caccgcctac atacctcgct 1920

ctgctaatcc tgttaccagt ggctgctgcc agtggcgata agtcgtgtct taccgggttg 1980

gactcaagac gatagttacc ggataaggcg cagcggtcgg gctgaacggg gggttcgtgc 2040

acacagccca gcttggagcg aacgacctac accgaactga gatacctaca gcgtgagcta 2100

tgagaaagcg ccacgcttcc cgaagggaga aaggcggaca ggtatccggt aagcggcagg 2160

gtcggaacag gagagcgcac gagggagctt ccagggggaa acgcctggta tctttatagt 2220

cctgtcgggt ttcgccacct ctgacttgag cgtcgatttt tgtgatgctc gtcagggggg 2280

cggagcctat ggaaaaacgc cagcaacgcg gcctttttac ggttcctggc cttttgctgg 2340

ccttttgctc acatgttctt tcctgcgtta tcccctgatt ctgtggataa ccgtattacc 2400

gcctttgagt gagctgatac cgctcgccgc agccgaacga ccgagcgcag cgagtcagtg 2460

agcgaggaag cggaagagcg cccaatacgc aaaccgcctc tccccgcgcg ttggccgatt 2520

cattaatgca gctggcacga caggtttccc gactggaaag cgggcagtga gcgcaacgca 2580

attaatgtga gttagctcac tcattaggca ccccaggctt tacactttat gcttccggct 2640

cgtacgcaac taacatgaat gaatacgata tacatcaaag actatgatac gcagtattgc 2700

acactgtacg agtaagagca ctagccactg cactcaagtg aaaccgttgc ccgggtacga 2760

gtatgagtat gtacagtatg tttagtattg tacttggaca gtgcttgtat cgtacattct 2820

caagtgtcaa acataaatat ccgttgctat atcctcgcac caccacgtag ctcgctatat 2880

ccctgtgttg aatccatcca tcttggattg ccaattgtgc acacagaacc gggcactcac 2940

ttccccatcc acacttgcgg ccgcttagac ctttcgcttt ttcttgggat cggctctgga 3000

gtcgccacca agctgagaca ggtcgattcg ggtctcgtac aggccagtga tggactggtg 3060

aatcagggtg gcatcgagaa cctccttggt ggatgtgtac cgctttcggt cgatagtggt 3120

atcgaagtac ttgaaagctg caggagcacc caggttggta agagtaaaca ggtgaatgat 3180

gttctccgcc tgttctcgaa tgggtttgtc ccgatgcttg ttgtaggcag agagcacctt 3240

gtccaagttg gcatcagcca ggatgactcg cttcgaaaac tcggaaatct gctcgataat 3300

ctcgtcgagg taatgtttgt gctgctcaac gaagagttgc ttctgttcgt tgtcctcggg 3360

agaacccttg agcttctcgt agtgagaagc cagatagaga aagttgacgt acttcgaagg 3420

caaggcaagc tcgtttccct tctgcagctc gccagcggag gcgagcatac gctttcgacc 3480

gttctccagt tcgaacagag agtacttggg cagcttgata atgaggtctt tcttgacctc 3540

cttgtaaccc ttggcttcca agaagtcgat gggattcttc tcgaagctcg atcgctccat 3600

gatggtaatt ccgagcagct ccttgacgga cttgagcttt ttggacttgc ccttctcgac 3660

cttcgcaacg acaagcacgg aataggcgac ggtaggagaa tcgaagccac cgtatttctt 3720

gggatcccag tctttctttc gagcgatgag cttgtcggag tttcgcttgg gcagaatcga 3780

ctccttggag aatccgccag tctgaacctc ggttttcttg acgatgttga cctgaggcat 3840

cgacagaacc tttcgcacgg ttgcaaagtc tcgacccttg tcccacacga tctctccagt 3900

ttcgccgttg gtctcgataa gtggtctctt tcgaatctct ccgttggcca aggtgatctc 3960

ggtcttgaaa aagttcatga tgttggagta aaagaagtac ttggcagtag ccttgccaat 4020

ctcctgttcg gacttggcaa tcatctttcg aacgtcgtag accttgtaat cgccgtaaac 4080

gaactcgctt tcgagcttgg ggtatttctt gatgagcgca gtgccaacga cggcgttgag 4140

gtaagcatcg tgggcatggt ggtaattgtt gatctctcgc accttgtaga actgaaagtc 4200

ctttcggaaa tcggagacca gtttggactt gagagtaatc accttgacct ctcggatgag 4260

cttgtcgttc tcgtcgtact tggtgttcat ccgagaatcg agaatctgtg cgacgtgctt 4320

tgtgatctgt ctggtctcga cgagttgacg cttgatgaag ccagccttgt cgagctcgga 4380

cagaccgcct cgctcggcct tggtaagatt gtcgaacttt cgctgggtaa tgagcttggc 4440

gttgagcagc tgtcgccagt agttcttcat ctttttgacc acctcttcgc tgggaacgtt 4500

gtccgacttg cctctgttct tgtcggatcg tgtaaggacc ttgttgtcga tagaatcgtc 4560

cttgagaaag gattgaggga caatgtggtc cacatcgtag tcgctgagac gattgatgtc 4620

cagttcctga tccacgtaca tgtctcgacc attctgcaga tagtagagat acagcttctc 4680

gttctgcagt tgagtgttct cgacgggatg ctccttgaga atctgggatc ccagctcctt 4740

gatgccttcc tcgattcgct tcatccgctc tcgcgagttt ttctgaccct tttgagttgt 4800

ctggttctct ctggccatct cgatcacaat gttctcgggc ttgtgacgtc ccatgacctt 4860

caccagctcg tcgacaacct tgacagtctg gagaatgcct ttcttgatgg ctggcgaacc 4920

agccaggttg gcaatatgtt cgtgcaagct gtcgccctga ccggacactt gtgccttctg 4980

gatgtcctcc ttgaaggtaa gagaatcgtc gtgaatgagc tgcatgaagt ttcggttggc 5040

aaagccatcg gacttgagaa agtccagaat ggtctttccg gactgcttgt ctctgatgcc 5100

gttgatgagc tttcgcgaaa gtcttcccca gccggtgtat ctacgtcgct tgagttgttt 5160

catgaccttg tcgtcgaaca ggtgagcgta tgtcttgagt cgttcctcga tcatctcccg 5220

atcttcgaac agggtaagag tgagcacgat gtcctccaga atgtcctcgt tttcctcgtt 5280

gtcgagaaaa tccttgtcct tgataatctt gagcagatcg tgataggtgc ccaaagaggc 5340

gttgaatcgg tcctcaactc cggaaatctc gacgctgtcg aaacactcga ttttcttgaa 5400

gtagtcctcc ttgagctgct taacagtgac ctttcggttg gtcttgaaca ggagatcgac 5460

aatggctttc ttctgttcgc cagacaagaa ggcaggcttt cgcattccct cggtaacgta 5520

cttgactttg gtgagttcgt tgtagactgt aaagtactcg tagagcagcg aatgcttggg 5580

aagaaccttc tcgttgggca gattcttgtc gaagttggtc attcgctcga tgaaggactg 5640

tgcagaggca cccttgtcca cgacttcctc gaagttccag ggagtgatgg tttcctcgga 5700

ctttcgagtc atccaagcaa atcgagagtt tcctctggca agaggaccaa catagtaggg 5760

gattcgaaag gtaagaatct tctcgatctt ctctcggttg tccttgagaa aggggtagaa 5820

gtcttcctga cgtcgaagaa tggcgtgcag ctcaccgagg tggatctgat gaggaatgct 5880

gccgttgtcg aaggttcgtt gcttccgaag cagatcctct cgattgagct tgacaagcag 5940

ttcctcggtt ccgtccatct tctcgagaat tggcttgatg aacttgtaga actcttcctg 6000

agaggctccg ccgtcgatgt atccagcgta gccgttcttc gactgatcga aaaagatctc 6060

cttgtacttc tcgggcagtt gctgtcggac aagagccttg agcagtgtga gatcctgatg 6120

gtgctcgtcg tatcgcttga tcatggaggc agaaagggga gcctttgtga tctcggtgtt 6180

gactcgcaga atgtcagaca agagaatagc atccgaaagg ttcttggcag cgagaaacag 6240

gtcggcgtac tgatcgccaa tctgtgcaag caggttgtcg aggtcatcgt cgtaggtgtc 6300

cttggacagc tggagcttgg cgtcctccgc cagatcgaag ttggacttga agttgggtgt 6360

gagaccaaga gaaagggcaa tgaggttgcc aaacagtccg ttctttttct cgccaggaag 6420

ttgggcaatg aggttctcca gtcgtctgct cttcgagagt cgagcagaca agatggcctt 6480

tgcatcgact ccggaggcat tgatggggtt ttcctcgaac agctggttgt aggtctgaac 6540

gagctgaatg aacagcttgt ccacatcgct gttgtcggga ttgagatcgc cctcgatgag 6600

gaaatgacct cgaaacttga tcatgtgtgc cagagcgagg tagataagtc tgagatccgc 6660

cttgtcggtg gaatcgacga gtttctttcg gagatggtag atggtaggat acttctcgtg 6720

gtaagcaacc tcgtccacaa tgttgccaaa gatgggatga cgctcgtgtt tcttgtcttc 6780

ctcgacgagg aaggattcct ccagtcgatg aaagaacgaa tcgtccacct tggccatctc 6840

gttggaaaag atctcctgca ggtagcagat tcggttcttc cgtcgggtgt aacgtcgccg 6900

agcagttcgc ttgagtctgg tagcttcggc agtctcgcca gaatcgaaca acagggcacc 6960

aatgaggttt ttcttgatgg agtgtcgatc ggtgtttccg aggaccttga atttcttgga 7020

gggcaccttg tactcgtcgg tgatgacagc ccagccgaca gagttggttc caatgtccag 7080

gccgatggag tatttcttgt ccatggtgtg atgtgtagtt tagatttcga atctgtgggg 7140

aaagaaagga aaaaagagac tggcaaccga ttgggagagc cactgtttat atatacccta 7200

gacaagcccc ccgcttgtaa gatgttggtc aatgtaaacc agtattaagg ttggcaagtg 7260

caggagaagc aaggtgtggg taccgagcaa tggaaatgtg cggaaggcaa aaaaatgagg 7320

ccacggccta ttgtcggggc tatatccagg gggcgattga agtacactaa catgacatgt 7380

gtccacagac cctcaatctg gcctgatgag ccaaatccat acgcgctttc gcagctctaa 7440

aggctataac aagtcacacc accctgctcg acctcagcgc cctcactttt tgttaagaca 7500

aactgtacac gctgttccag cgttttctgc ctgcacctgg tgggacattt ggtgcaacct 7560

aaagtgctcg gaacctctgt ggtgtccaga tcagcgcagc agttccgagg tagttttgag 7620

gcccttagat gatggtttaa accttaagcc cgctcataac ttcgtatagc atacattata 7680

cgaacggtag gttgcgggat agacgccgac ggagggcaat ggcgctatgg aaccttgcgg 7740

atatccatac gccgcggcgg actgcgtccg aaccagctcc agcagcgttt tttccgggcc 7800

attgagccga ctgcgacccc gccaacgtgt cttggcccac gcactcatgt catgttggtg 7860

ttgggaggcc actttttaag tagcacaagg cacctagctc gcagcaaggt gtccgaacca 7920

aagaagcggc tgcagtggtg caaacggggc ggaaacggcg ggaaaaagcc acgggggcac 7980

gaattgaggc acgccctcga atttgagacg agtcacggcc ccattcgccc gcgcaatggc 8040

tcgccaacgc ccggtctttt gcaccacatc aggttacccc aagccaaacc tttgtgttaa 8100

aaagcttaac atattatacc gaacgtaggt ttgggcgggc ttgctccgtc tgtccaaggc 8160

aacatttata taagggtctg catcgccggc tcaattgaat cttttttctt cttctcttct 8220

ctatattcat tcttgaatta aacacacatc aaccatggcc aaaaagcctg aactcaccgc 8280

gacgtctgtc gagaagtttc tgatcgaaaa gttcgacagc gtctccgacc tgatgcagct 8340

ctcggagggc gaagaatctc gtgctttcag cttcgatgta ggagggcgtg gatatgtcct 8400

gcgggtaaat agctgcgccg atggtttcta caaagatcgt tatgtttatc ggcactttgc 8460

atcggccgcg ctcccgattc cggaagtgct tgacattggg gagttcagcg agagcctgac 8520

ctattgcatc tcccgccgtg cacagggtgt cacgttgcaa gacctgcctg aaaccgaact 8580

gcccgctgtt ctgcagccgg tcgcggaggc tatggatgcg atcgctgcgg ccgatcttag 8640

ccagacgagc gggttcggcc cattcggacc gcaaggaatc ggtcaataca ctacatggcg 8700

tgatttcata tgcgcgattg ctgatcccca tgtgtatcac tggcaaactg tgatggacga 8760

caccgtcagt gcgtccgtcg cgcaggctct cgatgagctg atgctttggg ccgaggactg 8820

ccccgaagtc cggcacctcg tgcacgcgga tttcggctcc aacaatgtcc tgacggacaa 8880

tggccgcata acagcggtca ttgactggag cgaggcgatg ttcggggatt cccaatacga 8940

ggtcgccaac atcttcttct ggaggccgtg gttggcttgt atggagcagc agacgcgcta 9000

cttcgagcgg aggcatccgg agcttgcagg atcgccgcgg ctccgggcgt atatgctccg 9060

cattggtctt gaccaactct atcagagctt ggttgacggc aatttcgatg atgcagcttg 9120

ggcgcagggt cgatgcgacg caatcgtccg atccggagcc gggactgtcg ggcgtacaca 9180

aatcgcccgc agaagcgcgg ccgtctggac cgatggctgt gtagaagtac tcgccgatag 9240

tggaaaccga cgccccagca ctcgtccgag ggcaaaggaa tagcggccgc aagtgtggat 9300

ggggaagtga gtgcccggtt ctgtgtgcac aattggcaat ccaagatgga tggattcaac 9360

acagggatat agcgagctac gtggtggtgc gaggatatag caacggatat ttatgtttga 9420

cacttgagaa tgtacgatac aagcactgtc caagtacaat actaaacata ctgtacatac 9480

tcatactcgt acccgggcaa cggtttcact tgagtgcagt ggctagtgct cttactcgta 9540

cagtgtgcta ccgttcgtat agcatacatt atacgaagtt atcatagtct taattaaggg 9600

tttcttggtc tagttggtca tggcatccgc tttacacgcg gaacgtcggc agttcgatcc 9660

tgtcagaaat cattcaaacg attacccacc ctcgttttag agctagaaat agcaagttaa 9720

aataaggcta gtccgttatc aacttgaaaa agtggcaccg agtcggtgct tttttttttg 9780

ttttttatcg attgagcatc cgttgatttc cgaacagatc ccaatattac acccaagtag 9840

catgcataag ctaaaagtaa ctcgcagcgc acaccgtgca gattcataag tctatgatta 9900

attgaacgcc aataacccgg cttactacaa gtacaagtag gtatacatag cggtaatgaa 9960

tcattagaaa aaaaaaaaac aaaaaaaaac aaaacaaact gttgtggatg catcaacagt 10020

agtacatagt tgtacgatgt acttgtactt gtaaaagcaa aaatgtacaa tatctcaggg 10080

agcgcaactt ttacgttcga agaacaatgt accgcatacc gcattctaga ttctgcggaa 10140

cgtctaacct ggaaatacga tttttttttt ctttcatttt ttttgcttct tcaaaagtat 10200

ggtaatttcc taccattaca gttgacactg aacgaggggg gattgaattt aagcaaaaaa 10260

ttaaatcaaa atacctttat gtatccagcc catgtaataa acaaaaggat tatataacaa 10320

gaaataaata tataccttta atggatcatt agaataaaaa taaatacgag aagcacacca 10380

gagaagcttt ttgattgcca ctataccgct actttggtat atcttattat aattgttgaa 10440

tttgcaagat agaatgtcat tcattggaga gaaatccaag gaatatgtgg gatgaaatga 10500

ctagaagtat gaacaatgag aatagtacat acttgtacct gtatttctag aagagagaaa 10560

gacagttgag tgtgtgattc tcgtccaata ataatctcaa tagtaacgtg tgaatagctg 10620

ttctttgata gttgatattt ctcgatgact atttatgttg tacaagggat ttttttcgtt 10680

gctgttgatt tcgaattagg caatgcagat atcatttatg ctatccatat ttaagatttc 10740

ccatacgcat ttataacatt tattctacat aaattgttaa atgaacgaac tgccattata 10800

aattgtttcc taaataggaa gtgtttttca taaagcaagt aagttgtcta ataatactaa 10860

gtaataaaaa taagttcata caatatattt tgagaacatc atttggaggc ggtagatgga 10920

gtctgtttat tattaaacaa tgcgagatga ccccttaaat attgagaaca tcagttggag 10980

gcggcagatg gagtctgtct atttagcaat gggacatgac tgtcagtatc atcatgatgt 11040

atatatataa tacatataat attatataac acgatttttt taaattattg gcccgaaaat 11100

taatcagtgt agactggatc tcggcagtct ctcggatgta gaattaggtt tccttgaggc 11160

gaagatcggt ttgtgtgaca tgaattcgat atcaagctta tcgacaccat cgacctcgag 11220

ggggggcccg gtacccaatt cgccctatag tgagtcgtat tacaattcac tggccgtcgt 11280

tttacaacgt cgtgactggg aaaaccctgg cgttacccaa cttaatcgcc ttgcagc 11337

<210>  120
<211>  214
<212>  DNA
<213>  Artificial Sequence

<220>
<223>  Artificial Sequence

<400>  120
ttaattaaga tgtggtggct caatggtaga gctttcgact ccagtcttct cggttgtaat      60

cgaagggttg caggttcaat tcctgtccat gtcattcaaa cgattaccca ccctcgtttt      120

agagctagaa atagcaagtt aaaataaggc tagtccgtta tcaacttgaa aaagtggcac      180

cgagtcggtg cttttttttt tgttttttat cgat                                  214


<210>  121
<211>  86
<212>  DNA
<213>  Artificial Sequence

<220>
<223>  Artificial Sequence

<400>  121
gatgtggtgg ctcaatggta gagctttcga ctccagtctt ctcggttgta atcgaagggt      60

tgcaggttca attcctgtcc atgtca                                           86


<210>  122
<211>  11348
<212>  DNA
<213>  Artificial Sequence

<220>
<223>  Artificial Sequence

<400>  122
acatccccct ttcgccagct ggcgtaatag cgaagaggcc cgcaccgatc gcccttccca      60

acagttgcgc agcctgaatg gcgaatggaa attgtaagcg ttaatatttt gttaaaattc      120

gcgttaaatt tttgttaaat cagctcattt tttaaccaat aggccgaaat cggcaaaatc      180

ccttataaat caaaagaata gaccgagata gggttgagtg ttgttccagt ttggaacaag      240

agtccactat taaagaacgt ggactccaac gtcaaagggc gaaaaaccgt ctatcagggc      300

gatggcccac tacgtgaacc atcaccctaa tcaagttttt tggggtcgag gtgccgtaaa      360

gcactaaatc ggaaccctaa agggagcccc cgatttagag cttgacgggg aaagccggcg      420

aacgtggcga gaaaggaagg gaagaaagcg aaaggagcgg gcgctagggc gctggcaagt      480

gtagcggtca cgctgcgcgt aaccaccaca cccgccgcgc ttaatgcgcc gctacagggc      540

gcgtcaggtg gcacttttcg gggaaatgtg cgcggaaccc ctatttgttt atttttctaa      600

atacattcaa atatgtatcc gctcatgaga caataaccct gataaatgct tcaataatat      660

tgaaaaagga agagtatgag tattcaacat ttccgtgtcg cccttattcc cttttttgcg 720

gcattttgcc ttcctgtttt tgctcaccca gaaacgctgg tgaaagtaaa agatgctgaa 780

gatcagttgg gtgcacgagt gggttacatc gaactggatc tcaacagcgg taagatcctt 840

gagagttttc gccccgaaga acgttttcca atgatgagca cttttaaagt tctgctatgt 900

ggcgcggtat tatcccgtat tgacgccggg caagagcaac tcggtcgccg catacactat 960

tctcagaatg acttggttga gtactcacca gtcacagaaa agcatcttac ggatggcatg 1020

acagtaagag aattatgcag tgctgccata accatgagtg ataacactgc ggccaactta 1080

cttctgacaa cgatcggagg accgaaggag ctaaccgctt ttttgcacaa catgggggat 1140

catgtaactc gccttgatcg ttgggaaccg gagctgaatg aagccatacc aaacgacgag 1200

cgtgacacca cgatgcctgt agcaatggca acaacgttgc gcaaactatt aactggcgaa 1260

ctacttactc tagcttcccg gcaacaatta atagactgga tggaggcgga taaagttgca 1320

ggaccacttc tgcgctcggc ccttccggct ggctggttta ttgctgataa atctggagcc 1380

ggtgagcgtg ggtctcgcgg tatcattgca gcactggggc cagatggtaa gccctcccgt 1440

atcgtagtta tctacacgac ggggagtcag gcaactatgg atgaacgaaa tagacagatc 1500

gctgagatag gtgcctcact gattaagcat tggtaactgt cagaccaagt ttactcatat 1560

atactttaga ttgatttaaa acttcatttt taatttaaaa ggatctaggt gaagatcctt 1620

tttgataatc tcatgaccaa aatcccttaa cgtgagtttt cgttccactg agcgtcagac 1680

cccgtagaaa agatcaaagg atcttcttga gatccttttt ttctgcgcgt aatctgctgc 1740

ttgcaaacaa aaaaaccacc gctaccagcg gtggtttgtt tgccggatca agagctacca 1800

actctttttc cgaaggtaac tggcttcagc agagcgcaga taccaaatac tgtccttcta 1860

gtgtagccgt agttaggcca ccacttcaag aactctgtag caccgcctac atacctcgct 1920

ctgctaatcc tgttaccagt ggctgctgcc agtggcgata agtcgtgtct taccgggttg 1980

gactcaagac gatagttacc ggataaggcg cagcggtcgg gctgaacggg gggttcgtgc 2040

acacagccca gcttggagcg aacgacctac accgaactga gatacctaca gcgtgagcta 2100

tgagaaagcg ccacgcttcc cgaagggaga aaggcggaca ggtatccggt aagcggcagg 2160

gtcggaacag gagagcgcac gagggagctt ccagggggaa acgcctggta tctttatagt 2220

cctgtcgggt ttcgccacct ctgacttgag cgtcgatttt tgtgatgctc gtcagggggg 2280

cggagcctat ggaaaaacgc cagcaacgcg gcctttttac ggttcctggc cttttgctgg 2340

ccttttgctc acatgttctt tcctgcgtta tcccctgatt ctgtggataa ccgtattacc 2400

gcctttgagt gagctgatac cgctcgccgc agccgaacga ccgagcgcag cgagtcagtg 2460

agcgaggaag cggaagagcg cccaatacgc aaaccgcctc tccccgcgcg ttggccgatt 2520

cattaatgca gctggcacga caggtttccc gactggaaag cgggcagtga gcgcaacgca 2580

attaatgtga gttagctcac tcattaggca ccccaggctt tacactttat gcttccggct 2640

cgtacgcaac taacatgaat gaatacgata tacatcaaag actatgatac gcagtattgc 2700

acactgtacg agtaagagca ctagccactg cactcaagtg aaaccgttgc ccgggtacga 2760

gtatgagtat gtacagtatg tttagtattg tacttggaca gtgcttgtat cgtacattct 2820

caagtgtcaa acataaatat ccgttgctat atcctcgcac caccacgtag ctcgctatat 2880

ccctgtgttg aatccatcca tcttggattg ccaattgtgc acacagaacc gggcactcac 2940

ttccccatcc acacttgcgg ccgcttagac ctttcgcttt ttcttgggat cggctctgga 3000

gtcgccacca agctgagaca ggtcgattcg ggtctcgtac aggccagtga tggactggtg 3060

aatcagggtg gcatcgagaa cctccttggt ggatgtgtac cgctttcggt cgatagtggt 3120

atcgaagtac ttgaaagctg caggagcacc caggttggta agagtaaaca ggtgaatgat 3180

gttctccgcc tgttctcgaa tgggtttgtc ccgatgcttg ttgtaggcag agagcacctt 3240

gtccaagttg gcatcagcca ggatgactcg cttcgaaaac tcggaaatct gctcgataat 3300

ctcgtcgagg taatgtttgt gctgctcaac gaagagttgc ttctgttcgt tgtcctcggg 3360

agaacccttg agcttctcgt agtgagaagc cagatagaga aagttgacgt acttcgaagg 3420

caaggcaagc tcgtttccct tctgcagctc gccagcggag gcgagcatac gctttcgacc 3480

gttctccagt tcgaacagag agtacttggg cagcttgata atgaggtctt tcttgacctc 3540

cttgtaaccc ttggcttcca agaagtcgat gggattcttc tcgaagctcg atcgctccat 3600

gatggtaatt ccgagcagct ccttgacgga cttgagcttt ttggacttgc ccttctcgac 3660

cttcgcaacg acaagcacgg aataggcgac ggtaggagaa tcgaagccac cgtatttctt 3720

gggatcccag tctttctttc gagcgatgag cttgtcggag tttcgcttgg gcagaatcga 3780

ctccttggag aatccgccag tctgaacctc ggtttcttgg acgatgttga cctgaggcat 3840

cgacagaacc tttcgcacgg ttgcaaagtc tcgacccttg tcccacacga tctctccagt 3900

ttcgccgttg gtctcgataa gtggtctctt tcgaatctct ccgttggcca aggtgatctc 3960

ggtcttgaaa aagttcatga tgttggagta aaagaagtac ttggcagtag ccttgccaat 4020

ctcctgttcg gacttggcaa tcatctttcg aacgtcgtag accttgtaat cgccgtaaac 4080

gaactcgctt tcgagcttgg ggtatttctt gatgagcgca gtgccaacga cggcgttgag 4140

gtaagcatcg tgggcatggt ggtaattgtt gatctctcgc accttgtaga actgaaagtc 4200

ctttcggaaa tcggagacca gtttggactt gagagtaatc accttgacct ctcggatgag 4260

cttgtcgttc tcgtcgtact tggtgttcat ccgagaatcg agaatctgtg cgacgtgctt 4320

tgtgatctgt ctggtctcga cgagttgacg cttgatgaag ccagccttgt cgagctcgga 4380

cagaccgcct cgctcggcct tggtaagatt gtcgaacttt cgctgggtaa tgagcttggc 4440

gttgagcagc tgtcgccagt agttcttcat ctttttgacc acctcttcgc tgggaacgtt 4500

gtccgacttg cctctgttct tgtcggatcg tgtaaggacc ttgttgtcga tagaatcgtc 4560

cttgagaaag gattgaggga caatgtggtc cacatcgtag tcgctgagac gattgatgtc 4620

cagttcctga tccacgtaca tgtctcgacc attctgcaga tagtagagat acagcttctc 4680

gttctgcagt tgagtgttct cgacgggatg ctccttgaga atctgggatc ccagctcctt 4740

gatgccttcc tcgattcgct tcatccgctc tcgcgagttt ttctgaccct tttgagttgt 4800

ctggttctct ctggccatct cgatcacaat gttctcgggc ttgtgacgtc ccatgacctt 4860

caccagctcg tcgacaacct tgacagtctg gagaatgcct ttcttgatgg ctggcgaacc 4920

agccaggttg gcaatatgtt cgtgcaagct gtcgccctga ccggacactt gtgccttctg 4980

gatgtcctcc ttgaaggtaa gagaatcgtc gtgaatgagc tgcatgaagt ttcggttggc 5040

aaagccatcg gacttgagaa agtccagaat ggtctttccg gactgcttgt ctctgatgcc 5100

gttgatgagc tttcgcgaaa gtcttcccca gccggtgtat ctacgtcgct tgagttgttt 5160

catgaccttg tcgtcgaaca ggtgagcgta tgtcttgagt cgttcctcga tcatctcccg 5220

atcttcgaac agggtaagag tgagcacgat gtcctccaga atgtcctcgt tttcctcgtt 5280

gtcgagaaaa tccttgtcct tgataatctt gagcagatcg tgataggtgc ccaaagaggc 5340

gttgaatcgg tcctcaactc cggaaatctc gacgctgtcg aaacactcga ttttcttgaa 5400

gtagtcctcc ttgagctgct taacagtgac ctttcggttg gtcttgaaca ggagatcgac 5460

aatggctttc ttctgttcgc cagacaagaa ggcaggcttt cgcattccct cggtaacgta 5520

cttgactttg gtgagttcgt tgtagactgt aaagtactcg tagagcagcg aatgcttggg 5580

aagaaccttc tcgttgggca gattcttgtc gaagttggtc attcgctcga tgaaggactg 5640

tgcagaggca cccttgtcca cgacttcctc gaagttccag ggagtgatgg tttcctcgga 5700

ctttcgagtc atccaagcaa atcgagagtt tcctctggca agaggaccaa catagtaggg 5760

gattcgaaag gtaagaatct tctcgatctt ctctcggttg tccttgagaa aggggtagaa 5820

gtcttcctga cgtcgaagaa tggcgtgcag ctcaccgagg tggatctgat gaggaatgct 5880

gccgttgtcg aaggttcgtt gcttccgaag cagatcctct cgattgagct tgacaagcag 5940

ttcctcggtt ccgtccatct tctcgagaat tggcttgatg aacttgtaga actcttcctg 6000

agaggctccg ccgtcgatgt atccagcgta gccgttcttc gactgatcga aaaagatctc 6060

cttgtacttc tcgggcagtt gctgtcggac aagagccttg agcagtgtga gatcctgatg 6120

gtgctcgtcg tatcgcttga tcatggaggc agaaagggga gcctttgtga tctcggtgtt 6180

gactcgcaga atgtcagaca agagaatagc atccgaaagg ttcttggcag cgagaaacag 6240

gtcggcgtac tgatcgccaa tctgtgcaag caggttgtcg aggtcatcgt cgtaggtgtc 6300

cttggacagc tggagcttgg cgtcctccgc cagatcgaag ttggacttga agttgggtgt 6360

gagaccaaga gaaagggcaa tgaggttgcc aaacagtccg ttctttttct cgccaggaag 6420

ttgggcaatg aggttctcca gtcgtctgct cttcgagagt cgagcagaca agatggcctt 6480

tgcatcgact ccggaggcat tgatggggtt ttcctcgaac agctggttgt aggtctgaac 6540

gagctgaatg aacagcttgt ccacatcgct gttgtcggga ttgagatcgc cctcgatgag 6600

gaaatgacct cgaaacttga tcatgtgtgc cagagcgagg tagataagtc tgagatccgc 6660

cttgtcggtg gaatcgacga gtttctttcg gagatggtag atggtaggat acttctcgtg 6720

gtaagcaacc tcgtccacaa tgttgccaaa gatgggatga cgctcgtgtt tcttgtcttc 6780

ctcgacgagg aaggattcct ccagtcgatg aaagaacgaa tcgtccacct tggccatctc 6840

gttggaaaag atctcctgca ggtagcagat tcggttcttc cgtcgggtgt aacgtcgccg 6900

agcagttcgc ttgagtctgg tagcttcggc agtctcgcca gaatcgaaca acagggcacc 6960

aatgaggttt ttcttgatgg agtgtcgatc ggtgtttccg aggaccttga atttcttgga 7020

gggcaccttg tactcgtcgg tgatgacagc ccagccgaca gagttggttc caatgtccag 7080

gccgatggag tatttcttgt ccatggtgtg atgtgtagtt tagatttcga atctgtgggg 7140

aaagaaagga aaaaagagac tggcaaccga ttgggagagc cactgtttat atatacccta 7200

gacaagcccc ccgcttgtaa gatgttggtc aatgtaaacc agtattaagg ttggcaagtg 7260

caggagaagc aaggtgtggg taccgagcaa tggaaatgtg cggaaggcaa aaaaatgagg 7320

ccacggccta ttgtcggggc tatatccagg gggcgattga agtacactaa catgacatgt 7380

gtccacagac cctcaatctg gcctgatgag ccaaatccat acgcgctttc gcagctctaa 7440

aggctataac aagtcacacc accctgctcg acctcagcgc cctcactttt tgttaagaca 7500

aactgtacac gctgttccag cgttttctgc ctgcacctgg tgggacattt ggtgcaacct 7560

aaagtgctcg gaacctctgt ggtgtccaga tcagcgcagc agttccgagg tagttttgag 7620

gcccttagat gatggtttaa accttaagcc cgctcataac ttcgtatagc atacattata 7680

cgaacggtag gttgcgggat agacgccgac ggagggcaat ggcgctatgg aaccttgcgg 7740

atatccatac gccgcggcgg actgcgtccg aaccagctcc agcagcgttt tttccgggcc 7800

attgagccga ctgcgacccc gccaacgtgt cttggcccac gcactcatgt catgttggtg 7860

ttgggaggcc actttttaag tagcacaagg cacctagctc gcagcaaggt gtccgaacca 7920

aagaagcggc tgcagtggtg caaacggggc ggaaacggcg ggaaaaagcc acgggggcac 7980

gaattgaggc acgccctcga atttgagacg agtcacggcc ccattcgccc gcgcaatggc 8040

tcgccaacgc ccggtctttt gcaccacatc aggttacccc aagccaaacc tttgtgttaa 8100

aaagcttaac atattatacc gaacgtaggt ttgggcgggc ttgctccgtc tgtccaaggc 8160

aacatttata taagggtctg catcgccggc tcaattgaat cttttttctt cttctcttct 8220

ctatattcat tcttgaatta aacacacatc aaccatggcc aaaaagcctg aactcaccgc 8280

gacgtctgtc gagaagtttc tgatcgaaaa gttcgacagc gtctccgacc tgatgcagct 8340

ctcggagggc gaagaatctc gtgctttcag cttcgatgta ggagggcgtg gatatgtcct 8400

gcgggtaaat agctgcgccg atggtttcta caaagatcgt tatgtttatc ggcactttgc 8460

atcggccgcg ctcccgattc cggaagtgct tgacattggg gagttcagcg agagcctgac 8520

ctattgcatc tcccgccgtg cacagggtgt cacgttgcaa gacctgcctg aaaccgaact 8580

gcccgctgtt ctgcagccgg tcgcggaggc tatggatgcg atcgctgcgg ccgatcttag 8640

ccagacgagc gggttcggcc cattcggacc gcaaggaatc ggtcaataca ctacatggcg 8700

tgatttcata tgcgcgattg ctgatcccca tgtgtatcac tggcaaactg tgatggacga 8760

caccgtcagt gcgtccgtcg cgcaggctct cgatgagctg atgctttggg ccgaggactg 8820

ccccgaagtc cggcacctcg tgcacgcgga tttcggctcc aacaatgtcc tgacggacaa 8880

tggccgcata acagcggtca ttgactggag cgaggcgatg ttcggggatt cccaatacga 8940

ggtcgccaac atcttcttct ggaggccgtg gttggcttgt atggagcagc agacgcgcta 9000

cttcgagcgg aggcatccgg agcttgcagg atcgccgcgg ctccgggcgt atatgctccg 9060

cattggtctt gaccaactct atcagagctt ggttgacggc aatttcgatg atgcagcttg 9120

ggcgcagggt cgatgcgacg caatcgtccg atccggagcc gggactgtcg ggcgtacaca 9180

aatcgcccgc agaagcgcgg ccgtctggac cgatggctgt gtagaagtac tcgccgatag 9240

tggaaaccga cgccccagca ctcgtccgag ggcaaaggaa tagcggccgc aagtgtggat 9300

ggggaagtga gtgcccggtt ctgtgtgcac aattggcaat ccaagatgga tggattcaac 9360

acagggatat agcgagctac gtggtggtgc gaggatatag caacggatat ttatgtttga 9420

cacttgagaa tgtacgatac aagcactgtc caagtacaat actaaacata ctgtacatac 9480

tcatactcgt acccgggcaa cggtttcact tgagtgcagt ggctagtgct cttactcgta 9540

cagtgtgcta ccgttcgtat agcatacatt atacgaagtt atcatagtct taattaagat 9600

gtggtggctc aatggtagag ctttcgactc cagtcttctc ggttgtaatc gaagggttgc 9660

aggttcaatt cctgtccatg tcattcaaac gattacccac cctcgtttta gagctagaaa 9720

tagcaagtta aaataaggct agtccgttat caacttgaaa aagtggcacc gagtcggtgc 9780

tttttttttt gttttttatc gattgagcat ccgttgattt ccgaacagat cccaatatta 9840

cacccaagta gcatgcataa gctaaaagta actcgcagcg cacaccgtgc agattcataa 9900

gtctatgatt aattgaacgc caataacccg gcttactaca agtacaagta ggtatacata 9960

gcggtaatga atcattagaa aaaaaaaaaa caaaaaaaaa caaaacaaac tgttgtggat 10020

gcatcaacag tagtacatag ttgtacgatg tacttgtact tgtaaaagca aaaatgtaca 10080

atatctcagg gagcgcaact tttacgttcg aagaacaatg taccgcatac cgcattctag 10140

attctgcgga acgtctaacc tggaaatacg attttttttt tctttcattt tttttgcttc 10200

ttcaaaagta tggtaatttc ctaccattac agttgacact gaacgagggg ggattgaatt 10260

taagcaaaaa attaaatcaa aataccttta tgtatccagc ccatgtaata aacaaaagga 10320

ttatataaca agaaataaat atataccttt aatggatcat tagaataaaa ataaatacga 10380

gaagcacacc agagaagctt tttgattgcc actataccgc tactttggta tatcttatta 10440

taattgttga atttgcaaga tagaatgtca ttcattggag agaaatccaa ggaatatgtg 10500

ggatgaaatg actagaagta tgaacaatga gaatagtaca tacttgtacc tgtatttcta 10560

gaagagagaa agacagttga gtgtgtgatt ctcgtccaat aataatctca atagtaacgt 10620

gtgaatagct gttctttgat agttgatatt tctcgatgac tatttatgtt gtacaaggga 10680

tttttttcgt tgctgttgat ttcgaattag gcaatgcaga tatcatttat gctatccata 10740

tttaagattt cccatacgca tttataacat ttattctaca taaattgtta aatgaacgaa 10800

ctgccattat aaattgtttc ctaaatagga agtgtttttc ataaagcaag taagttgtct 10860

aataatacta agtaataaaa ataagttcat acaatatatt ttgagaacat catttggagg 10920

cggtagatgg agtctgttta ttattaaaca atgcgagatg accccttaaa tattgagaac 10980

atcagttgga ggcggcagat ggagtctgtc tatttagcaa tgggacatga ctgtcagtat 11040

catcatgatg tatatatata atacatataa tattatataa cacgattttt ttaaattatt 11100

ggcccgaaaa ttaatcagtg tagactggat ctcggcagtc tctcggatgt agaattaggt 11160

ttccttgagg cgaagatcgg tttgtgtgac atgaattcga tatcaagctt atcgacacca 11220

tcgacctcga gggggggccc ggtacccaat tcgccctata gtgagtcgta ttacaattca 11280

ctggccgtcg ttttacaacg tcgtgactgg gaaaaccctg gcgttaccca acttaatcgc 11340

cttgcagc 11348

<210> 123
<211> 211
<212> DNA
<213> Artificial Sequence

<220>
<223> Artificial Sequence

<400> 123
ttaactcccg gtggccaagc tggtttaagg cgcgagactg taatggaaaa ttgtcatctt 60

gagatcgggc gttcgactcg cccccgggag attcaaacga ttacccaccc tcgttttaga 120

gctagaaata gcaagttaaa ataaggctag tccgttatca acttgaaaaa gtggcaccga 180

gtcggtgctt ttttttttgt tttttatcga t 211

<210>  124
<211>  87
<212>  DNA
<213>  Artificial Sequence

<220>
<223>  Artificial Sequence

<400>  124
ctcccggtgg ccaagctggt ttaaggcgcg agactgtaat ggaaaattgt catcttgaga      60

tcgggcgttc gactcgcccc cgggaga                                          87


<210>  125
<211>  11349
<212>  DNA
<213>  Artificial Sequence

<220>
<223>  Artificial Sequence

<400>  125
acatccccct ttcgccagct ggcgtaatag cgaagaggcc cgcaccgatc gcccttccca      60

acagttgcgc agcctgaatg gcgaatggaa attgtaagcg ttaatatttt gttaaaattc     120

gcgttaaatt tttgttaaat cagctcattt tttaaccaat aggccgaaat cggcaaaatc     180

ccttataaat caaaagaata gaccgagata gggttgagtg ttgttccagt ttggaacaag     240

agtccactat taaagaacgt ggactccaac gtcaaagggc gaaaaaccgt ctatcagggc     300

gatggcccac tacgtgaacc atcaccctaa tcaagttttt tggggtcgag gtgccgtaaa     360

gcactaaatc ggaaccctaa agggagcccc cgatttagag cttgacgggg aaagccggcg     420

aacgtggcga gaaaggaagg gaagaaagcg aaaggagcgg gcgctagggc gctggcaagt     480

gtagcggtca cgctgcgcgt aaccaccaca cccgccgcgc ttaatgcgcc gctacagggc     540

gcgtcaggtg gcacttttcg gggaaatgtg cgcggaaccc ctatttgttt atttttctaa     600

atacattcaa atatgtatcc gctcatgaga caataaccct gataaatgct tcaataatat     660

tgaaaaagga agagtatgag tattcaacat ttccgtgtcg cccttattcc cttttttgcg     720

gcattttgcc ttcctgtttt tgctcaccca gaaacgctgg tgaaagtaaa agatgctgaa     780

gatcagttgg gtgcacgagt gggttacatc gaactggatc tcaacagcgg taagatcctt     840

gagagttttc gccccgaaga acgttttcca atgatgagca cttttaaagt tctgctatgt     900

ggcgcggtat tatcccgtat tgacgccggg caagagcaac tcggtcgccg catacactat     960

tctcagaatg acttggttga gtactcacca gtcacagaaa agcatcttac ggatggcatg    1020

acagtaagag aattatgcag tgctgccata accatgagtg ataacactgc ggccaactta    1080

cttctgacaa cgatcggagg accgaaggag ctaaccgctt ttttgcacaa catgggggat    1140

catgtaactc gccttgatcg ttgggaaccg gagctgaatg aagccatacc aaacgacgag    1200

cgtgacacca cgatgcctgt agcaatggca acaacgttgc gcaaactatt aactggcgaa 1260

ctacttactc tagcttcccg gcaacaatta atagactgga tggaggcgga taaagttgca 1320

ggaccacttc tgcgctcggc ccttccggct ggctggttta ttgctgataa atctggagcc 1380

ggtgagcgtg ggtctcgcgg tatcattgca gcactggggc cagatggtaa gccctcccgt 1440

atcgtagtta tctacacgac ggggagtcag gcaactatgg atgaacgaaa tagacagatc 1500

gctgagatag gtgcctcact gattaagcat tggtaactgt cagaccaagt ttactcatat 1560

atactttaga ttgatttaaa acttcatttt taatttaaaa ggatctaggt gaagatcctt 1620

tttgataatc tcatgaccaa aatcccttaa cgtgagtttt cgttccactg agcgtcagac 1680

cccgtagaaa agatcaaagg atcttcttga gatccttttt ttctgcgcgt aatctgctgc 1740

ttgcaaacaa aaaaaccacc gctaccagcg gtggtttgtt tgccggatca agagctacca 1800

actctttttc cgaaggtaac tggcttcagc agagcgcaga taccaaatac tgtccttcta 1860

gtgtagccgt agttaggcca ccacttcaag aactctgtag caccgcctac atacctcgct 1920

ctgctaatcc tgttaccagt ggctgctgcc agtggcgata agtcgtgtct taccgggttg 1980

gactcaagac gatagttacc ggataaggcg cagcggtcgg gctgaacggg gggttcgtgc 2040

acacagccca gcttggagcg aacgacctac accgaactga gatacctaca gcgtgagcta 2100

tgagaaagcg ccacgcttcc cgaagggaga aaggcggaca ggtatccggt aagcggcagg 2160

gtcggaacag gagagcgcac gagggagctt ccagggggaa acgcctggta tctttatagt 2220

cctgtcgggt ttcgccacct ctgacttgag cgtcgatttt tgtgatgctc gtcagggggg 2280

cggagcctat ggaaaaacgc cagcaacgcg gcctttttac ggttcctggc cttttgctgg 2340

ccttttgctc acatgttctt tcctgcgtta tcccctgatt ctgtggataa ccgtattacc 2400

gcctttgagt gagctgatac cgctcgccgc agccgaacga ccgagcgcag cgagtcagtg 2460

agcgaggaag cggaagagcg cccaatacgc aaaccgcctc tccccgcgcg ttggccgatt 2520

cattaatgca gctggcacga caggtttccc gactggaaag cgggcagtga gcgcaacgca 2580

attaatgtga gttagctcac tcattaggca ccccaggctt tacactttat gcttccggct 2640

cgtacgcaac taacatgaat gaatacgata tacatcaaag actatgatac gcagtattgc 2700

acactgtacg agtaagagca ctagccactg cactcaagtg aaaccgttgc ccgggtacga 2760

gtatgagtat gtacagtatg tttagtattg tacttggaca gtgcttgtat cgtacattct 2820

caagtgtcaa acataaatat ccgttgctat atcctcgcac caccacgtag ctcgctatat 2880

ccctgtgttg aatccatcca tcttggattg ccaattgtgc acacagaacc gggcactcac 2940

ttccccatcc acacttgcgg ccgcttagac ctttcgcttt ttcttgggat cggctctgga 3000

gtcgccacca agctgagaca ggtcgattcg ggtctcgtac aggccagtga tggactggtg 3060

aatcagggtg gcatcgagaa cctccttggt ggatgtgtac cgctttcggt cgatagtggt 3120

atcgaagtac ttgaaagctg caggagcacc caggttggta agagtaaaca ggtgaatgat 3180

gttctccgcc tgttctcgaa tgggtttgtc ccgatgcttg ttgtaggcag agagcacctt 3240

gtccaagttg gcatcagcca ggatgactcg cttcgaaaac tcggaaatct gctcgataat 3300

ctcgtcgagg taatgtttgt gctgctcaac gaagagttgc ttctgttcgt tgtcctcggg 3360

agaacccttg agcttctcgt agtgagaagc cagatagaga aagttgacgt acttcgaagg 3420

caaggcaagc tcgtttccct tctgcagctc gccagcggag gcgagcatac gctttcgacc 3480

gttctccagt tcgaacagag agtacttggg cagcttgata atgaggtctt tcttgacctc 3540

cttgtaaccc ttggcttcca agaagtcgat gggattcttc tcgaagctcg atcgctccat 3600

gatggtaatt ccgagcagct ccttgacgga cttgagcttt ttggacttgc ccttctcgac 3660

cttcgcaacg acaagcacgg aataggcgac ggtaggagaa tcgaagccac cgtatttctt 3720

gggatcccag tctttctttc gagcgatgag cttgtcggag tttcgcttgg gcagaatcga 3780

ctccttggag aatccgccag tctgaacctc ggtttcttgg acgatgttga cctgaggcat 3840

cgacagaacc tttcgcacgg ttgcaaagtc tcgacccttg tcccacacga tctctccagt 3900

ttcgccgttg gtctcgataa gtggtctctt tcgaatctct ccgttggcca aggtgatctc 3960

ggtcttgaaa aagttcatga tgttggagta aaagaagtac ttggcagtag ccttgccaat 4020

ctcctgttcg gacttggcaa tcatctttcg aacgtcgtag accttgtaat cgccgtaaac 4080

gaactcgctt tcgagcttgg ggtatttctt gatgagcgca gtgccaacga cggcgttgag 4140

gtaagcatcg tgggcatggt ggtaattgtt gatctctcgc accttgtaga actgaaagtc 4200

ctttcggaaa tcggagacca gtttggactt gagagtaatc accttgacct ctcggatgag 4260

cttgtcgttc tcgtcgtact tggtgttcat ccgagaatcg agaatctgtg cgacgtgctt 4320

tgtgatctgt ctggtctcga cgagttgacg cttgatgaag ccagccttgt cgagctcgga 4380

cagaccgcct cgctcggcct tggtaagatt gtcgaacttt cgctgggtaa tgagcttggc 4440

gttgagcagc tgtcgccagt agttcttcat ctttttgacc acctcttcgc tgggaacgtt 4500

gtccgacttg cctctgttct tgtcggatcg tgtaaggacc ttgttgtcga tagaatcgtc 4560

cttgagaaag gattgaggga caatgtggtc cacatcgtag tcgctgagac gattgatgtc 4620

cagttcctga tccacgtaca tgtctcgacc attctgcaga tagtagagat acagcttctc 4680

gttctgcagt tgagtgttct cgacgggatg ctccttgaga atctgggatc ccagctcctt 4740

gatgccttcc tcgattcgct tcatccgctc tcgcgagttt ttctgaccct tttgagttgt 4800

ctggttctct ctggccatct cgatcacaat gttctcgggc ttgtgacgtc ccatgacctt 4860

caccagctcg tcgacaacct tgacagtctg gagaatgcct ttcttgatgg ctggcgaacc 4920

agccaggttg gcaatatgtt cgtgcaagct gtcgccctga ccggacactt gtgccttctg 4980

gatgtcctcc ttgaaggtaa gagaatcgtc gtgaatgagc tgcatgaagt ttcggttggc 5040

aaagccatcg gacttgagaa agtccagaat ggtctttccg gactgcttgt ctctgatgcc 5100

gttgatgagc tttcgcgaaa gtcttcccca gccggtgtat ctacgtcgct tgagttgttt 5160

catgaccttg tcgtcgaaca ggtgagcgta tgtcttgagt cgttcctcga tcatctcccg 5220

atcttcgaac agggtaagag tgagcacgat gtcctccaga atgtcctcgt tttcctcgtt 5280

gtcgagaaaa tccttgtcct tgataatctt gagcagatcg tgataggtgc ccaaagaggc 5340

gttgaatcgg tcctcaactc cggaaatctc gacgctgtcg aaacactcga ttttcttgaa 5400

gtagtcctcc ttgagctgct taacagtgac ctttcggttg gtcttgaaca ggagatcgac 5460

aatggctttc ttctgttcgc cagacaagaa ggcaggcttt cgcattccct cggtaacgta 5520

cttgactttg gtgagttcgt tgtagactgt aaagtactcg tagagcagcg aatgcttggg 5580

aagaaccttc tcgttgggca gattcttgtc gaagttggtc attcgctcga tgaaggactg 5640

tgcagaggca cccttgtcca cgacttcctc gaagttccag ggagtgatgg tttcctcgga 5700

ctttcgagtc atccaagcaa atcgagagtt tcctctggca agaggaccaa catagtaggg 5760

gattcgaaag gtaagaatct tctcgatctt ctctcggttg tccttgagaa aggggtagaa 5820

gtcttcctga cgtcgaagaa tggcgtgcag ctcaccgagg tggatctgat gaggaatgct 5880

gccgttgtcg aaggttcgtt gcttccgaag cagatcctct cgattgagct tgacaagcag 5940

ttcctcggtt ccgtccatct tctcgagaat tggcttgatg aacttgtaga actcttcctg 6000

agaggctccg ccgtcgatgt atccagcgta gccgttcttc gactgatcga aaaagatctc 6060

cttgtacttc tcgggcagtt gctgtcggac aagagccttg agcagtgtga gatcctgatg 6120

gtgctcgtcg tatcgcttga tcatggaggc agaaagggga gcctttgtga tctcggtgtt 6180

gactcgcaga atgtcagaca agagaatagc atccgaaagg ttcttggcag cgagaaacag 6240

gtcggcgtac tgatcgccaa tctgtgcaag caggttgtcg aggtcatcgt cgtaggtgtc 6300

cttggacagc tggagcttgg cgtcctccgc cagatcgaag ttggacttga agttgggtgt 6360

gagaccaaga gaaagggcaa tgaggttgcc aaacagtccg ttctttttct cgccaggaag 6420

ttgggcaatg aggttctcca gtcgtctgct cttcgagagt cgagcagaca agatggcctt 6480

tgcatcgact ccggaggcat tgatggggtt ttcctcgaac agctggttgt aggtctgaac 6540

gagctgaatg aacagcttgt ccacatcgct gttgtcggga ttgagatcgc cctcgatgag 6600

gaaatgacct cgaaacttga tcatgtgtgc cagagcgagg tagataagtc tgagatccgc 6660

cttgtcggtg gaatcgacga gtttctttcg gagatggtag atggtaggat acttctcgtg 6720

gtaagcaacc tcgtccacaa tgttgccaaa gatgggatga cgctcgtgtt tcttgtcttc 6780

ctcgacgagg aaggattcct ccagtcgatg aaagaacgaa tcgtccacct tggccatctc 6840

gttggaaaag atctcctgca ggtagcagat tcggttcttc cgtcgggtgt aacgtcgccg 6900

agcagttcgc ttgagtctgg tagcttcggc agtctcgcca gaatcgaaca acagggcacc 6960

aatgaggttt ttcttgatgg agtgtcgatc ggtgtttccg aggaccttga atttcttgga 7020

gggcaccttg tactcgtcgg tgatgacagc ccagccgaca gagttggttc caatgtccag 7080

gccgatggag tatttcttgt ccatggtgtg atgtgtagtt tagatttcga atctgtgggg 7140

aaagaaagga aaaaagagac tggcaaccga ttgggagagc cactgtttat atatacccta 7200

gacaagcccc ccgcttgtaa gatgttggtc aatgtaaacc agtattaagg ttggcaagtg 7260

caggagaagc aaggtgtggg taccgagcaa tggaaatgtg cggaaggcaa aaaaatgagg 7320

ccacggccta ttgtcggggc tatatccagg gggcgattga agtacactaa catgacatgt 7380

gtccacagac cctcaatctg gcctgatgag ccaaatccat acgcgctttc gcagctctaa 7440

aggctataac aagtcacacc accctgctcg acctcagcgc cctcactttt tgttaagaca 7500

aactgtacac gctgttccag cgttttctgc ctgcacctgg tgggacattt ggtgcaacct 7560

aaagtgctcg gaacctctgt ggtgtccaga tcagcgcagc agttccgagg tagttttgag 7620

gcccttagat gatggtttaa accttaagcc cgctcataac ttcgtatagc atacattata 7680

cgaacggtag gttgcgggat agacgccgac ggagggcaat ggcgctatgg aaccttgcgg 7740

atatccatac gccgcggcgg actgcgtccg aaccagctcc agcagcgttt tttccgggcc 7800

attgagccga ctgcgacccc gccaacgtgt cttggcccac gcactcatgt catgttggtg 7860

ttgggaggcc actttttaag tagcacaagg cacctagctc gcagcaaggt gtccgaacca 7920

aagaagcggc tgcagtggtg caaacggggc ggaaacggcg ggaaaaagcc acgggggcac 7980

gaattgaggc acgccctcga atttgagacg agtcacggcc ccattcgccc gcgcaatggc 8040

tcgccaacgc ccggtctttt gcaccacatc aggttacccc aagccaaacc tttgtgttaa 8100

aaagcttaac atattatacc gaacgtaggt ttgggcgggc ttgctccgtc tgtccaaggc 8160

aacatttata taagggtctg catcgccggc tcaattgaat cttttttctt cttctcttct 8220

ctatattcat tcttgaatta aacacacatc aaccatggcc aaaaagcctg aactcaccgc 8280

gacgtctgtc gagaagtttc tgatcgaaaa gttcgacagc gtctccgacc tgatgcagct 8340

ctcggagggc gaagaatctc gtgctttcag cttcgatgta ggagggcgtg gatatgtcct 8400

gcgggtaaat agctgcgccg atggtttcta caaagatcgt tatgtttatc ggcactttgc 8460

atcggccgcg ctcccgattc cggaagtgct tgacattggg gagttcagcg agagcctgac 8520

ctattgcatc tcccgccgtg cacagggtgt cacgttgcaa gacctgcctg aaaccgaact 8580

gcccgctgtt ctgcagccgg tcgcggaggc tatggatgcg atcgctgcgg ccgatcttag 8640

ccagacgagc gggttcggcc cattcggacc gcaaggaatc ggtcaataca ctacatggcg 8700

tgatttcata tgcgcgattg ctgatcccca tgtgtatcac tggcaaactg tgatggacga 8760

caccgtcagt gcgtccgtcg cgcaggctct cgatgagctg atgctttggg ccgaggactg 8820

ccccgaagtc cggcacctcg tgcacgcgga tttcggctcc aacaatgtcc tgacggacaa 8880

tggccgcata acagcggtca ttgactggag cgaggcgatg ttcggggatt cccaatacga 8940

ggtcgccaac atcttcttct ggaggccgtg gttggcttgt atggagcagc agacgcgcta 9000

cttcgagcgg aggcatccgg agcttgcagg atcgccgcgg ctccgggcgt atatgctccg 9060

cattggtctt gaccaactct atcagagctt ggttgacggc aatttcgatg atgcagcttg 9120

ggcgcagggt cgatgcgacg caatcgtccg atccggagcc gggactgtcg ggcgtacaca 9180

aatcgcccgc agaagcgcgg ccgtctggac cgatggctgt gtagaagtac tcgccgatag 9240

tggaaaccga cgccccagca ctcgtccgag ggcaaaggaa tagcggccgc aagtgtggat 9300

ggggaagtga gtgcccggtt ctgtgtgcac aattggcaat ccaagatgga tggattcaac 9360

acagggatat agcgagctac gtggtggtgc gaggatatag caacggatat ttatgtttga 9420

cacttgagaa tgtacgatac aagcactgtc caagtacaat actaaacata ctgtacatac 9480

tcatactcgt acccgggcaa cggtttcact tgagtgcagt ggctagtgct cttactcgta 9540

cagtgtgcta ccgttcgtat agcatacatt atacgaagtt atcatagtct taattaactc 9600

ccggtggcca agctggttta aggcgcgaga ctgtaatgga aaattgtcat cttgagatcg 9660

ggcgttcgac tcgcccccgg gagattcaaa cgattaccca ccctcgtttt agagctagaa 9720

atagcaagtt aaaataaggc tagtccgtta tcaacttgaa aaagtggcac cgagtcggtg 9780

cttttttttt tgttttttat cgattgagca tccgttgatt tccgaacaga tcccaatatt 9840

acacccaagt agcatgcata agctaaaagt aactcgcagc gcacaccgtg cagattcata 9900

agtctatgat taattgaacg ccaataaccc ggcttactac aagtacaagt aggtatacat 9960

agcggtaatg aatcattaga aaaaaaaaaa acaaaaaaaa acaaaacaaa ctgttgtgga 10020

tgcatcaaca gtagtacata gttgtacgat gtacttgtac ttgtaaaagc aaaaatgtac 10080

aatatctcag ggagcgcaac ttttacgttc gaagaacaat gtaccgcata ccgcattcta 10140

gattctgcgg aacgtctaac ctggaaatac gatttttttt ttctttcatt ttttttgctt 10200

cttcaaaagt atggtaattt cctaccatta cagttgacac tgaacgaggg gggattgaat 10260

ttaagcaaaa aattaaatca aaataccttt atgtatccag cccatgtaat aaacaaaagg 10320

attatataac aagaaataaa tatatacctt taatggatca ttagaataaa aataaatacg 10380

agaagcacac cagagaagct ttttgattgc cactataccg ctactttggt atatcttatt 10440

ataattgttg aatttgcaag atagaatgtc attcattgga gagaaatcca aggaatatgt 10500

gggatgaaat gactagaagt atgaacaatg agaatagtac atacttgtac ctgtatttct 10560

agaagagaga aagacagttg agtgtgtgat tctcgtccaa taataatctc aatagtaacg 10620

tgtgaatagc tgttctttga tagttgatat ttctcgatga ctatttatgt tgtacaaggg 10680

atttttttcg ttgctgttga tttcgaatta ggcaatgcag atatcattta tgctatccat 10740

atttaagatt tcccatacgc atttataaca tttattctac ataaattgtt aaatgaacga 10800

actgccatta taaattgttt cctaaatagg aagtgttttt cataaagcaa gtaagttgtc 10860

taataatact aagtaataaa aataagttca tacaatatat tttgagaaca tcatttggag 10920

gcggtagatg gagtctgttt attattaaac aatgcgagat gaccccttaa atattgagaa 10980

catcagttgg aggcggcaga tggagtctgt ctatttagca atgggacatg actgtcagta 11040

tcatcatgat gtatatatat aatacatata atattatata acacgatttt tttaaattat 11100

tggcccgaaa attaatcagt gtagactgga tctcggcagt ctctcggatg tagaattagg 11160

tttccttgag gcgaagatcg gtttgtgtga catgaattcg atatcaagct tatcgacacc 11220

atcgacctcg aggggggccc cggtacccaa ttcgccctat agtgagtcgt attacaattc 11280

actggccgtc gttttacaac gtcgtgactg ggaaaaccct ggcgttaccc aacttaatcg 11340

ccttgcagc 11349

<210> 126
<211> 224
<212> DNA
<213> Artificial Sequence

<220>
<223> Artificial Sequence

<400> 126
ttaattaagc catcatagta tagtggttag tacaccacgt tgtggagtgt acttcagttc 60

ataacaagcc cgtggaaacc tcagttcgat tctgggtgat ggcattcaaa cgattaccca 120

ccctcgtttt agagctagaa atagcaagtt aaaataaggc tagtccgtta tcaacttgaa 180

aaagtggcac cgagtcggtg cttttttttt tgttttttat cgat 224

<210> 127
<211> 96
<212> DNA
<213> Artificial Sequence

<220>
<223> Artificial Sequence

<400> 127
gccatcatag tatagtggtt agtacaccac gttgtggagt gtacttcagt tcataacaag 60

cccgtggaaa cctcagttcg attctgggtg atggca 96

<210> 128
<211> 11358
<212> DNA
<213> Artificial Sequence

<220>
<223>  Artificial Sequence

<400>  128
acatccccct ttcgccagct ggcgtaatag cgaagaggcc cgcaccgatc gcccttccca      60

acagttgcgc agcctgaatg gcgaatggaa attgtaagcg ttaatatttt gttaaaattc     120

gcgttaaatt tttgttaaat cagctcattt tttaaccaat aggccgaaat cggcaaaatc     180

ccttataaat caaaagaata gaccgagata gggttgagtg ttgttccagt ttggaacaag     240

agtccactat taaagaacgt ggactccaac gtcaaagggc gaaaaaccgt ctatcagggc     300

gatggcccac tacgtgaacc atcaccctaa tcaagttttt tggggtcgag gtgccgtaaa     360

gcactaaatc ggaaccctaa agggagcccc cgatttagag cttgacgggg aaagccggcg     420

aacgtggcga gaaaggaagg gaagaaagcg aaaggagcgg gcgctagggc gctggcaagt     480

gtagcggtca cgctgcgcgt aaccaccaca cccgccgcgc ttaatgcgcc gctacagggc     540

gcgtcaggtg gcacttttcg gggaaatgtg cgcggaaccc ctatttgttt atttttctaa     600

atacattcaa atatgtatcc gctcatgaga caataaccct gataaatgct tcaataatat     660

tgaaaaagga agagtatgag tattcaacat ttccgtgtcg cccttattcc cttttttgcg     720

gcattttgcc ttcctgtttt tgctcaccca gaaacgctgg tgaaagtaaa agatgctgaa     780

gatcagttgg gtgcacgagt gggttacatc gaactggatc tcaacagcgg taagatcctt     840

gagagttttc gccccgaaga acgttttcca atgatgagca cttttaaagt tctgctatgt     900

ggcgcggtat tatcccgtat tgacgccggg caagagcaac tcggtcgccg catacactat     960

tctcagaatg acttggttga gtactcacca gtcacagaaa agcatcttac ggatggcatg    1020

acagtaagag aattatgcag tgctgccata accatgagtg ataacactgc ggccaactta    1080

cttctgacaa cgatcggagg accgaaggag ctaaccgctt ttttgcacaa catgggggat    1140

catgtaactc gccttgatcg ttgggaaccg gagctgaatg aagccatacc aaacgacgag    1200

cgtgacacca cgatgcctgt agcaatggca acaacgttgc gcaaactatt aactggcgaa    1260

ctacttactc tagcttcccg gcaacaatta atagactgga tggaggcgga taaagttgca    1320

ggaccacttc tgcgctcggc ccttccggct ggctggttta ttgctgataa atctggagcc    1380

ggtgagcgtg ggtctcgcgg tatcattgca gcactggggc cagatggtaa gccctcccgt    1440

atcgtagtta tctacacgac ggggagtcag gcaactatgg atgaacgaaa tagacagatc    1500

gctgagatag gtgcctcact gattaagcat tggtaactgt cagaccaagt ttactcatat    1560

atactttaga ttgatttaaa acttcatttt taatttaaaa ggatctaggt gaagatcctt    1620

tttgataatc tcatgaccaa aatcccttaa cgtgagtttt cgttccactg agcgtcagac    1680

cccgtagaaa agatcaaagg atcttcttga gatccttttt ttctgcgcgt aatctgctgc    1740

ttgcaaacaa aaaaaccacc gctaccagcg gtggtttgtt tgccggatca agagctacca    1800

actctttttc cgaaggtaac tggcttcagc agagcgcaga taccaaatac tgtccttcta 1860

gtgtagccgt agttaggcca ccacttcaag aactctgtag caccgcctac atacctcgct 1920

ctgctaatcc tgttaccagt ggctgctgcc agtggcgata agtcgtgtct taccgggttg 1980

gactcaagac gatagttacc ggataaggcg cagcggtcgg gctgaacggg gggttcgtgc 2040

acacagccca gcttggagcg aacgacctac accgaactga gatacctaca gcgtgagcta 2100

tgagaaagcg ccacgcttcc cgaagggaga aaggcggaca ggtatccggt aagcggcagg 2160

gtcggaacag gagagcgcac gagggagctt ccagggggaa acgcctggta tctttatagt 2220

cctgtcgggt ttcgccacct ctgacttgag cgtcgatttt tgtgatgctc gtcagggggg 2280

cggagcctat ggaaaaacgc cagcaacgcg gcctttttac ggttcctggc cttttgctgg 2340

ccttttgctc acatgttctt tcctgcgtta tcccctgatt ctgtggataa ccgtattacc 2400

gcctttgagt gagctgatac cgctcgccgc agccgaacga ccgagcgcag cgagtcagtg 2460

agcgaggaag cggaagagcg cccaatacgc aaaccgcctc tccccgcgcg ttggccgatt 2520

cattaatgca gctggcacga caggtttccc gactggaaag cgggcagtga gcgcaacgca 2580

attaatgtga gttagctcac tcattaggca ccccaggctt tacactttat gcttccggct 2640

cgtacgcaac taacatgaat gaatacgata tacatcaaag actatgatac gcagtattgc 2700

acactgtacg agtaagagca ctagccactg cactcaagtg aaaccgttgc ccgggtacga 2760

gtatgagtat gtacagtatg tttagtattg tacttggaca gtgcttgtat cgtacattct 2820

caagtgtcaa acataaatat ccgttgctat atcctcgcac caccacgtag ctcgctatat 2880

ccctgtgttg aatccatcca tcttggattg ccaattgtgc acacagaacc gggcactcac 2940

ttccccatcc acacttgcgg ccgcttagac ctttcgcttt ttcttgggat cggctctgga 3000

gtcgccacca agctgagaca ggtcgattcg ggtctcgtac aggccagtga tggactggtg 3060

aatcagggtg gcatcgagaa cctccttggt ggatgtgtac cgctttcggt cgatagtggt 3120

atcgaagtac ttgaaagctg caggagcacc caggttggta agagtaaaca ggtgaatgat 3180

gttctccgcc tgttctcgaa tgggtttgtc ccgatgcttg ttgtaggcag agagcacctt 3240

gtccaagttg gcatcagcca ggatgactcg cttcgaaaac tcggaaatct gctcgataat 3300

ctcgtcgagg taatgtttgt gctgctcaac gaagagttgc ttctgttcgt tgtcctcggg 3360

agaacccttg agcttctcgt agtgagaagc cagatagaga aagttgacgt acttcgaagg 3420

caaggcaagc tcgtttccct tctgcagctc gccagcggag gcgagcatac gctttcgacc 3480

gttctccagt tcgaacagag agtacttggg cagcttgata atgaggtctt tcttgacctc 3540

cttgtaaccc ttggcttcca agaagtcgat gggattcttc tcgaagctcg atcgctccat 3600

gatggtaatt ccgagcagct ccttgacgga cttgagcttt ttggacttgc ccttctcgac 3660

cttcgcaacg acaagcacgg aataggcgac ggtaggagaa tcgaagccac cgtatttctt 3720

gggatcccag tctttctttc gagcgatgag cttgtcggag tttcgcttgg gcagaatcga 3780

ctccttggag aatccgccag tctgaacctc ggttttcttg acgatgttga cctgaggcat 3840

cgacagaacc tttcgcacgg ttgcaaagtc tcgacccttg tcccacacga tctctccagt 3900

ttcgccgttg gtctcgataa gtggtctctt tcgaatctct ccgttggcca aggtgatctc 3960

ggtcttgaaa aagttcatga tgttggagta aaagaagtac ttggcagtag ccttgccaat 4020

ctcctgttcg gacttggcaa tcatctttcg aacgtcgtag accttgtaat cgccgtaaac 4080

gaactcgctt tcgagcttgg ggtatttctt gatgagcgca gtgccaacga cggcgttgag 4140

gtaagcatcg tgggcatggt ggtaattgtt gatctctcgc accttgtaga actgaaagtc 4200

ctttcggaaa tcggagacca gtttggactt gagagtaatc accttgacct ctcggatgag 4260

cttgtcgttc tcgtcgtact tggtgttcat ccgagaatcg agaatctgtg cgacgtgctt 4320

tgtgatctgt ctggtctcga cgagttgacg cttgatgaag ccagccttgt cgagctcgga 4380

cagaccgcct cgctcggcct tggtaagatt gtcgaacttt cgctgggtaa tgagcttggc 4440

gttgagcagc tgtcgccagt agttcttcat ctttttgacc acctcttcgc tgggaacgtt 4500

gtccgacttg cctctgttct tgtcggatcg tgtaaggacc ttgttgtcga tagaatcgtc 4560

cttgagaaag gattgaggga caatgtggtc cacatcgtag tcgctgagac gattgatgtc 4620

cagttcctga tccacgtaca tgtctcgacc attctgcaga tagtagagat acagcttctc 4680

gttctgcagt tgagtgttct cgacgggatg ctccttgaga atctgggatc ccagctcctt 4740

gatgccttcc tcgattcgct tcatccgctc tcgcgagttt ttctgaccct tttgagttgt 4800

ctggttctct ctggccatct cgatcacaat gttctcgggc ttgtgacgtc ccatgacctt 4860

caccagctcg tcgacaacct tgacagtctg gagaatgcct ttcttgatgg ctggcgaacc 4920

agccaggttg gcaatatgtt cgtgcaagct gtcgccctga ccggacactt gtgccttctg 4980

gatgtcctcc ttgaaggtaa gagaatcgtc gtgaatgagc tgcatgaagt ttcggttggc 5040

aaagccatcg gacttgagaa agtccagaat ggtctttccg gactgcttgt ctctgatgcc 5100

gttgatgagc tttcgcgaaa gtcttcccca gccggtgtat ctacgtcgct tgagttgttt 5160

catgaccttg tcgtcgaaca ggtgagcgta tgtcttgagt cgttcctcga tcatctcccg 5220

atcttcgaac agggtaagag tgagcacgat gtcctccaga atgtcctcgt tttcctcgtt 5280

gtcgagaaaa tccttgtcct tgataatctt gagcagatcg tgataggtgc ccaaagaggc 5340

gttgaatcgg tcctcaactc cggaaatctc gacgctgtcg aaacactcga ttttcttgaa 5400

gtagtcctcc ttgagctgct taacagtgac ctttcggttg gtcttgaaca ggagatcgac 5460

aatggctttc ttctgttcgc cagacaagaa ggcaggcttt cgcattccct cggtaacgta 5520

cttgactttg gtgagttcgt tgtagactgt aaagtactcg tagagcagcg aatgcttggg 5580

aagaaccttc tcgttgggca gattcttgtc gaagttggtc attcgctcga tgaaggactg 5640

tgcagaggca cccttgtcca cgacttcctc gaagttccag ggagtgatgg tttcctcgga 5700

ctttcgagtc atccaagcaa atcgagagtt tcctctggca agaggaccaa catagtaggg 5760

gattcgaaag gtaagaatct tctcgatctt ctctcggttg tccttgagaa aggggtagaa 5820

gtcttcctga cgtcgaagaa tggcgtgcag ctcaccgagg tggatctgat gaggaatgct 5880

gccgttgtcg aaggttcgtt gcttccgaag cagatcctct cgattgagct tgacaagcag 5940

ttcctcggtt ccgtccatct tctcgagaat tggcttgatg aacttgtaga actcttcctg 6000

agaggctccg ccgtcgatgt atccagcgta gccgttcttc gactgatcga aaaagatctc 6060

cttgtacttc tcgggcagtt gctgtcggac aagagccttg agcagtgtga gatcctgatg 6120

gtgctcgtcg tatcgcttga tcatggaggc agaaagggga gcctttgtga tctcggtgtt 6180

gactcgcaga atgtcagaca agagaatagc atccgaaagg ttcttggcag cgagaaacag 6240

gtcggcgtac tgatcgccaa tctgtgcaag caggttgtcg aggtcatcgt cgtaggtgtc 6300

cttggacagc tggagcttgg cgtcctccgc cagatcgaag ttggacttga agttgggtgt 6360

gagaccaaga gaaagggcaa tgaggttgcc aaacagtccg ttctttttct cgccaggaag 6420

ttgggcaatg aggttctcca gtcgtctgct cttcgagagt cgagcagaca agatggcctt 6480

tgcatcgact ccggaggcat tgatggggtt ttcctcgaac agctggttgt aggtctgaac 6540

gagctgaatg aacagcttgt ccacatcgct gttgtcggga ttgagatcgc cctcgatgag 6600

gaaatgacct cgaaacttga tcatgtgtgc cagagcgagg tagataagtc tgagatccgc 6660

cttgtcggtg gaatcgacga gtttctttcg gagatggtag atggtaggat acttctcgtg 6720

gtaagcaacc tcgtccacaa tgttgccaaa gatgggatga cgctcgtgtt tcttgtcttc 6780

ctcgacgagg aaggattcct ccagtcgatg aaagaacgaa tcgtccacct tggccatctc 6840

gttggaaaag atctcctgca ggtagcagat tcggttcttc cgtcgggtgt aacgtcgccg 6900

agcagttcgc ttgagtctgg tagcttcggc agtctcgcca gaatcgaaca acagggcacc 6960

aatgaggttt ttcttgatgg agtgtcgatc ggtgtttccg aggaccttga atttcttgga 7020

gggcaccttg tactcgtcgg tgatgacagc ccagccgaca gagttggttc caatgtccag 7080

gccgatggag tatttcttgt ccatggtgtg atgtgtagtt tagatttcga atctgtgggg 7140

aaagaaagga aaaaagagac tggcaaccga ttgggagagc cactgtttat atatacccta 7200

gacaagcccc ccgcttgtaa gatgttggtc aatgtaaacc agtattaagg ttggcaagtg 7260

caggagaagc aaggtgtggg taccgagcaa tggaaatgtg cggaaggcaa aaaaatgagg 7320

ccacggccta ttgtcggggc tatatccagg gggcgattga agtacactaa catgacatgt 7380

gtccacagac cctcaatctg gcctgatgag ccaaatccat acgcgctttc gcagctctaa 7440

aggctataac aagtcacacc accctgctcg acctcagcgc cctcactttt tgttaagaca 7500

aactgtacac gctgttccag cgttttctgc ctgcacctgg tgggacattt ggtgcaacct 7560

aaagtgctcg gaacctctgt ggtgtccaga tcagcgcagc agttccgagg tagttttgag 7620

gcccttagat gatggtttaa accttaagcc cgctcataac ttcgtatagc atacattata 7680

cgaacggtag gttgcgggat agacgccgac ggagggcaat ggcgctatgg aaccttgcgg 7740

atatccatac gccgcggcgg actgcgtccg aaccagctcc agcagcgttt tttccgggcc 7800

attgagccga ctgcgacccc gccaacgtgt cttggcccac gcactcatgt catgttggtg 7860

ttgggaggcc actttttaag tagcacaagg cacctagctc gcagcaaggt gtccgaacca 7920

aagaagcggc tgcagtggtg caaacggggc ggaaacggcg ggaaaaagcc acgggggcac 7980

gaattgaggc acgccctcga atttgagacg agtcacggcc ccattcgccc gcgcaatggc 8040

tcgccaacgc ccggtctttt gcaccacatc aggttacccc aagccaaacc tttgtgttaa 8100

aaagcttaac atattatacc gaacgtaggt ttgggcgggc ttgctccgtc tgtccaaggc 8160

aacatttata taagggtctg catcgccggc tcaattgaat cttttttctt cttctcttct 8220

ctatattcat tcttgaatta aacacacatc aaccatggcc aaaaagcctg aactcaccgc 8280

gacgtctgtc gagaagtttc tgatcgaaaa gttcgacagc gtctccgacc tgatgcagct 8340

ctcggagggc gaagaatctc gtgctttcag cttcgatgta ggagggcgtg gatatgtcct 8400

gcgggtaaat agctgcgccg atggtttcta caaagatcgt tatgtttatc ggcactttgc 8460

atcggccgcg ctcccgattc cggaagtgct tgacattggg gagttcagcg agagcctgac 8520

ctattgcatc tcccgccgtg cacagggtgt cacgttgcaa gacctgcctg aaaccgaact 8580

gcccgctgtt ctgcagccgg tcgcggaggc tatggatgcg atcgctgcgg ccgatcttag 8640

ccagacgagc gggttcggcc cattcggacc gcaaggaatc ggtcaataca ctacatggcg 8700

tgatttcata tgcgcgattg ctgatcccca tgtgtatcac tggcaaactg tgatggacga 8760

caccgtcagt gcgtccgtcg cgcaggctct cgatgagctg atgctttggg ccgaggactg 8820

ccccgaagtc cggcacctcg tgcacgcgga tttcggctcc aacaatgtcc tgacggacaa 8880

tggccgcata acagcggtca ttgactggag cgaggcgatg ttcggggatt cccaatacga 8940

ggtcgccaac atcttcttct ggaggccgtg gttggcttgt atggagcagc agacgcgcta 9000

cttcgagcgg aggcatccgg agcttgcagg atcgccgcgg ctccgggcgt atatgctccg 9060

cattggtctt gaccaactct atcagagctt ggttgacggc aatttcgatg atgcagcttg 9120

ggcgcagggt cgatgcgacg caatcgtccg atccggagcc gggactgtcg ggcgtacaca 9180

aatcgcccgc agaagcgcgg ccgtctggac cgatggctgt gtagaagtac tcgccgatag 9240

tggaaaccga cgccccagca ctcgtccgag ggcaaaggaa tagcggccgc aagtgtggat 9300

ggggaagtga gtgcccggtt ctgtgtgcac aattggcaat ccaagatgga tggattcaac 9360

acagggatat agcgagctac gtggtggtgc gaggatatag caacggatat ttatgtttga 9420

cacttgagaa tgtacgatac aagcactgtc caagtacaat actaaacata ctgtacatac 9480

tcatactcgt acccgggcaa cggtttcact tgagtgcagt ggctagtgct cttactcgta 9540

cagtgtgcta ccgttcgtat agcatacatt atacgaagtt atcatagtct taattaagcc 9600

atcatagtat agtggttagt acaccacgtt gtggagtgta cttcagttca taacaagccc 9660

gtggaaacct cagttcgatt ctgggtgatg gcattcaaac gattacccac cctcgtttta 9720

gagctagaaa tagcaagtta aaataaggct agtccgttat caacttgaaa aagtggcacc 9780

gagtcggtgc tttttttttt gttttttatc gattgagcat ccgttgattt ccgaacagat 9840

cccaatatta cacccaagta gcatgcataa gctaaaagta actcgcagcg cacaccgtgc 9900

agattcataa gtctatgatt aattgaacgc caataacccg gcttactaca agtacaagta 9960

ggtatacata gcggtaatga atcattagaa aaaaaaaaaa caaaaaaaaa caaaacaaac 10020

tgttgtggat gcatcaacag tagtacatag ttgtacgatg tacttgtact tgtaaaagca 10080

aaaatgtaca atatctcagg gagcgcaact tttacgttcg aagaacaatg taccgcatac 10140

cgcattctag attctgcgga acgtctaacc tggaaatacg attttttttt tctttcattt 10200

tttttgcttc ttcaaaagta tggtaatttc ctaccattac agttgacact gaacgagggg 10260

ggattgaatt taagcaaaaa attaaatcaa aataccttta tgtatccagc ccatgtaata 10320

aacaaaagga ttatataaca agaaataaat atataccttt aatggatcat tagaataaaa 10380

ataaatacga gaagcacacc agagaagctt tttgattgcc actataccgc tactttggta 10440

tatcttatta taattgttga atttgcaaga tagaatgtca ttcattggag agaaatccaa 10500

ggaatatgtg ggatgaaatg actagaagta tgaacaatga gaatagtaca tacttgtacc 10560

tgtatttcta gaagagagaa agacagttga gtgtgtgatt ctcgtccaat aataatctca 10620

atagtaacgt gtgaatagct gttctttgat agttgatatt tctcgatgac tatttatgtt 10680

gtacaaggga tttttttcgt tgctgttgat ttcgaattag gcaatgcaga tatcatttat 10740

gctatccata tttaagattt cccatacgca tttataacat ttattctaca taaattgtta 10800

aatgaacgaa ctgccattat aaattgtttc ctaaatagga agtgtttttc ataaagcaag 10860

taagttgtct aataatacta agtaataaaa ataagttcat acaatatatt ttgagaacat 10920

catttggagg cggtagatgg agtctgttta ttattaaaca atgcgagatg accccttaaa 10980

tattgagaac atcagttgga ggcggcagat ggagtctgtc tatttagcaa tgggacatga 11040

ctgtcagtat catcatgatg tatatatata atacatataa tattatataa cacgattttt 11100

ttaaattatt ggcccgaaaa ttaatcagtg tagactggat ctcggcagtc tctcggatgt 11160

agaattaggt ttccttgagg cgaagatcgg tttgtgtgac atgaattcga tatcaagctt 11220

atcgacacca tcgacctcga gggggggccc ggtacccaat tcgccctata gtgagtcgta 11280

ttacaattca ctggccgtcg ttttacaacg tcgtgactgg gaaaaccctg gcgttaccca 11340

acttaatcgc cttgcagc 11358

<210> 129
<211> 224
<212> DNA
<213> Artificial Sequence

<220>
<223> Artificial Sequence

<400> 129
ttaattaagc catcatagta tagtggttag tacaccacgt tgtggagtgt acttcagttc 60

ataacaagcc cgtggaaacc tcagttcgat tctgggtgat ggcattcaaa cgattaccca 120

ccctcgtttt agagctagaa atagcaagtt aaaataaggc tagtccgtta tcaacttgaa 180

aaagtggcac cgagtcggtg cttttttttt tgttttttat cgat 224

<210> 130
<211> 96
<212> DNA
<213> Artificial Sequence

<220>
<223> Artificial Sequence

<400> 130
gccatcatag tatagtggtt agtacaccac gttgtggagt gtacttcagt tcataacaag 60

cccgtggaaa cctcagttcg attctgggtg atggca 96

<210> 131
<211> 11358
<212> DNA
<213> Artificial Sequence

<220>
<223> Artificial Sequence

<400> 131
acatccccct ttcgccagct ggcgtaatag cgaagaggcc cgcaccgatc gcccttccca 60

acagttgcgc agcctgaatg gcgaatggaa attgtaagcg ttaatatttt gttaaaattc 120

gcgttaaatt tttgttaaat cagctcattt tttaaccaat aggccgaaat cggcaaaatc 180

ccttataaat caaaagaata gaccgagata gggttgagtg ttgttccagt ttggaacaag 240

agtccactat taaagaacgt ggactccaac gtcaaagggc gaaaaaccgt ctatcagggc 300

gatggcccac tacgtgaacc atcaccctaa tcaagttttt tggggtcgag gtgccgtaaa 360

gcactaaatc ggaaccctaa agggagcccc cgatttagag cttgacgggg aaagccggcg 420

aacgtggcga gaaaggaagg gaagaaagcg aaaggagcgg gcgctagggc gctggcaagt 480

gtagcggtca cgctgcgcgt aaccaccaca cccgccgcgc ttaatgcgcc gctacagggc 540

gcgtcaggtg gcacttttcg gggaaatgtg cgcggaaccc ctatttgttt atttttctaa 600

atacattcaa atatgtatcc gctcatgaga caataaccct gataaatgct tcaataatat 660

tgaaaaagga agagtatgag tattcaacat ttccgtgtcg cccttattcc cttttttgcg 720

gcattttgcc ttcctgtttt tgctcaccca gaaacgctgg tgaaagtaaa agatgctgaa 780

gatcagttgg gtgcacgagt gggttacatc gaactggatc tcaacagcgg taagatcctt 840

gagagttttc gccccgaaga acgttttcca atgatgagca cttttaaagt tctgctatgt 900

ggcgcggtat tatcccgtat tgacgccggg caagagcaac tcggtcgccg catacactat 960

tctcagaatg acttggttga gtactcacca gtcacagaaa agcatcttac ggatggcatg 1020

acagtaagag aattatgcag tgctgccata accatgagtg ataacactgc ggccaactta 1080

cttctgacaa cgatcggagg accgaaggag ctaaccgctt ttttgcacaa catgggggat 1140

catgtaactc gccttgatcg ttgggaaccg gagctgaatg aagccatacc aaacgacgag 1200

cgtgacacca cgatgcctgt agcaatggca acaacgttgc gcaaactatt aactggcgaa 1260

ctacttactc tagcttcccg gcaacaatta atagactgga tggaggcgga taaagttgca 1320

ggaccacttc tgcgctcggc ccttccggct ggctggttta ttgctgataa atctggagcc 1380

ggtgagcgtg ggtctcgcgg tatcattgca gcactggggc cagatggtaa gccctcccgt 1440

atcgtagtta tctacacgac ggggagtcag gcaactatgg atgaacgaaa tagacagatc 1500

gctgagatag gtgcctcact gattaagcat tggtaactgt cagaccaagt ttactcatat 1560

atactttaga ttgatttaaa acttcatttt taatttaaaa ggatctaggt gaagatcctt 1620

tttgataatc tcatgaccaa aatcccttaa cgtgagtttt cgttccactg agcgtcagac 1680

cccgtagaaa agatcaaagg atcttcttga gatccttttt ttctgcgcgt aatctgctgc 1740

ttgcaaacaa aaaaaccacc gctaccagcg gtggtttgtt tgccggatca agagctacca 1800

actctttttc cgaaggtaac tggcttcagc agagcgcaga taccaaatac tgtccttcta 1860

gtgtagccgt agttaggcca ccacttcaag aactctgtag caccgcctac atacctcgct 1920

ctgctaatcc tgttaccagt ggctgctgcc agtggcgata agtcgtgtct taccgggttg 1980

gactcaagac gatagttacc ggataaggcg cagcggtcgg gctgaacggg gggttcgtgc 2040

acacagccca gcttggagcg aacgacctac accgaactga gatacctaca gcgtgagcta 2100

tgagaaagcg ccacgcttcc cgaagggaga aaggcggaca ggtatccggt aagcggcagg 2160

gtcggaacag gagagcgcac gagggagctt ccagggggaa acgcctggta tctttatagt 2220

cctgtcgggt ttcgccacct ctgacttgag cgtcgatttt tgtgatgctc gtcagggggg 2280

cggagcctat ggaaaaacgc cagcaacgcg gcctttttac ggttcctggc cttttgctgg 2340

ccttttgctc acatgttctt tcctgcgtta tcccctgatt ctgtggataa ccgtattacc 2400

gcctttgagt gagctgatac cgctcgccgc agccgaacga ccgagcgcag cgagtcagtg 2460

agcgaggaag cggaagagcg cccaatacgc aaaccgcctc tccccgcgcg ttggccgatt 2520

cattaatgca gctggcacga caggtttccc gactggaaag cgggcagtga gcgcaacgca 2580

attaatgtga gttagctcac tcattaggca ccccaggctt tacactttat gcttccggct 2640

cgtacgcaac taacatgaat gaatacgata tacatcaaag actatgatac gcagtattgc 2700

acactgtacg agtaagagca ctagccactg cactcaagtg aaaccgttgc ccgggtacga 2760

gtatgagtat gtacagtatg tttagtattg tacttggaca gtgcttgtat cgtacattct 2820

caagtgtcaa acataaatat ccgttgctat atcctcgcac caccacgtag ctcgctatat 2880

ccctgtgttg aatccatcca tcttggattg ccaattgtgc acacagaacc gggcactcac 2940

ttccccatcc acacttgcgg ccgcttagac ctttcgcttt ttcttgggat cggctctgga 3000

gtcgccacca agctgagaca ggtcgattcg ggtctcgtac aggccagtga tggactggtg 3060

aatcagggtg gcatcgagaa cctccttggt ggatgtgtac cgctttcggt cgatagtggt 3120

atcgaagtac ttgaaagctg caggagcacc caggttggta agagtaaaca ggtgaatgat 3180

gttctccgcc tgttctcgaa tgggtttgtc ccgatgcttg ttgtaggcag agagcacctt 3240

gtccaagttg gcatcagcca ggatgactcg cttcgaaaac tcggaaatct gctcgataat 3300

ctcgtcgagg taatgtttgt gctgctcaac gaagagttgc ttctgttcgt tgtcctcggg 3360

agaacccttg agcttctcgt agtgagaagc cagatagaga aagttgacgt acttcgaagg 3420

caaggcaagc tcgtttccct tctgcagctc gccagcggag gcgagcatac gctttcgacc 3480

gttctccagt tcgaacagag agtacttggg cagcttgata atgaggtctt tcttgacctc 3540

cttgtaaccc ttggcttcca agaagtcgat gggattcttc tcgaagctcg atcgctccat 3600

gatggtaatt ccgagcagct ccttgacgga cttgagcttt ttggacttgc ccttctcgac 3660

cttcgcaacg acaagcacgg aataggcgac ggtaggagaa tcgaagccac cgtatttctt 3720

gggatcccag tctttctttc gagcgatgag cttgtcggag tttcgcttgg gcagaatcga 3780

ctccttggag aatccgccag tctgaacctc ggttttcttg acgatgttga cctgaggcat 3840

cgacagaacc tttcgcacgg ttgcaaagtc tcgacccttg tcccacacga tctctccagt 3900

ttcgccgttg gtctcgataa gtggtctctt tcgaatctct ccgttggcca aggtgatctc 3960

ggtcttgaaa aagttcatga tgttggagta aaagaagtac ttggcagtag ccttgccaat 4020

ctcctgttcg gacttggcaa tcatctttcg aacgtcgtag accttgtaat cgccgtaaac 4080

gaactcgctt tcgagcttgg ggtatttctt gatgagcgca gtgccaacga cggcgttgag 4140

gtaagcatcg tgggcatggt ggtaattgtt gatctctcgc accttgtaga actgaaagtc 4200

ctttcggaaa tcggagacca gtttggactt gagagtaatc accttgacct ctcggatgag 4260

cttgtcgttc tcgtcgtact tggtgttcat ccgagaatcg agaatctgtg cgacgtgctt 4320

tgtgatctgt ctggtctcga cgagttgacg cttgatgaag ccagccttgt cgagctcgga 4380

cagaccgcct cgctcggcct tggtaagatt gtcgaacttt cgctgggtaa tgagcttggc 4440

gttgagcagc tgtcgccagt agttcttcat ctttttgacc acctcttcgc tgggaacgtt 4500

gtccgacttg cctctgttct tgtcggatcg tgtaaggacc ttgttgtcga tagaatcgtc 4560

cttgagaaag gattgaggga caatgtggtc cacatcgtag tcgctgagac gattgatgtc 4620

cagttcctga tccacgtaca tgtctcgacc attctgcaga tagtagagat acagcttctc 4680

gttctgcagt tgagtgttct cgacgggatg ctccttgaga atctggatcc ccagctcctt 4740

gatgccttcc tcgattcgct tcatccgctc tcgcgagttt ttctgaccct tttgagttgt 4800

ctggttctct ctggccatct cgatcacaat gttctcgggc ttgtgacgtc ccatgacctt 4860

caccagctcg tcgacaacct tgacagtctg gagaatgcct ttcttgatgg ctggcgaacc 4920

agccaggttg gcaatatgtt cgtgcaagct gtcgccctga ccggacactt gtgccttctg 4980

gatgtcctcc ttgaaggtaa gagaatcgtc gtgaatgagc tgcatgaagt ttcggttggc 5040

aaagccatcg gacttgagaa agtccagaat ggtctttccg gactgcttgt ctctgatgcc 5100

gttgatgagc tttcgcgaaa gtcttcccca gccggtgtat ctacgtcgct tgagttgttt 5160

catgaccttg tcgtcgaaca ggtgagcgta tgtcttgagt cgttcctcga tcatctcccg 5220

atcttcgaac agggtaagag tgagcacgat gtcctccaga atgtcctcgt tttcctcgtt 5280

gtcgagaaaa tccttgtcct tgataatctt gagcagatcg tgataggtgc ccaaagaggc 5340

gttgaatcgg tcctcaactc cggaaatctc gacgctgtcg aaacactcga ttttcttgaa 5400

gtagtcctcc ttgagctgct taacagtgac ctttcggttg gtcttgaaca ggagatcgac 5460

aatggctttc ttctgttcgc cagacaagaa ggcaggcttt cgcattccct cggtaacgta 5520

cttgactttg gtgagttcgt tgtagactgt aaagtactcg tagagcagcg aatgcttggg 5580

aagaaccttc tcgttgggca gattcttgtc gaagttggtc attcgctcga tgaaggactg 5640

tgcagaggca cccttgtcca cgacttcctc gaagttccag ggagtgatgg tttcctcgga 5700

ctttcgagtc atccaagcaa atcgagagtt tcctctggca agaggaccaa catagtaggg 5760

gattcgaaag gtaagaatct tctcgatctt ctctcggttg tccttgagaa aggggtagaa 5820

gtcttcctga cgtcgaagaa tggcgtgcag ctcaccgagg tggatctgat gaggaatgct 5880

gccgttgtcg aaggttcgtt gcttccgaag cagatcctct cgattgagct tgacaagcag 5940

ttcctcggtt ccgtccatct tctcgagaat tggcttgatg aacttgtaga actcttcctg 6000

agaggctccg ccgtcgatgt atccagcgta gccgttcttc gactgatcga aaaagatctc 6060

cttgtacttc tcgggcagtt gctgtcggac aagagccttg agcagtgtga gatcctgatg 6120

gtgctcgtcg tatcgcttga tcatggaggc agaaagggga gcctttgtga tctcggtgtt 6180

gactcgcaga atgtcagaca agagaatagc atccgaaagg ttcttggcag cgagaaacag 6240

gtcggcgtac tgatcgccaa tctgtgcaag caggttgtcg aggtcatcgt cgtaggtgtc 6300

cttggacagc tggagcttgg cgtcctccgc cagatcgaag ttggacttga agttgggtgt 6360

gagaccaaga gaaagggcaa tgaggttgcc aaacagtccg ttctttttct cgccaggaag 6420

ttgggcaatg aggttctcca gtcgtctgct cttcgagagt cgagcagaca agatggcctt 6480

tgcatcgact ccggaggcat tgatggggtt ttcctcgaac agctggttgt aggtctgaac 6540

gagctgaatg aacagcttgt ccacatcgct gttgtcggga ttgagatcgc cctcgatgag 6600

gaaatgacct cgaaacttga tcatgtgtgc cagagcgagg tagataagtc tgagatccgc 6660

cttgtcggtg gaatcgacga gtttctttcg gagatggtag atggtaggat acttctcgtg 6720

gtaagcaacc tcgtccacaa tgttgccaaa gatgggatga cgctcgtgtt tcttgtcttc 6780

ctcgacgagg aaggattcct ccagtcgatg aaagaacgaa tcgtccacct tggccatctc 6840

gttggaaaag atctcctgca ggtagcagat tcggttcttc cgtcgggtgt aacgtcgccg 6900

agcagttcgc ttgagtctgg tagcttcggc agtctcgcca gaatcgaaca acagggcacc 6960

aatgaggttt ttcttgatgg agtgtcgatc ggtgtttccg aggaccttga atttcttgga 7020

gggcaccttg tactcgtcgg tgatgacagc ccagccgaca gagttggttc caatgtccag 7080

gccgatggag tatttcttgt ccatggtgtg atgtgtagtt tagatttcga atctgtgggg 7140

aaagaaagga aaaaagagac tggcaaccga ttgggagagc cactgtttat atatacccta 7200

gacaagcccc ccgcttgtaa gatgttggtc aatgtaaacc agtattaagg ttggcaagtg 7260

caggagaagc aaggtgtggg taccgagcaa tggaaatgtg cggaaggcaa aaaaatgagg 7320

ccacggccta ttgtcggggc tatatccagg gggcgattga agtacactaa catgacatgt 7380

gtccacagac cctcaatctg gcctgatgag ccaaatccat acgcgctttc gcagctctaa 7440

aggctataac aagtcacacc accctgctcg acctcagcgc cctcactttt tgttaagaca 7500

aactgtacac gctgttccag cgttttctgc ctgcacctgg tgggacattt ggtgcaacct 7560

aaagtgctcg gaacctctgt ggtgtccaga tcagcgcagc agttccgagg tagttttgag 7620

gcccttagat gatggtttaa accttaagcc cgctcataac ttcgtatagc atacattata 7680

cgaacggtag gttgcgggat agacgccgac ggagggcaat ggcgctatgg aaccttgcgg 7740

atatccatac gccgcggcgg actgcgtccg aaccagctcc agcagcgttt tttccgggcc 7800

attgagccga ctgcgacccc gccaacgtgt cttggcccac gcactcatgt catgttggtg 7860

ttgggaggcc actttttaag tagcacaagg cacctagctc gcagcaaggt gtccgaacca 7920

aagaagcggc tgcagtggtg caaacggggc ggaaacggcg ggaaaaagcc acgggggcac 7980

gaattgaggc acgccctcga atttgagacg agtcacggcc ccattcgccc gcgcaatggc 8040

tcgccaacgc ccggtctttt gcaccacatc aggttacccc aagccaaacc tttgtgttaa 8100

aaagcttaac atattatacc gaacgtaggt ttgggcgggc ttgctccgtc tgtccaaggc 8160

aacatttata taagggtctg catcgccggc tcaattgaat cttttttctt cttctcttct 8220

ctatattcat tcttgaatta aacacacatc aaccatggcc aaaaagcctg aactcaccgc 8280

gacgtctgtc gagaagtttc tgatcgaaaa gttcgacagc gtctccgacc tgatgcagct 8340

ctcggagggc gaagaatctc gtgctttcag cttcgatgta ggagggcgtg gatatgtcct 8400

gcgggtaaat agctgcgccg atggtttcta caaagatcgt tatgtttatc ggcactttgc 8460

atcggccgcg ctcccgattc cggaagtgct tgacattggg gagttcagcg agagcctgac 8520

ctattgcatc tcccgccgtg cacagggtgt cacgttgcaa gacctgcctg aaaccgaact 8580

gcccgctgtt ctgcagccgg tcgcggaggc tatggatgcg atcgctgcgg ccgatcttag 8640

ccagacgagc gggttcggcc cattcggacc gcaaggaatc ggtcaataca ctacatggcg 8700

tgatttcata tgcgcgattg ctgatcccca tgtgtatcac tggcaaactg tgatggacga 8760

caccgtcagt gcgtccgtcg cgcaggctct cgatgagctg atgctttggg ccgaggactg 8820

ccccgaagtc cggcacctcg tgcacgcgga tttcggctcc aacaatgtcc tgacggacaa 8880

tggccgcata acagcggtca ttgactggag cgaggcgatg ttcggggatt cccaatacga 8940

ggtcgccaac atcttcttct ggaggccgtg gttggcttgt atggagcagc agacgcgcta 9000

cttcgagcgg aggcatccgg agcttgcagg atcgccgcgg ctccgggcgt atatgctccg 9060

cattggtctt gaccaactct atcagagctt ggttgacggc aatttcgatg atgcagcttg 9120

ggcgcagggt cgatgcgacg caatcgtccg atccggagcc gggactgtcg ggcgtacaca 9180

aatcgcccgc agaagcgcgg ccgtctggac cgatggctgt gtagaagtac tcgccgatag 9240

tggaaaccga cgccccagca ctcgtccgag ggcaaaggaa tagcggccgc aagtgtggat 9300

ggggaagtga gtgcccggtt ctgtgtgcac aattggcaat ccaagatgga tggattcaac 9360

acagggatat agcgagctac gtggtggtgc gaggatatag caacggatat ttatgtttga 9420

cacttgagaa tgtacgatac aagcactgtc caagtacaat actaaacata ctgtacatac 9480

tcatactcgt acccgggcaa cggtttcact tgagtgcagt ggctagtgct cttactcgta 9540

cagtgtgcta ccgttcgtat agcatacatt atacgaagtt atcatagtct taattaagcc 9600

atcatagtat agtggttagt acaccacgtt gtggagtgta cttcagttca taacaagccc 9660

gtggaaacct cagttcgatt ctgggtgatg gcattcaaac gattacccac cctcgtttta 9720

gagctagaaa tagcaagtta aaataaggct agtccgttat caacttgaaa aagtggcacc 9780

gagtcggtgc tttttttttt gttttttatc gattgagcat ccgttgattt ccgaacagat 9840

cccaatatta cacccaagta gcatgcataa gctaaaagta actcgcagcg cacaccgtgc 9900

agattcataa gtctatgatt aattgaacgc caataacccg gcttactaca agtacaagta 9960

ggtatacata gcggtaatga atcattagaa aaaaaaaaaa caaaaaaaaa caaaacaaac 10020

tgttgtggat gcatcaacag tagtacatag ttgtacgatg tacttgtact tgtaaaagca 10080

aaaatgtaca atatctcagg gagcgcaact tttacgttcg aagaacaatg taccgcatac 10140

cgcattctag attctgcgga acgtctaacc tggaaatacg attttttttt tctttcattt 10200

tttttgcttc ttcaaaagta tggtaatttc ctaccattac agttgacact gaacgagggg 10260

ggattgaatt taagcaaaaa attaaatcaa aataccttta tgtatccagc ccatgtaata 10320

aacaaaagga ttatataaca agaaataaat atataccttt aatggatcat tagaataaaa 10380

ataaatacga gaagcacacc agagaagctt tttgattgcc actataccgc tactttggta 10440

tatcttatta taattgttga atttgcaaga tagaatgtca ttcattggag agaaatccaa 10500

ggaatatgtg ggatgaaatg actagaagta tgaacaatga gaatagtaca tacttgtacc 10560

tgtatttcta gaagagagaa agacagttga gtgtgtgatt ctcgtccaat aataatctca 10620

atagtaacgt gtgaatagct gttctttgat agttgatatt tctcgatgac tatttatgtt 10680

gtacaaggga tttttttcgt tgctgttgat ttcgaattag gcaatgcaga tatcatttat 10740

gctatccata tttaagattt cccatacgca tttataacat ttattctaca taaattgtta 10800

aatgaacgaa ctgccattat aaattgtttc ctaaatagga agtgtttttc ataaagcaag 10860

taagttgtct aataatacta agtaataaaa ataagttcat acaatatatt ttgagaacat 10920

catttggagg cggtagatgg agtctgttta ttattaaaca atgcgagatg accccttaaa 10980

tattgagaac atcagttgga ggcggcagat ggagtctgtc tatttagcaa tgggacatga 11040

ctgtcagtat catcatgatg tatatatata atacatataa tattatataa cacgattttt 11100

ttaaattatt ggcccgaaaa ttaatcagtg tagactggat ctcggcagtc tctcggatgt 11160

agaattaggt ttccttgagg cgaagatcgg tttgtgtgac atgaattcga tatcaagctt 11220

atcgacacca tcgacctcga gggggggccc ggtacccaat tcgccctata gtgagtcgta 11280

ttacaattca ctggccgtcg ttttacaacg tcgtgactgg gaaaaccctg gcgttaccca 11340

acttaatcgc cttgcagc 11358

<210> 132
<211> 694
<212> DNA
<213> Artificial Sequence

<220>
<223> Artificial Sequence

<400> 132
ttaaattttt tttgattttc ttttttgacc ccgtcttcaa ttacacttcc caactgggaa 60

cacccctctt tatcgaccca ttttaggtaa tttaccctag cccattgtct ccataaggaa 120

tattacccta acccacagtc cagggtgccc aggtccttct ttggccaaat tttaacttcg 180

gtcctatggc acagcggtag cgcgtgagat tgcaaatctt aaggtcccga gttcgaatct 240

cggtgggacc tagttatttt tgatagataa tttcgtgatg attagaaact taacgcaaaa 300

taatgcctgg ctagctcaat cggtagagcg tgagactctt atacaagaaa tctcaaggct 360

gtgggttcaa gccccacgtc gggctagccg ctcgagtgct caagctcggt tttagagcta 420

gaaatagcaa gttaaaataa ggctagtccg ttatcaactt gaaaaagtgg caccgagtcg 480

gtgctccgat atagtgtagg ggctatcaca tcacgctctc atcaagaagt cttcttgaga 540

accgtggaga ccggggttcg attccccgta tcggagttca aacgattacc caccctcgtt 600

ttagagctag aaatagcaag ttaaaataag gctagtccgt tatcaacttg aaaaagtggc 660

accgagtcgg tgcttttttt tttgtttttt atcg 694

<210> 133
<211> 11834
<212> DNA
<213> Artificial Sequence

<220>
<223> Artificial Sequence

<400> 133
attgagcatc cgttgatttc cgaacagatc ccaatattac acccaagtag catgcataag 60

ctaaaagtaa ctcgcagcgc acaccgtgca gattcataag tctatgatta attgaacgcc 120

aataacccgg cttactacaa gtacaagtag gtatacatag cggtaatgaa tcattagaaa 180

aaaaaaaaac aaaaaaaaac aaaacaaact gttgtggatg catcaacagt agtacatagt 240

tgtacgatgt acttgtactt gtaaaagcaa aaatgtacaa tatctcaggg agcgcaactt 300

ttacgttcga agaacaatgt accgcatacc gcattctaga ttctgcggaa cgtctaacct 360

ggaaatacga tttttttttt ctttcatttt ttttgcttct tcaaaagtat ggtaatttcc 420

taccattaca gttgacactg aacgaggggg gattgaattt aagcaaaaaa ttaaatcaaa 480

atacctttat gtatccagcc catgtaataa acaaaaggat tatataacaa gaaataaata 540

tataccttta atggatcatt agaataaaaa taaatacgag aagcacacca gagaagcttt 600

ttgattgcca ctataccgct actttggtat atcttattat aattgttgaa tttgcaagat 660

agaatgtcat tcattggaga gaaatccaag gaatatgtgg gatgaaatga ctagaagtat 720

gaacaatgag aatagtacat acttgtacct gtatttctag aagagagaaa gacagttgag 780

tgtgtgattc tcgtccaata ataatctcaa tagtaacgtg tgaatagctg ttctttgata 840

gttgatattt ctcgatgact atttatgttg tacaagggat ttttttcgtt gctgttgatt 900

tcgaattagg caatgcagat atcatttatg ctatccatat ttaagatttc ccatacgcat 960

ttataacatt tattctacat aaattgttaa atgaacgaac tgccattata aattgtttcc 1020
taaataggaa gtgtttttca taaagcaagt aagttgtcta ataatactaa gtaataaaaa 1080
taagttcata caatatattt tgagaacatc atttggaggc ggtagatgga gtctgtttat 1140
tattaaacaa tgcgagatga ccccttaaat attgagaaca tcagttggag gcggcagatg 1200
gagtctgtct atttagcaat gggacatgac tgtcagtatc atcatgatgt atatatataa 1260
tacatataat attatataac acgatttttt taaattattg gcccgaaaat taatcagtgt 1320
agactggatc tcggcagtct ctcggatgta gaattaggtt tccttgaggc gaagatcggt 1380
ttgtgtgaca tgaattcgat atcaagctta tcgacaccat cgacctcgag ggggggcccg 1440
gtacccaatt cgccctatag tgagtcgtat tacaattcac tggccgtcgt tttacaacgt 1500
cgtgactggg aaaaccctgg cgttacccaa cttaatcgcc ttgcagcaca tccccctttc 1560
gccagctggc gtaatagcga agaggcccgc accgatcgcc cttcccaaca gttgcgcagc 1620
ctgaatggcg aatggaaatt gtaagcgtta atattttgtt aaaattcgcg ttaaattttt 1680
gttaaatcag ctcatttttt aaccaatagg ccgaaatcgg caaaatccct tataaatcaa 1740
aagaatagac cgagataggg ttgagtgttg ttccagtttg gaacaagagt ccactattaa 1800
agaacgtgga ctccaacgtc aaagggcgaa aaaccgtcta tcagggcgat ggcccactac 1860
gtgaaccatc accctaatca agttttttgg ggtcgaggtg ccgtaaagca ctaaatcgga 1920
accctaaagg gagcccccga tttagagctt gacggggaaa gccggcgaac gtggcgagaa 1980
aggaagggaa gaaagcgaaa ggagcgggcg ctagggcgct ggcaagtgta gcggtcacgc 2040
tgcgcgtaac caccacaccc gccgcgctta atgcgccgct acagggcgcg tcaggtggca 2100
cttttcgggg aaatgtgcgc ggaaccccta tttgtttatt tttctaaata cattcaaata 2160
tgtatccgct catgagacaa taaccctgat aaatgcttca ataatattga aaaaggaaga 2220
gtatgagtat tcaacatttc cgtgtcgccc ttattccctt ttttgcggca ttttgccttc 2280
ctgtttttgc tcacccagaa acgctggtga aagtaaaaga tgctgaagat cagttgggtg 2340
cacgagtggg ttacatcgaa ctggatctca acagcggtaa gatccttgag agttttcgcc 2400
ccgaagaacg ttttccaatg atgagcactt ttaaagttct gctatgtggc gcggtattat 2460
cccgtattga cgccgggcaa gagcaactcg gtcgccgcat acactattct cagaatgact 2520
tggttgagta ctcaccagtc acagaaaagc atcttacgga tggcatgaca gtaagagaat 2580
tatgcagtgc tgccataacc atgagtgata acactgcggc caacttactt ctgacaacga 2640
tcggaggacc gaaggagcta accgcttttt tgcacaacat gggggatcat gtaactcgcc 2700
ttgatcgttg ggaaccggag ctgaatgaag ccataccaaa cgacgagcgt gacaccacga 2760
tgcctgtagc aatggcaaca acgttgcgca aactattaac tggcgaacta cttactctag 2820
cttcccggca acaattaata gactggatgg aggcggataa agttgcagga ccacttctgc 2880

gctcggccct tccggctggc tggtttattg ctgataaatc tggagccggt gagcgtgggt 2940

ctcgcggtat cattgcagca ctggggccag atggtaagcc ctcccgtatc gtagttatct 3000

acacgacggg gagtcaggca actatggatg aacgaaatag acagatcgct gagataggtg 3060

cctcactgat taagcattgg taactgtcag accaagttta ctcatatata ctttagattg 3120

atttaaaact tcatttttaa tttaaaagga tctaggtgaa gatccttttt gataatctca 3180

tgaccaaaat cccttaacgt gagttttcgt tccactgagc gtcagacccc gtagaaaaga 3240

tcaaaggatc ttcttgagat cctttttttc tgcgcgtaat ctgctgcttg caaacaaaaa 3300

aaccaccgct accagcggtg gtttgtttgc cggatcaaga gctaccaact ctttttccga 3360

aggtaactgg cttcagcaga gcgcagatac caaatactgt ccttctagtg tagccgtagt 3420

taggccacca cttcaagaac tctgtagcac cgcctacata cctcgctctg ctaatcctgt 3480

taccagtggc tgctgccagt ggcgataagt cgtgtcttac cgggttggac tcaagacgat 3540

agttaccgga taaggcgcag cggtcgggct gaacgggggg ttcgtgcaca cagcccagct 3600

tggagcgaac gacctacacc gaactgagat acctacagcg tgagctatga gaaagcgcca 3660

cgcttcccga agggagaaag gcggacaggt atccggtaag cggcagggtc ggaacaggag 3720

agcgcacgag ggagcttcca gggggaaacg cctggtatct ttatagtcct gtcgggtttc 3780

gccacctctg acttgagcgt cgatttttgt gatgctcgtc aggggggcgg agcctatgga 3840

aaaacgccag caacgcggcc tttttacggt tcctggcctt ttgctggcct tttgctcaca 3900

tgttctttcc tgcgttatcc cctgattctg tggataaccg tattaccgcc tttgagtgag 3960

ctgataccgc tcgccgcagc cgaacgaccg agcgcagcga gtcagtgagc gaggaagcgg 4020

aagagcgccc aatacgcaaa ccgcctctcc ccgcgcgttg gccgattcat taatgcagct 4080

ggcacgacag gtttcccgac tggaaagcgg gcagtgagcg caacgcaatt aatgtgagtt 4140

agctcactca ttaggcaccc caggctttac actttatgct tccggctcgt acgcaactaa 4200

catgaatgaa tacgatatac atcaaagact atgatacgca gtattgcaca ctgtacgagt 4260

aagagcacta gccactgcac tcaagtgaaa ccgttgcccg ggtacgagta tgagtatgta 4320

cagtatgttt agtattgtac ttggacagtg cttgtatcgt acattctcaa gtgtcaaaca 4380

taaatatccg ttgctatatc ctcgcaccac cacgtagctc gctatatccc tgtgttgaat 4440

ccatccatct tggattgcca attgtgcaca cagaaccggg cactcacttc cccatccaca 4500

cttgcggccg cttagacctt tcgctttttc ttgggatcgg ctctggagtc gccaccaagc 4560

tgagacaggt cgattcgggt ctcgtacagg ccagtgatgg actggtgaat cagggtggca 4620

tcgagaacct ccttggtgga tgtgtaccgc tttcggtcga tagtggtatc gaagtacttg 4680

aaagctgcag gagcacccag gttggtaaga gtaaacaggt gaatgatgtt ctccgcctgt 4740

tctcgaatgg gtttgtcccg atgcttgttg taggcagaga gcaccttgtc caagttggca 4800

tcagccagga tgactcgctt cgaaaactcg gaaatctgct cgataatctc gtcgaggtaa 4860

tgtttgtgct gctcaacgaa gagttgcttc tgttcgttgt cctcgggaga acccttgagc 4920

ttctcgtagt gagaagccag atagagaaag ttgacgtact tcgaaggcaa ggcaagctcg 4980

tttcccttct gcagctcgcc agcggaggcg agcatacgct ttcgaccgtt ctccagttcg 5040

aacagagagt acttgggcag cttgataatg aggtctttct tgacctcctt gtaacccttg 5100

gcttccaaga agtcgatggg attcttctcg aagctcgatc gctccatgat ggtaattccg 5160

agcagctcct tgacggactt gagctttttg gacttgccct tctcgacctt cgcaacgaca 5220

agcacggaat aggcgacggt aggagaatcg aagccaccgt atttcttggg atcccagtct 5280

ttctttcgag cgatgagctt gtcggagttt cgcttgggca gaatcgactc cttggagaat 5340

ccgccagtct gaacctcggt tttcttgacg atgttgacct gaggcatcga cagaaccttt 5400

cgcacggttg caaagtctcg acccttgtcc cacacgatct ctccagtttc gccgttggtc 5460

tcgataagtg gtctctttcg aatctctccg ttggccaagg tgatctcggt cttgaaaaag 5520

ttcatgatgt tggagtaaaa gaagtacttg gcagtagcct tgccaatctc ctgttcggac 5580

ttggcaatca tctttcgaac gtcgtagacc ttgtaatcgc cgtaaacgaa ctcgctttcg 5640

agcttggggt atttcttgat gagcgcagtg ccaacgacgg cgttgaggta agcatcgtgg 5700

gcatggtggt aattgttgat ctctcgcacc ttgtagaact gaaagtcctt tcggaaatcg 5760

gagaccagtt tggacttgag agtaatcacc ttgacctctc ggatgagctt gtcgttctcg 5820

tcgtacttgg tgttcatccg agaatcgaga atctgtgcga cgtgctttgt gatctgtctg 5880

gtctcgacga gttgacgctt gatgaagcca gccttgtcga gctcggacag accgcctcgc 5940

tcggccttgg taagattgtc gaactttcgc tgggtaatga gcttggcgtt gagcagctgt 6000

cgccagtagt tcttcatctt tttgaccacc tcttcgctgg gaacgttgtc cgacttgcct 6060

ctgttcttgt cggatcgtgt aaggaccttg ttgtcgatag aatcgtcctt gagaaaggat 6120

tgagggacaa tgtggtccac atcgtagtcg ctgagacgat tgatgtccag ttcctgatcc 6180

acgtacatgt ctcgaccatt ctgcagatag tagagataca gcttctcgtt ctgcagttga 6240

gtgttctcga cgggatgctc cttgagaatc tgggatccca gctccttgat gccttcctcg 6300

attcgcttca tccgctctcg cgagtttttc tgaccctttt gagttgtctg gttctctctg 6360

gccatctcga tcacaatgtt ctcgggcttg tgacgtccca tgaccttcac cagctcgtcg 6420

acaaccttga cagtctggag aatgcctttc ttgatggctg gcgaaccagc caggttggca 6480

atatgttcgt gcaagctgtc gccctgaccg gacacttgtg ccttctggat gtcctccttg 6540

aaggtaagag aatcgtcgtg aatgagctgc atgaagtttc ggttggcaaa gccatcggac 6600

ttgagaaagt ccagaatggt ctttccggac tgcttgtctc tgatgccgtt gatgagcttt 6660

cgcgaaagtc ttccccagcc ggtgtatcta cgtcgcttga gttgtttcat gaccttgtcg 6720

tcgaacaggt gagcgtatgt cttgagtcgt tcctcgatca tctcccgatc ttcgaacagg 6780

gtaagagtga gcacgatgtc ctccagaatg tcctcgtttt cctcgttgtc gagaaaatcc 6840

ttgtccttga taatcttgag cagatcgtga taggtgccca aagaggcgtt gaatcggtcc 6900

tcaactccgg aaatctcgac gctgtcgaaa cactcgattt tcttgaagta gtcctccttg 6960

agctgcttaa cagtgacctt tcggttggtc ttgaacagga gatcgacaat ggctttcttc 7020

tgttcgccag acaagaaggc aggctttcgc attccctcgg taacgtactt gactttggtg 7080

agttcgttgt agactgtaaa gtactcgtag agcagcgaat gcttgggaag aaccttctcg 7140

ttgggcagat tcttgtcgaa gttggtcatt cgctcgatga aggactgtgc agaggcaccc 7200

ttgtccacga cttcctcgaa gttccaggga gtgatggttt cctcggactt tcgagtcatc 7260

caagcaaatc gagagtttcc tctggcaaga ggaccaacat agtaggggat tcgaaaggta 7320

agaatcttct cgatcttctc tcggttgtcc ttgagaaagg ggtagaagtc ttcctgacgt 7380

cgaagaatgg cgtgcagctc accgaggtgg atctgatgag gaatgctgcc gttgtcgaag 7440

gttcgttgct tccgaagcag atcctctcga ttgagcttga caagcagttc ctcggttccg 7500

tccatcttct cgagaattgg cttgatgaac ttgtagaact cttcctgaga ggctccgccg 7560

tcgatgtatc cagcgtagcc gttcttcgac tgatcgaaaa agatctcctt gtacttctcg 7620

ggcagttgct gtcggacaag agccttgagc agtgtgagat cctgatggtg ctcgtcgtat 7680

cgcttgatca tggaggcaga aaggggagcc tttgtgatct cggtgttgac tcgcagaatg 7740

tcagacaaga gaatagcatc cgaaaggttc ttggcagcga gaaacaggtc ggcgtactga 7800

tcgccaatct gtgcaagcag gttgtcgagg tcatcgtcgt aggtgtcctt ggacagctgg 7860

agcttggcgt cctccgccag atcgaagttg gacttgaagt tgggtgtgag accaagagaa 7920

agggcaatga ggttgccaaa cagtccgttc tttttctcgc caggaagttg ggcaatgagg 7980

ttctccagtc gtctgctctt cgagagtcga gcagacaaga tggcctttgc atcgactccg 8040

gaggcattga tggggttttc ctcgaacagc tggttgtagg tctgaacgag ctgaatgaac 8100

agcttgtcca catcgctgtt gtcgggattg agatcgccct cgatgaggaa atgacctcga 8160

aacttgatca tgtgtgccag agcgaggtag ataagtctga gatccgcctt gtcggtggaa 8220

tcgacgagtt tctttcggag atggtagatg gtaggatact tctcgtggta agcaacctcg 8280

tccacaatgt tgccaaagat gggatgacgc tcgtgtttct tgtcttcctc gacgaggaag 8340

gattcctcca gtcgatgaaa gaacgaatcg tccaccttgg ccatctcgtt ggaaaagatc 8400

tcctgcaggt agcagattcg gttcttccgt cgggtgtaac gtcgccgagc agttcgcttg 8460

agtctggtag cttcggcagt ctcgccagaa tcgaacaaca gggcaccaat gaggtttttc 8520

ttgatggagt gtcgatcggt gtttccgagg accttgaatt tcttggaggg caccttgtac 8580

tcgtcggtga tgacagccca gccgacagag ttggttccaa tgtccaggcc gatggagtat 8640

ttcttgtcca tggtgtgatg tgtagtttag atttcgaatc tgtggggaaa gaaaggaaaa 8700

aagagactgg caaccgattg ggagagccac tgtttatata taccctagac aagccccccg 8760

cttgtaagat gttggtcaat gtaaaccagt attaaggttg gcaagtgcag gagaagcaag 8820

gtgtgggtac cgagcaatgg aaatgtgcgg aaggcaaaaa aatgaggcca cggcctattg 8880

tcggggctat atccaggggg cgattgaagt acactaacat gacatgtgtc cacagaccct 8940

caatctggcc tgatgagcca aatccatacg cgctttcgca gctctaaagg ctataacaag 9000

tcacaccacc ctgctcgacc tcagcgccct cactttttgt taagacaaac tgtacacgct 9060

gttccagcgt tttctgcctg cacctggtgg gacatttggt gcaacctaaa gtgctcggaa 9120

cctctgtggt gtccagatca gcgcagcagt tccgaggtag ttttgaggcc cttagatgat 9180

ggtttaaacc ttaagcccgc tcataacttc gtatagcata cattatacga acggtaggtt 9240

gcgggataga cgccgacgga gggcaatggc gctatggaac cttgcggata tccatacgcc 9300

gcggcggact gcgtccgaac cagctccagc agcgtttttt ccgggccatt gagccgactg 9360

cgaccccgcc aacgtgtctt ggcccacgca ctcatgtcat gttggtgttg ggaggccact 9420

ttttaagtag cacaaggcac ctagctcgca gcaaggtgtc cgaaccaaag aagcggctgc 9480

agtggtgcaa acggggcgga aacggcggga aaaagccacg ggggcacgaa ttgaggcacg 9540

ccctcgaatt tgagacgagt cacggcccca ttcgcccgcg caatggctcg ccaacgcccg 9600

gtcttttgca ccacatcagg ttaccccaag ccaaaccttt gtgttaaaaa gcttaacata 9660

ttataccgaa cgtaggtttg ggcgggcttg ctccgtctgt ccaaggcaac atttatataa 9720

gggtctgcat cgccggctca attgaatctt ttttcttctt ctcttctcta tattcattct 9780

tgaattaaac acacatcaac catggccaaa aagcctgaac tcaccgcgac gtctgtcgag 9840

aagtttctga tcgaaaagtt cgacagcgtc tccgacctga tgcagctctc ggagggcgaa 9900

gaatctcgtg ctttcagctt cgatgtagga gggcgtggat atgtcctgcg ggtaaatagc 9960

tgcgccgatg gtttctacaa agatcgttat gtttatcggc actttgcatc ggccgcgctc 10020

ccgattccgg aagtgcttga cattggggag ttcagcgaga gcctgaccta ttgcatctcc 10080

cgccgtgcac agggtgtcac gttgcaagac ctgcctgaaa ccgaactgcc cgctgttctg 10140

cagccggtcg cggaggctat ggatgcgatc gctgcggccg atcttagcca gacgagcggg 10200

ttcggcccat tcggaccgca aggaatcggt caatacacta catggcgtga tttcatatgc 10260

gcgattgctg atccccatgt gtatcactgg caaactgtga tggacgacac cgtcagtgcg 10320

tccgtcgcgc aggctctcga tgagctgatg ctttgggccg aggactgccc cgaagtccgg 10380

cacctcgtgc acgcggattt cggctccaac aatgtcctga cggacaatgg ccgcataaca 10440

gcggtcattg actggagcga ggcgatgttc ggggattccc aatacgaggt cgccaacatc 10500

ttcttctgga ggccgtggtt ggcttgtatg gagcagcaga cgcgctactt cgagcggagg 10560

catccggagc ttgcaggatc gccgcggctc cgggcgtata tgctccgcat tggtcttgac 10620

caactctatc agagcttggt tgacggcaat ttcgatgatg cagcttgggc gcagggtcga 10680

tgcgacgcaa tcgtccgatc cggagccggg actgtcgggc gtacacaaat cgcccgcaga 10740

agcgcggccg tctggaccga tggctgtgta gaagtactcg ccgatagtgg aaaccgacgc 10800

cccagcactc gtccgagggc aaaggaatag cggccgcaag tgtggatggg gaagtgagtg 10860

cccggttctg tgtgcacaat tggcaatcca agatggatgg attcaacaca gggatatagc 10920

gagctacgtg gtggtgcgag gatatagcaa cggatattta tgtttgacac ttgagaatgt 10980

acgatacaag cactgtccaa gtacaatact aaacatactg tacatactca tactcgtacc 11040

cgggcaacgg tttcacttga gtgcagtggc tagtgctctt actcgtacag tgtgctaccg 11100

ttcgtatagc atacattata cgaagttatc atagtcttaa ttaaattttt tttgattttc 11160

ttttttgacc ccgtcttcaa ttacacttcc caactgggaa cacccctctt tatcgaccca 11220

ttttaggtaa tttaccctag cccattgtct ccataaggaa tattacccta acccacagtc 11280

cagggtgccc aggtccttct ttggccaaat tttaacttcg gtcctatggc acagcggtag 11340

cgcgtgagat tgcaaatctt aaggtcccga gttcgaatct cggtgggacc tagttatttt 11400

tgatagataa tttcgtgatg attagaaact taacgcaaaa taatgcctgg ctagctcaat 11460

cggtagagcg tgagactctt atacaagaaa tctcaaggct gtgggttcaa gccccacgtc 11520

gggctagccg ctcgagtgct caagctcggt tttagagcta gaaatagcaa gttaaaataa 11580

ggctagtccg ttatcaactt gaaaaagtgg caccgagtcg gtgctccgat atagtgtagg 11640

ggctatcaca tcacgctctc atcaagaagt cttcttgaga accgtggaga ccggggttcg 11700

attccccgta tcggagttca aacgattacc caccctcgtt ttagagctag aaatagcaag 11760

ttaaaataag gctagtccgt tatcaacttg aaaaagtggc accgagtcgg tgcttttttt 11820

tttgtttttt atcg 11834

## Description

### CROSS REFERENCE TO RELATED APPLICATIONS

This application claims the benefit of U.S. Provisional Application No. 62/269,121, filed December 18, 2015, which is hereby incorporated by referenced in its entirety.

### FIELD

The disclosure relates to the field of molecular biology, in particular, to methods for producing guide RNAs and methods for altering the genome of a cell.

### REFERENCE TO SEQUENCE LISTING SUBMITTED ELECTRONICALLY

The official copy of the sequence listing is submitted electronically via EFS-Web as an ASCII formatted sequence listing with a file named CL6507-WO-PCT_SequenceListing.txt created on November 11, 2016 and having a size 418 kilobytes and is filed concurrently with the specification. The sequence listing contained in this ASCII formatted document is part of the specification and is herein incorporated by reference in its entirety.

### BACKGROUND

Recombinant DNA technology has made it possible to insert DNA sequences at targeted genomic locations and/or modify (edit) specific endogenous chromosomal sequences, thus altering the organism's phenotype. Site-specific integration techniques, which employ site-specific recombination systems, as well as other types of recombination technologies, have been used to generate targeted insertions of genes of interest in a variety of organism. Genome-editing techniques such as designer zinc finger nucleases (ZFNs), transcription activator-like effector nucleases (TALENs), homing meganucleases, engineered nucleases are available for producing targeted genome perturbations, but these systems tend to have a low specificity and employ designed nucleases that need to be redesigned for each target site, which renders them costly and time-consuming to prepare. CRISPR-associated (Cas) RNA-guided endonuclease systems have been developed as a means for introducing site-specific DNA strand breaks at specific target sites. These nuclease based systems can create a single strand or double strand break (DSB) in a target nucleotide, which can increase the frequency of homologous recombination at the target locus.

Inhibition of gene expression can be accomplished, for example, by interrupting or deleting the DNA sequence of the gene, resulting in "knock-out" of the gene. Gene knock-outs mostly have been carried out through homologous recombination (HR), a technique applicable across a wide array of organisms from bacteria to mammals. Another tool for studying gene function can be through genetic "knock-in", which is also usually performed by HR. HR for purposes of gene targeting (knock-out or knock-in) can use the presence of an exogenously supplied DNA having homology with the target site. Although gene targeting by HR is a powerful tool, it can be a complex, labor-intensive procedure. Most studies using HR have generally been limited to knock-out of a single gene rather than multiple genes in a pathway, since HR is generally difficult to scale-up in a cost-effective manner. This difficulty is exacerbated in organisms in which HR is not efficient. Such low efficiency typically forces practitioners to rely on selectable phenotypes or exogenous markers to help identify cells in which a desired HR event occurred.

Thus there remains a need for new and more efficient genome engineering technologies that are affordable, easy to set up, scalable, and amenable to targeting multiple positions within the genome of an organism.

### BRIEF SUMMARY

Compositions and methods are provided for editing nucleotides and/or altering target sites in the genome of a cell. The methods and compositions employ a recombinant DNA construct comprising a tRNA promoter operably linked to a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a cell such as a microbial cell. The present disclosure further describes methods and compositions employing a recombinant DNA construct comprising a tRNA promoter operably linked to a spacer sequence and a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast.

In one embodiment of the disclosure, the disclosure comprises a recombinant DNA construct comprising a tRNA promoter operably linked to a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast. The tRNA promoter is selected from the group consisting of a tRNA or a tRNA fragment capable of functioning as a promoter sequence. The tRNA can be selected from the group consisting of a tRNA-Lys, tRNA-Val, tRNA-Glu, tRNA Leu, tRNA-ile, tRNA-trp, tRNA-tyr, tRNA-his, or any one combination thereof. The tRNA fragment can be selected from the group consisting of a polynucleotide comprising the S-, D-, A-, V-, and T- domains of a tRNA, a polynucleotide comprising the S-, D-, V-, and T-domains of the tRNA, a polynucleotide comprising the S-, D-, and T- domains of the tRNA, and a polynucleotide comprising the S-, and T- domains of the tRNA.

In one embodiment of the disclosure, the disclosure comprises a recombinant DNA construct comprising a tRNA promoter operably linked to a spacer sequence and a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast. The spacer sequence can be a DNA sequence encoding a polynucleotide selected from the group consisting of a polynucleotide comprising a S-, D-, A-, V-, and T- domains of a tRNA, a polynucleotide comprising the S-, D-, V-, and T- domains of the tRNA, a polynucleotide comprising the S-, D-, and T- domains of the tRNA, and a polynucleotide comprising the S-, and T- domains of the tRNA.

Also provided is a non-conventional yeast comprising any one of the recombinant DNA constructs described herein. The non-conventional yeast can be a member of a genus selected from the group consisting of *Yarrowia, Pichia, Schwanniomyces, Kluyveromyces, Arxula, Trichosporon, Candida, Ustilago, Torulopsis, Zygosaccharomyces, Trigonopsis, Cryptococcus, Rhodotorula, Phaffia, Sporobolomyces,* and *Pachysolen*
In one embodiment of the disclosure, the method comprises a method for modifying a target site on a chromosome or episome in a non-conventional yeast, the method comprising providing to a non-conventional yeast at least a first recombinant DNA construct as described herein and a second recombinant DNA construct encoding a Cas endonuclease, wherein the Cas endonuclease introduces a single or double-strand break at said target site. The method can further comprise comprising identifying at least one non-conventional yeast cell that has a modification at said target site, wherein the modification includes at least one deletion, addition or substitution of one or more nucleotides in said target site.

These methods can further comprise identifying the mutation efficiency in said non-conventional yeasts. In one embodiment, the mutation efficiency can be least 2, 3, 4, 5, 6, 7, 8, 9, 10, 20 fold higher compared to a method for modifying a target site in said non-conventional yeast utilizing a ribozyme linked single guide RNA.

In one embodiment of the disclosure, the method comprises a method for editing a nucleotide sequence on a chromosome or episome in a non-conventional yeast, the method comprising providing to a non-conventional yeast a polynucleotide modification template DNA, a first recombinant DNA construct comprising a DNA sequence encoding a Cas endonuclease, and a second recombinant DNA construct described herein, wherein the Cas9 endonuclease introduces a single or double-strand break at a target site in the chromosome or episome of said yeast, wherein said polynucleotide modification template DNA comprises at least one nucleotide modification of said nucleotide sequence.

In one embodiment of the disclosure, the disclose comprises a method for a recombinant DNA construct comprising a promoter operably linked to a spacer sequence and a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast. The spacer sequence can be a DNA sequence encoding a polynucleotide selected from the group consisting of a polynucleotide comprising a S-, D-, A-, V-, and T- domains of a tRNA, a polynucleotide comprising the S-, D-, V-, and T- domains of the tRNA, a polynucleotide comprising the S-, D-, and T- domains of the tRNA, and a polynucleotide comprising the S-, and T- domains of the tRNA. The promoter can be a RNA Polymerase II or RNA polymerase III promoter.

In one embodiment of the disclosure, the method comprises a method for modifying multiple target sites on a chromosome or episome in a non-conventional yeast, the method comprising providing to a non-conventional yeast at least a first recombinant DNA construct comprising a DNA sequence encoding a Cas endonuclease, and at least a second recombinant DNA construct comprising a promoter operably linked to a sequence comprising more than one tRNA-guideRNA cassettes encoding more than one tRNA-guideRNAs targeting multiple target sites in the genome of said non-conventional yeast, wherein the Cas9 endonuclease introduces a single or double-strand break at each of said multiple target sites.

Also provided are nucleic acid constructs, microbial cells, produced by the methods described herein. Additional embodiments of the methods and compositions of the present disclosure are shown herein.

### BRIEF DESCRIPTION OF THE DRAWINGS AND THE SEQUENCE LISTING

The disclosure can be more fully understood from the following detailed description and the accompanying drawings and Sequence Listing, which form a part of this application. The sequence descriptions and sequence listing attached hereto comply with the rules governing nucleotide and amino acid sequence disclosures in patent applications as set forth in 37 C.F.R. §§1.821-1.825. The sequence descriptions contain the three letter codes for amino acids as defined in 37 C.F.R. §§ 1.821-1.825, which are incorporated herein by reference.

### Figures

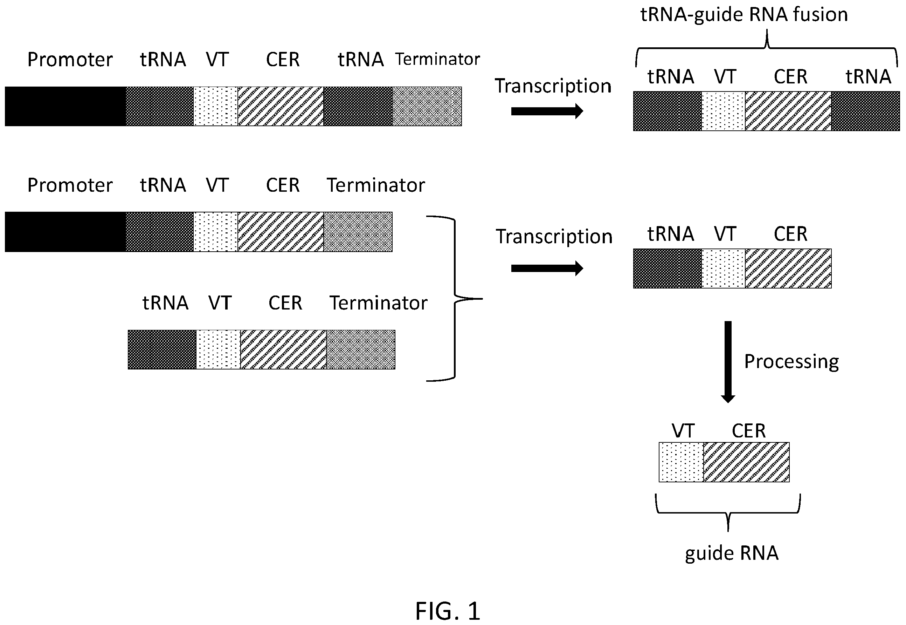

**Figure 1** depicts the structure of three tRNA-gRNA expression cassettes (SEQ ID NOs: 27, 101, and 34) listed to the left. Where applicable, the cassette is composed of a promoter (shown in solid black), DNA encoding tRNA (shown in solid gray), DNA encoding a variable targeting domain (shown in black dots), DNA encoding a CER domain (shown in horizontal line) and a transcriptional terminator (shown in dot fill). Following transcription of the tRNA-gRNA expression cassette, host proteins process the premature tRNA-gRNA fusion transcript giving rise to a functional single guide RNA (sgRNA)

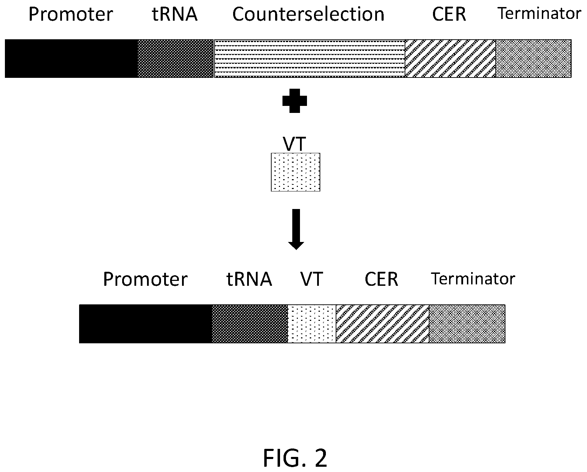

**Figure 2** depicts the structure of a high throughput tRNA-gRNA expression cassette. The cassette is composed of a promoter (shown in solid black), a tRNA (shown in solid gray), a counterselection marker flanked by two restriction sites (shown in horizontal fill), a DNA encoding the CER (shown in horizontal line fill) and a transcriptional terminator (shown in dot fill). When a DNA duplex, containing a DNA encoding a variable targeting domain with the correct overhanging ends (VT, shown as vertical stripe fill), is mixed with a plasmid containing an expression cassette in the presence of restriction enzymes and DNA ligase, the counterselection cassette (horizontal stripe fill) can be replaced by the VT domain (Vertical stripe). These events can be selected *in vitro* by selecting for the absence of the counter selection cassette. The product is a functional tRNA-gRNA expression cassette.

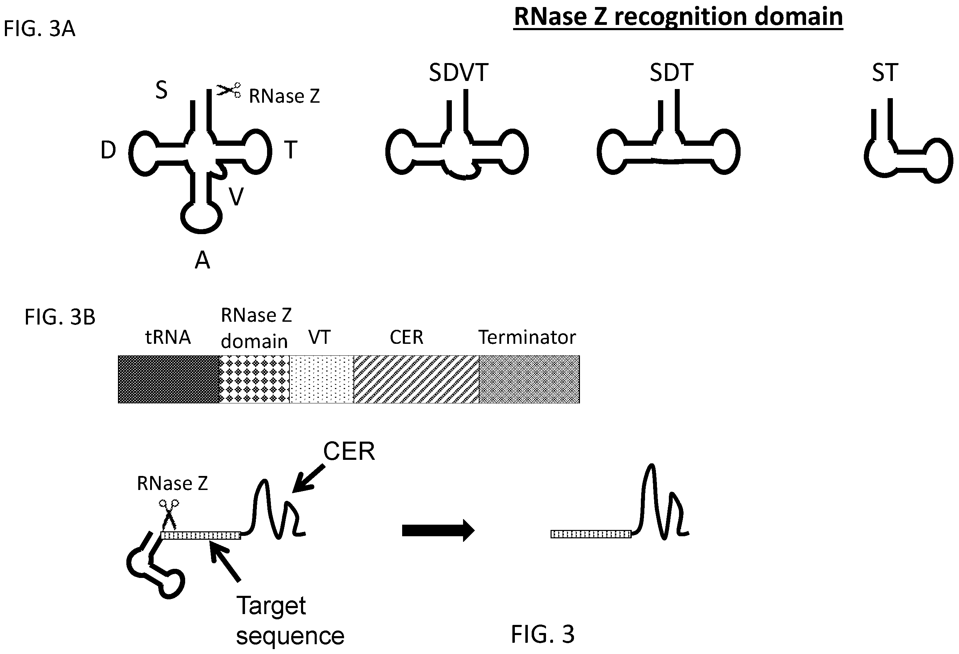

**Figure 3A** depicts the structure for various RNase Z recognition domains derived from a tRNA; S, D, A, V and T refer to the common names for subdomains of tRNA. S refers to the acceptor stem; D refers to the diuridine RM; A refers to the anticodon arm; V refers to the variable loop; T refers to the TΨC arm.
**Figure 3B** depicts cleavage between the ST RNase Z domain and the tRNA leading to functional gRNA-CER.

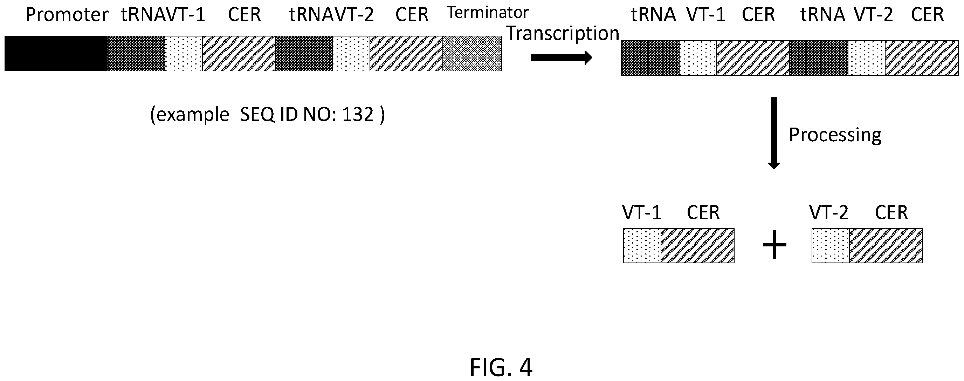

**Figure 4** depicts the structure of tRNA-gRNA-tRNA-gRNA expression cassette that enables the production of multiple functional gRNAs (multiplexing). The cassette is composed of a promoter (shown in solid black), DNA encoding tRNAs (shown in solid gray), DNA encoding two variable targeting domains (VR-1 and VR-2; shown in black dots), DNA encoding CER domains (shown in horizontal line) and a transcriptional terminator (shown in dot fill). Following transcription of the tRNA-gRNA-tRNA-gRNA expression cassette, host proteins process the premature tRNA-gRNA-tRNA-gRNA fusion transcript giving rise to two functional single guide RNAs (sgRNA).

### Sequences

**Table 1**

| **Summary of Nucleic Acid and Protein SEQ ID Numbers** | | |
|---|---|---|
| | Nucleic acid | Protein |
| Description | SEQ ID NO. | SEQ ID NO. |
| Cas9 endonuclease, *Streptococcus pyogenes* | 1 | |
| Yarrowia codon optimized Cas9 | 2 | |
| SV40 Nuclear localization signal | | 3 |
| FBA1 promoter | 4 | |
| Yarrowia optimized expression cassette | 5 | |
| pZufCas9 | 6 | |
| Aarl-removal 1 primer | 7 | |
| Aarl-removal 2 primer | 8 | |
| pRF109 | 9 | |
| Aar1- Cas9 ORF (Aar1 -Cas9CG gene) | 10 | |
| pRF141 | 11 | |
| high-throughput cloning cassette | 12 | |
| yl52 promoter | 13 | |
| DNA encoding the HDV ribozyme | 14 | |
| rpsL counterselectable marker | 15 | |
| DNA encoding Cas9 CER domain | 16 | |
| SUP4 terminator | 17 | |
| pRF291 | 18 | |
| Can1-1 F oligo for HDV plasmid | 19 | |
| Can1-1 R oligo for HDV plasmid | 20 | |
| DNA encoding Can1-1 VT domain | 21 | |
| Can1-1 target site | 22 | |
| CAN1 gene, *Yarrowia lipolytica* | 23 | |
| pRF434 | 24 | |
| Hygromycin resistance cassette | 25 | |
| ura3-1 target site | 26 | |
| 5' and 3' flanked tRNA gRNA expression cassette | 27 | |
| tRNA Lysine | 28 | |
| tRNA Glutamine | 29 | |
| pFB8 | 30 | |
| 5' flanked tRNA expression plasmid cassette | 31 | |
| DNA sequences upstream to tRNA Lysine | 32 | |
| pFB5 | 33 | |
| 5' flanked tRNA gRNA expression plasmid cassette lacking upstream promoter sequences | 34 | |
| Can1-2 target site | 35 | |
| pFB33 | 36 | |
| 5' flanked tRNA expression plasmid cassette lacking upstream promoter sequences | 37 | |
| tRNA Valine | 38 | |
| pFB32 | 39 | |
| variable targeting domain (VT) cloning cassette for tRNA constructs | 40 | |
| pFB12 | 41 | |
| Can1-1 F oligo for tRNA plasmid | 42 | |
| Can1-1 R for tRNA plasmid | 43 | |
| 44 SDVT RNase Z recognition oligo F | 44 | |
| 45 SDVT RNase Z recognition oligo R | 45 | |
| SDVT RNase Z recognition domain | 46 | |
| pFB105 | 47 | |
| SDT RNase Z recognition oligo F | 48 | |
| SDT RNase Z recognition oligo R | 49 | |
| SDT RNase Z recognition domain | 50 | |
| pFB108 | 51 | |
| ST RNase Z recognition oligo F | 52 | |
| ST RNase Z recognition oligo R | 53 | |
| ST RNase Z recognition domain | 54 | |
| pFB109 | 55 | |
| Can1 oligos (see Table 3) | 56-99 | |
| pFB65 | 100 | |
| 5' flanked tRNA gRNA expression plasmid cassette | 101 | |
| SDVT SpacerRNA-gRNA construct | 102 | |
| SDT SpacerRNA-gRNA construc | 103 | |
| ST SpacerRNA-gRNA construct | 104 | |
| 5' flanked tRNA-leu expression plasmid cassette | 105 | |
| tRNA-leu | 106 | |
| pFB111 | 107 | |
| 5' flanked tRNA-leu(2) expression plasmid cassette | 108 | |
| tRNA-leu(2) | 109 | |
| pFB112 | 110 | |
| 5' flanked tRNA-leu(3) expression plasmid cassette | 111 | |
| tRNA-leu(3) | 112 | |
| pFB113 | 113 | |
| 5' flanked tRNA-ile expression plasmid cassette | 114 | |
| tRNA-ile | 115 | |
| pFB115 | 116 | |
| 5' flanked tRNA-val expression plasmid cassette | 117 | |
| tRNA-val | 118 | |
| pFB116 | 119 | |
| 5' flanked tRNA-trp expression plasmid cassette | 120 | |
| tRNA-trp | 121 | |
| pFB117 | 122 | |
| 5' flanked tRNA-tyr expression plasmid cassette | 123 | |
| tRNA-tyr | 124 | |
| pFB118 | 125 | |
| 5' flanked tRNA-his expression plasmid cassette | 126 | |
| tRNA-his | 127 | |
| pFB120 | 128 | |
| 5' flanked tRNA-his(2) expression plasmid cassette | 129 | |
| tRNA-his(2) | 130 | |
| pFB121 | 131 | |
| tRNA-gRNA-tRNA-gRNA expression cassette | 132 | |
| pFB9 | 133 | |

### DETAILED DESCRIPTION

Compositions and methods are provided for editing nucleotides and/or altering target sites in the genome of a cell. The methods and compositions employ a recombinant DNA construct comprising a tRNA promoter operably linked to a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a cell such as a microbial cell. The present disclosure further describes methods and compositions employing a recombinant DNA construct comprising a tRNA promoter operably linked to a spacer sequence and a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast.

CRISPR (clustered regularly interspaced short palindromic repeats) loci refers to certain genetic loci encoding factors of class I, II, or III DNA cleavage systems, for example, used by bacterial and archaeal cells to destroy foreign DNA (Horvath and Barrangou, 2010, Science 327:167-170). Components of CRISPR systems are taken advantage of herein in a heterologous manner for DNA targeting in cells.

The type II CRISPR/Cas system from bacteria employs a crRNA (CRISPR RNA) and tracrRNA (trans-activating CRISPR RNA) to guide the Cas endonuclease to its DNA target. The crRNA contains a region complementary to one strand of the double strand DNA target and a region that base pairs with the tracrRNA (trans-activating CRISPR RNA) forming a RNA duplex that directs the Cas endonuclease to cleave the DNA target. CRISPR systems belong to different classes, with different repeat patterns, sets of genes, and species ranges. The number of CRISPR-associated genes at a given CRISPR locus can vary between species (Haft et al. (2005) Computational Biology, PLoS Comput Biol 1(6): e60. doi:10.1371/journal.pcbi.0010060).

The term "Cas gene" herein refers to a gene that is generally coupled, associated or close to, or in the vicinity of flanking CRISPR loci. The terms "Cas gene", "CRISPR-associated (Cas) gene" are used interchangeably herein. The term "Cas endonuclease" herein refers to a protein encoded by a Cas gene. A Cas endonuclease herein, when in complex with a suitable polynucleotide component, is capable of recognizing, binding to, and optionally nicking or cleaving all or part of a specific DNA target sequence. A Cas endonuclease described herein comprises one or more nuclease domains. Cas endonucleases of the disclosure includes those having a HNH or HNH-like nuclease domain and / or a RuvC or RuvC-like nuclease domain. A Cas endonuclease of the disclosure includes a Cas9 protein, a Cpf1 protein, a C2c1 protein, a C2c2 protein, a C2c3 protein, Cas3, Cas 5, Cas7, Cas8, Cas10, or complexes of these.

As used herein, the terms "guide polynucleotide/Cas endonuclease complex", "guide polynucleotide/Cas endonuclease system", " guide polynucleotide/Cas complex", "guide polynucleotide/Cas system", "guided Cas system" are used interchangeably herein and refer to at least one guide polynucleotide and at least one Cas endonuclease that are capable of forming a complex, wherein said guide polynucleotide/Cas endonuclease complex can direct the Cas endonuclease to a DNA target site, enabling the Cas endonuclease to recognize, bind to, and optionally nick or cleave (introduce a single or double strand break) the DNA target site. A guide polynucleotide/Cas endonuclease complex herein can comprise Cas protein(s) and suitable polynucleotide component(s) of any of the four known CRISPR systems (Horvath and Barrangou, 2010, Science 327:167-170) such as a type I, II, or III CRISPR system. A Cas endonuclease unwinds the DNA duplex at the target sequence and optionally cleaves at least one DNA strand, as mediated by recognition of the target sequence by a polynucleotide (such as, but not limited to, a crRNA or guide RNA) that is in complex with the Cas protein. Such recognition and cutting of a target sequence by a Cas endonuclease typically occurs if the correct protospacer-adjacent motif (PAM) is located at or adjacent to the 3' end of the DNA target sequence. Alternatively, a Cas protein herein may lack DNA cleavage or nicking activity, but can still specifically bind to a DNA target sequence when complexed with a suitable RNA component. (See also U.S. Patent Application US 2015-0082478 A1, published on March 19, 2015 and US 2015-0059010 A1, published on February 26, 2015, both are hereby incorporated in its entirety by reference).

A guide polynucleotide/Cas endonuclease complex can cleave one or both strands of a DNA target sequence. A guide polynucleotide/Cas endonuclease complex that can cleave both strands of a DNA target sequence typically comprises a Cas protein that has all of its endonuclease domains in a functional state (e.g., wild type endonuclease domains or variants thereof retaining some or all activity in each endonuclease domain). Thus, a wild type Cas protein (e.g., a Cas9 protein disclosed herein), or a variant thereof retaining some or all activity in each endonuclease domain of the Cas protein, is a suitable example of a Cas endonuclease that can cleave both strands of a DNA target sequence. A Cas9 protein comprising functional RuvC and HNH nuclease domains is an example of a Cas protein that can cleave both strands of a DNA target sequence. A guide polynucleotide/Cas endonuclease complex that can cleave one strand of a DNA target sequence can be characterized herein as having nickase activity (e.g., partial cleaving capability). A Cas nickase typically comprises one functional endonuclease domain that allows the Cas to cleave only one strand (i.e., make a nick) of a DNA target sequence. For example, a Cas9 nickase may comprise (i) a mutant, dysfunctional RuvC domain and (ii) a functional HNH domain (e.g., wild type HNH domain). As another example, a Cas9 nickase may comprise (i) a functional RuvC domain (e.g., wild type RuvC domain) and (ii) a mutant, dysfunctional HNH domain. Non-limiting examples of Cas9 nickases suitable for use herein are disclosed in U.S. Patent Appl. Publ. No. 2014/0189896, which is incorporated herein by reference.

A pair of Cas9 nickases can be used to increase the specificity of DNA targeting. In general, this can be done by providing two Cas9 nickases that, by virtue of being associated with RNA components with different guide sequences, target and nick nearby DNA sequences on opposite strands in the region for desired targeting. Such nearby cleavage of each DNA strand creates a double strand break (i.e., a DSB with single-stranded overhangs), which is then recognized as a substrate for non-homologous-end-joining, NHEJ (prone to imperfect repair leading to mutations) or homologous recombination, HR. Each nick in these embodiments can be at least about 5, 10, 15, 20, 30, 40, 50, 60, 70, 80, 90, or 100 (or any integer between 5 and 100) bases apart from each other, for example. One or two Cas9 nickase proteins herein can be used in a Cas9 nickase pair. For example, a Cas9 nickase with a mutant RuvC domain, but functioning HNH domain (i.e., Cas9 HNH+/RuvC-), could be used (e.g., *Streptococcus pyogenes* Cas9 HNH+/RuvC-). Each Cas9 nickase (e.g., Cas9 HNH+/RuvC-) would be directed to specific DNA sites nearby each other (up to 100 base pairs apart) by using suitable RNA components herein with guide RNA sequences targeting each nickase to each specific DNA site.

A Cas protein can be part of a fusion protein comprising one or more heterologous protein domains (e.g., 1, 2, 3, or more domains in addition to the Cas protein). Such a fusion protein may comprise any additional protein sequence, and optionally a linker sequence between any two domains, such as between Cas and a first heterologous domain. Examples of protein domains that may be fused to a Cas protein herein include, without limitation, epitope tags (e.g., histidine [His], V5, FLAG, influenza hemagglutinin [HA], myc, VSV-G, thioredoxin [Trx]), reporters (e.g., glutathione-5-transferase [GST], horseradish peroxidase [HRP], chloramphenicol acetyltransferase [CAT], beta-galactosidase, beta-glucuronidase [GUS], luciferase, green fluorescent protein [GFP], HcRed, DsRed, cyan fluorescent protein [CFP], yellow fluorescent protein [YFP], blue fluorescent protein [BFP]), and domains having one or more of the following activities: methylase activity, demethylase activity, transcription activation activity (e.g., VP16 or VP64), transcription repression activity, transcription release factor activity, histone modification activity, RNA cleavage activity and nucleic acid binding activity. A Cas protein can also be in fusion with a protein that binds DNA molecules or other molecules, such as maltose binding protein (MBP), S-tag, Lex A DNA binding domain (DBD), GAL4A DNA binding domain, and herpes simplex virus (HSV) VP16.

A Cas protein herein can be from any of the following genera: *Aeropyrum, Pyrobaculum, Sulfolobus, Archaeoglobus, Haloarcula, Methanobacteriumn, Methanococcus, Methanosarcina, Methanopyrus, Pyrococcus, Picrophilus, Thernioplasnia, Corynebacterium, Mycobacterium, Streptomyces, Aquifrx, Porphvromonas, Chlorobium, Thermus, Bacillus, Listeria, Staphylococcus, Clostridium, Thermoanaerobacter, Mycoplasma, Fusobacterium, Azarcus, Chromobacterium, Neisseria, Nitrosomonas, Desulfovibrio, Geobacter, Myrococcus, Campylobacter, Wolinella, Acinetobacter, Erwinia, Escherichia, Legionella, Methylococcus, Pasteurella, Photobacterium, Salmonella, Xanthomonas, Yersinia, Streptococcus, Treponema, Francisella,* or *Thermotoga.* See also US patent applications 62/162,377 filed May 15, 2015 and 62/162,353 filed May 15, 2015 (both applications incorporated herein by reference) for more examples of Cas proteins.

A guide polynucleotide/Cas endonuclease complex in certain embodiments can bind to a DNA target site sequence, but does not cleave any strand at the target site sequence. Such a complex may comprise a Cas protein in which all of its nuclease domains are mutant, dysfunctional. For example, a Cas9 protein herein that can bind to a DNA target site sequence, but does not cleave any strand at the target site sequence, may comprise both a mutant, dysfunctional RuvC domain and a mutant, dysfunctional HNH domain. A Cas protein herein that binds, but does not cleave, a target DNA sequence can be used to modulate gene expression, for example, in which case the Cas protein could be fused with a transcription factor (or portion thereof) (e.g., a repressor or activator, such as any of those disclosed herein).

The Cas endonuclease gene herein can encode a Type II Cas9 endonuclease, such as but not limited to, Cas9 genes listed in SEQ ID NOs: 462, 474, 489, 494, 499, 505, and 518 of WO2007/025097, published March 1, 2007, and incorporated herein by reference. In another embodiment, the Cas endonuclease gene is a microbe or optimized Cas9 endonuclease gene. The Cas endonuclease gene can be operably linked to a SV40 nuclear targeting signal upstream of the Cas codon region and a bipartite VirD2 nuclear localization signal (Tinland et al. (1992) Proc. Natl. Acad. Sci. USA 89:7442-6) downstream of the Cas codon region.

The Cas endonuclease gene includes a plant or microbial codon optimized *Streptococcus pyogenes* Cas9 gene that can recognize any genomic sequence of the form N(12-30)NGG can in principle be targeted or a Cas9 endonuclease originated from an organism selected from the group consisting of *Brevibacillus laterosporus, Lactobacillus reuteri* Mlc3, *Lactobacillus rossiae* DSM 15814, *Pediococcus pentosaceus* SL4, *Lactobacillus nodensis* JCM 14932, *Sulfurospirillum sp.* SCADC, *Bifidobacterium thermophilum* DSM 20210, *Loktanella vestfoldensis, Sphingomonas sanxanigenens* NX02, *Epilithonimonas tenax* DSM 16811, *Sporocytophaga myxococcoides* and *Psychroflexus torquis* ATCC 700755, wherein said Cas9 endonuclease can form a guide RNA/Cas endonuclease complex capable of recognizing, binding to, and optionally nicking or cleaving all or part of a DNA target sequence. Other Cas endonuclease systems have been described in US patent applications 62/162,377 filed May 15, 2015 and 62/162,353 filed May 15, 2015, both applications incorporated herein by reference.

"Cas9" (formerly referred to as Cas5, Csn1, or Csx12) herein refers to a Cas endonuclease of a type II CRISPR system that forms a complex with a crNucleotide and a tracrNucleotide, or with a single guide polynucleotide, for specifically recognizing and cleaving all or part of a DNA target sequence. Cas9 protein comprises a RuvC nuclease domain and an HNH (H-N-H) nuclease domain, each of which can cleave a single DNA strand at a target sequence (the concerted action of both domains leads to DNA double-strand cleavage, whereas activity of one domain leads to a nick). In general, the RuvC domain comprises subdomains I, II and III, where domain I is located near the N-terminus of Cas9 and subdomains II and III are located in the middle of the protein, flanking the HNH domain (Hsu et al, Cell 157:1262-1278). A type II CRISPR system includes a DNA cleavage system utilizing a Cas9 endonuclease in complex with at least one polynucleotide component. For example, a Cas9 can be in complex with a CRISPR RNA (crRNA) and a trans-activating CRISPR RNA (tracrRNA). In another example, a Cas9 can be in complex with a single guide RNA.

The amino acid sequence of a Cas9 protein described herein, as well as certain other Cas proteins herein, may be derived from a *Streptococcus* (e.g., S. *pyogenes, S. pneumoniae, S. thermophilus, S. agalactiae, S. parasanguinis, S. oralis, S. salivarius, S. macacae, S. dysgalactiae, S. anginosus, S. constellatus, S. pseudoporcinus, S. mutans*), *Listeria* (e.g., *L. innocua*), *Spiroplasma* (e.g., *S. apis, S. syrphidicola), Peptostreptococcaceae, Atopobium, Porphyromonas* (e.g., *P. catoniae*), *Prevotella* (e.g., *P. intermedia*), *Veillonella, Treponema* (e.g., *T. socranskii, T. denticola), Capnocytophaga, Finegoldia* (e.g., *F. magna*), *Coriobacteriaceae* (e.g., *C. bacterium), Olsenella* (e.g., *O*. *profusa*), *Haemophilus* (e.g., *H. sputorum, H. pittmaniae), Pasteurella* (e.g., *P. bettyae*), *Olivibacter* (e.g., *O*. *sitiensis*), *Epilithonimonas* (e.g., *E. tenax*), *Mesonia* (e.g., *M. mobilis), Lactobacillus* (e.g., *L. plantarum), Bacillus* (e.g., *B. cereus*), *Aquimarina* (e.g., *A. muelleri*), *Chryseobacterium* (e.g., *C. palustre*), *Bacteroides* (e.g., *B. graminisolvens*), *Neisseria* (e.g., *N. meningitidis*), *Francisella* (e.g., *F. novicida*), or *Flavobacterium* (e.g., *F. frigidarium, F. soli*) species, for example. As another example, a Cas9 protein can be any of the Cas9 proteins disclosed in Chylinski et al. (RNA Biology 10:726-737 and US patent application 62/162377, filed May 15, 2015), which are incorporated herein by reference.

Accordingly, the sequence of a Cas9 protein herein can comprise, for example, any of the Cas9 amino acid sequences disclosed in GenBank Accession Nos. G3ECR1 (*S. thermophilus*), WP_026709422, WP_027202655, WP_027318179, WP_027347504, WP_027376815, WP_027414302, WP_027821588, WP_027886314, WP_027963583, WP_028123848, WP_028298935, Q03JI6 (*S. thermophilus*), EGP66723, EGS38969, EGV05092, EHI65578 (*S. pseudoporcinus*), EIC75614 (*S. oralis),* EID22027 (S. *constellatus*), EIJ69711, EJP22331 (*S. oralis),* EJP26004 (*S. anginosus*), EJP30321, EPZ44001 (*S. pyogenes),* EPZ46028 (*S. pyogenes),* EQL78043 (*S. pyogenes),* EQL78548 (*S. pyogenes),* ERL10511, ERL12345, ERL19088 (*S. pyogenes),* ESA57807 (*S. pyogenes),* ESA59254 (*S. pyogenes),* ESU85303 (*S. pyogenes),* ETS96804, UC75522, EGR87316 (*S. dysgalactiae*), EGS33732, EGV01468 (*S. oralis),* EHJ52063 (*S. macacae*), EID26207 (*S. oralis),* EID33364, EIG27013 (*S. parasanguinis*), EJF37476, EJO19166 *(Streptococcus* sp. BS35b), EJU16049, EJU32481, YP_006298249, ERF61304, ERK04546, ETJ95568 (*S. agalactiae*), TS89875, ETS90967 *(Streptococcus* sp. SR4), ETS92439, EUB27844 *(Streptococcus* sp. BS21), AFJ08616, EUC82735 *(Streptococcus* sp. CM6), EWC92088, EWC94390, EJP25691, YP_008027038, YP_008868573, AGM26527, AHK22391, AHB36273, Q927P4, G3ECR1, or Q99ZW2 (*S. pyogenes),* which are incorporated by reference. A variant of any of these Cas9 protein sequences may be used, but should have specific binding activity, and optionally endonucleolytic activity, toward DNA when associated with an RNA component herein. Such a variant may comprise an amino acid sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to the amino acid sequence of the reference Cas9.

Alternatively, a Cas9 protein may comprise an amino acid sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to any of the foregoing amino acid sequences, for example. Such a variant Cas9 protein should have specific binding activity, and optionally cleavage or nicking activity, toward DNA when associated with an RNA component herein.

A Cas protein herein such as a Cas9 can comprise a heterologous nuclear localization sequence (NLS). A heterologous NLS amino acid sequence herein may be of sufficient strength to drive accumulation of a Cas protein in a detectable amount in the nucleus of a yeast cell herein, for example. An NLS may comprise one (monopartite) or more (e.g., bipartite) short sequences (e.g., 2 to 20 residues) of basic, positively charged residues (e.g., lysine and/or arginine), and can be located anywhere in a Cas amino acid sequence but such that it is exposed on the protein surface. An NLS may be operably linked to the N-terminus or C-terminus of a Cas protein herein, for example. Two or more NLS sequences can be linked to a Cas protein, for example, such as on both the N- and C-termini of a Cas protein. Non-limiting examples of suitable NLS sequences herein include those disclosed in U.S. Patent No. 7309576, which is incorporated herein by reference.

The Cas endonuclease can comprise a modified form of the Cas9 polypeptide. The modified form of the Cas9 polypeptide can include an amino acid change (e.g., deletion, insertion, or substitution) that reduces the naturally-occurring nuclease activity of the Cas9 protein. For example, in some instances, the modified form of the Cas9 protein has less than 50%, less than 40%, less than 30%, less than 20%, less than 10%, less than 5%, or less than 1% of the nuclease activity of the corresponding wild-type Cas9 polypeptide (US patent application US20140068797 A1, published on March 6, 2014). In some cases, the modified form of the Cas9 polypeptide has no substantial nuclease activity and is referred to as catalytically "inactivated Cas9" or "deactivated cas9 (dCas9)." Catalytically inactivated Cas9 variants include Cas9 variants that contain mutations in the HNH and RuvC nuclease domains. These catalytically inactivated Cas9 variants are capable of interacting with sgRNA and binding to the target site in vivo but cannot cleave either strand of the target DNA.

A catalytically inactive Cas9 can be fused to a heterologous sequence (US patent application US20140068797 A1, published on March 6, 2014). Suitable fusion partners include, but are not limited to, a polypeptide that provides an activity that indirectly increases transcription by acting directly on the target DNA or on a polypeptide (e.g., a histone or other DNA-binding protein) associated with the target DNA. Additional suitable fusion partners include, but are not limited to, a polypeptide that provides for methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity, or demyristoylation activity. Further suitable fusion partners include, but are not limited to, a polypeptide that directly provides for increased transcription of the target nucleic acid (e.g., a transcription activator or a fragment thereof, a protein or fragment thereof that recruits a transcription activator, a small molecule/drug-responsive transcription regulator, etc.). A catalytically inactive Cas9 can also be fused to a Fokl nuclease to generate double strand breaks (Guilinger et al. Nature biotechnology, volume 32, number 6, June 2014).

The terms "functional fragment ", "fragment that is functionally equivalent" and "functionally equivalent fragment" of a Cas endonuclease are used interchangeably herein, and refer to a portion or subsequence of the Cas endonuclease sequence of the present disclosure in which the ability to recognize, bind to, and optionally nick or cleave (introduce a single or double strand break in) the target site is retained.

The terms "functional variant ", "Variant that is functionally equivalent" and "functionally equivalent variant" of a Cas endonuclease are used interchangeably herein, and refer to a variant of the Cas endonuclease of the present disclosure in which the ability to recognize, bind to, and optionally nick or cleave (introduce a single or double strand break in) the target site is retained. Fragments and variants can be obtained via methods such as site-directed mutagenesis and synthetic construction.

Any guided endonuclease can be used in the methods disclosed herein. Such endonucleases include, but are not limited to Cas9 and Cpf1 endonucleases. Many endonucleases have been described to date that can recognize specific PAM sequences (see for example Jinek et al. (2012) Science 337 p 816-821, US patent applications 62/162377 filed May 15, 2015 and 62/162353 filed May 15, 2015 and Zetsche B et al. 2015. Cell 163, 1013) and cleave the target DNA at a specific position. It is understood that based on the methods and embodiments described herein utilizing a guided Cas system one can now tailor these methods such that they can utilize any guided endonuclease system.

The term "off-target site effects" and "off-target effects" are used interchangeably and include any alteration in an off-target site that is due to the activity of an endonuclease cleavage, wherein the alteration include, for example: (i) a replacement of at least one nucleotide, (ii) a deletion of at least one nucleotide, (iii) an insertion of at least one nucleotide, or (iv) any combination of (i) - (iii), as well as any integration of a template or donor DNA at an unintended site. The unintended site can be any site in the genome of the organism that is not the target site.

Several approaches have been explored to improve the specificity and decrease off-target site effects of Cas endonucleases, including reducing the amount of enzyme active in the cell, shortening the section of the guide RNA complementary to the target, deploying pairs of engineered nicking Cas9s (Nicolas et al. Human Gene Therapy. 2015, 26(7): 425-431), and structure-guided protein engineering (Slaymaker et al. Science. 2015. Science DOI: 10.1126/science.aad5227). Many of these approaches remain to have limitations, often decreasing on-target editing efficiency.

Described in US patent application cl6501, incorporated herein by reference, are methods for decreasing off-target site effects in a cell while remaining and/or increasing on-target editing efficiency using small molecules such as NHEJ inhibitors or HDR enhancers.

The endonuclease can be provided to a cell by any method known in the art, for example, but not limited to transient introduction methods, transfection, microinjection, and/or topical application or indirectly via recombination constructs. The endonuclease can be provided as a protein or as a guided polynucleotide complex directly to a cell or indirectly via recombination constructs. The endonuclease can be introduced into a cell transiently or can be incorporated into the genome of the host cell using any method known in the art. Uptake of the endonuclease and/or the guided polynucleotide into the cell can be facilitated with a Cell Penetrating Peptide (CPP) as described in US application 62/075999, filed November 06, 2014.

Endonucleases are enzymes that cleave the phosphodiester bond within a polynucleotide chain, and include restriction endonucleases that cleave DNA at specific sites without damaging the bases. Restriction endonucleases include Type I, Type II, Type III, and Type IV endonucleases, which further include subtypes. In the Type I and Type III systems, both the methylase and restriction activities are contained in a single complex. Endonucleases also include meganucleases, also known as homing endonucleases (HEases), which like restriction endonucleases, bind and cut at a specific recognition site, however the recognition sites for meganucleases are typically longer, about 18 bp or more (patent application PCT/US12/30061, filed on March 22, 2012). Meganucleases have been classified into four families based on conserved sequence motifs, the families are the LAGLIDADG, GIY-YIG, H-N-H, and His-Cys box families. These motifs participate in the coordination of metal ions and hydrolysis of phosphodiester bonds. HEases are notable for their long recognition sites, and for tolerating some sequence polymorphisms in their DNA substrates. The naming convention for meganuclease is similar to the convention for other restriction endonuclease. Meganucleases are also characterized by prefix F-, I-, or PI- for enzymes encoded by free-standing ORFs, introns, and inteins, respectively. One step in the recombination process involves polynucleotide cleavage at or near the recognition site. This cleaving activity can be used to produce a double-strand break. For reviews of site-specific recombinases and their recognition sites, see, Sauer (1994) Curr Op Biotechnol 5:521-7; and Sadowski (1993) FASEB 7:760-7. In some examples the recombinase is from the Integrase or Resolvase families.

TAL effector nucleases (TALEN) are a class of sequence-specific nucleases that can be used to make double-strand breaks at specific target sequences in the genome of a plant or other organism. (Miller et al. (2011) Nature Biotechnology 29:143-148). Zinc finger nucleases (ZFNs) are engineered double-strand break inducing agents comprised of a zinc finger DNA binding domain and a double-strand-break-inducing agent domain. Recognition site specificity is conferred by the zinc finger domain, which typically comprising two, three, or four zinc fingers, for example having a C2H2 structure, however other zinc finger structures are known and have been engineered. Zinc finger domains are amenable for designing polypeptides which specifically bind a selected polynucleotide recognition sequence. ZFNs include an engineered DNA-binding zinc finger domain linked to a non-specific endonuclease domain, for example nuclease domain from a Type IIs endonuclease such as Fokl. Additional functionalities can be fused to the zinc-finger binding domain, including transcriptional activator domains, transcription repressor domains, and methylases. In some examples, dimerization of nuclease domain is required for cleavage activity. Each zinc finger recognizes three consecutive base pairs in the target DNA. For example, a 3 finger domain recognized a sequence of 9 contiguous nucleotides, with a dimerization requirement of the nuclease, two sets of zinc finger triplets are used to bind an 18 nucleotide recognition sequence.

As used herein, the term "guide polynucleotide", relates to a polynucleotide sequence that can form a complex with a Cas endonuclease and enables the Cas endonuclease to recognize, bind to, and optionally cleave a DNA target site. The guide polynucleotide can be a single molecule or a double molecule. The guide polynucleotide sequence can be a RNA sequence, a DNA sequence, or a combination thereof (a RNA-DNA combination sequence). Optionally, the guide polynucleotide can comprise at least one nucleotide, phosphodiester bond or linkage modification such as, but not limited, to Locked Nucleic Acid (LNA), 5-methyl dC, 2,6-Diaminopurine, 2'-Fluoro A, 2'-Fluoro U, 2'-O-Methyl RNA, phosphorothioate bond, linkage to a cholesterol molecule, linkage to a polyethylene glycol molecule, linkage to a spacer 18 (hexaethylene glycol chain) molecule, or 5' to 3' covalent linkage resulting in circularization. A guide polynucleotide that solely comprises ribonucleic acids is also referred to as a "guide RNA" or "gRNA" (See also U.S. Patent Application US 2015-0082478 A1, published on March 19, 2015 and US 2015-0059010 A1, published on February 26, 2015, both are hereby incorporated in its entirety by reference).

The guide polynucleotide can be a double molecule (also referred to as duplex guide polynucleotide) comprising a crNucleotide sequence and a tracrNucleotide sequence. The crNucleotide includes a first nucleotide sequence domain (referred to as Variable Targeting domain or VT domain) that can hybridize to a nucleotide sequence in a target DNA and a second nucleotide sequence (also referred to as a tracr mate sequence) that is part of a Cas endonuclease recognition (CER) domain. The tracr mate sequence can hybridized to a tracrNucleotide along a region of complementarity and together form the Cas endonuclease recognition domain or CER domain. The CER domain is capable of interacting with a Cas endonuclease polypeptide. The crNucleotide and the tracrNucleotide of the duplex guide polynucleotide can be RNA, DNA, and/or RNA-DNA- combination sequences. In some embodiments, the crNucleotide molecule of the duplex guide polynucleotide is referred to as "crDNA" (when composed of a contiguous stretch of DNA nucleotides) or "crRNA" (when composed of a contiguous stretch of RNA nucleotides), or "crDNA-RNA" (when composed of a combination of DNA and RNA nucleotides). The crNucleotide can comprise a fragment of the cRNA naturally occurring in Bacteria and Archaea. The size of the fragment of the cRNA naturally occurring in Bacteria and Archaea that can be present in a crNucleotide disclosed herein can range from, but is not limited to, 2, 3, 4, 5, 6, 7, 8, 9,10, 11, 12, 13, 14, 15, 16, 17, 18, 19, 20 or more nucleotides. In some embodiments the tracrNucleotide is referred to as "tracrRNA" (when composed of a contiguous stretch of RNA nucleotides) or "tracrDNA" (when composed of a contiguous stretch of DNA nucleotides) or "tracrDNA-RNA" (when composed of a combination of DNA and RNA nucleotides. In one embodiment, the RNA that guides the RNA/ Cas9 endonuclease complex is a duplexed RNA comprising a duplex crRNA-tracrRNA.
The tracrRNA (trans-activating CRISPR RNA) contains, in the 5'-to-3' direction, (i) a sequence that anneals with the repeat region of CRISPR type II crRNA and (ii) a stem loop-containing portion (Deltcheva et al., Nature 471:602-607). The duplex guide polynucleotide can form a complex with a Cas endonuclease, wherein said guide polynucleotide/Cas endonuclease complex (also referred to as a guide polynucleotide/Cas endonuclease system) can direct the Cas endonuclease to a genomic target site, enabling the Cas endonuclease to recognize, bind to, and optionally nick or cleave (introduce a single or double strand break) into the target site. (See also U.S. Patent Application US 2015-0082478 A1, published on March 19, 2015 and US 2015-0059010 A1, published on February 26, 2015, both are hereby incorporated in its entirety by reference.)

The guide polynucleotide can also be a single molecule (also referred to as single guide polynucleotide) comprising a crNucleotide sequence linked to a tracrNucleotide sequence. The single guide polynucleotide comprises a first nucleotide sequence domain (referred to as Variable Targeting domain or VT domain) that can hybridize to a nucleotide sequence in a target DNA and a Cas endonuclease recognition domain (CER domain), that interacts with a Cas endonuclease polypeptide. By "domain" it is meant a contiguous stretch of nucleotides that can be RNA, DNA, and/or RNA-DNA-combination sequence. The VT domain and /or the CER domain of a single guide polynucleotide can comprise a RNA sequence, a DNA sequence, or a RNA-DNA-combination sequence. The single guide polynucleotide being comprised of sequences from the crNucleotide and the tracrNucleotide may be referred to as "single guide RNA" (when composed of a contiguous stretch of RNA nucleotides) or "single guide DNA" (when composed of a contiguous stretch of DNA nucleotides) or "single guide RNA-DNA" (when composed of a combination of RNA and DNA nucleotides). The single guide polynucleotide can form a complex with a Cas endonuclease, wherein said guide polynucleotide/Cas endonuclease complex (also referred to as a guide polynucleotide/Cas endonuclease system) can direct the Cas endonuclease to a genomic target site, enabling the Cas endonuclease to recognize, bind to, and optionally nick or cleave (introduce a single or double strand break) the target site. (See also U.S. Patent Application US 2015-0082478 A1, published on March 19, 2015 and US 2015-0059010 A1, published on February 26, 2015, both are hereby incorporated in its entirety by reference.)

The term "variable targeting domain" or "VT domain" is used interchangeably herein and includes a nucleotide sequence that can hybridize (is complementary) to one strand (nucleotide sequence) of a double strand DNA target site. The % complementation between the first nucleotide sequence domain (VT domain) and the target sequence can be at least 50%, 51%, 52%, 53%, 54%, 55%, 56%, 57%, 58%, 59%, 60%, 61%, 62%, 63%, 63%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. The variable targeting domain can be at least 12, 13, 14, 15, 16, 17, 18, 19, 20, 21, 22, 23, 24, 25, 26, 27, 28, 29 or 30 nucleotides in length. In some embodiments, the variable targeting domain comprises a contiguous stretch of 12 to 30 nucleotides. The variable targeting domain can be composed of a DNA sequence, a RNA sequence, a modified DNA sequence, a modified RNA sequence, or any combination thereof.

The term "Cas endonuclease recognition domain" or "CER domain" (of a guide polynucleotide) is used interchangeably herein and includes a nucleotide sequence that interacts with a Cas endonuclease polypeptide. A CER domain comprises a tracrNucleotide mate sequence followed by a tracrNucleotide sequence. The CER domain can be composed of a DNA sequence, a RNA sequence, a modified DNA sequence, a modified RNA sequence (see for example US 2015-0059010 A1, published on February 26, 2015, incorporated in its entirety by reference herein), or any combination thereof.

The nucleotide sequence linking the crNucleotide and the tracrNucleotide of a single guide polynucleotide can comprise a RNA sequence, a DNA sequence, or a RNA-DNA combination sequence. In one embodiment, the nucleotide sequence linking the crNucleotide and the tracrNucleotide of a single guide polynucleotide can be at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 78, 79, 80, 81, 82, 83, 84, 85, 86, 87, 88, 89, 90, 91, 92, 93, 94, 95, 96, 97, 98, 99 or 100 nucleotides in length. In another embodiment, the nucleotide sequence linking the crNucleotide and the tracrNucleotide of a single guide polynucleotide can comprise a tetraloop sequence, such as, but not limiting to a GAAA tetraloop sequence.

Nucleotide sequence modification of the guide polynucleotide, VT domain and/or CER domain can be selected from, but not limited to , the group consisting of a 5' cap, a 3' polyadenylated tail, a riboswitch sequence, a stability control sequence, a sequence that forms a dsRNA duplex, a modification or sequence that targets the guide poly nucleotide to a subcellular location, a modification or sequence that provides for tracking , a modification or sequence that provides a binding site for proteins , a Locked Nucleic Acid (LNA), a 5-methyl dC nucleotide, a 2,6-Diaminopurine nucleotide, a 2'-Fluoro A nucleotide, a 2'-Fluoro U nucleotide; a 2'-O-Methyl RNA nucleotide, a phosphorothioate bond, linkage to a cholesterol molecule, linkage to a polyethylene glycol molecule, linkage to a spacer 18 molecule, a 5' to 3' covalent linkage, or any combination thereof. These modifications can result in at least one additional beneficial feature, wherein the additional beneficial feature is selected from the group of a modified or regulated stability, a subcellular targeting, tracking, a fluorescent label, a binding site for a protein or protein complex, modified binding affinity to complementary target sequence, modified resistance to cellular degradation, and increased cellular permeability.

The terms "functional fragment ", "fragment that is functionally equivalent" and "functionally equivalent fragment" of a guide RNA, crRNA or tracrRNA are used interchangeably herein, and refer to a portion or subsequence of the guide RNA, crRNA or tracrRNA, respectively, of the present disclosure in which the ability to function as a guide RNA, crRNA or tracrRNA, respectively, is retained.

The terms "functional variant ", "Variant that is functionally equivalent" and "functionally equivalent variant" of a guide RNA, crRNA or tracrRNA (respectively) are used interchangeably herein, and refer to a variant of the guide RNA, crRNA or tracrRNA, respectively, of the present disclosure in which the ability to function as a guide RNA, crRNA or tracrRNA, respectively, is retained.

The terms "single guide RNA" and "sgRNA" are used interchangeably herein and relate to a synthetic fusion of two RNA molecules, a crRNA (CRISPR RNA) comprising a variable targeting domain (linked to a tracr mate sequence that hybridizes to a tracrRNA), fused to a tracrRNA (trans-activating CRISPR RNA). The single guide RNA can comprise a crRNA or crRNA fragment and a tracrRNA or tracrRNA fragment of the type II CRISPR/Cas system that can form a complex with a type II Cas endonuclease, wherein said guide RNA/Cas endonuclease complex can direct the Cas endonuclease to a DNA target site, enabling the Cas endonuclease to recognize, bind to, and optionally nick or cleave (introduce a single or double strand break) the DNA target site.

There remains a need for improved expression systems of guide RNAs in cels. Described herein are compositions and methods that express precursor tRNA-gRNA molecules that can be optionally processed by internal cellular mechanisms to result in functional single guide RNAs capable of guiding a Cas endonuclease to its target site.

In one embodiment of the disclosure, the disclosure describes a recombinant DNA construct comprising a tRNA promoter operably linked to a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast.

In another embodiment of the disclosure, the disclosure describes a recombinant DNA construct comprising a promoter operably linked to a spacer sequence and a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast. The promoter operably linked to said spacer sequence can be any functional promoter such as but not limited to a tRNA promoter, a Pol-II promoter, a Pol-III promoter, or any one combination thereof.

The terms "guide RNA/Cas endonuclease complex", "guide RNA/Cas endonuclease system", " guide RNA/Cas complex", "guide RNA/Cas system", "gRNA/Cas complex", "gRNA/Cas system", "RNA-guided endonuclease" , "RGEN" are used interchangeably herein and refer to at least one RNA component and at least one Cas endonuclease that are capable of forming a complex , wherein said guide RNA/Cas endonuclease complex can direct the Cas endonuclease to a DNA target site, enabling the Cas endonuclease to recognize, bind to, and optionally nick or cleave (introduce a single or double strand break) the DNA target site. A guide RNA/Cas endonuclease complex herein can comprise Cas protein(s) and suitable RNA component(s) of any of the four known CRISPR systems (Horvath and Barrangou, 2010, Science 327:167-170) such as a type I, II, or III CRISPR system. A guide RNA/Cas endonuclease complex can comprise a Type II Cas9 endonuclease and at least one RNA component (e.g., a crRNA and tracrRNA, or a gRNA). (See also U.S. Patent Application US 2015-0082478 A1, published on March 19, 2015 and US 2015-0059010 A1, published on February 26, 2015, both are hereby incorporated in its entirety by reference).

The guide polynucleotide can be introduced into a cell transiently, as single stranded polynucleotide or a double stranded polynucleotide, using any method known in the art such as, but not limited to, particle bombardment, *Agrobacterium transformation* or topical applications. The guide polynucleotide can also be introduced indirectly into a cell by introducing a recombinant DNA molecule (via methods such as, but not limited to, particle bombardment or *Agrobacterium transformation*) comprising a heterologous nucleic acid fragment encoding a guide polynucleotide, operably linked to a specific promoter that is capable of transcribing the guide RNA in said cell.

A RNA polymerase III promoter (Pol-III promoter) can allow for transcription of RNA with precisely defined, unmodified, 5'- and 3'-ends (DiCarlo et al., Nucleic Acids Res. 41: 4336-4343; Ma et al., Mol. Ther. Nucleic Acids 3:e161; yl52 promoter, Marck et al. 2006. Nuceic Acid Res. 34(6):1816-1835)
RNA polymerase II promoters include a FBA1 promoter (Hong et al. 2012. Yeast. 29:59-72; see also US application 62/036652, filed on August 13, 2014, incorporated herein in its entirety by reference.

The term "tRNA promoter" as used herein refers to a DNA fragment encoding a tRNA, or a fragment thereof, that has promoter activity in a cell.

The tRNA promoter includes a DNA encoding any one tRNA known in the art such as but limiting to tRNA-Lysine (tRNA-Lys; see Acker et al. 2008. Nucleic acid res. 36(18):5832-5844), a tRNA-Glutamine (tRNA-Glu), a tRNA-Valine (tRNA Val; Marck et al. 2006. Nuceic Acid Res. 34(6):1816-1835) or any other tRNA active in a cell, a tRNA-leucine (tRNA Leu, tRNA-leu(2), tRNA-leu(3)), a tRNA-isoleucine (tRNA-ile), a tRNA-tryptophan (tRNA-trp), a tRNA-tyrosine (tRNA-tyr), a tRNA-histidine (tRNA-his; tRNA-his). As described herein, in a microbial cell (such as, but not limited to *Yarrowia*) having a recombinant DNA construct comprising a tRNA operably linked to a DNA encoding a guide RNA (gRNA), the DNA fragment encoding the tRNA can act as a promoter capable of expression of a tRNA-guide RNA fusion molecule.

The terms "tRNA-guide RNA expression cassette", "tRNA-gRNA expression cassette", "tRNA-guide RNA recombinant DNA construct" or "tRNA-gRNA recombinant DNA construct" are used interchangeable used herein and refer to any expression cassette (recombinant DNA construct) that encodes a tRNA-guide RNA fusion molecule, wherein the tRNA is fused to the guide RNA at its 5' and/or 3' end. Such fusions include a tRNA-gRNA-tRNA fusion, a tRNA-gRNA-tRNA-gRNA fusion, and a gRNA-tRNA fusion. As described herein, any one of such tRNA-guide RNA fusion molecule can be further processed in microbial cells by host proteins giving rise to a functional guide RNA (gRNA).

In one embodiment of the disclosure, the disclosure describes a recombinant DNA construct comprising a promoter operably linked to a spacer sequence and a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast, wherein the spacer sequence is a DNA sequence encoding a polynucleotide selected from the group consisting of a polynucleotide comprising a S-, D-, A-, V-, and T- domains of a tRNA, a polynucleotide comprising the S-, D-, V-, and T- domains of the tRNA, a polynucleotide comprising the S-, D-, and T- domains of the tRNA, and a polynucleotide comprising the S-, and T- domains of the tRNA.

RNaseZ recognition domains can be selected based on the interface of tRNA and RNase Z as determined from the crystal structure of RNase Z (de la Sierra-Gallay et al. 2005. Nature.433(7026): 657-661) and from RNase Z cleavage assays of tRNA with various arm deletions (Schiffer et al. 2001. Biochemistry. 40:8264-8272).

The terms "target site", "target sequence", "target site sequence, "target DNA", "target locus", "genomic target site", "genomic target sequence", "genomic target locus" and "protospacer", are used interchangeably herein and refer to a polynucleotide sequence such as, but not limited to, a nucleotide sequence on a chromosome, episome, or any other DNA molecule in the genome (including chromosomal, choloroplastic, mitochondrial DNA, plasmid DNA) of a cell, at which a guide polynucleotide/Cas endonuclease complex can recognize, bind to, and optionally nick or cleave . The target site can be an endogenous site in the genome of a cell, or alternatively, the target site can be heterologous to the cell and thereby not be naturally occurring in the genome of the cell, or the target site can be found in a heterologous genomic location compared to where it occurs in nature. As used herein, terms "endogenous target sequence" and "native target sequence" are used interchangeable herein to refer to a target sequence that is endogenous or native to the genome of a cell and is at the endogenous or native position of that target sequence in the genome of the cell. Cells include, but are not limited to, human, non-human, animal, bacterial, fungal, insect, yeast, non-conventional yeast, and plant cells as well as plants and seeds produced by the methods described herein. An "artificial target site" or "artificial target sequence" are used interchangeably herein and refer to a target sequence that has been introduced into the genome of a cell. Such an artificial target sequence can be identical in sequence to an endogenous or native target sequence in the genome of a cell but be located in a different position (i.e., a non-endogenous or non-native position) in the genome of a cell.

An "altered target site", "altered target sequence", "modified target site", "modified target sequence" are used interchangeably herein and refer to a target sequence as disclosed herein that comprises at least one alteration when compared to non-altered target sequence. Such "alterations" include, for example: (i) replacement of at least one nucleotide, (ii) a deletion of at least one nucleotide, (iii) an insertion of at least one nucleotide, or (iv) any combination of (i) - (iii).

Methods for "modifying a target site" and for "altering a target site" are used interchangeably herein and refer to methods for producing an altered target site.

The length of the target DNA sequence (target site) can vary, and includes, for example, target sites that are at least 12, 13, 14, 15, 16, 17, 18, 19, 20, 21, 22, 23, 24, 25, 26, 27, 28, 29, 30 or more nucleotides in length. It is further possible that the target site can be palindromic, that is, the sequence on one strand reads the same in the opposite direction on the complementary strand. The nick/cleavage site can be within the target sequence or the nick/cleavage site could be outside of the target sequence. In another variation, the cleavage could occur at nucleotide positions immediately opposite each other to produce a blunt end cut or, in other Cases, the incisions could be staggered to produce single-stranded overhangs, also called "sticky ends", which can be either 5' overhangs, or 3' overhangs. Active variants of genomic target sites can also be used. Such active variants can comprise at least 65%, 70%, 75%, 80%, 85%, 90%, 91%, 92%, 93%, 94%, 95%, 96%, 97%, 98%, 99% or more sequence identity to the given target site, wherein the active variants retain biological activity and hence are capable of being recognized and cleaved by an Cas endonuclease. Assays to measure the single or double-strand break of a target site by an endonuclease are known in the art and generally measure the overall activity and specificity of the agent on DNA substrates containing recognition sites.

A "protospacer adjacent motif" (PAM) herein refers to a short nucleotide sequence adjacent to a target sequence (protospacer) that is recognized (targeted) by a guide polynucleotide/Cas endonuclease system described herein. The Cas endonuclease may not successfully recognize a target DNA sequence if the target DNA sequence is not followed by a PAM sequence. The sequence and length of a PAM herein can differ depending on the Cas protein or Cas protein complex used. The PAM sequence can be of any length but is typically 1, 2, 3, 4, 5, 6, 7, 8, 9, 10, 11, 12, 13, 14, 15, 16, 17, 18, 19 or 20 nucleotides long.

The terms "targeting", "gene targeting" and "DNA targeting" are used interchangeably herein. DNA targeting herein may be the specific introduction of a knock-out, edit, or knock-in at a particular DNA sequence, such as in a chromosome or plasmid of a cell. In general, DNA targeting can be performed herein by cleaving one or both strands at a specific DNA sequence in a cell with an endonuclease associated with a suitable polynucleotide component. Such DNA cleavage, if a double-strand break (DSB), can prompt NHEJ or HDR processes which can lead to modifications at the target site.

A targeting method herein can be performed in such a way that two or more DNA target sites are targeted in the method, for example. Such a method can optionally be characterized as a multiplex method. Two, three, four, five, six, seven, eight, nine, ten, or more target sites can be targeted at the same time in certain embodiments. A multiplex method is typically performed by a targeting method herein in which multiple different RNA components are provided, each designed to guide a guide polynucleotide/Cas endonuclease complex to a unique DNA target site. (US application 62/036652, filed on August 13, 2014, incorporated herein in its entirety by reference).

In certain embodiments, a recombinant DNA construct can comprise (i) a promoter operably linked to (ii) a sequence comprising more than one tRNA-guide RNA component cassettes (i.e., tandem cassettes). A transcript expressed from such a recombinant DNA construct can have, for example, 2, 3, 4, 5, 6, 7, 8, 9, 10, or more tRNA-gRNA component cassettes. The tRNA can allow for cleavage and separation of the guide RNA components from downstream transcript sequence. Each guide RNA component in such embodiments typically is designed to guide an Cas endonuclease herein to a unique DNA target site. Thus, such a recombinant DNA construct can be used in a non-conventional yeast accordingly to target multiple different target sites at the same time, for example; such use can optionally be characterized as a multiplexing method.

The terms "knock-out", "gene knock-out" and "genetic knock-out" are used interchangeably herein. A knock-out represents a DNA sequence of a cell that has been rendered partially or completely inoperative by targeting with a Cas protein; such a DNA sequence prior to knock-out could have encoded an amino acid sequence, or could have had a regulatory function (e.g., promoter), for example. A knock-out may be produced by an indel (insertion or deletion of nucleotide bases in a target DNA sequence through NHEJ), or by specific removal of sequence that reduces or completely destroys the function of sequence at or near the targeting site.

The guide polynucleotide/Cas endonuclease system can be used in combination with a co-delivered polynucleotide modification template to allow for editing (modification) of a genomic nucleotide sequence of interest. (See also U.S. Patent Application US 2015-0082478 A1, published on March 19, 2015 and WO2015/026886 A1, published on February 26, 2015, both are hereby incorporated in its entirety by reference.)

A "modified nucleotide" or "edited nucleotide" refers to a nucleotide sequence of interest that comprises at least one alteration when compared to its non-modified nucleotide sequence. Such "alterations" include, for example: (i) replacement of at least one nucleotide, (ii) a deletion of at least one nucleotide, (iii) an insertion of at least one nucleotide, or (iv) any combination of (i) - (iii).

The term "polynucleotide modification template" includes a polynucleotide that comprises at least one nucleotide modification when compared to the nucleotide sequence to be edited. A nucleotide modification can be at least one nucleotide substitution, addition or deletion. Optionally, the polynucleotide modification template can further comprise homologous nucleotide sequences flanking the at least one nucleotide modification, wherein the flanking homologous nucleotide sequences provide sufficient homology to the desired nucleotide sequence to be edited.

Genome editing can be accomplished using any method of gene editing available. For example, gene editing can be accomplished through the introduction into a host cell of a polynucleotide modification template (sometimes also referred to as a gene repair oligonucleotide) containing a targeted modification to a gene within the genome of the host cell. The polynucleotide modification template for use in such methods can be either single-stranded or double-stranded. Examples of such methods are generally described, for example, in US Publication No. 2013/0019349.

In some embodiments, gene editing may be facilitated through the induction of a double-stranded break (DSB) in a defined position in the genome near the desired alteration. DSBs can be induced using any DSB-inducing agent available, including, but not limited to, TALENs, meganucleases, zinc finger nucleases, Cas9-gRNA systems (based on bacterial CRISPR-Cas systems), and the like. In some embodiments, the introduction of a DSB can be combined with the introduction of a polynucleotide modification template.

The process for editing a genomic sequence combining DSB and modification templates generally comprises: providing to a host cell, a DSB-inducing agent, or a nucleic acid encoding a DSB-inducing agent, that recognizes a target sequence in the chromosomal sequence and is able to induce a DSB in the genomic sequence, and at least one polynucleotide modification template comprising at least one nucleotide alteration when compared to the nucleotide sequence to be edited. The polynucleotide modification template can further comprise nucleotide sequences flanking the at least one nucleotide alteration, in which the flanking sequences are substantially homologous to the chromosomal region flanking the DSB. Genome editing using DSB-inducing agents, such as Cas9-gRNA complexes, has been described, for example in U.S. Patent Application US 2015-0082478 A1, published on March 19, 2015, WO2015/026886 A1, published on February 26, 2015, US application 62/023246, filed on July 07, 2014, and US application 62/036,652, filed on August 13, 2014, all of which are incorporated by reference herein.

The terms "knock-in", "gene knock-in, "gene insertion" and "genetic knock-in" are used interchangeably herein. A knock-in represents the replacement or insertion of a DNA sequence at a specific DNA sequence in cell by targeting with a Cas protein (by HR, wherein a suitable donor DNA polynucleotide is also used). Examples of knock-ins are a specific insertion of a heterologous amino acid coding sequence in a coding region of a gene, or a specific insertion of a transcriptional regulatory element in a genetic locus.

Various methods and compositions can be employed to obtain a cell or organism having a polynucleotide of interest inserted in a target site for a Cas endonuclease. Such methods can employ homologous recombination to provide integration of the polynucleotide of Interest at the target site. In one method provided, a polynucleotide of interest is provided to the organism cell in a donor DNA construct. As used herein, "donor DNA" is a DNA construct that comprises a polynucleotide of Interest to be inserted into the target site of a Cas endonuclease. The donor DNA construct further comprises a first and a second region of homology that flank the polynucleotide of Interest. The first and second regions of homology of the donor DNA share homology to a first and a second genomic region, respectively, present in or flanking the target site of the cell or organism genome. By "homology" is meant DNA sequences that are similar. For example, a "region of homology to a genomic region" that is found on the donor DNA is a region of DNA that has a similar sequence to a given "genomic region" in the cell or organism genome. A region of homology can be of any length that is sufficient to promote homologous recombination at the cleaved target site. For example, the region of homology can comprise at least 5-10, 5-15, 5-20, 5-25, 5-30, 5-35, 5-40, 5-45, 5- 50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800, 5-2900, 5-3000, 5-3100 or more bases in length such that the region of homology has sufficient homology to undergo homologous recombination with the corresponding genomic region. "Sufficient homology" indicates that two polynucleotide sequences have sufficient structural similarity to act as substrates for a homologous recombination reaction. The structural similarity includes overall length of each polynucleotide fragment, as well as the sequence similarity of the polynucleotides. Sequence similarity can be described by the percent sequence identity over the whole length of the sequences, and/or by conserved regions comprising localized similarities such as contiguous nucleotides having 100% sequence identity, and percent sequence identity over a portion of the length of the sequences.

The amount of homology or sequence identity shared by a target and a donor polynucleotide can vary and includes total lengths and/or regions having unit integral values in the ranges of about 1-20 bp, 20-50 bp, 50-100 bp, 75-150 bp, 100-250 bp, 150-300 bp, 200-400 bp, 250-500 bp, 300-600 bp, 350-750 bp, 400-800 bp, 450-900 bp, 500-1000 bp, 600-1250 bp, 700-1500 bp, 800-1750 bp, 900-2000 bp, 1-2.5 kb, 1.5-3 kb, 2-4 kb, 2.5-5 kb, 3-6 kb, 3.5-7 kb, 4-8 kb, 5-10 kb, or up to and including the total length of the target site. These ranges include every integer within the range, for example, the range of 1-20 bp includes 1, 2, 3, 4, 5, 6, 7, 8, 9, 10, 11, 12, 13, 14, 15, 16, 17, 18, 19 and 20 bps. The amount of homology can also described by percent sequence identity over the full aligned length of the two polynucleotides which includes percent sequence identity of about at least 50%, 55%, 60%, 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. Sufficient homology includes any combination of polynucleotide length, global percent sequence identity, and optionally conserved regions of contiguous nucleotides or local percent sequence identity, for example sufficient homology can be described as a region of 75-150 bp having at least 80% sequence identity to a region of the target locus. Sufficient homology can also be described by the predicted ability of two polynucleotides to specifically hybridize under high stringency conditions, see, for example, Sambrook et al., (1989) Molecular Cloning: A Laboratory Manual, (Cold Spring Harbor Laboratory Press, NY); Current Protocols in Molecular Biology, Ausubel et al., Eds (1994) Current Protocols, (Greene Publishing Associates, Inc. and John Wiley & Sons, Inc.); and, Tijssen (1993) Laboratory Techniques in Biochemistry and Molecular Biology--Hybridization with Nucleic Acid Probes, (Elsevier, New York).

As used herein, a "genomic region" is a segment of a chromosome in the genome of a cell that is present on either side of the target site or, alternatively, also comprises a portion of the target site. The genomic region can comprise at least 5-10, 5-15, 5-20, 5-25, 5-30, 5-35, 5-40, 5-45, 5- 50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800. 5-2900, 5-3000, 5-3100 or more bases such that the genomic region has sufficient homology to undergo homologous recombination with the corresponding region of homology.

Polynucleotides of interest and/or traits can be stacked together in a complex trait locus as described in US 2013/0263324-A1, published October 3, 2013 and in PCT/US13/22891, published January 24, 2013, both applications are hereby incorporated by reference. The guide polynucleotide/Cas9 endonuclease system described herein provides for an efficient system to generate double strand breaks and allows for traits to be stacked in a complex trait locus.

The structural similarity between a given genomic region and the corresponding region of homology found on the donor DNA can be any degree of sequence identity that allows for homologous recombination to occur. For example, the amount of homology or sequence identity shared by the "region of homology" of the donor DNA and the "genomic region" of the organism genome can be at least 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity, such that the sequences undergo homologous recombination

The region of homology on the donor DNA can have homology to any sequence flanking the target site. While in some embodiments the regions of homology share significant sequence homology to the genomic sequence immediately flanking the target site, it is recognized that the regions of homology can be designed to have sufficient homology to regions that may be further 5' or 3' to the target site. In still other embodiments, the regions of homology can also have homology with a fragment of the target site along with downstream genomic regions. In one embodiment, the first region of homology further comprises a first fragment of the target site and the second region of homology comprises a second fragment of the target site, wherein the first and second fragments are dissimilar.

As used herein, "homologous recombination" includes the exchange of DNA fragments between two DNA molecules at the sites of homology. The frequency of homologous recombination is influenced by a number of factors. Different organisms vary with respect to the amount of homologous recombination and the relative proportion of homologous to non-homologous recombination. Generally, the length of the region of homology affects the frequency of homologous recombination events: the longer the region of homology, the greater the frequency. The length of the homology region needed to observe homologous recombination is also species-variable. In many cases, at least 5 kb of homology has been utilized, but homologous recombination has been observed with as little as 25-50 bp of homology. See, for example, Singer et al., (1982) Cell 31:25-33; Shen and Huang, (1986) Genetics 112:441-57; Watt et al., (1985) Proc. Natl. Acad. Sci. USA 82:4768-72, Sugawara and Haber, (1992) Mol Cell Biol 12:563-75, Rubnitz and Subramani, (1984) Mol Cell Biol 4:2253-8; Ayares et al., (1986) Proc. Natl. Acad. Sci. USA 83:5199-203; Liskay et al., (1987) Genetics 115:161-7.

Homology-directed repair (HDR) is a mechanism in cells to repair double-stranded and single stranded DNA breaks. Homology-directed repair includes homologous recombination (HR) and single-strand annealing (SSA) (Lieber. 2010 Annu. Rev. Biochem. 79:181-211). The most common form of HDR is called homologous recombination (HR), which has the longest sequence homology requirements between the donor and acceptor DNA. Other forms of HDR include single-stranded annealing (SSA) and breakage-induced replication, and these require shorter sequence homology relative to HR. Homology-directed repair at nicks (single-stranded breaks) can occur via a mechanism distinct from HDR at double-strand breaks (Davis and Maizels (2014) PNAS (0027-8424), 111 (10), p. E924-E932).

Alteration of the genome of a plant cell, for example, through homologous recombination (HR), is a powerful tool for genetic engineering. Homologous recombination has been demonstrated in plants (Halfter et al., (1992) Mol Gen Genet 231:186-93) and insects (Dray and Gloor, 1997, Genetics 147:689-99). Homologous recombination has also been accomplished in other organisms. For example, at least 150-200 bp of homology was required for homologous recombination in the parasitic protozoan Leishmania (Papadopoulou and Dumas, (1997) Nucleic Acids Res 25:4278-86). In the filamentous fungus *Aspergillus nidulans,* gene replacement has been accomplished with as little as 50 bp flanking homology (Chaveroche et al., (2000) Nucleic Acids Res 28:e97). Targeted gene replacement has also been demonstrated in the ciliate *Tetrahymena thermophila* (Gaertig et al., (1994) Nucleic Acids Res 22:5391-8). In mammals, homologous recombination has been most successful in the mouse using pluripotent embryonic stem cell lines (ES) that can be grown in culture, transformed, selected and introduced into a mouse embryo (Watson et al., 1992, Recombinant DNA, 2nd Ed., (Scientific American Books distributed by WH Freeman & Co.).

Error-prone DNA repair mechanisms can produce mutations at double-strand break sites. The Non-Homologous-End-Joining (NHEJ) pathways are the most common repair mechanism to bring the broken ends together (Bleuyard et al., (2006) DNA Repair 5:1-12). The structural integrity of chromosomes is typically preserved by the repair, but deletions, insertions, or other rearrangements are possible. The two ends of one double-strand break are the most prevalent substrates of NHEJ (Kirik et al., (2000) EMBO J 19:5562-6), however if two different double-strand breaks occur, the free ends from different breaks can be ligated and result in chromosomal deletions (Siebert and Puchta, (2002) Plant Cell 14:1121-31), or chromosomal translocations between different chromosomes (Pacher et al., (2007) Genetics 175:21-9). Microhomology-mediated end joining MMEH is described in US patent application US2014/0242702, published on August 28, 2014, incorporated hereinin its entirety .

It is understood by anyone skilled in the art that the Cas endonuclease used in the methods described herein can be substituted by any double strand break inducing agent such us but not limited to TAL nucleases (TALENs), designer zinc-finger nucleases, engineered meganucleases and homing meganucleases.

Episomal DNA molecules can also be ligated into the double-strand break, for example, integration of T-DNAs into chromosomal double-strand breaks (Chilton and Que, (2003) Plant Physiol 133:956-65; Salomon and Puchta, (1998) EMBO J 17:6086-95). Once the sequence around the double-strand breaks is altered, for example, by exonuclease activities involved in the maturation of double-strand breaks, gene conversion pathways can restore the original structure if a homologous sequence is available, such as a homologous chromosome in non-dividing somatic cells, or a sister chromatid after DNA replication (Molinier et al., (2004) Plant Cell 16:342-52). Ectopic and/or epigenic DNA sequences may also serve as a DNA repair template for homologous recombination (Puchta, (1999) Genetics 152:1173-81).

Once a double-strand break is induced in the DNA, the cell's DNA repair mechanism is activated to repair the break. Error-prone DNA repair mechanisms can produce mutations at double-strand break sites. The most common repair mechanism to bring the broken ends together is the nonhomologous end-joining (NHEJ) pathway (Bleuyard et al., (2006) DNA Repair 5:1-12). The structural integrity of chromosomes is typically preserved by the repair, but deletions, insertions, or other rearrangements are possible (Siebert and Puchta, (2002) Plant Cell 14:1121-31; Pacher et al., (2007) Genetics 175:21-9).

Alternatively, the double-strand break can be repaired by homologous recombination between homologous DNA sequences. Once the sequence around the double-strand break is altered, for example, by exonuclease activities involved in the maturation of double-strand breaks, gene conversion pathways can restore the original structure if a homologous sequence is available, such as a homologous chromosome in non-dividing somatic cells, or a sister chromatid after DNA replication (Molinier et al., (2004) Plant Cell 16:342-52). Ectopic and/or epigenic DNA sequences may also serve as a DNA repair template for homologous recombination (Puchta, (1999) Genetics 152:1173-81).

DNA double-strand breaks appear to be an effective factor to stimulate homologous recombination pathways (Puchta et al., (1995) Plant Mol Biol 28:281-92; Tzfira and White, (2005) Trends Biotechnol 23:567-9; Puchta, (2005) J Exp Bot 56:1-14). Using DNA-breaking agents, a two- to nine-fold increase of homologous recombination was observed between artificially constructed homologous DNA repeats in plants (Puchta et al., (1995) Plant Mol Biol 28:281-92). In maize protoplasts, experiments with linear DNA molecules demonstrated enhanced homologous recombination between plasmids (Lyznik et al., (1991) Mol Gen Genet 230:209-18).

The donor DNA may be introduced by any means known in the art. The donor DNA may be provided by any transformation method known in the art including, for example, Agrobacterium-mediated transformation or biolistic particle bombardment. The donor DNA may be present transiently in the cell or it could be introduced via a viral replicon. In the presence of the Cas endonuclease and the target site, the donor DNA is inserted into the transformed plant's genome. (see guide language)

Further uses for guide RNA/Cas endonuclease systems have been described (See U.S. Patent Application US 2015-0082478 A1, published on March 19, 2015, WO2015/026886 A1, published on February 26, 2015, US 2015-0059010 A1, published on February 26, 2015, US application 62/023246, filed on July 07, 2014, and US application 62/036,652, filed on August 13, 2014, all of which are incorporated by reference herein) and include but are not limited to modifying or replacing nucleotide sequences of interest (such as a regulatory elements), insertion of polynucleotides of interest, gene knock-out, gene-knock in, modification of splicing sites and/or introducing alternate splicing sites, modifications of nucleotide sequences encoding a protein of interest, amino acid and/or protein fusions, and gene silencing by expressing an inverted repeat into a gene of interest.

Polynucleotides of interest are further described herein and include polynucleotides reflective of the commercial markets and interests of those involved in the development of the crop. Polynucleotides/polypeptides of interest include, but are not limited to, herbicide-resistance coding sequences, insecticidal coding sequences, nematicidal coding sequences, antimicrobial coding sequences, antifungal coding sequences, antiviral coding sequences, abiotic and biotic stress tolerance coding sequences, or sequences modifying plant traits such as yield, grain quality, nutrient content, starch quality and quantity, nitrogen fixation and/or utilization, fatty acids, and oil content and/or composition.

Furthermore, it is recognized that the polynucleotide of interest may also comprise antisense sequences complementary to at least a portion of the messenger RNA (mRNA) for a targeted gene sequence of interest. Antisense nucleotides are constructed to hybridize with the corresponding mRNA. Modifications of the antisense sequences may be made as long as the sequences hybridize to and interfere with expression of the corresponding mRNA. In this manner, antisense constructions having 70%, 80%, or 85% sequence identity to the corresponding antisense sequences may be used. Furthermore, portions of the antisense nucleotides may be used to disrupt the expression of the target gene. Generally, sequences of at least 50 nucleotides, 100 nucleotides, 200 nucleotides, or greater may be used.

In addition, the polynucleotide of interest may also be used in the sense orientation to suppress the expression of endogenous genes in plants. Methods for suppressing gene expression in plants using polynucleotides in the sense orientation are known in the art. The methods generally involve transforming plants with a DNA construct comprising a promoter that drives expression in a plant operably linked to at least a portion of a nucleotide sequence that corresponds to the transcript of the endogenous gene. Typically, such a nucleotide sequence has substantial sequence identity to the sequence of the transcript of the endogenous gene, generally greater than about 65% sequence identity, about 85% sequence identity, or greater than about 95% sequence identity. See, U.S. Patent Nos. 5,283,184 and 5,034,323; herein incorporated in its entirety by reference.

The polynucleotide of interest can also be a phenotypic marker. A phenotypic marker is screenable or a selectable marker that includes visual markers and selectable markers whether it is a positive or negative selectable marker. Any phenotypic marker can be used. Specifically, a selectable or screenable marker comprises a DNA segment that allows one to identify, or select for or against a molecule or a cell that contains it, often under particular conditions. These markers can encode an activity, such as, but not limited to, production of RNA, peptide, or protein, or can provide a binding site for RNA, peptides, proteins, inorganic and organic compounds or compositions and the like.

As used herein, "nucleic acid" means a polynucleotide and includes a single or a double-stranded polymer of deoxyribonucleotide or ribonucleotide bases. Nucleic acids may also include fragments and modified nucleotides. Thus, the terms "polynucleotide", "nucleic acid sequence", "nucleotide sequence" and "nucleic acid fragment" are used interchangeably to denote a polymer of RNA and/or DNA that is single- or double-stranded, optionally containing synthetic, non-natural, or altered nucleotide bases. Nucleotides (usually found in their 5'-monophosphate form) are referred to by their single letter designation as follows: "A" for adenosine or deoxyadenosine (for RNA or DNA, respectively), "C" for cytosine or deoxycytosine, "G" for guanosine or deoxyguanosine, "U" for uridine, "T" for deoxythymidine, "R" for purines (A or G), "Y" for pyrimidines (C or T), "K" for G or T, "H" for A or C or T, "I" for inosine, and "N" for any nucleotide.

"Open reading frame" is abbreviated ORF.

The terms "subfragment that is functionally equivalent" and "functionally equivalent subfragment" are used interchangeably herein. These terms refer to a portion or subsequence of an isolated nucleic acid fragment in which the ability to alter gene expression or produce a certain phenotype is retained whether or not the fragment or subfragment encodes an active enzyme. For example, the fragment or subfragment can be used in the design of genes to produce the desired phenotype in a transformed plant. Genes can be designed for use in suppression by linking a nucleic acid fragment or subfragment thereof, whether or not it encodes an active enzyme, in the sense or antisense orientation relative to a plant promoter sequence.

The term "conserved domain" or "motif" means a set of amino acids conserved at specific positions along an aligned sequence of evolutionarily related proteins. While amino acids at other positions can vary between homologous proteins, amino acids that are highly conserved at specific positions indicate amino acids that are essential to the structure, the stability, or the activity of a protein. Because they are identified by their high degree of conservation in aligned sequences of a family of protein homologues, they can be used as identifiers, or "signatures", to determine if a protein with a newly determined sequence belongs to a previously identified protein family.

Polynucleotide and polypeptide sequences, variants thereof, and the structural relationships of these sequences can be described by the terms "homology", "homologous", "substantially identical", "substantially similar" and "corresponding substantially" which are used interchangeably herein. These refer to polypeptide or nucleic acid fragments wherein changes in one or more amino acids or nucleotide bases do not affect the function of the molecule, such as the ability to mediate gene expression or to produce a certain phenotype. These terms also refer to modification(s) of nucleic acid fragments that do not substantially alter the functional properties of the resulting nucleic acid fragment relative to the initial, unmodified fragment. These modifications include deletion, substitution, and/or insertion of one or more nucleotides in the nucleic acid fragment.

Substantially similar nucleic acid sequences encompassed may be defined by their ability to hybridize (under moderately stringent conditions, e.g., 0.5X SSC, 0.1% SDS, 60°C) with the sequences exemplified herein, or to any portion of the nucleotide sequences disclosed herein and which are functionally equivalent to any of the nucleic acid sequences disclosed herein. Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions.

The term "selectively hybridizes" includes reference to hybridization, under stringent hybridization conditions, of a nucleic acid sequence to a specified nucleic acid target sequence to a detectably greater degree (e.g., at least 2-fold over background) than its hybridization to non-target nucleic acid sequences and to the substantial exclusion of non-target nucleic acids. Selectively hybridizing sequences typically have about at least 80% sequence identity, or 90% sequence identity, up to and including 100% sequence identity (i.e., fully complementary) with each other.

The term "stringent conditions" or "stringent hybridization conditions" includes reference to conditions under which a probe will selectively hybridize to its target sequence in an *in vitro* hybridization assay. Stringent conditions are sequence-dependent and will be different in different circumstances. By controlling the stringency of the hybridization and/or washing conditions, target sequences can be identified which are 100% complementary to the probe (homologous probing). Alternatively, stringency conditions can be adjusted to allow some mismatching in sequences so that lower degrees of similarity are detected (heterologous probing). Generally, a probe is less than about 1000 nucleotides in length, optionally less than 500 nucleotides in length.

Typically, stringent conditions will be those in which the salt concentration is less than about 1.5 M Na ion, typically about 0.01 to 1.0 M Na ion concentration (or other salt(s)) at pH 7.0 to 8.3, and at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C.

"Sequence identity" or "identity" in the context of nucleic acid or polypeptide sequences refers to the nucleic acid bases or amino acid residues in two sequences that are the same when aligned for maximum correspondence over a specified comparison window.

The term "percentage of sequence identity" refers to the value determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the results by 100 to yield the percentage of sequence identity. Useful examples of percent sequence identities include, but are not limited to, 50%, 55%, 60%, 65%, 70%, 75%, 80%, 85%, 90% or 95%, or any integer percentage from 50% to 100%. These identities can be determined using any of the programs described herein.

Sequence alignments and percent identity or similarity calculations may be determined using a variety of comparison methods designed to detect homologous sequences including, but not limited to, the MegAlign™ program of the LASERGENE bioinformatics computing suite (DNASTAR Inc., Madison, WI). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

The "Clustal V method of alignment" corresponds to the alignment method labeled Clustal V (described by Higgins and Sharp, (1989) CABIOS 5:151-153; Higgins et al., (1992) Comput Appl Biosci 8:189-191) and found in the MegAlign™ program of the LASERGENE bioinformatics computing suite (DNASTAR Inc., Madison, WI). For multiple alignments, the default values correspond to GAP PENALTY=10 and GAP LENGTH PENALTY=10. Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a "percent identity" by viewing the "sequence distances" table in the same program.

The "Clustal W method of alignment" corresponds to the alignment method labeled Clustal W (described by Higgins and Sharp, (1989) CABIOS 5:151-153; Higgins et al., (1992) ComputAppl Biosci 8:189-191) and found in the MegAlign™ v6.1 program of the LASERGENE bioinformatics computing suite (DNASTAR Inc., Madison, WI). Default parameters for multiple alignment (GAP PENALTY=10, GAP LENGTH PENALTY=0.2, Delay Divergen Seqs (%)=30, DNA Transition Weight=0.5, Protein Weight Matrix=Gonnet Series, DNA Weight Matrix=IUB). After alignment of the sequences using the Clustal W program, it is possible to obtain a "percent identity" by viewing the "sequence distances" table in the same program.

Unless otherwise stated, sequence identity/similarity values provided herein refer to the value obtained using GAP Version 10 (GCG, Accelrys, San Diego, CA) using the following parameters: % identity and % similarity for a nucleotide sequence using a gap creation penalty weight of 50 and a gap length extension penalty weight of 3, and the nwsgapdna.cmp scoring matrix; % identity and % similarity for an amino acid sequence using a GAP creation penalty weight of 8 and a gap length extension penalty of 2, and the BLOSUM62 scoring matrix (Henikoff and Henikoff, (1989) Proc. Natl. Acad. Sci. USA 89:10915). GAP uses the algorithm of Needleman and Wunsch, (1970) J Mol Biol 48:443-53, to find an alignment of two complete sequences that maximizes the number of matches and minimizes the number of gaps. GAP considers all possible alignments and gap positions and creates the alignment with the largest number of matched bases and the fewest gaps, using a gap creation penalty and a gap extension penalty in units of matched bases.

"BLAST" is a searching algorithm provided by the National Center for Biotechnology Information (NCBI) used to find regions of similarity between biological sequences. The program compares nucleotide or protein sequences to sequence databases and calculates the statistical significance of matches to identify sequences having sufficient similarity to a query sequence such that the similarity would not be predicted to have occurred randomly. BLAST reports the identified sequences and their local alignment to the query sequence.

It is well understood by one skilled in the art that many levels of sequence identity are useful in identifying polypeptides from other species or modified naturally or synthetically wherein such polypeptides have the same or similar function or activity. Useful examples of percent identities include, but are not limited to, 50%, 55%, 60%, 65%, 70%, 75%, 80%, 85%, 90% or 95%, or any integer percentage from 50% to 100%. Indeed, any integer amino acid identity from 50% to 100% may be useful in describing the present disclosure, such as 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%.

"Gene" includes a nucleic acid fragment that expresses a functional molecule such as, but not limited to, a specific protein, including regulatory sequences preceding (5' non-coding sequences) and following (3' non-coding sequences) the coding sequence. "Native gene" refers to a gene as found in nature with its own regulatory sequences.

A "mutated gene" is a gene that has been altered through human intervention. Such a "mutated gene" has a sequence that differs from the sequence of the corresponding non-mutated gene by at least one nucleotide addition, deletion, or substitution. In certain embodiments of the disclosure, the mutated gene comprises an alteration that results from a guide polynucleotide/Cas endonuclease system as disclosed herein. A mutated plant is a plant comprising a mutated gene.

As used herein, a "targeted mutation" is a mutation in a native gene that was made by altering a target sequence within the native gene using a method involving a double-strand-break-inducing agent that is capable of inducing a double-strand break in the DNA of the target sequence as disclosed herein or known in the art.

The guide RNA/Cas endonuclease induced targeted mutation can occur in a nucleotide sequence that is located within or outside a genomic target site that is recognized and cleaved by a Cas endonuclease.

Mutation efficiency can be calculated as described herein (see Examples). The mutation efficiency caused by a guideRNA /Cas endonuclease system wherein the guide RNA originates from a recombinant DNA expression cassette comprising a tRNA promoter operably linked to a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast, can be at least 2, 3, 4, 5, 6, 7, 8, 9, 10, 20 fold higher compared to the mutation efficiency caused by a guideRNA /Cas endonuclease system wherein the guide RNA originates from, or is a ribozyme linked single guide RNA.

The term "genome" as it applies to a plant cells encompasses not only chromosomal DNA found within the nucleus, but organelle DNA found within subcellular components (e.g., mitochondria, or plastid) of the cell.

A "codon-modified gene" or "codon-preferred gene" or "codon-optimized gene" is a gene having its frequency of codon usage designed to mimic the frequency of preferred codon usage of the host cell.

An "allele" is one of several alternative forms of a gene occupying a given locus on a chromosome. When all the alleles present at a given locus on a chromosome are the same, that plant is homozygous at that locus. If the alleles present at a given locus on a chromosome differ, that plant is heterozygous at that locus.

"Coding sequence" refers to a polynucleotide sequence which codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to: promoters, translation leader sequences, 5' untranslated sequences, 3' untranslated sequences, introns, polyadenylation target sequences, RNA processing sites, effector binding sites, and stem-loop structures.

"A plant-optimized nucleotide sequence" is nucleotide sequence that has been optimized for increased expression in plants, particularly for increased expression in plants or in one or more plants of interest. For example, a plant-optimized nucleotide sequence can be synthesized by modifying a nucleotide sequence encoding a protein such as, for example, double-strand-break-inducing agent (*e.g.,* an endonuclease) as disclosed herein, using one or more plant-preferred codons for improved expression. See, for example, Campbell and Gowri (1990) Plant Physiol. 92:1-11 for a discussion of host-preferred codon usage.

Methods are available in the art for synthesizing plant-preferred genes. See, for example, U.S. Patent Nos. 5,380,831, and 5,436,391, and Murray et al. (1989) Nucleic Acids Res. 17:477-498, herein incorporated by reference. Additional sequence modifications are known to enhance gene expression in a plant host. These include, for example, elimination of: one or more sequences encoding spurious polyadenylation signals, one or more exon-intron splice site signals, one or more transposon-like repeats, and other such well-characterized sequences that may be deleterious to gene expression. The G-C content of the sequence may be adjusted to levels average for a given plant host, as calculated by reference to known genes expressed in the host plant cell. When possible, the sequence is modified to avoid one or more predicted hairpin secondary mRNA structures. Thus, "a plant-optimized nucleotide sequence" of the present disclosure comprises one or more of such sequence modifications.

A promoter is a region of DNA involved in recognition and binding of RNA polymerase and other proteins to initiate transcription. The promoter sequence consists of proximal and more distal upstream elements, the latter elements often referred to as enhancers. An "enhancer" is a DNA sequence that can stimulate promoter activity, and may be an innate element of the promoter or a heterologous element inserted to enhance the level or tissue-specificity of a promoter. Promoters may be derived in their entirety from a native gene, or be composed of different elements derived from different promoters found in nature, and/or comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity. Promoters that cause a gene to be expressed in most cell types at most times are commonly referred to as "constitutive promoters". It has been shown that certain promoters are able to direct RNA synthesis at a higher rate than others. These are called "strong promoters". Certain other promoters have been shown to direct RNA synthesis at higher levels only in particular types of cells or tissues and are often referred to as "tissue specific promoters", or "tissue-preferred promoters" if the promoter directs RNA synthesis preferably in certain tissues but also in other tissues at reduced levels. Since patterns of expression of a chimeric gene (or genes) introduced into a plant are controlled using promoters, there is an ongoing interest in the isolation of novel promoters which are capable of controlling the expression of a chimeric gene or (genes) at certain levels in specific tissue types or at specific plant developmental stages.

Chemical-regulated promoters can be used to modulate the expression of a gene in a plant through the application of an exogenous chemical regulator. The promoter may be a chemical-inducible promoter, where application of the chemical induces gene expression, or a chemical-repressible promoter, where application of the chemical represses gene expression (De Veylder et al., (1997) Plant Cell Physiol 38:568-77). Tissue-preferred promoters can be utilized to target enhanced expression within a particular plant tissue (Kawamata etal., (1997) Plant Cell Physio/38:792-803). Seed-preferred promoters include both seed-specific promoters active during seed development, as well as seed-germinating promoters active during seed germination (Thompson et al., 1989, BioEssays 10:108

The term "inducible promoter" refers to promoters that selectively express a coding sequence or functional RNA in response to the presence of an endogenous or exogenous stimulus, for example by chemical compounds (chemical inducers) or in response to environmental, hormonal, chemical, and/or developmental signals. Inducible or regulated promoters include, for example, promoters induced or regulated by light, heat, stress, flooding or drought, salt stress, osmotic stress, phytohormones, wounding, or chemicals such as ethanol, abscisic acid (ABA), jasmonate, salicylic acid, or safeners.

Examples of strong promoters useful in certain aspects herein (e.g., fungal and/or yeast cells) herein include those disclosed in U.S. Patent Appl. Publ. Nos. 2012/0252079 (DGAT2), 2012/0252093 (EL1), 2013/0089910 (ALK2), 2013/0089911 (SPS19), 2006/0019297 (GPD and GPM), 2011/0059496 (GPD and GPM), 2005/0130280 (FBA, FBAIN, FBAINm), 2006/0057690 (GPAT) and 2010/0068789 (YAT1), which are incorporated herein by reference. Other examples of strong promoters include XPR2 (U.S. Pat. No. 4937189; EP220864), GPD, GPM (U.S. Pat. Nos. 7259255 and 7459546), TEF (U.S. Pat. No. 6265185), GPDIN (U.S. Pat. No. 7459546, GPM/FBAIN (U.S. Pat. No. 7202356), FBA, FBAIN, FBAINm (U.S. Pat. No. 7202356), GPAT (U.S. Pat. No. 7264949), YAT1 (U.S. Pat. Appl. Publ. No. 2006/0094102) and EXP1(U.S. Pat. No. 7932077). Other examples of strong promoters useful in certain embodiments herein include PGK1, ADH1, TDH3, TEF1, PHO5, LEU2, and GAL1 promoters, as well as strong yeast promoters disclosed in Velculescu et al. (Cell 88:243-251), which is incorporated herein by reference.

"Translation leader sequence" refers to a polynucleotide sequence located between the promoter sequence of a gene and the coding sequence. The translation leader sequence is present in the mRNA upstream of the translation start sequence. The translation leader sequence may affect processing of the primary transcript to mRNA, mRNA stability or translation efficiency. Examples of translation leader sequences have been described (e.g., Turner and Foster, (1995) Mol Biotechnol 3:225-236).

"3' non-coding sequences", "transcription terminator" or "termination sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al., (1989) Plant Cell 1:671-680.

"RNA transcript" refers to the product resulting from RNA polymerase-catalyzed transcription of a DNA sequence. When the RNA transcript is a perfect complimentary copy of the DNA sequence, it is referred to as the primary transcript or pre-mRNA. A RNA transcript is referred to as the mature RNA or mRNA when it is a RNA sequence derived from post-transcriptional processing of the primary transcript pre mRNAt. "Messenger RNA" or "mRNA" refers to the RNA that is without introns and that can be translated into protein by the cell. "cDNA" refers to a DNA that is complementary to, and synthesized from, a mRNA template using the enzyme reverse transcriptase. The cDNA can be single-stranded or converted into double-stranded form using the Klenow fragment of DNA polymerase I. "Sense" RNA refers to RNA transcript that includes the mRNA and can be translated into protein within a cell or *in vitro.* "Antisense RNA" refers to an RNA transcript that is complementary to all or part of a target primary transcript or mRNA, and that blocks the expression of a target gene (see, e.g., U.S. Patent No. 5,107,065). The complementarity of an antisense RNA may be with any part of the specific gene transcript, i.e., at the 5' non-coding sequence, 3' non-coding sequence, introns, or the coding sequence. "Functional RNA" refers to antisense RNA, ribozyme RNA, or other RNA that may not be translated but yet has an effect on cellular processes. The terms "complement" and "reverse complement" are used interchangeably herein with respect to mRNA transcripts, and are meant to define the antisense RNA of the message.

The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., the coding sequence is under the transcriptional control of the promoter). Coding sequences can be operably linked to regulatory sequences in a sense or antisense orientation. In another example, the complementary RNA regions can be operably linked, either directly or indirectly, 5' to the target mRNA, or 3' to the target mRNA, or within the target mRNA, or a first complementary region is 5' and its complement is 3' to the target mRNA.

Standard recombinant DNA and molecular cloning techniques used herein are well known in the art and are described more fully in Sambrook et al., Molecular Cloning: A Laboratory Manual; Cold Spring Harbor Laboratory: Cold Spring Harbor, NY (1989). Transformation methods are well known to those skilled in the art and are described *infra.*

"PCR" or "polymerase chain reaction" is a technique for the synthesis of specific DNA segments and consists of a series of repetitive denaturation, annealing, and extension cycles. Typically, a double-stranded DNA is heat denatured, and two primers complementary to the 3' boundaries of the target segment are annealed to the DNA at low temperature, and then extended at an intermediate temperature. One set of these three consecutive steps is referred to as a "cycle".

The term "recombinant" refers to an artificial combination of two otherwise separated segments of sequence, e.g., by chemical synthesis, or manipulation of isolated segments of nucleic acids by genetic engineering techniques.

The terms "plasmid", "vector" and "cassette" refer to an extra chromosomal element often carrying genes that are not part of the central metabolism of the cell, and usually in the form of double-stranded DNA. Such elements may be autonomously replicating sequences, genome integrating sequences, phage, or nucleotide sequences, in linear or circular form, of a single- or double-stranded DNA or RNA, derived from any source, in which a number of nucleotide sequences have been joined or recombined into a unique construction which is capable of introducing a polynucleotide of interest into a cell. "Transformation cassette" refers to a specific vector containing a gene and having elements in addition to the gene that facilitates transformation of a particular host cell. "Expression cassette" refers to a specific vector containing a gene and having elements in addition to the gene that allow for expression of that gene in a host.

The terms "recombinant DNA molecule", "recombinant construct", "expression construct", "construct", "construct", and "recombinant DNA construct" are used interchangeably herein. A recombinant construct comprises an artificial combination of nucleic acid fragments, e.g., regulatory and coding sequences that are not all found together in nature. For example, a construct may comprise regulatory sequences and coding sequences that are derived from different sources, or regulatory sequences and coding sequences derived from the same source, but arranged in a manner different than that found in nature. Such a construct may be used by itself or may be used in conjunction with a vector. If a vector is used, then the choice of vector is dependent upon the method that will be used to transform host cells as is well known to those skilled in the art. For example, a plasmid vector can be used. The skilled artisan is well aware of the genetic elements that must be present on the vector in order to successfully transform, select and propagate host cells. The skilled artisan will also recognize that different independent transformation events may result in different levels and patterns of expression (Jones et al., (1985) EMBO J 4:2411-2418; De Almeida et al., (1989) Mol Gen Genetics 218:78-86), and thus that multiple events are typically screened in order to obtain lines displaying the desired expression level and pattern. Such screening may be accomplished standard molecular biological, biochemical, and other assays including Southern analysis of DNA, Northern analysis of mRNA expression, PCR, real time quantitative PCR (qPCR), reverse transcription PCR (RT-PCR), immunoblotting analysis of protein expression, enzyme or activity assays, and/or phenotypic analysis.

The term "expression", as used herein, refers to the production of a functional end-product (e.g., an mRNA, guide RNA, or a protein) in either precursor or mature form.

The term "providing" includes providing a nucleic acid (e.g., expression construct) or peptide, polypeitde or protein to a cell. Providing includes reference to the incorporation of a nucleic acid or polypeptide into a eukaryotic or prokaryotic cell where the nucleic acid may be incorporated into the genome of the cell, and includes reference to the transient provision of a nucleic acid or protein to the cell. Providing includes reference to stable or transient transformation methods, transfection, transduction, microinjection, electroporation, viral methods, *Agrobacterium*-mediated transformation, ballistic particle acceleration as well as sexually crossing. Thus, "providing" in the context of inserting a nucleic acid fragment (e.g., a recombinant DNA construct/expression construct, guide RNA, guide DNA, template DNA, donor DNA) into a cell, includes "transfection" or "transformation" or "transduction" and includes reference to the incorporation of a nucleic acid fragment into a eukaryotic or prokaryotic cell where the nucleic acid fragment may be incorporated into the genome of the cell (e.g., chromosome, plasmid, plastid, or mitochondrial DNA), converted into an autonomous replicon, or transiently expressed (e.g., transfected mRNA).

A variety of methods are known for contacting, providing, and/or introducing a composition (such as a nucleotide sequence, a peptide or a polypeptide) into an organisms including stable transformation methods, transient transformation methods, virus-mediated methods, sexual crossing and sexual breeding. Stable transformation indicates that the introduced polynucleotide integrates into the genome of the organism and is capable of being inherited by progeny thereof. Transient transformation indicates that the introduced composition is only temporarily expressed or present in the organism.

Protocols for contacting, providing, introducing polynucleotides and polypeptides to cells or organisms are known. and include microinjection (Crossway et al., (1986) Biotechniques 4:320-34 and U.S. Patent No. 6,300,543), meristem transformation (U.S. Patent No. 5,736,369), electroporation (Riggs et al., (1986) Proc. Natl. Acad. Sci. USA 83:5602-6, Agrobacterium-mediated transformation (U.S. Patent Nos. 5,563,055 and 5,981,840), direct gene transfer (Paszkowski et al., (1984) EMBO J 3:2717-22), and ballistic particle acceleration (U.S. Patent Nos. 4,945,050; 5,879,918; 5,886,244; 5,932,782; Tomes et al., (1995) "Direct DNA Transfer into Intact Plant Cells via Microprojectile Bombardment" in Plant Cell, Tissue, and Organ Culture: Fundamental Methods, ed. Gamborg & Phillips (Springer-Verlag, Berlin); McCabe et al., (1988) Biotechnology 6:923-6; Weissinger et al., (1988) Ann Rev Genet 22:421-77; Sanford et al., (1987) Particulate Science and Technology 5:27-37 (onion); Christou et al., (1988) Plant Physiol 87:671-4 (soybean); Finer and McMullen, (1991) In Vitro Cell Dev Biol 27P:175-82 (soybean); Singh et al., (1998) Theor Appl Genet 96:319-24 (soybean); Datta et al., (1990) Biotechnology 8:736-40 (rice); Klein et al., (1988) Proc. Natl. Acad. Sci. USA 85:4305-9 (maize); Klein et al., (1988) Biotechnology 6:559-63 (maize); U.S. Patent Nos. 5,240,855; 5,322,783 and 5,324,646; Klein et al., (1988) Plant Physiol 91:440-4 (maize); Fromm et al., (1990) Biotechnology 8:833-9 (maize); Hooykaas-Van Slogteren et al., (1984) Nature 311:763-4; U.S. Patent No. 5,736,369 (cereals); Bytebier et al., (1987) Proc. Natl. Acad. Sci. USA 84:5345-9 (Liliaceae); De Wet et al., (1985) in The Experimental Manipulation of Ovule Tissues, ed. Chapman et al., (Longman, New York), pp. 197-209 (pollen); Kaeppler et al., (1990) Plant Cell Rep 9:415-8) and Kaeppler et al., (1992) Theor Appl Genet 84:560-6 (whisker-mediated transformation); D'Halluin et al., (1992) Plant Cell 4:1495-505 (electroporation); Li et al., (1993) Plant Cell Rep 12:250-5; Christou and Ford (1995) Annals Botany 75:407-13 (rice) and Osjoda et al., (1996) Nat Biotechnol 14:745-50 (maize via Agrobacterium tumefaciens).

Alternatively, polynucleotides may be introduced into cells or organisms by contacting cells or organisms with a virus or viral nucleic acids. Generally, such methods involve incorporating a polynucleotide within a viral DNA or RNA molecule. In some examples a polypeptide of interest may be initially synthesized as part of a viral polyprotein, which is later processed by proteolysis *in vivo* or *in vitro* to produce the desired recombinant protein. Methods for introducing polynucleotides into plants and expressing a protein encoded therein, involving viral DNA or RNA molecules, are known, see, for example, U.S. Patent Nos. 5,889,191, 5,889,190, 5,866,785, 5,589,367 and 5,316,931. Transient transformation methods include, but are not limited to, the introduction of polypeptides, such as a double-strand break inducing agent, directly into the organism, the introduction of polynucleotides such as DNA and/or RNA polynucleotides, and the introduction of the RNA transcript, such as an mRNA encoding a double-strand break inducing agent, into the organism. Such methods include, for example, microinjection or particle bombardment. See, for example Crossway et al., (1986) Mol Gen Genet 202:179-85; Nomura et al., (1986) Plant Sci 44:53-8; Hepler et al., (1994) Proc. Natl. Acad. Sci. USA 91:2176-80; and, Hush et al., (1994) J Cell Sci 107:775-84.

Nucleid acids and proteins can be provided to a cell by any method including methods using molecules to facilitate the uptake of anyone or all componens of a guided Cas system (protein and/or nucleic acids), such as cell-penetrating peptides and nanocariers. See also US20110035836 Nanocarier based plant transfection and transduction, and EP 2821486 A1 Method of introducing nucleic acid into plant cells, incorporated herein by reference.

Providing a guide RNA/Cas endonuclease complex to a cell includes providing the individual components of said complex to the cell either directly or via recombination constructs, and includes providing the whole complex to the cell as well.

"Stable transformation" refers to the transfer of a nucleic acid fragment into a genome of a host organism, including both nuclear and organellar genomes, resulting in genetically stable inheritance. In contrast, "transient transformation" refers to the transfer of a nucleic acid fragment into the nucleus, or other DNA-containing organelle, of a host organism resulting in gene expression without integration or stable inheritance. Host organisms containing the transformed nucleic acid fragments are referred to as "transgenic" organisms.

The term "cell" herein refers to any type of cell such as a prokaryotic or eukaryotic cell. A eukaryotic cell has a nucleus and other membrane-enclosed structures (organelles), whereas a prokaryotic cell lacks a nucleus. A cell in certain embodiments can be a mammalian cell or non-mammalian cell. Non-mammalian cells can be eukaryotic or prokaryotic. For example, a non-mammalian cell herein can refer to a microbial cell or cell of a non-mammalian multicellular organism such as a plant, insect, nematode, avian species, amphibian, reptile, or fish.

The terms "control cell" and "suitable control cell" are used interchangeably herein and may be referenced with respect to a cell in which a particular modification (e.g., over-expression of a polynucleotide, down-regulation of a polynucleotide) has been made (i.e., an "experimental cell"). A control cell may be any cell that does not have or does not express the particular modification of the experimental cell. Thus, a control cell may be an untransformed wild type cell or may be genetically transformed but does not express the genetic transformation. For example, a control cell may be a direct parent of the experimental cell, which direct parent cell does not have the particular modification that is in the experimental cell. Alternatively, a control cell may be a parent of the experimental cell that is removed by one or more generations. Alternatively, a control cell may be a sibling of the experimental cell, which sibling does not comprise the particular modification that is present in the experimental cell.

A microbial cell herein can refer to a fungal cell (e.g., yeast cell), prokaryotic cell, protist cell (e.g., algal cell), euglenoid cell, stramenopile cell, or oomycete cell, for example. A prokaryotic cell herein can refer to a bacterial cell or archaeal cell, for example. Fungal cells (e.g., yeast cells), protist cells (e.g., algal cells), euglenoid cells, stramenopile cells, and oomycete cells represent examples of eukaryotic microbial cells. A eukaryotic microbial cell has a nucleus and other membrane-enclosed structures (organelles), whereas a prokaryotic cell lacks a nucleus.

The term "yeast" herein refers to fungal species that predominantly exist in unicellular form. Yeast can alternatively be referred to as "yeast cells". A yeast in certain aspects herein can be one that reproduces asexually (anamorphic) or sexually (teleomorphic). While yeast herein typically exist in unicellular form, certain types of these yeast may optionally be able to form pseudohyphae (strings of connected budding cells). In still further aspects, a yeast may be haploid or diploid, and/or may have the ability to exist in either of these ploidy forms. A yeast herein can be characterized as either a conventional yeast or non-conventional yeast, for example.

The term "conventional yeast" ("model yeast") herein generally refers to Saccharomyces or Schizosaccharomyces yeast species. Conventional yeast include yeast that favor homologous recombination (HR) DNA repair processes over repair processes mediated by non-homologous end-joining (NHEJ). Examples of conventional yeast herein include species of the genera *Saccharomyces* (e.g., *S. cerevisiae,* which is also known as budding yeast, baker's yeast, and/or brewer's yeast; *S*. *bayanus; S. boulardii; S. bulderi; S. cariocanus; S. cariocus; S. chevalieri; S. dairenensis; S. ellipsoideus; S. eubayanus; S. exiguus; S. florentinus; S. kluyveri; S. martiniae; S. monacensis; S. norbensis; S. paradoxus; S. pastorianus; S. spencerorum;* S. *turicensis; S. unisporus; S. uvarum; S. zonatus*) and *Schizosaccharomyces* (e.g., *S*. *pombe,* which is also known as fission yeast; *S*. *cryophilus; S. japonicus; S. octosporus*)*.*

The term "non-conventional yeast" herein refers to any yeast that is not a *Saccharomyces* (e.g., *S*. *cerevisiae*) or *Schizosaccharomyces* yeast species. Non-conventional yeast are described in Non-Conventional Yeasts in Genetics, Biochemistry and Biotechnology: Practical Protocols (K. Wolf, K.D. Breunig, G. Barth, Eds., Springer-Verlag, Berlin, Germany, 2003), which is incorporated herein by reference. Non-conventional yeast in certain embodiments may additionally (or alternatively) be yeast that favor non-homologous end-joining (NHEJ) DNA repair processes over repair processes mediated by homologous recombination (HR).

Conventional yeasts such as *S*. *cerevisiae* and *S*. *pombe* typically exhibit specific integration of donor DNA with short flanking homology arms (30-50 bp) with efficiencies routinely over 70%, whereas non-conventional yeasts such as *Pichia pastoris, Pichia stipitis, Hansenula polymorpha, Yarrowia lipolytica* and *Kluyveromyces lactis* usually show specific integration with similarly structured donor DNA at efficiencies of less than 1% (Chen et al., PLoS ONE 8:e57952). Thus, a preference for HR processes can be gauged, for example, by transforming yeast with a suitable donor DNA and determining the degree to which it is specifically recombined with a genomic site predicted to be targeted by the donor DNA. A preference for NHEJ (or low preference for HR), for example, would be manifest if such an assay yielded a high degree of random integration of the donor DNA in the yeast genome. Assays for determining the rate of specific (HR-mediated) and/or random (NHEJ-mediated) integration of DNA in yeast are known in the art (e.g., Ferreira and Cooper, Genes Dev. 18:2249-2254; Corrigan et al., PLoS ONE 8:e69628; Weaver et al., Proc. Natl. Acad. Sci. U.S.A. 78:6354-6358; Keeney and Boeke, Genetics 136:849-856).

Given their low level of HR activity, non-conventional yeast herein can (i) exhibit a rate of specific targeting by a suitable donor DNA having 30-50 bp flanking homology arms of less than about 1%, 2%, 3%, 4%, 5%, 6%, 7%, or 8%, for example, and/or (ii) exhibit a rate of random integration of the foregoing donor DNA of more than about 65%, 66%, 67%, 68%, 69%, 70%, 71%, 72%, 73%, 74%, or 75%, for example. These rates of (i) specific targeting and/or (ii) random integration of a suitable donor DNA can characterize a non-conventional yeast as it exists before being provided an RGEN as disclosed herein. An aim for providing an RGEN to a non-conventional yeast in certain embodiments is to create site-specific DNA single-strand breaks (SSB) or double-strand breaks (DSB) for biasing the yeast toward HR at the specific site. Thus, providing a suitable RGEN in a non-conventional yeast typically should allow the yeast to exhibit an increased rate of HR with a particular donor DNA. Such an increased rate can be at least about 2-, 3-, 4-, 5-, 6-, 7-, 8-, 9-, or 10-fold higher than the rate of HR in a suitable control (e.g., same non-conventional yeast transformed with the same donor DNA, but lacking a suitable RGEN).

A non-conventional yeast herein can be cultivated following any means known in the art, such as described in Non-Conventional Yeasts in Genetics, Biochemistry and Biotechnology Practical Protocols (K. Wolf, K.D. Breunig, G. Barth, Eds., Springer-Verlag, Berlin, Germany, 2003), Yeasts in Natural and Artificial Habitats (J.F.T. Spencer, D.M. Spencer, Eds., Springer-Verlag, Berlin, Germany, 1997), and/or Yeast Biotechnology: Diversity and Applications (T. Satyanarayana, G. Kunze, Eds., Springer, 2009), all of which are incorporated herein by reference.

Non-limiting examples of non-conventional yeast herein include yeasts of the following genera: *Yarrowia, Pichia, Schwanniomyces, Kluyveromyces, Arxula, Trichosporon, Candida, Ustilago, Torulopsis, Zygosaccharomyces, Trigonopsis, Cryptococcus, Rhodotorula, Phaffia, Sporobolomyces, Pachysolen,* and *Moniliella.* A suitable example of a *Yarrowia* species is *Y. lipolytica.* Suitable examples of *Pichia* species include *P. pastoris, P. methanolica, P. stipitis, P. anomala* and *P. angusta.* Suitable examples of *Schwanniomyces* species include *S*. *castellii, S. alluvius, S. hominis, S. occidentalis, S*. capriottii, *S*. *etchellsii, S. polymorphus, S. pseudopolymorphus, S. vanrijiae* and *S*. *yamadae.* Suitable examples of *Kluyveromyces* species include *K. lactis, K. marxianus, K. fragilis, K. drosophilarum, K. thermotolerans, K. phaseolosporus, K. vanudenii, K. waltii, K. africanus* and *K*. *polysporus.* Suitable examples of *Arxula* species include *A. adeninivorans* and *A*. *terrestre.* Suitable examples of *Trichosporon* species include *T. cutaneum, T. capitatum, T. inkin* and *T. beemeri.* Suitable examples of *Candida* species include *C*. *albicans, C. ascalaphidarum, C. amphixiae, C. antarctica, C. apicola, C. argentea, C. atlantica, C. atmosphaerica, C. blattae, C. bromeliacearum, C. carpophila, C. carvajalis, C. cerambycidarum, C. chauliodes, C. corydali, C. dosseyi, C. dubliniensis, C. ergatensis, C. fructus, C. glabrata, C. fermentati, C. guilliermondii, C. haemulonii, C. insectamens, C. insectorum, C. intermedia, C. jeffresii, C. kefyr, C*. *keroseneae, C*. *krusei, C. lusitaniae, C. lyxosophila, C. maltosa, C. marina, C. membranifaciens, C. milleri, C. mogii, C. oleophila, C. oregonensis, C. parapsilosis, C. quercitrusa, C. rugosa, C*. *sake, C*. *shehatea, C. temnochilae, C. tenuis, C. theae, C. tolerans, C. tropicalis, C. tsuchiyae, C. sinolaborantium, C. sojae, C. subhashii, C. viswanathii, C. utilis, C. ubatubensis* and *C. zemplinina.* Suitable examples of *Ustilago* species include *U. avenae, U. esculenta, U. hordei, U. maydis, U. nuda* and *U. tritici.* Suitable examples of *Torulopsis* species include *T. geochares, T. azyma, T. glabrata* and *T. candida.* Suitable examples of *Zygosaccharomyces* species include *Z*. *bailii, Z. bisporus, Z. cidri, Z. fermentati, Z. florentinus, Z. kombuchaensis, Z. lentus, Z. mellis, Z. microellipsoides, Z. mrakii, Z. pseudorouxii* and *Z*. *rouxii.* Suitable examples of *Trigonopsis* species include *T. variabilis.* Suitable examples of *Cryptococcus* species include *C. laurentii, C. albidus, C. neoformans, C. gattii, C. uniguttulatus, C. adeliensis, C. aerius, C. albidosimilis, C. antarcticus, C. aquaticus, C. ater, C. bhutanensis, C. consortionis, C. curvatus, C. phenolicus, C. skinneri, C. terreus* and *C*. *vishniacci.* Suitable examples of *Rhodotorula* species include *R. acheniorum, R. tula, R. acuta, R. americana, R. araucariae, R. arctica, R. armeniaca, R. aurantiaca, R. auriculariae, R. bacarum, R. benthica, R. biourgei, R. bogoriensis, R. bronchialis, R. buffonii, R. calyptogenae, R. chungnamensis, R. cladiensis, R. corallina, R. cresolica, R. crocea, R. cycloclastica, R. dairenensis, R. diffluens, R. evergladiensis, R. ferulica, R. foliorum, R. fragaria, R. fujisanensis, R. futronensis, R. gelatinosa, R. glacialis, R. glutinis, R. gracilis, R. graminis, R. grinbergsii, R. himalayensis, R. hinnulea, R. histolytica, R. hylophila, R. incarnata, R. ingeniosa, R. javanica, R. koishikawensis, R. lactosa, R. lamellibrachiae, R. laryngis, R. lignophila, R. lini, R. longissima, R. ludwigii, R. lysinophila, R. marina, R. martyniae-fragantis, R. matritensis, R. meli, R. minuta, R. mucilaginosa, R. nitens, R. nothofagi, R. oryzae, R. pacifica, R. pallida, R. peneaus, R. philyla, R. phylloplana, R. pilatii, R. pilimanae, R. pinicola, R. plicata, R. polymorpha, R. psychrophenolica, R. psychrophila, R. pustula, R. retinophila, R. rosacea, R. rosulata, R. rubefaciens, R. rubella, R. rubescens, R. rubra, R. rubrorugosa, R. rufula, R. rutila, R. sanguinea, R. sanniei, R. sartoryi, R. silvestris, R. simplex, R. sinensis, R. slooffiae, R. sonckii, R. straminea, R. subericola, R. suganii, R. taiwanensis, R. taiwaniana, R. terpenoidalis, R. terrea, R. texensis, R. tokyoensis, R. ulzamae, R. vanillica, R. vuilleminii, R. yarrowii, R. yunnanensis* and *R. zsoltii.* Suitable examples of *Phaffia* species include *P. rhodozyma.* Suitable examples of *Sporobolomyces* species include *S*. *alborubescens, S*. *bannaensis, S. beijingensis, S. bischofiae, S. clavatus, S. coprosmae, S. coprosmicola, S. corallinus, S. dimmenae, S. dracophylli, S. elongatus, S. gracilis, S. inositophilus, S. johnsonii, S*. *koalae, S*. *magnisporus, S*. *novozealandicus, S. odorus, S*. *patagonicus, S. productus, S*. *roseus, S*. *sasicola, S. shibatanus, S*. *singularis, S. subbrunneus, S*. *symmetricus, S*. *syzygii, S. taupoensis, S*. *tsugae, S. xanthus* and *S*. *yunnanensis.* Suitable examples of *Pachysolen* and *Moniliella* species include *P. tannophilus* and *M. pollinis,* respectively. Still other examples of non-conventional yeasts herein include *Pseudozyma* species (e.g., *S*. *antarctica*)*, Thodotorula* species (e.g., *T. bogoriensis*)*, Wickerhamiella* species (e.g., *W. domercqiae*)*,* and *Starmerella* species (e.g., *S*. *bombicola*)*.*

*Yarrowia lipolytica* is preferred in certain embodiments disclosed herein. Examples of suitable *Y. lipolytica* include the following isolates available from the American Type Culture Collection (ATCC, Manassas, VA): strain designations ATCC #20362, #8862, #8661, #8662, #9773, #15586, #16617, #16618, #18942, #18943, #18944, #18945, #20114, #20177, #20182, #20225, #20226, #20228, #20327, #20255, #20287, #20297, #20315, #20320, #20324, #20336, #20341, #20346, #20348, #20363, #20364, #20372, #20373, #20383, #20390, #20400, #20460, #20461, #20462, #20496, #20510, #20628, #20688, #20774, #20775, #20776, #20777, #20778, #20779, #20780, #20781, #20794, #20795, #20875, #20241, #20422, #20423, #32338, #32339, #32340, #32341, #34342, #32343, #32935, #34017, #34018, #34088, #34922, #34922, #38295, #42281, #44601, #46025, #46026, #46027, #46028, #46067, #46068, #46069, #46070, #46330, #46482, #46483, #46484, #46436, #60594, #62385, #64042, #74234, #76598, #76861, #76862, #76982, #90716, #90811, #90812, #90813, #90814, #90903, #90904, #90905, #96028, #201241, #201242, #201243, #201244, #201245, #201246, #201247, #201249, and/or #201847.

A fungal cell herein can be a yeast (e.g., as described above) or of any other fungal type such as a filamentous fungus. For instance, a fungus herein can be a Basidiomycetes, Zygomycetes, Chytridiomycetes, or Ascomycetes fungus. Examples of filamentous fungi herein include those of the genera *Trichoderma, Chrysosporium, Thielavia, Neurospora* (e.g., *N. crassa, N. sitophila*)*, Cryphonectria* (e.g., *C*. *parasitica*)*, Aureobasidium* (e.g., *A. pullulans*)*, Filibasidium, Piromyces, Cryplococcus, Acremonium, Tolypocladium, Scytalidium, Schizophyllum, Sporotrichum, Penicillium* (e.g., *P. bilaiae, P. camemberti, P. candidum, P. chrysogenum, P. expansum, P. funiculosum, P. glaucum, P. marneffei, P. roqueforti, P. verrucosum, P. viridicatum*), *Gibberella* (e.g., *G. acuminata, G*. *avenacea, G. baccata, G. circinata, G. cyanogena, G. fujikuroi, G. intricans, G. pulicaris, G. stilboides, G. tricincta, G*. zeae), *Myceliophthora, Mucor* (e.g., *M. rouxii, M. circinelloides*)*, Aspergillus* (e.g., *A. niger, A. oryzae, A. nidulans, A. flavus, A. lentulus, A. terreus, A. clavatus, A. fumigatus), Fusarium* (e.g., *F. graminearum, F. oxysporum, F. bubigenum, F. solani, F. oxysporum, F. verticillioides, F. proliferatum, F. venenatum*)*,* and *Humicola,* and anamorphs and teleomorphs thereof. The genus and species of fungi herein can be defined, if desired, by morphology as disclosed in Barnett and Hunter (Illustrated Genera of Imperfect Fungi, 3rd Edition, Burgess Publishing Company, 1972). A fungus can optionally be characterized as a pest/pathogen of a plant or animal (e.g., human) in certain embodiments.

*Trichoderma* species in certain aspects herein include *T. aggressivum, T. amazonicum, T. asperellum, T. atroviride, T. aureoviride, T. austrokoningii, T. brevicompactum, T. candidum, T. caribbaeum, T. catoptron, T. cremeum, T. ceramicum, T. cerinum, T. chlorosporum, T. chromospermum, T. cinnamomeum, T. citrinoviride, T. crassum, T. cremeum, T. dingleyeae, T. dorotheae, T. effusum, T. erinaceum, T. estonicum, T. fertile, T. gelatinosus, T. ghanense, T. hamatum, T. harzianum, T. helicum, T. intricatum, T. konilangbra, T. koningii, T. koningiopsis, T. longibrachiatum, T. longipile, T. minutisporum, T. oblongisporum, T. ovalisporum, T. petersenii, T. phyllostahydis, T. piluliferum, T. pleuroticola, T. pleurotum, T. polysporum, T. pseudokoningii, T. pubescens, T. reesei, T. rogersonii, T. rossicum, T. saturnisporum, T. sinensis, T. sinuosum, T. spirale, T. stramineum, T. strigosum, T. stromaticum, T. surrotundum, T. taiwanense, T. thailandicum, T. thelephoricolum, T. theobromicola, T. tomentosum, T. velutinum, T. virens, T. viride* and *T. viridescens.* A *Trichoderma* species herein can be cultivated and/or manipulated as described in Trichoderma: Biology and Applications (P.K. Mukherjee et al., Eds., CABI, Oxfordshire, UK, 2013), for example, which is incorporated herein by reference.

A microbial cell in certain embodiments is an algal cell. For example, an algal cell can be from any of the following: Chlorophyta (green algae), Rhodophyta (red algae), Phaeophyceae (brown algae), Bacillariophycaeae (diatoms), and Dinoflagellata (dinoflagellates). An algal cell can be of amicroalgae (e.g., phytoplankton, microphytes, or planktonic algae) or macroalgae (kelp, seaweed) in other aspects. As further examples, an algal cell herein can be a *Porphyra* (purple laver), *Palmaria* species such as *P. palmata* (dulse), *Arthrospira* species such as *A*. *platensis* (*spirulina*)*, Chlorella* (e.g., *C. protothecoides*)*,* a *Chondrus* species such as *C. crispus* (Irish moss), *Aphanizomenon, Sargassum, Cochayuyo, Botryococcus* (e.g., *B. braunii*)*, Dunaliella* (e.g., *D. tertiolecta*), *Gracilaria, Pleurochrysis* (e.g., *P. carterae*)*, Ankistrodesmus, Cyclotella, Hantzschia, Nannochloris, Nannochloropsis, Nitzschia, Phaeodactylum* (e.g., *P. tricornutum*), *Scenedesmus, Stichococcus, Tetraselmis* (e.g., *T. suecica*)*, Thalassiosira* (e.g., *T. pseudonana*)*, Crypthecodinium* (e.g., *C. cohnii*)*, Neochloris* (e.g., *N. oleoabundans*), or *Schiochytrium.* An algal species herein can be cultivated and/or manipulated as described in Thompson (Algal Cell Culture. Encyclopedia of Life Support System (EOLSS), Biotechnology Vol 1, available at eolss.net/sample-chapters internet site), for example, which is incorporated herein by reference.

A protist cell herein can be selected from the class Ciliata (e.g., the genera *Tetrahymena, Paramecium, Colpidium, Colpoda, Glaucoma, Platyophrya, Vorticella, Potomacus, Pseudocohnilembus, Euplotes, Engelmaniella,* and *Stylonichia*), the subphylum Mastigophora (flagellates), the class Phytomastigophorea (e.g., the genera *Euglena, Astasia, Haematococcus,* and *Crypthecodinium*), the class Zoomastigophorea, the superclass Rhizopoda, the class Lobosea (e.g., the genus *Amoeba*), and the class Eumycetozoea (e.g., the genera *Dictyostelium* and *Physarum*), for example. Certain protist species herein can be cultivated and/or manipulated as described in ATCC® Protistology Culture Guide: tips and techniques for propagating protozoa and algae (2013, available at American Type Culture Collection internet site), for example, which is incorporated herein by reference. A protist can optionally be characterized as a pest/pathogen of a plant or animal (e.g., human) in certain embodiments.

A bacterial cell in certain embodiments can be those in the form of cocci, bacilli, spirochetes, spheroplasts, protoplasts, etc. Other non-limiting examples of bacteria include those that are Gram-negative and Gram-positive. Still other non-limiting examples of bacteria include those of the genera *Salmonella* (e.g., *S*. *typhi, S. enteritidis*)*, Shigella* (e.g., *S*. *dysenteriae*)*, Escherichia* (e.g., *E. coli*)*, Enterobacter, Serratia, Proteus, Yersinia, Citrobacter, Edwardsiella, Providencia, Klebsiella, Hafnia, Ewingella, Kluyvera, Morganella, Planococcus, Stomatococcus, Micrococcus, Staphylococcus* (e.g., *S*. *aureus, S. epidermidis*)*, Vibrio* (e.g., *V. cholerae*)*, Aeromonas, Plessiomonas, Haemophilus* (e.g., *H. influenzae*)*, Actinobacillus, Pasteurella, Mycoplasma* (e.g., *M. pneumonia*)*, Ureaplasma, Rickettsia, Coxiella, Rochalimaea, Ehrlichia, Streptococcus* (e.g., *S*. *pyogenes, S. mutans, S. pneumoniae*)*, Enterococcus* (e.g., *E. faecalis*)*, Aerococcus, Gemella, Lactococcus* (e.g., *L. lactis*)*, Leuconostoc* (e.g., *L. mesenteroides*)*, Pedicoccus, Bacillus* (e.g., B. *cereus,* B. *subtilis, B. thuringiensis*)*, Corynebacterium* (e.g., *C. diphtheriae*)*, Arcanobacterium, Actinomyces, Rhodococcus, Listeria* (e.g., *L. monocytogenes*)*, Erysipelothrix, Gardnerella, Neisseria* (e.g., *N. meningitidis, N. gonorrhoeae*)*, Campylobacter, Arcobacter, Wolinella, Helicobacter* (e.g., *H. pylori*)*, Achromobacter, Acinetobacter, Agrobacterium* (e.g., *A. tumefaciens*)*, Alcaligenes, Chryseomonas, Comamonas, Eikenella, Flavimonas, Flavobacterium, Moraxella, Oligella, Pseudomonas* (e.g., *P. aeruginosa*)*, Shewanella, Weeksella, Xanthomonas, Bordetella, Franciesella, Brucella, Legionella, Afipia, Bartonella, Calymmatobacterium, Cardiobacterium, Streptobacillus, Spirillum, Peptostreptococcus, Peptococcus, Sarcinia, Coprococcus, Ruminococcus, Propionibacterium, Mobiluncus, Bifidobacterium, Eubacterium, Lactobacillus* (e.g., *L. lactis, L. acidophilus*)*, Rothia, Clostridium* (e.g., *C. botulinum, C. perfringens*)*, Bacteroides, Porphyromonas, Prevotella, Fusobacterium, Bilophila, Leptotrichia, Wolinella, Acidaminococcus, Megasphaera, Veilonella, Norcardia, Actinomadura, Norcardiopsis, Streptomyces, Micropolysporas, Thermoactinomycetes, Mycobacterium* (e.g., *M. tuberculosis, M. bovis, M. leprae*)*, Treponema, Borrelia* (e.g., *B. burgdorferi*)*, Leptospira, and Chlamydiae.* A bacteria can optionally be characterized as a pest/pathogen of a plant or animal (e.g., human) in certain embodiments. Bacteria can be comprised in a mixed microbial population (e.g., containing other bacteria, or containing yeast and/or other bacteria) in certain embodiments.

An archaeal cell in certain embodiments can be from any Archaeal phylum, such as Euryarchaeota, Crenarchaeota, Nanoarchaeota, Korarchaeota, Aigarchaeota, or Thaumarchaeota. Archaeal cells herein can be extremophilic (e.g., able to grow and/or thrive in physically or geochemically extreme conditions that are detrimental to most life), for example. Some examples of extremophilic archaea include those that are thermophilic (e.g., can grow at temperatures between 45-122 °C), hyperthermophilic (e.g., can grow at temperatures between 80-122°C), acidophilic (e.g., can grow at pH levels of 3 or below), alkaliphilic (e.g., can grow at pH levels of 9 or above), and/or halophilic (e.g., can grow in high salt concentrations [e.g., 20-30% NaCl]). Examples of archaeal species include those of the genera *Halobacterium* (e.g., *H. volcanii*)*, Sulfolobus* (e.g., *S. solfataricus, S. acidocaldarius*)*, Thermococcus* (e.g., *T. alcaliphilus, T. celer, T. chitonophagus, T. gammatolerans, T. hydrothermalis, T. kodakarensis, T. litoralis, T. peptonophilus, T. profundus, T. stetteri), Methanocaldococcus* (e.g., *M. thermolithotrophicus, M. jannaschii), Methanococcus* (e.g., *M. maripaludis*)*, Methanothermobacter* (e.g., *M. marburgensis, M. thermautotrophicus*)*, Archaeoglobus* (e.g., *A. fulgidus*)*, Nitrosopumilus* (e.g., *N. maritimus*)*, Metallosphaera* (e.g., *M. sedula*)*, Ferroplasma, Thermoplasma, Methanobrevibacter* (e.g., *M. smithii*)*,* and *Methanosphaera* (e.g., *M. stadtmanae*)*.*

Examples of insect cells herein include *Spodoptera frugiperda* cells, *Trichoplusia ni* cells, *Bombyx mori* cells and the like. *S*. *frugiperda* cells include Sf9 and Sf21, for instance. *T. ni* ovary cells include HIGH FIVE cells (alias BTI-TN-5B1-4, manufactured by Invitrogen), for example. B. mori cells include N4, for example. Certain insect cells herein can be cultivated and/or manipulated as described in Growth and Maintenance of Insect cell lines (2010, Invitrogen, Manual part no. 25-0127, MAN0000030), for example, which is incorporated herein by reference. In other aspects, an insect cell can be a cell of a plant pest/pathogen such as an armyworm, black cutworm, corn earworm, corn flea beetle, corn leaf aphid, corn root aphid, European corn borer, fall armyworm, granulate cutworm, Japanese beetle, lesser cornstalk borer, maize billbug, melanotus communis, seedcorn maggot, sod webworms, sorghum midge, sorghum webworm, southern corn billbug, southern corn rootworm, southern cornstalk borer, southern potato wireworm, spider mite, stalk borer, sugarcane beetle, tobacco wireworm, white grub, aphid, boll weevil, bollworm complex, cabbage looper, tarnished plant bug, thrip, two spotted spider mite, yellow striped armyworm, alfalfa weevil, clover leaf weevil, clover root curculio, fall armyworm, grasshopper, meadow spittlebug, pea aphid, potato leafhopper, sod webworm, variegated cutworm, lesser cornstalk borer, tobacco thrip, wireworm, cereal leaf beetle, chinch bug, English grain aphid, greenbug, hessian fly, bean leaf beetle, beet armyworm, blister beetle, grape colaspis, green cloverworm, Mexican bean beetle, soybean looper, soybean stem borer, stink bug, three-cornered alfalfa hopper, velvetbean caterpillar, budworm, cabbage looper, cutworm, green june beetle, green peach aphid, hornworm, potato tuberworm, southern mole cricket, suckfly, tobacco flea beetle, vegetable weevil, or whitefringed beetle. Alternatively, an insect cell can be a cell of a pest/pathogen of an animal (e.g., human).

A nematode cell, for example, can be of a nematode from any of the following genera: *Meloidogyne* (root-knot nematode), *Pratylenchus* (lesion nematode), *Heterodera* (cyst nematode), *Globodera* (cyst nematode), *Ditylenchus* (stem and bulb nematode), *Tylenchulus* (citrus nematode), *Xiphinema* (dagger nematode), *Radopholus* (burrowing nematode), *Rotylenchulus* (reniform nematode), *Helicotylenchus* (spiral nematode), or *Belonolaimus* (sting nematode). A nematode can optionally be characterized as a pest/pathogen of a plant or animal (e.g., human) in certain embodiments. A nematode can be *C*. *elegans* in other aspects.

A fish cell herein can be any of those as disclosed in U.S. Patent Nos. 7408095 and 7217564, and Tissue Culture of Fish Cell Lines (T. Ott, NWFHS Laboratory Procedures Manual - Second Edition, Chapter 10, 2004), for example, which are incorporated herein by reference. These references also disclose information regarding cultivating and/or manipulating fish cells. Non-limiting examples of fish cells can be from a teleost such as zebrafish, medaka, Giant rerio, or puffer fish.

Mammalian cells in certain embodiments can be human, non-human primate (e.g., monkey, ape), rodent (e.g., mouse, rat, hamster, guinea pig), rabbit, dog, cat, cow, pig, horse, goat, or sheep cells. Other examples of mammalian cells herein include primary epithelial cells (e.g., keratinocytes, cervical epithelial cells, bronchial epithelial cells, tracheal epithelial cells, kidney epithelial cells, retinal epithelial cells); established cell lines (e.g., 293 embryonic kidney cells, HeLa cervical epithelial cells, PER-C6 retinal cells, MDBK, CRFK, MDCK, CHO, BeWo, Chang cells, Detroit 562, Hep-2, KB, LS 180, LS 174T, NCI-H-548, RPMI 2650, SW-13, T24, WI-28 VA13, 2RA, WISH, BS-C-I, LLC-MK2, Clone M-3, RAG, TCMK-1, LLC-PK1, PK-15, GH1, GH3, L2, LLC-RC 256, MH1C1, XC, MDOK, VSW, TH-I, B1 cells); any epithelial, mesenchymal (e.g., fibroblast), neural, or muscular cell from any tissue or organ (e.g., skin, heart; liver; kidney; colon; intestine; esophagus; stomach; neural tissue such as brain or spinal cord; lung; vascular tissue; lymphoid tissue such as lymph gland, adenoid, tonsil, bone marrow, or blood; spleen); and fibroblast or fibroblast-like cell lines (e.g., TRG-2, IMR-33, Don cells, GHK-21, citrullinemia cells, Dempsey cells, Detroit 551, Detroit 510, Detroit 525, Detroit 529, Detroit 532, Detroit 539, Detroit 548, Detroit 573, HEL 299, IMR-90, MRC-5, WI-38, WI-26, MiCl1, CV-1, COS-1, COS-3, COS-7, Vero, DBS-FrhL-2, BALB/3T3, F9, SV-T2, M-MSV-BALB/3T3, K-BALB, BLO-11, NOR-10, C3H/IOTI/2, HSDM1C3, KLN205, McCoy cells, Mouse L cells, SCC-PSA1, Swiss/3T3 cells, Indian muntjac cells, SIRC, Jensen cells). Methods of culturing and manipulating mammalian cells lines are known in the art.

The term "plant" refers to whole plants, plant organs, plant tissues, seeds, plant cells, seeds and progeny of the same. Plant cells include, without limitation, cells from seeds, suspension cultures, embryos, meristematic regions, callus tissue, leaves, roots, shoots, gametophytes, sporophytes, pollen and microspores. Plant parts include differentiated and undifferentiated tissues including, but not limited to roots, stems, shoots, leaves, pollens, seeds, tumor tissue and various forms of cells and culture (e.g., single cells, protoplasts, embryos, and callus tissue). The plant tissue may be in plant or in a plant organ, tissue or cell culture. The term "plant organ" refers to plant tissue or a group of tissues that constitute a morphologically and functionally distinct part of a plant. The term "genome" refers to the entire complement of genetic material (genes and non-coding sequences) that is present in each cell of an organism, or virus or organelle; and/or a complete set of chromosomes inherited as a (haploid) unit from one parent. "Progeny" comprises any subsequent generation of a plant.

A transgenic plant includes, for example, a plant which comprises within its genome a heterologous polynucleotide introduced by a transformation step. The heterologous polynucleotide can be stably integrated within the genome such that the polynucleotide is passed on to successive generations. The heterologous polynucleotide may be integrated into the genome alone or as part of a recombinant DNA construct. A transgenic plant can also comprise more than one heterologous polynucleotide within its genome. Each heterologous polynucleotide may confer a different trait to the transgenic plant. A heterologous polynucleotide can include a sequence that originates from a foreign species, or, if from the same species, can be substantially modified from its native form. Transgenic can include any cell, cell line, callus, tissue, plant part or plant, the genotype of which has been altered by the presence of heterologous nucleic acid including those transgenics initially so altered as well as those created by sexual crosses or asexual propagation from the initial transgenic. The alterations of the genome (chromosomal or extra-chromosomal) by conventional plant breeding methods, by the genome editing procedure described herein that does not result in an insertion of a foreign polynucleotide, or by naturally occurring events such as random cross-fertilization, non-recombinant viral infection, non-recombinant bacterial transformation, non-recombinant transposition, or spontaneous mutation are not intended to be regarded as transgenic.

A fertile plant is a plant that produces viable male and female gametes and is self-fertile. Such a self-fertile plant can produce a progeny plant without the contribution from any other plant of a gamete and the genetic material contained therein. Male-sterile plants include plants that do not produce male gametes that are viable or otherwise capable of fertilization. Female-sterile plants include plants that do not produce female gametes that are viable or otherwise capable of fertilization. It is recognized that male-sterile and female-sterile plants can be female-fertile and male-fertile, respectively. It is further recognized that a male-fertile (but female-sterile) plant can produce viable progeny when crossed with a female-fertile plant and that a female-fertile (but male-sterile) plant can produce viable progeny when crossed with a male-fertile plant.

Any plant can be used, including monocot and dicot plants. Examples of monocot plants that can be used include, but are not limited to, corn (Zea *mays*)*,* rice (*Oryza sativa*)*,* rye (*Secale cereale*)*,* sorghum (*Sorghum bicolor, Sorghum vulgare*)*,* millet (e.g., pearl millet (*Pennisetum glaucum*)*,* proso millet (*Panicum miliaceum*)*,* foxtail millet (*Setaria italica*)*,* finger millet (*Eleusine coracana*))*,* wheat (*Triticum aestivum*)*,* sugarcane (*Saccharum spp.*)*,* oats (*Avena*)*,* barley (*Hordeum*)*,* switchgrass (*Panicum virgatum*)*,* pineapple (*Ananas comosus*)*,* banana (*Musa spp.*)*,* palm, ornamentals, turfgrasses, and other grasses. Examples of dicot plants that can be used include, but are not limited to, soybean (*Glycine max*)*,* canola (*Brassica napus* and *B. campestris*)*,* alfalfa (*Medicago sativa*)*,* tobacco (*Nicotiana tabacum*)*, Arabidopsis* (*Arabidopsis thaliana*)*,* sunflower (*Helianthus annuus*)*,* cotton (*Gossypium arboreum*)*,* and peanut (*Arachis hypogaea*)*,* tomato (*Solanum lycopersicum*)*,* potato (*Solanum tuberosum*) *etc.*

The term "dicot" refers to the subclass of angiosperm plants also knows as "dicotyledoneae" and includes reference to whole plants, plant organs (e.g., leaves, stems, roots, etc.), seeds, plant cells, and progeny of the same. Plant cell, as used herein includes, without limitation, seeds, suspension cultures, embryos, meristematic regions, callus tissue, leaves, roots, shoots, gametophytes, sporophytes, pollen, and microspores.

The terms "5'-cap" and "7-methylguanylate (m⁷G) cap" are used interchangeably herein. A 7-methylguanylate residue is located on the 5' terminus of RNA transcribed by RNA polymerase II (Pol II) in eukaryotes. A capped RNA herein has a 5'-cap, whereas an uncapped RNA does not have such a cap.

The terminology "uncapped", "not having a 5'-cap", and the like are used interchangeably herein to refer to RNA lacking a 5'-cap and optionally having, for example, a 5'-hydroxyl group instead of a 5'-cap. Uncapped RNA can better accumulate in the nucleus following transcription, since 5'-capped RNA is subject to nuclear export.

The terms "ribozyme", "ribonucleic acid enzyme" and "self-cleaving ribozyme" are used interchangeably herein. A ribozyme refers to one or more RNA sequences that form secondary, tertiary, and/or quaternary structure(s) that can cleave RNA at a specific site, particularly at a cis-site relative to the ribozyme sequence (i.e., autocatalytic, or self-cleaving). The general nature of ribozyme nucleolytic activity has been described (e.g., Lilley, Biochem. Soc. Trans. 39:641-646). A "hammerhead ribozyme" (HHR) may comprise a small catalytic RNA motif made up of three base-paired stems and a core of highly conserved, non-complementary nucleotides that are involved in catalysis. Pley et al. (Nature 372:68-74) and Hammann et al. (RNA 18:871 - 885), which are incorporated herein by reference, disclose hammerhead ribozyme structure and activity. A hammerhead ribozyme may comprise a "minimal hammerhead" sequence as disclosed by Scott et al. (Cell 81:991-1002, incorporated herein by reference), for example.

The term "increased" as used herein may refer to a quantity or activity that is at least about 1%, 2%, 3%, 4%, 5%, 6%, 7%, 8%, 9%, 10%, 11%, 12%, 13%, 14%, 15%, 16%, 17%, 18%, 19%, 20%, 50%, 100%, or 200% more than the quantity or activity for which the increased quantity or activity is being compared. The terms "increased", "elevated", "enhanced", "greater than", and "improved" are used interchangeably herein. The term "increased" can be used to characterize the expression of a polynucleotide encoding a protein, for example, where "increased expression" can also mean "over-expression".

A variety of methods are available to identify those cells having an altered genome at or near a target site without using a screenable marker phenotype. Such methods can be viewed as directly analyzing a target sequence to detect any change in the target sequence, including but not limited to PCR methods, sequencing methods, nuclease digestion, Southern blots, and any combination thereof.

Standard DNA isolation, purification, molecular cloning, vector construction, and verification/characterization methods are well established, see, for example Sambrook et al., (1989) Molecular Cloning: A Laboratory Manual, (Cold Spring Harbor Laboratory Press, NY). Vectors and constructs include circular plasmids, and linear polynucleotides, comprising a polynucleotide of interest and optionally other components including linkers, adapters, regulatory or analysis. In some examples a recognition site and/or target site can be contained within an intron, coding sequence, 5' UTRs, 3' UTRs, and/or regulatory regions.

The meaning of abbreviations is as follows: "sec" means second(s), "min" means minute(s), "h" means hour(s), "d" means day(s), "µL" means microliter(s), "mL" means milliliter(s), "L" means liter(s), "µM" means micromolar, "mM" means millimolar, "M" means molar, "mmol" means millimole(s), "µmole" mean micromole(s), "g" means gram(s), "µg" means microgram(s), "ng" means nanogram(s), "U" means unit(s), "bp" means base pair(s) and "kb" means kilobase(s).

Non-limiting examples of compositions and methods disclosed herein are as follows:
1. A recombinant DNA construct comprising a tRNA promoter operably linked to a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast.
2. The recombinant DNA of claim 1, wherein the tRNA promoter is selected from the group consisting of a tRNA or a tRNA fragment capable of functioning as a promoter sequence.
3. The recombinant DNA of claim 2, wherein the tRNA is selected from the group consisting of a tRNA-Lys, tRNA-Val, tRNA-Glu, tRNA Leu, tRNA-ile, tRNA-trp, tRNA-tyr, tRNA-his.
4. The recombinant DNA of claim 2, wherein the tRNA fragment is selected from the group consisting of a polynucleotide comprising the S-, D-, A-, V-, and T- domains of a tRNA, a polynucleotide comprising the S-, D-, V-, and T- domains of the tRNA, a polynucleotide comprising the S-, D-, and T- domains of the tRNA, and a polynucleotide comprising the S-, and T- domains of the tRNA.
5. A recombinant DNA construct comprising a tRNA promoter operably linked to a spacer sequence and a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast.
6. The recombinant DNA of claim 5, wherein the spacer sequence is a DNA sequence encoding a polynucleotide selected from the group consisting of a polynucleotide comprising a S-, D-, A-, V-, and T- domains of a tRNA, a polynucleotide comprising the S-, D-, V-, and T- domains of the tRNA, a polynucleotide comprising the S-, D-, and T- domains of the tRNA, and a polynucleotide comprising the S-, and T-domains of the tRNA.
7. The recombinant DNA of claim 5, wherein recombinant DNA encodes for a spacer RNA-guideRNA fusion molecule, wherein the spacer RNA can be cleaved off by a RNAse Z.
8. A non-conventional yeast comprising the recombinant DNA of any one of claims 1-7.
9. The non-conventional yeast of claim 8, wherein said yeast is a member of a genus selected from the group consisting of *Yarrowia, Pichia, Schwanniomyces, Kluyveromyces, Arxula, Trichosporon, Candida, Ustilago, Torulopsis, Zygosaccharomyces, Trigonopsis, Cryptococcus, Rhodotorula, Phaffia, Sporobolomyces,* and *Pachysolen*
10. A single guide RNA encoded by the recombinant DNA of any one of claims 1-7.
11. An expression vector comprising at least one recombinant DNA of any one of claims 1-7.
12. The expression vector of claim 11, further comprising a nucleotide encoding a Cas endonuclease.
13. The expression vector of claim 11, wherein the vector further comprises at least one nucleotide encoding a polynucleotide modification template or donor DNA.
14. A method for modifying a target site on a chromosome or episome in a non-conventional yeast, the method comprising providing to a non-conventional yeast at least a first recombinant DNA construct of claim 1or claim 5 and a second recombinant DNA construct encoding a Cas endonuclease, wherein the Cas endonuclease introduces a single or double-strand break at said target site.
15. The method of claim 14, wherein the at least first recombinant DNA construct of claim 1 and second recombinant DNA construct are located on the same polynucleotide or an separate polynucleotides.
16. The method of any of claims 14-15, further comprising identifying at least one non-conventional yeast cell that has a modification at said target site, wherein the modification includes at least one deletion, addition or substitution of one or more nucleotides in said target site.
17. The method of any of claims 14-15, further comprising providing a donor DNA to said yeast, wherein said donor DNA comprises a polynucleotide of interest.
18. The method of claim 17, further comprising identifying at least one yeast cell comprising in its chromosome or episome the polynucleotide of interest integrated at said target site.
19. The methods of any one of claims 14-15, further comprising identifying the mutation efficiency in said non-conventional yeast.
20. The method of claim 19, wherein the mutation efficiency is at least 2, 3, 4, 5, 6, 7, 8, 9, 10, 20 fold higher compared to a method for modifying a target site in said non-conventional yeast utilizing a ribozyme linked single guide RNA.
21. A method for editing a nucleotide sequence on a chromosome or episome in a non-conventional yeast, the method comprising providing to a non-conventional yeast a polynucleotide modification template DNA, a first recombinant DNA construct comprising a DNA sequence encoding a Cas endonuclease, and a second recombinant DNA construct of claim 1 or claim 5, wherein the Cas endonuclease introduces a single or double-strand break at a target site in the chromosome or episome of said yeast, wherein said polynucleotide modification template DNA comprises at least one nucleotide modification of said nucleotide sequence.
22. A method for silencing a nucleotide sequence on a chromosome or episome in a non-conventional yeast, the method comprising providing to a non-conventional yeast, at least a first recombinant DNA construct comprising a DNA sequence encoding an inactivated Cas endonuclease, and at least a second recombinant DNA construct of claim 1 or claim 5, wherein said tRNA-guide RNA fusion molecule and the inactivated Cas endonuclease can form a complex that binds to said nucleotide sequence in the chromosome or episome of said yeast, thereby blocking transcription of said nucleotide sequence.
23. A recombinant DNA construct comprising a promoter operably linked to a spacer sequence and a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast.
24. The recombinant DNA of claim 23, wherein the spacer sequence is a DNA sequence encoding a polynucleotide selected from the group consisting of a polynucleotide comprising a S-, D-, A-, V-, and T- domains of a tRNA, a polynucleotide comprising the S-, D-, V-, and T- domains of the tRNA, a polynucleotide comprising the S-, D-, and T- domains of the tRNA, and a polynucleotide comprising the S-, and T- domains of the tRNA.
25. The recombinant DNA of claim 23, wherein the promoter is a RNA Polymerase II or RNA polymerase III promoter.
26. A method for modifying multiple target sites on a chromosome or episome in a non-conventional yeast, the method comprising providing to a non-conventional yeast at least a first recombinant DNA construct comprising a DNA sequence encoding a Cas endonuclease, and at least a second recombinant DNA construct comprising a promoter operably linked to a sequence comprising more than one tRNA-guideRNA cassettes encoding more than one tRNA-guideRNAs targeting multiple target sites in the genome of said non-conventional yeast, wherein the Cas endonuclease introduces a single or double-strand break at each of said multiple target sites.

### EXAMPLES

In the following Examples, unless otherwise stated, parts and percentages are by weight and degrees are Celsius. It should be understood that these Examples, while indicating embodiments of the disclosure, are given by way of illustration only. From the above discussion and these Examples, one skilled in the art can make various changes and modifications of the disclosure to adapt it to various usages and conditions. Such modifications are also intended to fall within the scope of the appended claims.

### EXAMPLE 1

### Expression of tRNA-gRNA-tRNA, tRNA-gRNA-tRNA-gRNA and tRNA-gRNA fusion molecules as precursors for single guide RNAs and Cas9 editing

This example discusses the use of single guide RNAs (sgRNAs) that are obtained from premature tRNA-guide RNA fusion molecules, where the guide RNA is flanked on the 5' and/or the 3' end by *Yarrowia lipolytica* tRNAs. Recombinant DNA constructs (expression cassettes) were produced encoding the premature tRNA-guide RNA fusion molecules as described below. The transcribed premature tRNA-guide RNA fusion molecules are processed by host enzymes yielding single guide RNA's (sgRNAs) uncapped at the 5' end and where relevant at the 3' end.

### Cas9 recombinant DNA constructs

In order to test a sgRNA/Cas endonuclease system in *Yarrowia,* the Cas9 gene from *Streptococcus pyrogenes* M1 GAS (SF370 (SEQ ID NO: 1) was *Yarrowia* codon optimized per standard techniques known in the art (SEQ ID NO: 2). In order to localize the Cas9 protein to the nucleus of the cells, *Simian virus 40* (SV40) monopartite (PKKKRKV, SEQ ID NO: 3) nuclear localization signal was incorporated at the carboxyl terminus of the Cas9 protein. The Yarrowia codon optimized Cas9 gene was fused to a Yarrowia constitutive promoter, FBA1 (SEQ ID NO: 4), by standard molecular biology techniques. An example of a Yarrowia codon optimized Cas9 expression cassette containing the FBA1 promoter and the Yarrowia optimized Cas9-NLS fusion is shown in SEQ ID NO: 5. The Cas9 expression cassette was cloned into the plasmid pZuf resulting in pZufCas9 (SEQ ID NO 6).

Plasmid pZuf-Cas9CS (SEQ ID NO: 6) was mutagenized using Agilent QuickChange and the following primers:
Aarl-removal-1 :
   (AGAAGTATCCTACCATCTACcatctccGAAAGAAACTCGTCGATTCC; SEQ ID NO: 7) and
Aarl-removal-2:
   (GGAATCGACGAGTTTCTTTCggagatgGTAGATGGTAGGATACTTCT; SEQ ID NO: 8) to remove the endogenous Aarl site present in the *Yarrowia* codon optimized Cas9 gene present in pZuf-Cas9CS generating pRF109 (SEQ ID NO: 9). The modified Aar1-Cas9CS gene (SEQ ID NO: 10) was cloned as a Ncol/Notl fragment from pRF109 (SEQ ID NO: 9) into the Ncol/Notl site of pZufCas9CS (SEQU ID NO: 6) replacing the existing Cas9 gene (SEQ ID NO: 2) with the Aar1- Cas9 gene (SEQ ID NO: 10) generating pRF141 (SEQ ID NO: 11). Next, the Pacl-Clal insert (SEQ ID NO: 12) was cloned into pRF141 yielding pRF291 (SEQ ID NO: 18).

### tRNA based recombinant DNA constructs

Plasmid pRF434 (SEQ ID NO: 24) was constructed by replacing the *URA3* selectable marker present in pRF291 (SEQ ID NO: 18) between the Pacl and Pmel restriction sites with a hygromycin resistance expression cassette (SEQ ID NO: 25).

A tRNA-gRNA-tRNA expression cassette (SEQ ID NO: 27 was constructed that was composed of a yl52 promoter (SEQ ID NO: 13), a DNA sequence encoding the Yarrowia tRNA-Lys (SEQ ID NO: 28), a DNA sequence encoding a variable targeting domain targeting the ura3-1 target sequence in Yarrowia (SEQ ID NO:26), a DNA encoding the CER domain (SEQ ID NO: 16), a DNA sequence encoding the Yarrowia tRNA-Glu (SEQ ID NO:29) and the *S*. *cerevisiae SUP4* terminator (SEQ ID NO: 17). This tRNA-guide RNA expression cassette (SEQ ID NO: 27) contains Pacl and Clal restriction enzyme sites and was cloned into pRF434 to generate pFB8 (SEQ ID NO: 30).

A tRNA-gRNA-tRNA-gRNA expression cassette (SEQ ID NO: 132) was constructed that was composed of a yl52 promoter (SEQ ID NO: 13), a DNA sequence encoding the Yarrowia tRNA-Lys (SEQ ID NO: 28), a DNA sequence encoding a variable targeting domain targeting the ura3-1 target sequence in Yarrowia (SEQ ID NO:26), a DNA encoding the CER domain (SEQ ID NO: 16), a DNA sequence encoding the Yarrowia tRNA-Glu (SEQ ID NO:29), a DNA sequence encoding a variable targeting domain targeting the can1-1 target sequence in Yarrowia (SEQ ID NO:22) and the *S*. *cerevisiae SUP4* terminator (SEQ ID NO: 17). This tRNA-guide RNA expression cassette (SEQ ID NO: 132) contains Pacl and Clal restriction enzyme sites and was cloned into pRF434 to generate pFB9 (SEQ ID NO: 133).

A tRNA-gRNA expression cassette (SEQ ID NO:31) was constructed that was composed of the chromosomal derived 507 base pair upstream sequences to and including tRNA-Lys (SEQ ID NO: 32), a DNA sequence encoding the tRNA-Lys (SEQ ID NO: 28), a DNA sequence encoding a variable targeting domain targeting the ura3-1 target sequence in Yarrowia (SEQ ID NO:26), a DNA encoding the CER domain (SEQ ID NO: 16) and the *S*. *cerevisiae SUP4* terminator (SEQ ID NO: 17). This tRNA-guide RNA expression cassette (SEQ ID NO: 31) contains Pacl and Clal restriction enzyme sites and was cloned into pRF434 to generate pFB5 (SEQ ID NO: 79).

A tRNA-gRNA expression cassette (SEQ ID NO: 34) lacking a promoter 5' upstream of the DNA sequence encoding a tRNA sequence was composed of a DNA sequence encoding the tRNA-Lys (SEQ ID NO: 28), a DNA sequence encoding a variable targeting domain targeting the can-2 target sequence in Yarrowia (SEQ ID NO:35), a DNA encoding the CER domain (SEQ ID NO: 16) and the *S*. *cerevisiae SUP4* terminator (SEQ ID NO: 17). This tRNA-guide RNA expression cassette (SEQ ID NO: 34) contains Pacl and Clal restriction enzyme sites and was cloned into pRF434 to generate pFB33 (SEQ ID NO: 36).

A tRNA-gRNA expression cassette (SEQ ID NO: 37) lacking a promoter 5' upstream of the DNA sequence encoding a tRNA sequence was composed of a DNA sequence encoding the tRNA-Val (SEQ ID NO: 38), a DNA sequence encoding a variable targeting domain targeting the can-2 target sequence in Yarrowia (SEQ ID NO: 35), a DNA sequence encoding the CER domain (SEQ ID NO: 16) and the *S*. *cerevisiae SUP4* terminator (SEQ ID NO: 17). This tRNA-guide RNA expression cassette (SEQ ID NO: 37) contains Pacl and Clal restriction enzyme sites and was cloned into pRF434 to generate pFB32 (SEQ ID NO: 39).

A tRNA-gRNA expression cassette (SEQ ID NO: 105) lacking a promoter 5' upstream of the DNA sequence encoding a tRNA sequence was composed of a DNA sequence encoding the tRNA-leu (SEQ ID NO: 106), a DNA sequence encoding a variable targeting domain targeting the can-1 target sequence in Yarrowia (SEQ ID NO: 22), a DNA sequence encoding the CER domain (SEQ ID NO: 16) and the *S*. *cerevisiae SUP4* terminator (SEQ ID NO: 17). This tRNA-guide RNA expression cassette (SEQ ID NO: 105) contains Pacl and Clal restriction enzyme sites and was cloned into pRF434 to generate pFB111 (SEQ ID NO: 107).

A tRNA-gRNA expression cassette (SEQ ID NO: 108) lacking a promoter 5' upstream of the DNA sequence encoding a tRNA sequence was composed of a DNA sequence encoding the tRNA-leu(2) (SEQ ID NO: 109), a DNA sequence encoding a variable targeting domain targeting the can-1 target sequence in Yarrowia (SEQ ID NO: 22), a DNA sequence encoding the CER domain (SEQ ID NO: 16) and the *S*. *cerevisiae SUP4* terminator (SEQ ID NO: 17). This tRNA-guide RNA expression cassette (SEQ ID NO: 108) contains Pacl and Clal restriction enzyme sites and was cloned into pRF434 to generate pFB112 (SEQ ID NO: 110).

A tRNA-gRNA expression cassette (SEQ ID NO: 111) lacking a promoter 5' upstream of the DNA sequence encoding a tRNA sequence was composed of a DNA sequence encoding the tRNA-leu(3) (SEQ ID NO: 112), a DNA sequence encoding a variable targeting domain targeting the can-1 target sequence in Yarrowia (SEQ ID NO: 22), a DNA sequence encoding the CER domain (SEQ ID NO: 16) and the *S*. *cerevisiae SUP4* terminator (SEQ ID NO: 17). This tRNA-guide RNA expression cassette (SEQ ID NO: 111) contains Pacl and Clal restriction enzyme sites and was cloned into pRF434 to generate pFB113 (SEQ ID NO: 113).

A tRNA-gRNA expression cassette (SEQ ID NO: 114) lacking a promoter 5' upstream of the DNA sequence encoding a tRNA sequence was composed of a DNA sequence encoding the tRNA-ile (SEQ ID NO: 115), a DNA sequence encoding a variable targeting domain targeting the can-1 target sequence in Yarrowia (SEQ ID NO: 22), a DNA sequence encoding the CER domain (SEQ ID NO: 16) and the *S*. *cerevisiae SUP4* terminator (SEQ ID NO: 17). This tRNA-guide RNA expression cassette (SEQ ID NO: 114) contains Pacl and Clal restriction enzyme sites and was cloned into pRF434 to generate pFB115 (SEQ ID NO: 116).

A tRNA-gRNA expression cassette (SEQ ID NO: 117) lacking a promoter 5' upstream of the DNA sequence encoding a tRNA sequence was composed of a DNA sequence encoding the tRNA-val (SEQ ID NO: 118), a DNA sequence encoding a variable targeting domain targeting the can-1 target sequence in Yarrowia (SEQ ID NO: 22), a DNA sequence encoding the CER domain (SEQ ID NO: 16) and the *S*. *cerevisiae SUP4* terminator (SEQ ID NO: 17). This tRNA-guide RNA expression cassette (SEQ ID NO: 117) contains Pacl and Clal restriction enzyme sites and was cloned into pRF434 to generate pFB116 (SEQ ID NO: 119).

A tRNA-gRNA expression cassette (SEQ ID NO: 120) lacking a promoter 5' upstream of the DNA sequence encoding a tRNA sequence was composed of a DNA sequence encoding the tRNA-trp (SEQ ID NO: 121), a DNA sequence encoding a variable targeting domain targeting the can-1 target sequence in Yarrowia (SEQ ID NO: 22), a DNA sequence encoding the CER domain (SEQ ID NO: 16) and the *S*. *cerevisiae SUP4* terminator (SEQ ID NO: 17). This tRNA-guide RNA expression cassette (SEQ ID NO: 120) contains Pacl and Clal restriction enzyme sites and was cloned into pRF434 to generate pFB116 (SEQ ID NO: 122).

A tRNA-gRNA expression cassette (SEQ ID NO: 123) lacking a promoter 5' upstream of the DNA sequence encoding a tRNA sequence was composed of a DNA sequence encoding the tRNA-tyr (SEQ ID NO: 124), a DNA sequence encoding a variable targeting domain targeting the can-1 target sequence in Yarrowia (SEQ ID NO: 22), a DNA sequence encoding the CER domain (SEQ ID NO: 16) and the *S*. *cerevisiae SUP4* terminator (SEQ ID NO: 17). This tRNA-guide RNA expression cassette (SEQ ID NO: 123) contains Pacl and Clal restriction enzyme sites and was cloned into pRF434 to generate pFB118 (SEQ ID NO: 125).

A tRNA-gRNA expression cassette (SEQ ID NO: 126) lacking a promoter 5' upstream of the DNA sequence encoding a tRNA sequence was composed of a DNA sequence encoding the tRNA-his (SEQ ID NO: 127), a DNA sequence encoding a variable targeting domain targeting the can-1 target sequence in Yarrowia (SEQ ID NO: 22), a DNA sequence encoding the CER domain (SEQ ID NO: 16) and the *S*. *cerevisiae SUP4* terminator (SEQ ID NO: 17). This tRNA-guide RNA expression cassette (SEQ ID NO: 126) contains Pacl and Clal restriction enzyme sites and was cloned into pRF434 to generate pFB120 (SEQ ID NO: 128).

A tRNA-gRNA expression cassette (SEQ ID NO: 129) lacking a promoter 5' upstream of the DNA sequence encoding a tRNA sequence was composed of a DNA sequence encoding the tRNA-his(2) (SEQ ID NO: 130), a DNA sequence encoding a variable targeting domain targeting the can-1 target sequence in Yarrowia (SEQ ID NO: 22), a DNA sequence encoding the CER domain (SEQ ID NO: 16) and the *S*. *cerevisiae SUP4* terminator (SEQ ID NO: 17). This tRNA-guide RNA expression cassette (SEQ ID NO: 129) contains Pacl and Clal restriction enzyme sites and was cloned into pRF434 to generate pFB121 (SEQ ID NO: 131).

### Transformation of Yarrowia lipolytica with Cas9 and tRNA-gRNA expression constructs

*Yarrowia lipolytica* ATCC20362 cells were grown for 24 hours on YPD medium plates (Teknova) at 30°C. 1 loop of cells were resuspended in transformation buffer (35% polyethylene glycol average molecular weight of 3550, 100 mM lithium acetate, 100 mM dithiothreitol, 10 mM Tris, 1mM EDTA pH 6.0). 100 µl of cell suspension was mixed with 300 ng of plasmid DNA. Transformation mixtures were incubated at 39°C for 1 hour at 800 RPM. Cells were plated on YPD medium containing 250 mg/L of hygromycin sulfate (Calbiochem). Colonies were allowed to form at 30°C. 32 colonies from each transformation (with the exception of no DNA which had 0 colonies) were patched to YPD medium plates (Teknova) and CM plates containing either 450 mg/L 5-fluoroorotic acid (5FOA) or complete minimal plates lacking arginine containing 60µg/ml L-canavanine. 5FOA selects against cells with a functional *URA3* gene. L-canavanine is toxic to cells with a functional *CAN1* gene which is an importer of arginine and L-canavanine to the cells. Cells containing a loss of function allele in the *CAN1* gene will be phenotypically resistant to the presence of L-canavanine in the medium and will form colonies on plates containing L-canavanine. Cells containing a wild-type copy of the *CAN1* gene will be unable to grow on medium containing L-canavanine. The mode of action of L-canavanine is well known (Rosenthal G.A., The Biological effects and mode of action of L-Canavanine, a structural analog of L-arginine, The quarterly review of biology, volume 52, 1977, 155-178). Thus, Canavanine resistance is used to infer the mutation frequency as a result of base pair insertion or deletions (indel) from non-homologous end joining of the Cas9 double stranded induced breaks.

**Table 2**

| **5FOA or Canavanine resistance in *Yarrowia* from transformation of indicated plasmids.** | | | |
|---|---|---|---|
| Plasmid | tRNA-gRNA expression construct | Target site in Yarrowia (SEQ ID NO:) | Frequency of 5FOA or Canavanine resistance ± Standard Deviation¹ |
| pRF434 | none (no gRNA control) | none | 0.00 ± 0.00 |
| pFB8 | PRO-tRNAlys-ura3-1 gRNA-tRNAglu-TERM | Ura3-1 (SEQ ID NO: 26) | 0.70 ± 0.27 |
| pFB9 | PRO-tRNAlys-ura3-1gRNA-tRNAglu-can1-1-TERM | Ura3-1 (SEQ ID NO: 26) and Can1-1 (SEQ ID NO: 22) | 0.47² ± 0.34 |
| pFB65 | PRO-tRNAlys-can1-1 gRNA | Can1-1 (SEQ ID NO: 22) | 0.93 ± 0.05 |
| pFB5 | tRNAlys-ura3-1-TERM | Ura-3-1 (SEQ ID NO: 26) | 0.66 ± 0.22 |
| pFB33 | tRNAlys-can1-2-TERM | Can1-2 (SEQ ID NO: 35) | 0.84 ± 0.06 |
| pFB32 | tRNAval-can1-2-TERM | Can1-2 (SEQ ID NO: 35) | 0.77 ± 0.08 |
| pFB111 | tRNAleu-can1-1-TERM | Can1-1 (SEQ ID NO: 22) | 0.84 |
| pFB112 | tRNAleu(2)-can1-1-TERM | Can1-1 (SEQ ID NO: 22) | 0.88 |
| pFB113 | tRNA(leu3)-can1-1-TERM | Can1-1 (SEQ ID NO: 22) | 0.84 |
| pFB115 | tRNAile-can1-1-TERM | Can1-1 (SEQ ID NO: 22) | 0.91 |
| pFB116 | tRNAval-can1-1-TERM | Can1-1 (SEQ ID NO: 22) | 0.88 |
| pFB117 | tRNAtrp-can1-1-TERM | Can1-1 (SEQ ID NO: 22) | 0.91 |
| pFB118 | tRNAtyr-can1-1-TERM | Can1-1 (SEQ ID NO: 22) | 0.94 |
| pFB120 | tRNAhis-can1-1-TERM | Can1-1 (SEQ ID NO: 22) | 0.88 |
| pFB121 | tRNAhis(2)-can1-1-TERM | Can1-1 (SEQ ID NO: 22) | 0.81 |

| | | | |
|---|---|---|---|
| ¹Results represent the average and standard deviation for three independent experiments except pFB111-pFB121 represents one experiment. ²Indicates resistance to both Canavanine and 5FOA. PRO= promoter; TERM=terminator | | | |

Plasmid pRF434 (SEQ ID NO: 24) carries a Cas9 expression cassette but lacks a functional gRNA targeting ura3 or can1 and therefore did not yield cells resistant to either 5FOA or Canavanine (Table 2). Cells transformed with plasmids encoding a promoter (PRO, Table 2) expressing a gRNA targeting either ura3 or can1 with 5' or both 5' and 3' tRNAs yielded high frequency of resistance (Table 2, pFB8 (0.70), pFB65 (SEQ ID NO: 100, Table 2 0.93). Cells transformed with plasmid pFB9, which carries target sites for both ura1 and can1 (tRNA-gRNA-tRNA-gRNA, Figure 4), on average yielded high frequency of cells resistant to both Canavanine and 5FOA indicating that expression of tRNA flanked gRNAs can be used to target multiple sites in the same cell. Surprisingly, upstream promoter sequences were not necessary for efficient targeting of ura3 or can1 as cells transformed pFB5(SEQ ID NO:33), pFB33 (SEQ ID NO:36) or pFB32 (SEQ ID NO:39) all yielded resistant colonies (Table 1 0.66, 0.84 and 0.77 respectively).

### Example 2

### tRNA-gRNA based expression systems improves mutation frequency over ribozyme HDV-gRNA expression systems

This example describes the improvement in mutation frequency at a target site in *Yarrowia lipolytica* when using the tRNA-gRNA expression system compared to a ribozyme (HDV)-gRNA expression system. The ribozyme system for gRNA production, cleaves off upstream RNA leaving gRNA with the HDV ribozyme fused to the 5'gRNA target sequence (see US application 62/036652, filed on August 13, 2014, incorporated herein in its entirety by reference)). As described herein, the t-RNA-guide RNA expression system results in single guide RNA's not fused to the tRNA. To directly compare the two gRNA expression systems, the same 12 sites of the can1 gene were targeted by gRNA's produced by each of the two systems.

A high throughput cloning cassette for ribozyme based constructs (SEQ ID NO: 12) was produced and was composed of the yl52 promoter (SEQ ID NO: 13), a DNA sequence encoding the HDV ribozyme (SEQ ID NO: 14), an *Escherichia coli* counterselection cassette *rpsL* (SEQ ID NO: 15), a DNA encoding the CER domain (SEQ ID NO: 16) and the *S*. *cerevisiae SUP4* terminator (SEQ ID NO: 17). Flanking the ends of the high-throughput cloning cassette (SEQ ID NO: 12) were Pacl and Clal restriction enzyme recognition sites. The high-throughput cloning cassette (SEQ ID NO: 12) was cloned into the Pacl/Clal sites of pRF141 (SEQ ID NO: 11) to generate pRF291 (SEQ ID NO 14). The *rpsL* counterselection cassette (SEQ ID NO: 15) contains a WT copy of the *E*. *coli rpsL* gene with its native promoter and terminator. *rpsL* encodes the S12 ribosomal protein subunit (Escherichia coli and Salmonella typhimurium: Cellular and Molecular Biology, 1987 American Society of Microbiology). Some mutations in the S12 subunit cause resistance to the antibiotic streptomycin (Ozaki, M., et al. (1969) Nature 222(5191): 333-339) in a recessive manner (Lederberg, J, 1951, J Bacteriol 61(5): 549-550) such that if a wild-type copy of the *rpsL* gene is present the strain is phenotypically sensitive to streptomycin. Common cloning strains such as Top10 (Life technologies) have a mutated copy of *rpsL* on their chromosome such that the cells are resistant to streptomycin.

A high throughput tRNA-gRNA expression cassette (depicted in Figure 2, SEQ ID NO: 40) was produced as described in Example 1 and was composed of the yl52 promoter (SEQ ID NO: 13), a DNA sequence encoding the tRNA-Lys (SEQ ID NO: 28), an *Escherichia coli* counterselection cassette *rpsL* (SEQ ID NO: 15), a DNA encoding the CER domain (SEQ ID NO: 16) and the *S*. *cerevisiae SUP4* terminator (SEQ ID NO: 17). Flanking the ends of the high-throughput cloning cassette (SEQ ID NO: 40) were Pacl and Clal restriction enzyme recognition sites. The high-throughput cloning cassette (SEQ ID NO: 40) was cloned into the Pacl/Clal sites of pRF434 (SEQ ID NO: 24) to generate pFB12 (SEQ ID NO:41).

Cloning DNA encoding a variable targeting domain into pRF291 or pFB12 requires two partially complimentary oligonucleotides that when annealed contain the desired variable targeting domain as well as correct overhangs for cloning into the two Aarl sites present in the high-throughput cloning cassette. For example, to clone DNA encoding a variable targeting domain into pRF29,1 two oligonucleotides, Can1-1F (AATGGGACtcaaacgattacccaccctcGTTT, SEQ ID NO: 19) and Can1-1R (TCTAAAACgagggtgggtaatcgtttgaGTCC, SEQ ID NO: 20) containing the DNA encoding the variable targeting domain which targets the Can1-1 target site (SEQ ID NO: 22) in the *CAN1* gene of *Yarrowia lipolytica* (SEQ ID NO: 23), were resuspended in duplex buffer (30 mM HEPES pH 7.5, 100 mM Sodium Acetate) at 100 µM. Can1-1F (SEQ ID NO: 19) and Can1-1R (SEQ ID NO: 20) were mixed at a final concentration of 50µM each in a single tube, heated to 95°C for 5 minutes and cooled to 25°C at 0.1°C/min to anneal the two oligonucleotides to form a small duplex DNA molecule. A single tube digestion/ligation reaction was created containing 50 ng of pRF291, 2.5 µM of the small duplex DNA composed of Can1-1F (SEQ ID NO: 19) and Can1-1R (SEQ ID NO: 20), 1x T4 ligase buffer (50 mM Tris-HCl, 10 mM MgCl₂, 1 mM ATP, 10 mM DTT pH 7.5), 0.5 µM Aarl oligonucleotide, 2 units Aarl, 40 units T4 DNA ligase in a 20 µl final volume. A second control reaction lacking the duplexed Can1-1F and Can1-1R duplex was also assembled. The reactions were incubated at 37°C for 30 minutes. 10 µl of each reaction was transformed into Top10 *E*. *coli* cells as previously described (Green, M. R. & Sambrook, J. Molecular Cloning: A Laboratory Manual. Fourth Edition edn, (Cold Spring Harbor Laboratory Press, 2012)). In order to select for the presence of pRF291 where the duplex of Can1-1F (SEQ ID NO: 19) and Can1-1R (SEQ ID NO: 20) had replaced the *rpsL* counterselection marker flanked by Aarl restriction sites cells were plated on lysogeny broth solidified with 1.5% (w/v) Bacto agar containing 100µg/ml Ampicillin and 50 µg/ml Streptomycin.

The presence of pRF291 containing the high-throughput cloning cassette yielded colonies phenotypically resistant to the antibiotic ampicillin but sensitive to the antibiotic streptomycin due to the presence of the counterselection cassette on the plasmid. However, in cases where the counterselection cassette was removed via the Aarl enzyme and the Can1-1 variable targeting domain containing duplex DNA was ligated into the site (removing the recognition sequences for Aarl) the cells transformed with the plasmid had an ampicillin resistant, streptomycin resistant phenotype. pRF291 containing the DNA encoding the Can1-1 variable targeting domain replacing the counterselection cassette created a recombinant Can1-1 gRNA expression cassette (SEQ ID NO: 19) containing the yl52 promoter (SEQ ID NO: 13) fused to DNA encoding the HDV ribozyme (SEQ ID NO: 14) fused to DNA encoding the Can1-1 variable targeting domain (SEQ ID NO: 21) fused to DNA encoding the CER domain (SEQ ID NO: 16) fused to the *SUP4* terminator (SEQ ID NO: 17).

For cloning DNA encoding variable targeting domains into pFB12, a similar approach as described above was used, only the overhangs of the annealed oligos had sequence to anneal to the Aarl overhangs of pFB12. For example, cloning the DNA encoding the can1-1 variable targeting domain into pFB12, two oligonucleotides, Can1-1F (TCGGGCTAtcaaacgattacccaccctcGTTT, SEQ ID NO: 42) and Can1-1R (TCTAAAACgagggtgggtaatcgtttgaTAGC, SEQ ID NO: 43) were used to create the 5' flanked tRNA expression plasmid cassette (SEQ ID NO: 101) on pFB65 (SEQ ID NO: 100). The generation of HDV plasmids using pRF291 and tRNA plasmids using pFB12 was done using the oligonuclepotides shown in Table 3. This yielded both HDV and tRNA based plasmids targeting the same 12 sites in the can1 gene.

**Table 3**

| **Oligonucleotides used for cloning of Can1 targeting plasmids** | | | | | | |
|---|---|---|---|---|---|---|
| **Target site** | **HDV Plasmid** | **Forward oligonucleotide** | **Reverse oligonucleotide** | **tRNA Plasmid** | **Forward oligonucleotide** | **Reverse oligonucleotide** |
| Can1-1 | pRF303 | SEQ ID NO: 19 | SEQ ID NO: 20 | pFB65 | SEQ ID NO: 42 | SEQ ID NO: 43 |
| Can1-2 | pRF489 | SEQ ID NO: 56 | SEQ ID NO: 57 | pFB41 | SEQ ID NO: 78 | SEQ ID NO: 79 |
| Can1-3 | pRF490 | SEQ ID NO: 58 | SEQ ID NO: 59 | pFB42 | SEQ ID NO: 80 | SEQ ID NO: 81 |
| Can1-4 | pRF491 | SEQ ID NO: 60 | SEQ ID NO: 61 | pFB43 | SEQ ID NO: 82 | SEQ ID NO: 83 |
| Can1-5 | pRF492 | SEQ ID NO: 62 | SEQ ID NO: 63 | pFB44 | SEQ ID NO: 84 | SEQ ID NO: 85 |
| Can1-6 | pRF493 | SEQ ID NO: 64 | SEQ ID NO: 65 | pFB45 | SEQ ID NO: 86 | SEQ ID NO: 87 |
| Can1-7 | pRF495 | SEQ ID NO: 66 | SEQ ID NO: 67 | pFB47 | SEQ ID NO: 88 | SEQ ID NO: 89 |
| Can1-8 | pRF496 | SEQ ID NO: 68 | SEQ ID NO: 69 | pFB48 | SEQ ID NO: 90 | SEQ ID NO: 91 |
| Can1-9 | pRF497 | SEQ ID NO: 70 | SEQ ID NO: 71 | pFB49 | SEQ ID NO: 92 | SEQ ID NO: 93 |
| Can1-10 | pRF498 | SEQ ID NO: 72 | SEQ ID NO: 73 | pFB50 | SEQ ID NO: 94 | SEQ ID NO: 95 |
| Can1-11 | pRF499 | SEQ ID NO: 74 | SEQ ID NO: 75 | pFB51 | SEQ ID NO: 96 | SEQ ID NO: 973 |
| Can1-12 | pRF500 | SEQ ID NO: 76 | SEQ ID NO: 77 | pFB52 | SEQ ID NO: 98 | SEQ ID NO: 99 |

*Yarrowia lipolytica* ATCC20362 or a uracil auxotrophic derivative cells were grown for 24 hours on YPD medium plates (Teknova) at 30°C. 1 loop of cells were resuspended in transformation buffer (35% polyethylene glycol average molecular weight of 3550, 100 mM lithium acetate, 100 mM dithiothreitol, 10 mM Tris, 1mM EDTA pH 6.0). 100 µl of cell suspension was mixed with 300 ng of plasmid DNA. Transformation mixtures were incubated at 39°C for 1 hour at 800 RPM. Cells were plated on either complete minimal medium plates lacking uracil (Teknova) for HDV plasmids or YPD medium containing 250 mg/L of hygromycin sulfate (Calbiochem) for tRNA plasmids. Colonies were allowed to form at 30°C. Plates were replica plated (with the exception of no DNA which had 0 colonies) onto complete minimal plates lacking arginine containing 60µg/ml L-canavanine. Canavanine resistance frequencies of the same 12 target sites in the can1 gene are shown in Table 4.

**Table 4**

| **Comparison of frequency of Canavanine resistance between HDV and tRNA expression systems** | | | |
|---|---|---|---|
| | **Canavanine Resistance ± Standard Deviation¹** | | |
| **gRNA** | **HDV system** | **tRNA system** | **Fold increase** |
| no gRNA | 0.00±0.00 | 0.00±0.00 | 0.00 |
| Can1-1 | 0.75±0.21 | 0.93±0.05 | 1.23 |
| Can1-2 | 0.35±0.18 | 0.87±0.08 | 2.47 |
| Can1-3 | 0.78±0.19 | 0.91±0.07 | 1.16 |
| Can1-4 | 0.27±0.24 | 0.89±0.05 | 3.27 |
| Can1-5 | 0.35±0.11 | 0.62±0.06 | 1.77 |
| Can1-6 | 0.10±0.07 | 0.88±0.07 | 8.34 |
| Can1-7 | 0.14±0.15 | 0.92±0.12 | 6.63 |
| Can1-8 | 0.58±0.17 | 0.78±0.28 | 1.34 |
| Can1-9 | 0.25±0.07 | 0.86±0.19 | 3.46 |
| Can1-10 | 0.29±0.08 | 0.96±0.06 | 3.27 |
| Can1-11 | 0.07±0.08 | 0.93±0.06 | 13.52 |
| Can1-12 | 0.25±0.22 | 0.94±0.07 | 3.82 |

| | | | |
|---|---|---|---|
| ¹Results represent the average and standard deviation for three independent experiments. | | | |

In every instance the tRNA expression system yielded higher frequency of resistance when compared to the HDV based system (Table 4).

### Example 3

### Expression of gRNA with RNase Z recognition domains is sufficient for efficient Cas9 targeting

In this example gRNAs targeting the can1 gene were expressed by a recombinant DNA construct encoding a spacer RNA-gRNA precursor RNA fusion molecule, containing a 5' RNA domain (referred to as the spacer RNA domain) that can act as a substrate for RNase Z (Figure 3). Cleavage of this spacer RNA-gRNA precursor fusion molecule adjacent to the RNase Z recognition domains would leave an uncapped gRNA. RNase Z recognition domains were derived from the tRNA Valine described in Example 2.

Cloning DNA encoding RNase Z recognition domains into pFB12 requires two partially complimentary oligonucleotides that when annealed they contain the desired RNase Z recognition domain upstream the DNA encoding the guide RNA targeting the can1-2 target site as well as the correct overhangs for cloning into the two Aarl sites present in the high-throughput cloning cassette. Oligonucleotides were mixed at a final concentration of 50 µM each in a single tube, heated to 95°C for 5 minutes and cooled to 25°C at 0.1°C/min to anneal the two oligonucleotides to form a small duplex DNA molecule. The duplex DNA was ligated to 50 ng of pFB12. To clone the DNA encoding the SDVT RNase Z recognition domain, oligonucleotide SDVT RNase Z recognition oligo F (SEQ ID NO: 44) and SDVT RNase Z recognition oligo R (SEQ ID NO: 45) which contain the SDVT RNase Z recognition domain (SEQ ID NO: 46) and the DNA sequence encoding the can1-2 gRNA target sequence (SEQ ID NO: 35) were used to generate SDVT spacer RNA-gRNA construct (SEQ ID NO: 102) on pFB105 (SEQ ID NO:47). To clone the DNA encoding the SDT RNase Z recognition domain, oligonucleotide SDT RNase Z recognition oligo F (SEQ ID NO: 48) and SDT RNase Z recognition oligo R (SEQ ID NO:49) which contain the SDT RNase Z recognition domain (SEQ ID NO: 50) and the DNA sequence encoding the can1-2 gRNA target sequence (SEQ ID NO:35) were used to generate SDT spacer RNA-gRNA construct (SEQ ID NO: 103) on pFB108 (SEQ ID NO:51). To clone the DNA encoding the ST RNase Z recognition domain, oligonucleotide ST RNase Z recognition oligo F (SEQ ID NO:52) and ST RNase Z recognition oligo R (SEQ ID NO:53) which contain the ST RNase Z recognition domain (SEQ ID NO: 54) and the DNA sequence encoding the can1-2 gRNA target sequence (SEQ ID NO:35) were used to generate ST RNAspacer-gRNA construct (SEQ ID NO: 104) on pFB109 (SEQ ID NO:55).

*Yarrowia lipolytica* ATCC20362 cells were grown for 24 hours on YPD medium plates (Teknova) at 30°C. 1 loop of cells were resuspended in transformation buffer (35% polyethylene glycol average molecular weight of 3550, 100 mM lithium acetate, 100 mM dithiothreitol, 10 mM Tris, 1mM EDTA pH 6.0). 100 µl of cell suspension was mixed with 300 ng of plasmid DNA. Transformation mixtures were incubated at 39°C for 1 hour at 800 RPM. Cells were plated on YPD medium containing 250 mg/L of hygromycin sulfate (Calbiochem). Colonies were allowed to form at 30°C. 32 colonies from each transformation (with the exception of no DNA which had 0 colonies) were patched to YPD medium plates (Teknova) and complete minimal plates lacking arginine containing 60µg/ml L-canavanine.

The frequency of Canavanine resistance for can1 targeting gRNAs with the indicated RNase Z recognition domains at the 5' end is shown in Table 5. High frequencies of gene inactivation indicate the Rnase Z recognition domains are able to yield functional targeting gRNAs.

**Table 5**

| **Frequency of Canavanine resistance of Yarrowia cells transformed with DNA encoding RNA-spacer-gRNAs fusions targeting the can1 gene. The type of RNA-spacer domain is indicated by RNase Z recognition domains. 1 = 100% Can resistance.** | | | |
|---|---|---|---|
| **Plasmid** | **spacer RNA-gRNA expression construct** | **RNase Z recognition domain** | **Canavanine Resistance¹** |
| pRF434 | none (no gRNA control) (SEQ ID NO: 12) | none | 0 |
| pFB105 | SDVT-can1-1gRNA-TERM (SEQ ID NO: 102) | SDVT | 0.94 |
| pFB108 | SDT-can1-1gRNA-TERM (SEQ ID NO: 103) | SDT | 1.00 |
| pFB109 | ST-can1-1gRNA-TERM (SEQ ID NO: 104) | ST | 0.91 |

| | | | |
|---|---|---|---|
| ¹Frequency of resistance from a single experiment. | | | |

The following numbered paragraphs (paras.) contain further statements of various aspects of the present invention:-
1. A recombinant DNA construct comprising a tRNA promoter operably linked to a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast.
2. The recombinant DNA of para 1, wherein the tRNA promoter is selected from the group consisting of a tRNA or a tRNA fragment capable of functioning as a promoter sequence.
3. The recombinant DNA of para 2, wherein the tRNA is selected from the group consisting of tRNA-Lys, tRNA-Val, tRNA-Glu, tRNA Leu, tRNA-ile, tRNA-trp, tRNA-tyr, tRNA-his, and any one combination thereof.
4. The recombinant DNA of para 2, wherein the tRNA fragment is selected from the group consisting of a polynucleotide comprising the S-, D-, A-, V-, and T-domains of a tRNA, a polynucleotide comprising the S-, D-, V-, and T- domains of the tRNA, a polynucleotide comprising the S-, D-, and T- domains of the tRNA, and a polynucleotide comprising the S-, and T- domains of the tRNA.
5. A recombinant DNA construct comprising a tRNA promoter operably linked to a spacer sequence and a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast.
6. The recombinant DNA of para 5, wherein the spacer sequence is a DNA sequence encoding a polynucleotide selected from the group consisting of a polynucleotide comprising a S-, D-, A-, V-, and T- domains of a tRNA, a polynucleotide comprising the S-, D-, V-, and T- domains of the tRNA, a polynucleotide comprising the S-, D-, and T- domains of the tRNA, and a polynucleotide comprising the S-, and T- domains of the tRNA.
7. The recombinant DNA of para 5, wherein recombinant DNA encodes for a spacer RNA-guideRNA fusion molecule, wherein the spacer RNA can be cleaved off by an RNAse Z.
8. A non-conventional yeast comprising the recombinant DNA of any one of paras 1-7.
9. The non-conventional yeast of para 8, wherein said yeast is a member of a genus selected from the group consisting of *Yarrowia, Pichia, Schwanniomyces, Kluyveromyces, Arxula, Trichosporon, Candida, Ustilago, Torulopsis, Zygosaccharomyces, Trigonopsis, Cryptococcus, Rhodotorula, Phaffia, Sporobolomyces,* and *Pachysolen*
10. A single guide RNA encoded by the recombinant DNA of any one of paras 1-7.
11. An expression vector comprising at least one recombinant DNA of any one of paras 1-7.
12. The expression vector of para 11, further comprising a nucleotide encoding a Cas endonuclease.
13. The expression vector of para 11, wherein the vector further comprises at least one nucleotide encoding a polynucleotide modification template or donor DNA.
14. A method for modifying a target site on a chromosome or episome in a non-conventional yeast, the method comprising providing to a non-conventional yeast at least a first recombinant DNA construct of para 1or para 5 and a second recombinant DNA construct encoding a Cas endonuclease, wherein the Cas endonuclease introduces a single or double-strand break at said target site.
15. The method of para 14, wherein the at least a first recombinant DNA construct of para 1 and a second recombinant DNA construct are located on the same polynucleotide or on separate polynucleotides.
16. The method of any of paras 14-15, further comprising identifying at least one non-conventional yeast cell that has a modification at said target site, wherein the modification includes at least one deletion, addition or substitution of one or more nucleotides in said target site.
17. The method of any of paras 14-15, further comprising providing a donor DNA to said yeast, wherein said donor DNA comprises a polynucleotide of interest.
18. The method of para 17, further comprising identifying at least one yeast cell comprising in its chromosome or episome the polynucleotide of interest integrated at said target site.
19. The methods of any one of paras 14-15, further comprising identifying the mutation efficiency in said non-conventional yeast.
20. The method of para 19, wherein the mutation efficiency is at least 2, 3, 4, 5, 6, 7, 8, 9, 10, 20 fold higher compared to a method for modifying a target site in said non-conventional yeast utilizing a ribozyme linked single guide RNA.
21. A method for editing a nucleotide sequence on a chromosome or episome in a non-conventional yeast, the method comprising providing to a non-conventional yeast a polynucleotide modification template DNA, a first recombinant DNA construct comprising a DNA sequence encoding a Cas endonuclease, and a second recombinant DNA construct of para 1 or para 5, wherein the Cas endonuclease introduces a single or double-strand break at a target site in the chromosome or episome of said yeast, wherein said polynucleotide modification template DNA comprises at least one nucleotide modification of said nucleotide sequence.
22. A method for silencing a nucleotide sequence on a chromosome or episome in a non-conventional yeast, the method comprising providing to a non-conventional yeast, at least a first recombinant DNA construct comprising a DNA sequence encoding an inactivated Cas endonuclease, and at least a second recombinant DNA construct of para 1 or para 5, wherein said tRNA-guide RNA fusion molecule and the inactivated Cas endonuclease can form a complex that binds to said nucleotide sequence in the chromosome or episome of said yeast, thereby blocking transcription of said nucleotide sequence.
23. A recombinant DNA construct comprising a promoter operably linked to a spacer sequence and a polynucleotide encoding a single guide RNA, wherein said recombinant DNA construct does not comprise a nucleotide sequence encoding a ribozyme, wherein said guide RNA is capable of forming a guide RNA/Cas endonuclease complex, wherein said complex can bind to and cleave a target site sequence in the genome of a non-conventional yeast.
24. The recombinant DNA of para 23, wherein the spacer sequence is a DNA sequence encoding a polynucleotide selected from the group consisting of a polynucleotide comprising a S-, D-, A-, V-, and T- domains of a tRNA, a polynucleotide comprising the S-, D-, V-, and T- domains of the tRNA, a polynucleotide comprising the S-, D-, and T- domains of the tRNA, and a polynucleotide comprising the S-, and T- domains of the tRNA.
25. The recombinant DNA of para 23, wherein the promoter is a RNA Polymerase II or RNA polymerase III promoter.
26. A method for modifying multiple target sites on a chromosome or episome in a non-conventional yeast, the method comprising providing to a non-conventional yeast at least a first recombinant DNA construct comprising a DNA sequence encoding a Cas endonuclease, and at least a second recombinant DNA construct comprising a promoter operably linked to a sequence comprising more than one tRNA-guideRNA cassettes encoding more than one tRNA-guideRNAs targeting multiple target sites in the genome of said non-conventional yeast, wherein the Cas endonuclease introduces a single or double-strand break at each of said multiple target sites.

## Claims

1. A method for modifying multiple target sites on a chromosome or episome in a non-conventional yeast, the method comprising providing to a non-conventional yeast a DNA construct encoding a Cas endonuclease and recombinant DNA encoding a plurality of tRNA-guide RNA expression cassettes, wherein each tRNA-guide RNA expression cassette comprises a tRNA promoter operably linked to a polynucleotide encoding a single guide RNA, wherein each tRNA-guide RNA expression cassette does not comprise a nucleotide sequence encoding a ribozyme, wherein each guide RNA is capable of forming a guide RNA/Cas endonuclease complex, and wherein each of said complexes can bind to and cleave a different target site sequence in the genome of a non-conventional yeast.

2. The method of claim 1, wherein the tRNA promoter is selected from the group consisting of a tRNA or a tRNA fragment capable of functioning as a promoter sequence, wherein optionally the tRNA is selected from the group consisting of tRNA-Lys, tRNA-Val, tRNA-Glu, tRNA Leu, tRNA-ile, tRNA-trp, tRNA-tyr, tRNA-his, and any one combination thereof.

3. The method of claim 2, wherein the tRNA fragment is selected from the group consisting of a polynucleotide comprising the S-, D-, A-, V-, and T- domains of a tRNA, a polynucleotide comprising the S-, D-, V-, and T- domains of the tRNA, a polynucleotide comprising the S-, D-, and T- domains of the tRNA, and a polynucleotide comprising the S-, and T- domains of the tRNA.

4. The method of claim 2, wherein said non-conventional yeast is a member of a genus selected from the group consisting of *Yarrowia, Pichia, Schwanniomyces, Kluyveromyces, Arxula, Trichosporon, Candida, Ustilago, Torulopsis, Zygosaccharomyces, Trigonopsis, Cryptococcus, Rhodotorula, Phaffia, Sporobolomyces,* and *Pachysolen.*

5. The method of claim 1, further comprising identifying at least one non-conventional yeast cell that has a modification at said target sites, wherein the modification includes at least one deletion, addition or substitution of one or more nucleotides in said target sites.

6. The method of claim 1, further comprising providing at least one donor DNA to said yeast, wherein said at least one donor DNA comprises a polynucleotide of interest.

7. The method of claim 6, further comprising identifying at least one yeast cell comprising in its chromosome or episome the polynucleotide of interest integrated at one or more of said target sites.

8. The method of claim 1, further comprising identifying the mutation efficiency in said non-conventional yeast.
